# EUROPEAN PATENT APPLICATION

(11) **EP 3 636 278 A2**
(43) Date of publication of application: **15.04.2020**
(21) Application number: 19192151.9
(22) Date of filing: 25.06.2015
(51) Int. Cl.: A61K 39/08, C07K 14/33

(54) **CLOSTRIDIUM DIFFICILE IMMUNOGENIC COMPOSITION**

(30) Priority: 25.06.2014 GB 201411306; 26.06.2014 GB 201411371
(62) Divisional of application: 15733404.6
(71) Applicant: GlaxoSmithKline Biologicals S.A., 1330 Rixensart (BE)
(72) Inventor: CASTADO, Cindy, 1330 Rixensart (BE)
(74) Representative: Johnston, Caroline Louise

(57) **Abstract**

The present invention relates to immunogenic compositions comprising isolated *Clostridium difficile* CDTb. In particular the isolated *Clostridium difficile* CDTb protein has been mutated to modify pore forming ability, to modify heptamerisation ability, or is a truncated CDTb protein with the signal peptide and the prodomain removed and also the receptor binding domain removed and/or the CDTa binding domain removed.The invention also relates to fusion proteins comprising a CDTa protein and a CDTb protein. Vaccines comprising such immunogenic compositions and therapeutic uses of the same also form part of the invention.

SEQUENCE LISTING

<110>  GlaxoSmithKline Biologicals SA - (GSKBIOL   )

<120>  IMMUNOGENIC COMPOSITION

<130>  VB65721

<160>  76

<170>  PatentIn version 3.5

<210>  1
<211>  463
<212>  PRT
<213>  Clostridium difficile

<400>  1

Met Lys Lys Phe Arg Lys His Lys Arg Ile Ser Asn Cys Ile Ser Ile
1               5                   10                  15

Leu Leu Ile Leu Tyr Leu Thr Leu Gly Gly Leu Leu Pro Asn Asn Ile
            20                  25                  30

Tyr Ala Gln Asp Leu Gln Ser Tyr Ser Glu Lys Val Cys Asn Thr Thr
        35                  40                  45

Tyr Lys Ala Pro Ile Glu Arg Pro Glu Asp Phe Leu Lys Asp Lys Glu
    50                  55                  60

Lys Ala Lys Glu Trp Glu Arg Lys Glu Ala Glu Arg Ile Glu Gln Lys
65                  70                  75                  80

Leu Glu Arg Ser Glu Lys Glu Ala Leu Glu Ser Tyr Lys Lys Asp Ser
                85                  90                  95

Val Glu Ile Ser Lys Tyr Ser Gln Thr Arg Asn Tyr Phe Tyr Asp Tyr
            100                 105                 110

Gln Ile Glu Ala Asn Ser Arg Glu Lys Glu Tyr Lys Glu Leu Arg Asn
        115                 120                 125

Ala Ile Ser Lys Asn Lys Ile Asp Lys Pro Met Tyr Val Tyr Tyr Phe
    130                 135                 140

Glu Ser Pro Glu Lys Phe Ala Phe Asn Lys Val Ile Arg Thr Glu Asn
145                 150                 155                 160

Gln Asn Glu Ile Ser Leu Glu Lys Phe Asn Glu Phe Lys Glu Thr Ile
                165                 170                 175

Gln Asn Lys Leu Phe Lys Gln Asp Gly Phe Lys Asp Ile Ser Leu Tyr
        180                 185                 190

Glu Pro Gly Lys Gly Asp Glu Lys Pro Thr Pro Leu Leu Met His Leu
    195                 200                 205

Lys Leu Pro Arg Asn Thr Gly Met Leu Pro Tyr Thr Asn Thr Asn Asn
    210                 215                 220

Val Ser Thr Leu Ile Glu Gln Gly Tyr Ser Ile Lys Ile Asp Lys Ile
225                 230                 235                 240

Val Arg Ile Val Ile Asp Gly Lys His Tyr Ile Lys Ala Glu Ala Ser
                245                 250                 255

Val Val Ser Ser Leu Asp Phe Lys Asp Asp Val Ser Lys Gly Asp Ser
            260                 265                 270

Trp Gly Lys Ala Asn Tyr Asn Asp Trp Ser Asn Lys Leu Thr Pro Asn
        275                 280                 285

Glu Leu Ala Asp Val Asn Asp Tyr Met Arg Gly Gly Tyr Thr Ala Ile
    290                 295                 300

Asn Asn Tyr Leu Ile Ser Asn Gly Pro Val Asn Asn Pro Asn Pro Glu
305                 310                 315                 320

Leu Asp Ser Lys Ile Thr Asn Ile Glu Asn Ala Leu Lys Arg Glu Pro
                325                 330                 335

Ile Pro Thr Asn Leu Thr Val Tyr Arg Arg Ser Gly Pro Gln Glu Phe
            340                 345                 350

Gly Leu Thr Leu Thr Ser Pro Glu Tyr Asp Phe Asn Lys Leu Glu Asn
        355                 360                 365

Ile Asp Ala Phe Lys Ser Lys Trp Glu Gly Gln Ala Leu Ser Tyr Pro
    370                 375                 380

Asn Phe Ile Ser Thr Ser Ile Gly Ser Val Asn Met Ser Ala Phe Ala
385                 390                 395                 400

Lys Arg Lys Ile Val Leu Arg Ile Thr Ile Pro Lys Gly Ser Pro Gly
                405                 410                 415

Ala Tyr Leu Ser Ala Ile Pro Gly Tyr Ala Gly Glu Tyr Glu Val Leu
            420                 425                 430

Leu Asn His Gly Ser Lys Phe Lys Ile Asn Lys Ile Asp Ser Tyr Lys
        435                 440                 445

Asp Gly Thr Ile Thr Lys Leu Ile Val Asp Ala Thr Leu Ile Pro
    450                 455                 460

<210>  2
<211>  1392
<212>  DNA
<213>  Clostridium difficile

<400>  2
atgaaaaaat ttaggaaaca taaaaggatt agtaattgta tatctatatt gttgatatta      60

tatctaactt taggtggttt gttacctaat aacatttatg cacaagactt acaaagctat     120

agtgaaaaag tttgcaatac tacttacaag gctcctatag aaagaccaga agattttctt     180

aaagataaag aaaaggctaa agaatgggaa agaaaagaag cagaaagaat agagcaaaaa     240

cttgaaagat ctgaaaaaga agcattagaa tcatataaaa aagattctgt agaaataagt     300

aaatattctc agacaagaaa ttatttttat gattatcaaa tagaagcaaa ttctcgagaa     360

aaagaatata aagaacttcg aaatgctata tcaaaaaata aaatagataa acctatgtat     420

gtctattatt ttgaatctcc agaaaaattt gcatttaata aagtaataag aacagaaaat     480

caaaacgaaa tttcattaga aaaatttaat gagtttaaag aaactataca aaacaaatta     540

tttaagcaag atggatttaa agatatttct ttatatgaac ctggaaaagg tgatgaaaaa     600

cctacaccat tacttatgca cttaaaatta cctagaaata ctggtatgtt accatataca     660

aatactaaca atgtaagtac attaatagag caaggatata gtataaaaat agataaaatt     720

gttcgtatag ttatagatgg gaagcactat attaaagcag aagcatctgt tgtaagtagt     780

cttgatttta aagatgatgt aagtaagggg gattcttggg gtaaagcaaa ttataatgat     840

tggagtaata aattaacacc taatgaactt gctgatgtaa atgattatat gcgtggagga     900

tatactgcaa ttaataatta tttaatatca aatggtccag taaataatcc taacccagaa     960

ttagattcta aaatcacaaa cattgaaaat gcattaaaac gtgaacctat tccaactaat    1020

ttaactgtat atagaagatc tggtcctcaa gaatttggtt taactcttac ttcccctgaa    1080

tatgatttta acaaactaga aaatatagat gcttttaaat caaaatggga aggacaagca    1140

ctgtcttatc caaactttat tagtactagt attggtagtg tgaatatgag tgcatttgct    1200

aaaagaaaaa tagtactacg tataactata cctaaaggtt ctcctggagc ttatctatca    1260

gctattccag gttatgcagg tgaatatgaa gtgcttttaa atcatggaag caaatttaaa    1320

atcaataaaa ttgattctta caaagatggt actataacaa aattaattgt tgatgcaaca    1380

ttgatacctt aa                                                        1392

<210>  3
<211>  876
<212>  PRT
<213>  Clostridium difficile

<400>  3

Met Lys Ile Gln Met Arg Asn Lys Lys Val Leu Ser Phe Leu Thr Leu
1               5                   10                  15

Thr Ala Ile Val Ser Gln Ala Leu Val Tyr Pro Val Tyr Ala Gln Thr
            20                  25                  30

Ser Thr Ser Asn His Ser Asn Lys Lys Lys Glu Ile Val Asn Glu Asp
        35                  40                  45

Ile Leu Pro Asn Asn Gly Leu Met Gly Tyr Tyr Phe Thr Asp Glu His
    50                  55                  60

Phe Lys Asp Leu Lys Leu Met Ala Pro Ile Lys Asp Gly Asn Leu Lys
65                  70                  75                  80

Phe Glu Glu Lys Lys Val Asp Lys Leu Leu Asp Lys Asp Lys Ser Asp
                85                  90                  95

Val Lys Ser Ile Arg Trp Thr Gly Arg Ile Ile Pro Ser Lys Asp Gly
            100                 105                 110

Glu Tyr Thr Leu Ser Thr Asp Arg Asp Asp Val Leu Met Gln Val Asn
        115                 120                 125

Thr Glu Ser Thr Ile Ser Asn Thr Leu Lys Val Asn Met Lys Lys Gly
    130                 135                 140

Lys Glu Tyr Lys Val Arg Ile Glu Leu Gln Asp Lys Asn Leu Gly Ser
145                 150                 155                 160

Ile Asp Asn Leu Ser Ser Pro Asn Leu Tyr Trp Glu Leu Asp Gly Met
                165                 170                 175

Lys Lys Ile Ile Pro Glu Glu Asn Leu Phe Leu Arg Asp Tyr Ser Asn
            180                 185                 190

Ile Glu Lys Asp Asp Pro Phe Ile Pro Asn Asn Asn Phe Phe Asp Pro
        195                 200                 205

Lys Leu Met Ser Asp Trp Glu Asp Glu Asp Leu Asp Thr Asp Asn Asp
    210                 215                 220

Asn Ile Pro Asp Ser Tyr Glu Arg Asn Gly Tyr Thr Ile Lys Asp Leu
225                 230                 235                 240

Ile Ala Val Lys Trp Glu Asp Ser Phe Ala Glu Gln Gly Tyr Lys Lys
                245                 250                 255

Tyr Val Ser Asn Tyr Leu Glu Ser Asn Thr Ala Gly Asp Pro Tyr Thr
            260                 265                 270

Asp Tyr Glu Lys Ala Ser Gly Ser Phe Asp Lys Ala Ile Lys Thr Glu
        275                 280                 285

Ala Arg Asp Pro Leu Val Ala Ala Tyr Pro Ile Val Gly Val Gly Met
    290                 295                 300

Glu Lys Leu Ile Ile Ser Thr Asn Glu His Ala Ser Thr Asp Gln Gly
305                 310                 315                 320

Lys Thr Val Ser Arg Ala Thr Thr Asn Ser Lys Thr Glu Ser Asn Thr
                325                 330                 335

Ala Gly Val Ser Val Asn Val Gly Tyr Gln Asn Gly Phe Thr Ala Asn
            340                 345                 350

Val Thr Thr Asn Tyr Ser His Thr Thr Asp Asn Ser Thr Ala Val Gln
        355                 360                 365

Asp Ser Asn Gly Glu Ser Trp Asn Thr Gly Leu Ser Ile Asn Lys Gly
    370                 375                 380

Glu Ser Ala Tyr Ile Asn Ala Asn Val Arg Tyr Tyr Asn Thr Gly Thr
385                 390                 395                 400

Ala Pro Met Tyr Lys Val Thr Pro Thr Thr Asn Leu Val Leu Asp Gly
                405                 410                 415

Asp Thr Leu Ser Thr Ile Lys Ala Gln Glu Asn Gln Ile Gly Asn Asn
            420                 425                 430

Leu Ser Pro Gly Asp Thr Tyr Pro Lys Lys Gly Leu Ser Pro Leu Ala
        435                 440                 445

Leu Asn Thr Met Asp Gln Phe Ser Ser Arg Leu Ile Pro Ile Asn Tyr
    450                 455                 460

Asp Gln Leu Lys Lys Leu Asp Ala Gly Lys Gln Ile Lys Leu Glu Thr
465                 470                 475                 480

Thr Gln Val Ser Gly Asn Phe Gly Thr Lys Asn Ser Ser Gly Gln Ile
                485                 490                 495

Val Thr Glu Gly Asn Ser Trp Ser Asp Tyr Ile Ser Gln Ile Asp Ser
            500                 505                 510

Ile Ser Ala Ser Ile Ile Leu Asp Thr Glu Asn Glu Ser Tyr Glu Arg
        515                 520                 525

Arg Val Thr Ala Lys Asn Leu Gln Asp Pro Glu Asp Lys Thr Pro Glu
    530                 535                 540

Leu Thr Ile Gly Glu Ala Ile Glu Lys Ala Phe Gly Ala Thr Lys Lys
545                 550                 555                 560

Asp Gly Leu Leu Tyr Phe Asn Asp Ile Pro Ile Asp Glu Ser Cys Val
                565                 570                 575

Glu Leu Ile Phe Asp Asp Asn Thr Ala Asn Lys Ile Lys Asp Ser Leu
            580                 585                 590

Lys Thr Leu Ser Asp Lys Lys Ile Tyr Asn Val Lys Leu Glu Arg Gly
        595                 600                 605

Met Asn Ile Leu Ile Lys Thr Pro Thr Tyr Phe Thr Asn Phe Asp Asp
    610                 615                 620

Tyr Asn Asn Tyr Pro Ser Thr Trp Ser Asn Val Asn Thr Thr Asn Gln
625                 630                 635                 640

Asp Gly Leu Gln Gly Ser Ala Asn Lys Leu Asn Gly Glu Thr Lys Ile
                645                 650                 655

Lys Ile Pro Met Ser Glu Leu Lys Pro Tyr Lys Arg Tyr Val Phe Ser
            660                 665                 670

Gly Tyr Ser Lys Asp Pro Leu Thr Ser Asn Ser Ile Ile Val Lys Ile
        675                 680                 685

Lys Ala Lys Glu Glu Lys Thr Asp Tyr Leu Val Pro Glu Gln Gly Tyr
    690                 695                 700

Thr Lys Phe Ser Tyr Glu Phe Glu Thr Thr Glu Lys Asp Ser Ser Asn
705                 710                 715                 720

Ile Glu Ile Thr Leu Ile Gly Ser Gly Thr Thr Tyr Leu Asp Asn Leu

725                 730                 735

Ser Ile Thr Glu Leu Asn Ser Thr Pro Glu Ile Leu Asp Glu Pro Glu
            740                 745                 750

Val Lys Ile Pro Thr Asp Gln Glu Ile Met Asp Ala His Lys Ile Tyr
        755                 760                 765

Phe Ala Asp Leu Asn Phe Asn Pro Ser Thr Gly Asn Thr Tyr Ile Asn
    770                 775                 780

Gly Met Tyr Phe Ala Pro Thr Gln Thr Asn Lys Glu Ala Leu Asp Tyr
785                 790                 795                 800

Ile Gln Lys Tyr Arg Val Glu Ala Thr Leu Gln Tyr Ser Gly Phe Lys
                805                 810                 815

Asp Ile Gly Thr Lys Asp Lys Glu Met Arg Asn Tyr Leu Gly Asp Pro
            820                 825                 830

Asn Gln Pro Lys Thr Asn Tyr Val Asn Leu Arg Ser Tyr Phe Thr Gly
        835                 840                 845

Gly Glu Asn Ile Met Thr Tyr Lys Lys Leu Arg Ile Tyr Ala Ile Thr
    850                 855                 860

Pro Asp Asp Arg Glu Leu Leu Val Leu Ser Val Asp
865                 870                 875

<210>  4
<211>  2631
<212>  DNA
<213>  Clostridium difficile

<400>  4
atgaaaatac aaatgaggaa taaaaaggta ttaagttttt taacacttac agctatagtt      60

agtcaagcac tagtatatcc tgtatatgct caaactagta caagtaatca ttctaataag     120

aaaaaagaaa ttgtaaatga agatatactc ccaaacaatg gattaatggg atattatttc     180

acagatgagc actttaaaga tttaaaatta atggcaccca taaaagatgg taatttaaaa     240

tttgaagaaa agaaagtaga taaacttctg gataaagaca aatcagatgt aaaatctata     300

cgatggacag gaagaataat tccttctaag gatggtgaat atacattatc aactgataga     360

gatgatgtct taatgcaagt aaatactgag agtactatat caaatacact taaagttaat     420

atgaaaaagg gtaaagaata taaagttaga atagagctac aagataaaaa tttaggttca     480

atagataatt tatcatcacc taatctttat tgggaattag atggtatgaa gaaaattata     540

ccagaagaaa atttattctt aagagattat tctaatatag aaaaagatga tccatttatc 600

ccaaataaca atttctttga cccaaagttg atgtctgatt gggaagacga agatttggat 660

acagataatg ataatatacc agattcatat gaacgaaatg gatatactat taaggactta 720

attgcagtta agtgggaaga tagttttgca gaacaaggct ataagaaata tgtatcaaat 780

tatttagagt caaatactgc tggagatcca tatacagatt atgaaaaagc ttcaggttct 840

tttgacaagg ctataaagac tgaagcaaga gatccgttag ttgcagcata tccaattgtt 900

ggagtaggta tggaaaaatt aattatatct acaaatgaac atgcctctac tgatcaaggt 960

aaaactgttt ccagagctac tactaacagt aaaactgaat ctaatacagc tggtgtgtct 1020

gttaatgtag gatatcaaaa tggattcaca gctaatgtaa ctacaaatta ttcccataca 1080

acagataatt caactgctgt tcaagatagt aatggagaat catggaatac tggattaagt 1140

ataaacaaag gagaatctgc atatataaat gcaaatgtta gatattacaa cacaggtact 1200

gcacctatgt acaaagtgac accaacaaca aatttagtgt tagatggaga tacattatca 1260

actatcaaag cacaagaaaa tcaaattggc aataatctat ctcctggaga tacttatccc 1320

aaaaaagggc tttcacctct agctcttaac acaatggatc aatttagctc tagactgatt 1380

cctataaatt atgatcaatt aaaaaaatta gatgctggaa agcaaattaa attagaaaca 1440

acacaagtaa gtggaaattt tggtacaaaa aatagttctg gacaaatagt aacagaagga 1500

aatagttggt cagactatat aagtcaaatt gacagtattt ctgcatctat tatattagat 1560

acagagaatg aatcttacga aagaagagtt actgctaaaa atttacagga tccagaagat 1620

aaaacacctg aacttacaat tggagaagca attgaaaaag cttttggcgc tactaaaaaa 1680

gatggtttgt tatattttaa tgatatacca atagatgaaa gttgtgttga actcatattt 1740

gatgataata cagccaataa gattaaagat agtttaaaaa ctttgtctga taaaaagata 1800

tataatgtta aacttgaaag aggaatgaat atacttataa aaacaccaac ttactttact 1860

aattttgatg attataataa ttaccctagt acatggagta atgtcaatac tacgaatcaa 1920

gatggtttac aaggctcagc aaataaatta aatggtgaga cgaagattaa aatccctatg 1980

tctgagctaa aaccttataa acgttatgtt tttagtggat attcaaagga tcctttaaca 2040

tctaattcaa taattgtaaa gataaaagca aaagaagaga aaacggatta tttggtacca 2100

gaacaaggat atacaaaatt tagttatgaa tttgaaacta ctgaaaaaga ttcttctaat 2160

atagagataa cattaattgg tagtggtaca acatacttag ataacttatc tattacagag 2220

ctaaatagta ctcctgaaat acttgatgaa ccagaagtta aaattccaac tgaccaagaa 2280

ataatggatg cacataaaat atattttgca gatttaaatt ttaatccaag tacaggaaat 2340

acttatataa atggtatgta ttttgcacca acacaaacta ataaagaagc tctcgattat 2400

atccaaaaat atagagttga agctacttta caatattctg gatttaaaga tattggaact 2460

aaagataaag aaatgcgtaa ttatttagga gatccaaatc agcctaaaac taattatgtt     2520

aatcttagga gttattttac aggtggagaa aatattatga catacaagaa attaagaata     2580

tatgcaatta ctccagacga tagagagtta ttagttctta gtgttgatta g              2631

<210>  5
<211>  1073
<212>  PRT
<213>  Clostridium difficile

<400>  5

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205
Thr Phe Gly Gly Gly Asp His Pro Pro Lys Ser Asp Leu Glu Val Leu
    210                 215                 220
Phe Gln Gly Pro Leu Gly Ser His Met Glu Ile Val Asn Glu Asp Ile
225                 230                 235                 240
Leu Pro Asn Asn Gly Leu Met Gly Tyr Tyr Phe Thr Asp Glu His Phe
                245                 250                 255
Lys Asp Leu Lys Leu Met Ala Pro Ile Lys Asp Gly Asn Leu Lys Phe
            260                 265                 270
Glu Glu Lys Lys Val Asp Lys Leu Leu Asp Lys Asp Lys Ser Asp Val
        275                 280                 285
Lys Ser Ile Arg Trp Thr Gly Arg Ile Ile Pro Ser Lys Asp Gly Glu
    290                 295                 300
Tyr Thr Leu Ser Thr Asp Arg Asp Asp Val Leu Met Gln Val Asn Thr
305                 310                 315                 320
Glu Ser Thr Ile Ser Asn Thr Leu Lys Val Asn Met Lys Lys Gly Lys
                325                 330                 335
Glu Tyr Lys Val Arg Ile Glu Leu Gln Asp Lys Asn Leu Gly Ser Ile
            340                 345                 350
Asp Asn Leu Ser Ser Pro Asn Leu Tyr Trp Glu Leu Asp Gly Met Lys
        355                 360                 365
Lys Ile Ile Pro Glu Glu Asn Leu Phe Leu Arg Asp Tyr Ser Asn Ile
    370                 375                 380
Glu Lys Asp Asp Pro Phe Ile Pro Asn Asn Asn Phe Phe Asp Pro Lys
385                 390                 395                 400
Leu Met Ser Asp Trp Glu Asp Glu Asp Leu Asp Thr Asp Asn Asp Asn
                405                 410                 415
Ile Pro Asp Ser Tyr Glu Arg Asn Gly Tyr Thr Ile Lys Asp Leu Ile
            420                 425                 430
Ala Val Lys Trp Glu Asp Ser Phe Ala Glu Gln Gly Tyr Lys Lys Tyr
        435                 440                 445

Val Ser Asn Tyr Leu Glu Ser Asn Thr Ala Gly Asp Pro Tyr Thr Asp
    450                 455                 460

Tyr Glu Lys Ala Ser Gly Ser Phe Asp Lys Ala Ile Lys Thr Glu Ala
465                 470                 475                 480

Arg Asp Pro Leu Val Ala Ala Tyr Pro Ile Val Gly Val Gly Met Glu
                485                 490                 495

Lys Leu Ile Ile Ser Thr Asn Glu His Ala Ser Thr Asp Gln Gly Lys
            500                 505                 510

Thr Val Ser Arg Ala Thr Thr Asn Ser Lys Thr Glu Ser Asn Thr Ala
        515                 520                 525

Gly Val Ser Val Asn Val Gly Tyr Gln Asn Gly Phe Thr Ala Asn Val
    530                 535                 540

Thr Thr Asn Tyr Ser His Thr Thr Asp Asn Ser Thr Ala Val Gln Asp
545                 550                 555                 560

Ser Asn Gly Glu Ser Trp Asn Thr Gly Leu Ser Ile Asn Lys Gly Glu
                565                 570                 575

Ser Ala Tyr Ile Asn Ala Asn Val Arg Tyr Tyr Asn Thr Gly Thr Ala
            580                 585                 590

Pro Met Tyr Lys Val Thr Pro Thr Thr Asn Leu Val Leu Asp Gly Asp
        595                 600                 605

Thr Leu Ser Thr Ile Lys Ala Gln Glu Asn Gln Ile Gly Asn Asn Leu
    610                 615                 620

Ser Pro Gly Asp Thr Tyr Pro Lys Lys Gly Leu Ser Pro Leu Ala Leu
625                 630                 635                 640

Asn Thr Met Asp Gln Phe Ser Ser Arg Leu Ile Pro Ile Asn Tyr Asp
                645                 650                 655

Gln Leu Lys Lys Leu Asp Ala Gly Lys Gln Ile Lys Leu Glu Thr Thr
            660                 665                 670

Gln Val Ser Gly Asn Phe Gly Thr Lys Asn Ser Ser Gly Gln Ile Val
        675                 680                 685

Thr Glu Gly Asn Ser Trp Ser Asp Tyr Ile Ser Gln Ile Asp Ser Ile
    690                 695                 700

Ser Ala Ser Ile Ile Leu Asp Thr Glu Asn Glu Ser Tyr Glu Arg Arg
705                 710                 715                 720

Val Thr Ala Lys Asn Leu Gln Asp Pro Glu Asp Lys Thr Pro Glu Leu
                725                 730                 735

Thr Ile Gly Glu Ala Ile Glu Lys Ala Phe Gly Ala Thr Lys Lys Asp
            740                 745                 750

Gly Leu Leu Tyr Phe Asn Asp Ile Pro Ile Asp Glu Ser Cys Val Glu
        755                 760                 765

Leu Ile Phe Asp Asp Asn Thr Ala Asn Lys Ile Lys Asp Ser Leu Lys
    770                 775                 780

Thr Leu Ser Asp Lys Lys Ile Tyr Asn Val Lys Leu Glu Arg Gly Met
785                 790                 795                 800

Asn Ile Leu Ile Lys Thr Pro Thr Tyr Phe Thr Asn Phe Asp Asp Tyr
                805                 810                 815

Asn Asn Tyr Pro Ser Thr Trp Ser Asn Val Asn Thr Thr Asn Gln Asp
            820                 825                 830

Gly Leu Gln Gly Ser Ala Asn Lys Leu Asn Gly Glu Thr Lys Ile Lys
        835                 840                 845

Ile Pro Met Ser Glu Leu Lys Pro Tyr Lys Arg Tyr Val Phe Ser Gly
    850                 855                 860

Tyr Ser Lys Asp Pro Leu Thr Ser Asn Ser Ile Ile Val Lys Ile Lys
865                 870                 875                 880

Ala Lys Glu Glu Lys Thr Asp Tyr Leu Val Pro Glu Gln Gly Tyr Thr
                885                 890                 895

Lys Phe Ser Tyr Glu Phe Glu Thr Thr Glu Lys Asp Ser Ser Asn Ile
            900                 905                 910

Glu Ile Thr Leu Ile Gly Ser Gly Thr Thr Tyr Leu Asp Asn Leu Ser
        915                 920                 925

Ile Thr Glu Leu Asn Ser Thr Pro Glu Ile Leu Asp Glu Pro Glu Val
    930                 935                 940

Lys Ile Pro Thr Asp Gln Glu Ile Met Asp Ala His Lys Ile Tyr Phe

945                 950                 955                 960

Ala Asp Leu Asn Phe Asn Pro Ser Thr Gly Asn Thr Tyr Ile Asn Gly
                965                 970                 975

Met Tyr Phe Ala Pro Thr Gln Thr Asn Lys Glu Ala Leu Asp Tyr Ile
            980                 985                 990

Gln Lys Tyr Arg Val Glu Ala Thr  Leu Gln Tyr Ser Gly  Phe Lys Asp
        995                 1000                 1005

Ile Gly  Thr Lys Asp Lys Glu  Met Arg Asn Tyr Leu  Gly Asp Pro
    1010                 1015                 1020

Asn Gln  Pro Lys Thr Asn Tyr  Val Asn Leu Arg Ser  Tyr Phe Thr
    1025                 1030                 1035

Gly Gly  Glu Asn Ile Met Thr  Tyr Lys Lys Leu Arg  Ile Tyr Ala
    1040                 1045                 1050

Ile Thr  Pro Asp Asp Arg Glu  Leu Leu Val Leu Ser  Val Asp His
    1055                 1060                 1065

His His  His His His
    1070

<210>  6
<211>  3222
<212>  DNA
<213>  Clostridium difficile

<400>  6
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa     420

acatatttaa atggtgatca tgtaacccat cctgacttca tgttgtatga cgctcttgat     480

gttgttttat acatggaccc aatgtgcctg gatgcgttcc caaaattagt ttgttttaaa     540

aaacgtattg aagctatccc acaaattgat aagtacttga aatccagcaa gtatatagca     600

tggcctttgc agggctggca agccacgttt ggtggtggcg accatcctcc aaaatcggat     660

ctggaagttc tgttccaggg gcccctggga tcccatatgg aaattgtgaa tgaagatatt 720

ctgccgaata atggtctgat gggatactac tttaccgatg aacattttaa agatctgaaa 780

ctgatggcac cgattaaaga tggcaatctg aaatttgaag aaaaaaaagt ggataaactg 840

ctggataaag ataaaagtga tgtgaaaagc attcgttgga ccggtcgtat tattccgagc 900

aaagatggtg aatacaccct gagcaccgat cgtgatgatg ttctgatgca ggttaatacc 960

gaaagcacca ttagcaatac cctgaaagtg aatatgaaaa aaggcaaaga atataaagtg 1020

cgcattgaac tgcaggataa aaatctgggt agcattgata atctgagcag cccgaatctg 1080

tattgggaac tggatggtat gaaaaaaatc attccggaag aaaacctgtt tctgcgcgat 1140

tatagcaata ttgaaaaaga tgatccgttt attccgaata ataacttttt tgatccgaaa 1200

ctgatgagcg attgggaaga tgaagatctg gataccgata atgataatat tccggatagc 1260

tatgaacgca atggctatac cattaaagat ctgattgccg tgaaatggga agatagcttt 1320

gcagaacagg gctataagaa atatgtgagc aattatctgg aaagcaatac cgcaggcgat 1380

ccgtataccg attatgaaaa agcaagcggc agctttgata aagccattaa aaccgaagca 1440

cgtgatccgc tggttgcagc atatccgatt gttggtgttg gtatggaaaa actgattatt 1500

agcaccaatg aacatgcaag caccgatcag ggtaaaaccg ttagccgtgc aaccaccaat 1560

agcaaaaccg aaagcaatac agccggtgtt agcgttaatg ttggttatca gaatggtttt 1620

accgccaatg tgaccaccaa ttatagccat accaccgata atagcaccgc agttcaggat 1680

agcaatggtg aaagctggaa taccggtctg agcattaaca aaggtgaaag cgcatatatc 1740

aatgccaatg tgcgctatta taacaccggc accgcaccga tgtataaagt taccccgacc 1800

accaatctgg ttctggatgg tgataccctg agtaccatta aagcacaaga aaatcagatt 1860

ggcaataatc tgagtccggg tgatacctat ccgaaaaaag gtctgagtcc gctggcactg 1920

aataccatgg atcagtttag cagccgtctg attccgatta actatgatca gctgaaaaaa 1980

ctggatgccg gtaaacaaat caaactggaa accacccagg ttagcggtaa ttttggcacc 2040

aaaaattcaa gcggtcagat tgttaccgaa ggtaatagct ggtcagatta tatcagccag 2100

attgatagca ttagcgccag cattattctg gatacagaaa atgaaagcta tgaacgtcgt 2160

gtgaccgcaa aaaatctgca ggacccggaa gataaaacac cggaactgac cattggtgaa 2220

gcaattgaaa aagcatttgg tgccaccaaa aaagatggcc tgctgtattt taacgatatt 2280

ccgattgatg aaagctgcgt ggaactgatt tttgatgata ataccgccaa taaaatcaaa 2340

gatagcctga aaaccctgag cgacaaaaaa atctataatg tgaaactgga acgcggtatg 2400

aatattctga ttaaaacccc gacctatttt accaattttg atgattataa caattatccg 2460

agcacttgga gcaatgtgaa taccaccaat caggatggtc tgcagggtag cgcaaataaa 2520

ctgaatggtg aaaccaaaat caaaattccg atgagcgaac tgaaaccgta taaacgttat 2580

gtgtttagcg gctatagcaa agatccgctg accagcaata gcattattgt gaaaatcaaa 2640

gccaaagaag aaaaaaccga ttatctggtt ccggaacagg gttataccaa atttagctat 2700

gaatttgaaa ccaccgaaaa agatagcagt aatattgaaa ttaccctgat tggtagcggc 2760

accacctatc tggataatct gagtattacc gaactgaata gcacaccgga aattctggat 2820

gaaccggaag tgaaaattcc gaccgatcaa gaaattatgg atgcccataa aatctatttt 2880

gccgatctga actttaatcc gagcaccggc aatacctata ttaacggcat gtattttgca 2940

ccgacccaga ccaataaaga agccctggat tatattcaga aatatcgtgt tgaagccacc 3000

ctgcagtata gcggttttaa agatattggc accaaagata aagaaatgcg taattatctg 3060

ggcgatccga atcagccgaa aaccaattat gttaatctgc gcagctattt taccggtggc 3120

gaaaacatta tgacctacaa aaaactgcgc atttatgcca ttacaccgga tgatcgtgaa 3180

ctgctggttc tgagcgttga tcaccaccat catcatcatt aa 3222

<210> 7
<211> 672
<212> PRT
<213> Clostridium difficile

<400> 7

Met Ser Asp Trp Glu Asp Glu Asp Leu Asp Thr Asp Asn Asp Asn Ile
1               5                   10                  15

Pro Asp Ser Tyr Glu Arg Asn Gly Tyr Thr Ile Lys Asp Leu Ile Ala
            20                  25                  30

Val Lys Trp Glu Asp Ser Phe Ala Glu Gln Gly Tyr Lys Lys Tyr Val
        35                  40                  45

Ser Asn Tyr Leu Glu Ser Asn Thr Ala Gly Asp Pro Tyr Thr Asp Tyr
    50                  55                  60

Glu Lys Ala Ser Gly Ser Phe Asp Lys Ala Ile Lys Thr Glu Ala Arg
65                  70                  75                  80

Asp Pro Leu Val Ala Ala Tyr Pro Ile Val Gly Val Gly Met Glu Lys
                85                  90                  95

Leu Ile Ile Ser Thr Asn Glu His Ala Ser Thr Asp Gln Gly Lys Thr
            100                 105                 110

Val Ser Arg Ala Thr Thr Asn Ser Lys Thr Glu Ser Asn Thr Ala Gly
        115                 120                 125

Val Ser Val Asn Val Gly Tyr Gln Asn Gly Phe Thr Ala Asn Val Thr
    130                 135                 140

Thr Asn Tyr Ser His Thr Thr Asp Asn Ser Thr Ala Val Gln Asp Ser
145                 150                 155                 160

Asn Gly Glu Ser Trp Asn Thr Gly Leu Ser Ile Asn Lys Gly Glu Ser
                165                 170                 175

Ala Tyr Ile Asn Ala Asn Val Arg Tyr Tyr Asn Thr Gly Thr Ala Pro
            180                 185                 190

Met Tyr Lys Val Thr Pro Thr Thr Asn Leu Val Leu Asp Gly Asp Thr
        195                 200                 205

Leu Ser Thr Ile Lys Ala Gln Glu Asn Gln Ile Gly Asn Asn Leu Ser
    210                 215                 220

Pro Gly Asp Thr Tyr Pro Lys Lys Gly Leu Ser Pro Leu Ala Leu Asn
225                 230                 235                 240

Thr Met Asp Gln Phe Ser Ser Arg Leu Ile Pro Ile Asn Tyr Asp Gln
                245                 250                 255

Leu Lys Lys Leu Asp Ala Gly Lys Gln Ile Lys Leu Glu Thr Thr Gln
            260                 265                 270

Val Ser Gly Asn Phe Gly Thr Lys Asn Ser Ser Gly Gln Ile Val Thr
        275                 280                 285

Glu Gly Asn Ser Trp Ser Asp Tyr Ile Ser Gln Ile Asp Ser Ile Ser
    290                 295                 300

Ala Ser Ile Ile Leu Asp Thr Glu Asn Glu Ser Tyr Glu Arg Arg Val
305                 310                 315                 320

Thr Ala Lys Asn Leu Gln Asp Pro Glu Asp Lys Thr Pro Glu Leu Thr
                325                 330                 335

Ile Gly Glu Ala Ile Glu Lys Ala Phe Gly Ala Thr Lys Lys Asp Gly
            340                 345                 350

Leu Leu Tyr Phe Asn Asp Ile Pro Ile Asp Glu Ser Cys Val Glu Leu
        355                 360                 365

Ile Phe Asp Asp Asn Thr Ala Asn Lys Ile Lys Asp Ser Leu Lys Thr
    370                 375                 380

Leu Ser Asp Lys Lys Ile Tyr Asn Val Lys Leu Glu Arg Gly Met Asn
385                 390                 395                 400

Ile Leu Ile Lys Thr Pro Thr Tyr Phe Thr Asn Phe Asp Asp Tyr Asn
                405                 410                 415

Asn Tyr Pro Ser Thr Trp Ser Asn Val Asn Thr Thr Asn Gln Asp Gly
            420                 425                 430

Leu Gln Gly Ser Ala Asn Lys Leu Asn Gly Glu Thr Lys Ile Lys Ile
        435                 440                 445

Pro Met Ser Glu Leu Lys Pro Tyr Lys Arg Tyr Val Phe Ser Gly Tyr
    450                 455                 460

Ser Lys Asp Pro Leu Thr Ser Asn Ser Ile Ile Val Lys Ile Lys Ala
465                 470                 475                 480

Lys Glu Glu Lys Thr Asp Tyr Leu Val Pro Glu Gln Gly Tyr Thr Lys
                485                 490                 495

Phe Ser Tyr Glu Phe Glu Thr Thr Glu Lys Asp Ser Ser Asn Ile Glu
            500                 505                 510

Ile Thr Leu Ile Gly Ser Gly Thr Thr Tyr Leu Asp Asn Leu Ser Ile
        515                 520                 525

Thr Glu Leu Asn Ser Thr Pro Glu Ile Leu Asp Glu Pro Glu Val Lys
    530                 535                 540

Ile Pro Thr Asp Gln Glu Ile Met Asp Ala His Lys Ile Tyr Phe Ala
545                 550                 555                 560

Asp Leu Asn Phe Asn Pro Ser Thr Gly Asn Thr Tyr Ile Asn Gly Met
                565                 570                 575

Tyr Phe Ala Pro Thr Gln Thr Asn Lys Glu Ala Leu Asp Tyr Ile Gln
            580                 585                 590

Lys Tyr Arg Val Glu Ala Thr Leu Gln Tyr Ser Gly Phe Lys Asp Ile
        595                 600                 605

Gly Thr Lys Asp Lys Glu Met Arg Asn Tyr Leu Gly Asp Pro Asn Gln
    610                 615                 620

Pro Lys Thr Asn Tyr Val Asn Leu Arg Ser Tyr Phe Thr Gly Gly Glu
625                 630                 635                 640

Asn Ile Met Thr Tyr Lys Lys Leu Arg Ile Tyr Ala Ile Thr Pro Asp
                645                 650                 655

Asp Arg Glu Leu Leu Val Leu Ser Val Asp His His His His His His
            660                 665                 670

<210>  8
<211>  414
<212>  PRT
<213>  Clostridium difficile

<400>  8

Met Ser Asp Trp Glu Asp Glu Asp Leu Asp Thr Asp Asn Asp Asn Ile
1               5                   10                  15

Pro Asp Ser Tyr Glu Arg Asn Gly Tyr Thr Ile Lys Asp Leu Ile Ala
            20                  25                  30

Val Lys Trp Glu Asp Ser Phe Ala Glu Gln Gly Tyr Lys Lys Tyr Val
        35                  40                  45

Ser Asn Tyr Leu Glu Ser Asn Thr Ala Gly Asp Pro Tyr Thr Asp Tyr
    50                  55                  60

Glu Lys Ala Ser Gly Ser Phe Asp Lys Ala Ile Lys Thr Glu Ala Arg
65                  70                  75                  80

Asp Pro Leu Val Ala Ala Tyr Pro Ile Val Gly Val Gly Met Glu Lys
                85                  90                  95

Leu Ile Ile Ser Thr Asn Glu His Ala Ser Thr Asp Gln Gly Lys Thr
            100                 105                 110

Val Ser Arg Ala Thr Thr Asn Ser Lys Thr Glu Ser Asn Thr Ala Gly
        115                 120                 125

Val Ser Val Asn Val Gly Tyr Gln Asn Gly Phe Thr Ala Asn Val Thr
    130                 135                 140

Thr Asn Tyr Ser His Thr Thr Asp Asn Ser Thr Ala Val Gln Asp Ser
145                 150                 155                 160

Asn Gly Glu Ser Trp Asn Thr Gly Leu Ser Ile Asn Lys Gly Glu Ser
                165                 170                 175

Ala Tyr Ile Asn Ala Asn Val Arg Tyr Tyr Asn Thr Gly Thr Ala Pro
            180                 185                 190

Met Tyr Lys Val Thr Pro Thr Thr Asn Leu Val Leu Asp Gly Asp Thr
        195                 200                 205

Leu Ser Thr Ile Lys Ala Gln Glu Asn Gln Ile Gly Asn Asn Leu Ser
    210                 215                 220

Pro Gly Asp Thr Tyr Pro Lys Lys Gly Leu Ser Pro Leu Ala Leu Asn
225                 230                 235                 240

Thr Met Asp Gln Phe Ser Ser Arg Leu Ile Pro Ile Asn Tyr Asp Gln
                245                 250                 255

Leu Lys Lys Leu Asp Ala Gly Lys Gln Ile Lys Leu Glu Thr Thr Gln
            260                 265                 270

Val Ser Gly Asn Phe Gly Thr Lys Asn Ser Ser Gly Gln Ile Val Thr
        275                 280                 285

Glu Gly Asn Ser Trp Ser Asp Tyr Ile Ser Gln Ile Asp Ser Ile Ser
    290                 295                 300

Ala Ser Ile Ile Leu Asp Thr Glu Asn Glu Ser Tyr Glu Arg Arg Val
305                 310                 315                 320

Thr Ala Lys Asn Leu Gln Asp Pro Glu Asp Lys Thr Pro Glu Leu Thr
                325                 330                 335

Ile Gly Glu Ala Ile Glu Lys Ala Phe Gly Ala Thr Lys Lys Asp Gly
            340                 345                 350

Leu Leu Tyr Phe Asn Asp Ile Pro Ile Asp Glu Ser Cys Val Glu Leu
        355                 360                 365

Ile Phe Asp Asp Asn Thr Ala Asn Lys Ile Lys Asp Ser Leu Lys Thr
    370                 375                 380

Leu Ser Asp Lys Lys Ile Tyr Asn Val Lys Leu Glu Arg Gly Met Asn
385                 390                 395                 400

Ile Leu Ile Lys Thr Pro Gly Gly His His His His His His
                405                 410

<210> 9
<211> 588
<212> PRT
<213> Clostridium difficile

<400>  9

Met Ala Tyr Pro Ile Val Gly Val Gly Met Glu Lys Leu Ile Ile Ser
1               5                   10                  15

Thr Asn Glu His Ala Ser Thr Asp Gln Gly Lys Thr Val Ser Arg Ala
            20                  25                  30

Thr Thr Asn Ser Lys Thr Glu Ser Asn Thr Ala Gly Val Ser Val Asn
        35                  40                  45

Val Gly Tyr Gln Asn Gly Phe Thr Ala Asn Val Thr Thr Asn Tyr Ser
    50                  55                  60

His Thr Thr Asp Asn Ser Thr Ala Val Gln Asp Ser Asn Gly Glu Ser
65                  70                  75                  80

Trp Asn Thr Gly Leu Ser Ile Asn Lys Gly Glu Ser Ala Tyr Ile Asn
                85                  90                  95

Ala Asn Val Arg Tyr Tyr Asn Thr Gly Thr Ala Pro Met Tyr Lys Val
            100                 105                 110

Thr Pro Thr Thr Asn Leu Val Leu Asp Gly Asp Thr Leu Ser Thr Ile
        115                 120                 125

Lys Ala Gln Glu Asn Gln Ile Gly Asn Asn Leu Ser Pro Gly Asp Thr
    130                 135                 140

Tyr Pro Lys Lys Gly Leu Ser Pro Leu Ala Leu Asn Thr Met Asp Gln
145                 150                 155                 160

Phe Ser Ser Arg Leu Ile Pro Ile Asn Tyr Asp Gln Leu Lys Lys Leu
                165                 170                 175

Asp Ala Gly Lys Gln Ile Lys Leu Glu Thr Thr Gln Val Ser Gly Asn
            180                 185                 190

Phe Gly Thr Lys Asn Ser Ser Gly Gln Ile Val Thr Glu Gly Asn Ser
        195                 200                 205

Trp Ser Asp Tyr Ile Ser Gln Ile Asp Ser Ile Ser Ala Ser Ile Ile
    210                 215                 220

Leu Asp Thr Glu Asn Glu Ser Tyr Glu Arg Arg Val Thr Ala Lys Asn
225                 230                 235                 240

Leu Gln Asp Pro Glu Asp Lys Thr Pro Glu Leu Thr Ile Gly Glu Ala

                245                 250                 255

Ile Glu Lys Ala Phe Gly Ala Thr Lys Lys Asp Gly Leu Leu Tyr Phe
            260                 265                 270

Asn Asp Ile Pro Ile Asp Glu Ser Cys Val Glu Leu Ile Phe Asp Asp
        275                 280                 285

Asn Thr Ala Asn Lys Ile Lys Asp Ser Leu Lys Thr Leu Ser Asp Lys
    290                 295                 300

Lys Ile Tyr Asn Val Lys Leu Glu Arg Gly Met Asn Ile Leu Ile Lys
305                 310                 315                 320

Thr Pro Thr Tyr Phe Thr Asn Phe Asp Asp Tyr Asn Asn Tyr Pro Ser
                325                 330                 335

Thr Trp Ser Asn Val Asn Thr Thr Asn Gln Asp Gly Leu Gln Gly Ser
            340                 345                 350

Ala Asn Lys Leu Asn Gly Glu Thr Lys Ile Lys Ile Pro Met Ser Glu
        355                 360                 365

Leu Lys Pro Tyr Lys Arg Tyr Val Phe Ser Gly Tyr Ser Lys Asp Pro
    370                 375                 380

Leu Thr Ser Asn Ser Ile Ile Val Lys Ile Lys Ala Lys Glu Glu Lys
385                 390                 395                 400

Thr Asp Tyr Leu Val Pro Glu Gln Gly Tyr Thr Lys Phe Ser Tyr Glu
                405                 410                 415

Phe Glu Thr Thr Glu Lys Asp Ser Ser Asn Ile Glu Ile Thr Leu Ile
            420                 425                 430

Gly Ser Gly Thr Thr Tyr Leu Asp Asn Leu Ser Ile Thr Glu Leu Asn
        435                 440                 445

Ser Thr Pro Glu Ile Leu Asp Glu Pro Glu Val Lys Ile Pro Thr Asp
    450                 455                 460

Gln Glu Ile Met Asp Ala His Lys Ile Tyr Phe Ala Asp Leu Asn Phe
465                 470                 475                 480

Asn Pro Ser Thr Gly Asn Thr Tyr Ile Asn Gly Met Tyr Phe Ala Pro
                485                 490                 495

Thr Gln Thr Asn Lys Glu Ala Leu Asp Tyr Ile Gln Lys Tyr Arg Val
        500                 505                 510

Glu Ala Thr Leu Gln Tyr Ser Gly Phe Lys Asp Ile Gly Thr Lys Asp
    515                 520                 525

Lys Glu Met Arg Asn Tyr Leu Gly Asp Pro Asn Gln Pro Lys Thr Asn
    530                 535                 540

Tyr Val Asn Leu Arg Ser Tyr Phe Thr Gly Gly Glu Asn Ile Met Thr
545                 550                 555                 560

Tyr Lys Lys Leu Arg Ile Tyr Ala Ile Thr Pro Asp Asp Arg Glu Leu
                565                 570                 575

Leu Val Leu Ser Val Asp His His His His His His
        580                 585

<210>  10
<211>  1073
<212>  PRT
<213>  Clostridium difficile

<400>  10

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His Met Glu Ile Val Asn Glu Asp Ile
225                 230                 235                 240

Leu Pro Asn Asn Gly Leu Met Gly Tyr Tyr Phe Thr Asp Glu His Phe
                245                 250                 255

Lys Asp Leu Lys Leu Met Ala Pro Ile Lys Asp Gly Asn Leu Lys Phe
            260                 265                 270

Glu Glu Lys Lys Val Asp Lys Leu Leu Asp Lys Asp Lys Ser Asp Val
        275                 280                 285

Lys Ser Ile Arg Trp Thr Gly Arg Ile Ile Pro Ser Lys Asp Gly Glu
    290                 295                 300

Tyr Thr Leu Ser Thr Asp Arg Asp Asp Val Leu Met Gln Val Asn Thr
305                 310                 315                 320

Glu Ser Thr Ile Ser Asn Thr Leu Lys Val Asn Met Lys Lys Gly Lys
                325                 330                 335

Glu Tyr Lys Val Arg Ile Glu Leu Gln Asp Lys Asn Leu Gly Ser Ile
            340                 345                 350

Asp Asn Leu Ser Ser Pro Asn Leu Tyr Trp Glu Leu Asp Gly Met Lys
        355                 360                 365

Lys Ile Ile Pro Glu Glu Asn Leu Phe Leu Arg Asp Tyr Ser Asn Ile
    370                 375                 380

Glu Lys Asp Asp Pro Phe Ile Pro Asn Asn Asn Phe Phe Asp Pro Lys
385                 390                 395                 400

Leu Met Ser Asp Trp Glu Asp Glu Asp Leu Asp Thr Asp Asn Asp Asn
                405                 410                 415

Ile Pro Asp Ser Tyr Glu Arg Asn Gly Tyr Thr Ile Lys Asp Leu Ile
            420                 425                 430

Ala Val Lys Trp Glu Asp Ser Phe Ala Glu Gln Gly Tyr Lys Lys Tyr
        435                 440                 445

Val Ser Asn Tyr Leu Glu Ser Asn Thr Ala Gly Asp Pro Tyr Thr Asp
    450                 455                 460

Tyr Glu Lys Ala Ser Gly Ser Phe Asp Lys Ala Ile Lys Thr Glu Ala
465                 470                 475                 480

Arg Asp Pro Leu Val Ala Ala Tyr Pro Ile Val Gly Val Gly Met Glu
                485                 490                 495

Lys Leu Ile Ile Ser Thr Asn Glu His Ala Ser Thr Asp Gln Gly Lys
            500                 505                 510

Thr Val Ser Arg Ala Thr Thr Asn Ser Lys Thr Glu Ser Asn Thr Ala
        515                 520                 525

Gly Val Ser Val Asn Val Gly Tyr Gln Asn Gly Phe Thr Ala Asn Val
    530                 535                 540

Thr Thr Asn Tyr Ser His Thr Thr Asp Asn Ser Thr Ala Val Gln Asp
545                 550                 555                 560

Ser Asn Gly Glu Ser Trp Asn Thr Gly Leu Ser Ile Asn Lys Gly Glu
                565                 570                 575

Ser Ala Tyr Ile Asn Ala Asn Val Arg Tyr Tyr Asn Thr Gly Thr Ala
            580                 585                 590

Pro Met Tyr Lys Val Thr Pro Thr Thr Asn Leu Val Leu Asp Gly Asp
        595                 600                 605

Thr Leu Ser Thr Ile Lys Ala Gln Glu Asn Gln Ile Gly Asn Asn Leu
    610                 615                 620

Ser Pro Gly Asp Thr Tyr Pro Lys Lys Gly Leu Ser Pro Leu Ala Leu
625                 630                 635                 640

Asn Thr Met Asp Gln Arg Ser Ser Arg Leu Ile Pro Ile Asn Tyr Asp
                645                 650                 655

Gln Leu Lys Lys Leu Asp Ala Gly Lys Gln Ile Lys Leu Glu Thr Thr
            660                 665                 670

Gln Val Ser Gly Asn Phe Gly Thr Lys Asn Ser Ser Gly Gln Ile Val
        675                 680                 685

Thr Glu Gly Asn Ser Trp Ser Asp Tyr Ile Ser Gln Ile Asp Ser Ile
    690                 695                 700

Ser Ala Ser Ile Ile Leu Asp Thr Glu Asn Glu Ser Tyr Glu Arg Arg
705                 710                 715                 720

Val Thr Ala Lys Asn Leu Gln Asp Pro Glu Asp Lys Thr Pro Glu Leu
                725                 730                 735

Thr Ile Gly Glu Ala Ile Glu Lys Ala Phe Gly Ala Thr Lys Lys Asp
            740                 745                 750

Gly Leu Leu Tyr Phe Asn Asp Ile Pro Ile Asp Glu Ser Cys Val Glu
        755                 760                 765

Leu Ile Phe Asp Asp Asn Thr Ala Asn Lys Ile Lys Asp Ser Leu Lys
    770                 775                 780

Thr Leu Ser Asp Lys Lys Ile Tyr Asn Val Lys Leu Glu Arg Gly Met
785                 790                 795                 800

Asn Ile Leu Ile Lys Thr Pro Thr Tyr Phe Thr Asn Phe Asp Asp Tyr
                805                 810                 815

Asn Asn Tyr Pro Ser Thr Trp Ser Asn Val Asn Thr Thr Asn Gln Asp
            820                 825                 830

Gly Leu Gln Gly Ser Ala Asn Lys Leu Asn Gly Glu Thr Lys Ile Lys
        835                 840                 845

Ile Pro Met Ser Glu Leu Lys Pro Tyr Lys Arg Tyr Val Phe Ser Gly
    850                 855                 860

Tyr Ser Lys Asp Pro Leu Thr Ser Asn Ser Ile Ile Val Lys Ile Lys
865                 870                 875                 880

Ala Lys Glu Glu Lys Thr Asp Tyr Leu Val Pro Glu Gln Gly Tyr Thr

                885                 890                 895

Lys Phe Ser Tyr Glu Phe Glu Thr Thr Glu Lys Asp Ser Ser Asn Ile
            900                 905                 910

Glu Ile Thr Leu Ile Gly Ser Gly Thr Thr Tyr Leu Asp Asn Leu Ser
        915                 920                 925

Ile Thr Glu Leu Asn Ser Thr Pro Glu Ile Leu Asp Glu Pro Glu Val
    930                 935                 940

Lys Ile Pro Thr Asp Gln Glu Ile Met Asp Ala His Lys Ile Tyr Phe
945                 950                 955                 960

Ala Asp Leu Asn Phe Asn Pro Ser Thr Gly Asn Thr Tyr Ile Asn Gly
                965                 970                 975

Met Tyr Phe Ala Pro Thr Gln Thr Asn Lys Glu Ala Leu Asp Tyr Ile
            980                 985                 990

Gln Lys Tyr Arg Val Glu Ala Thr Leu Gln Tyr Ser Gly Phe Lys Asp
        995                 1000                1005

Ile Gly Thr Lys Asp Lys Glu Met Arg Asn Tyr Leu Gly Asp Pro
    1010                1015                1020

Asn Gln Pro Lys Thr Asn Tyr Val Asn Leu Arg Ser Tyr Phe Thr
    1025                1030                1035

Gly Gly Glu Asn Ile Met Thr Tyr Lys Lys Leu Arg Ile Tyr Ala
    1040                1045                1050

Ile Thr Pro Asp Asp Arg Glu Leu Leu Val Leu Ser Val Asp His
    1055                1060                1065

His His His His His
    1070

<210> 11
<211> 1073
<212> PRT
<213> Clostridium difficile

<400> 11

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu

20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His Met Glu Ile Val Asn Glu Asp Ile
225                 230                 235                 240

Leu Pro Asn Asn Gly Leu Met Gly Tyr Tyr Phe Thr Asp Glu His Phe
                245                 250                 255

Lys Asp Leu Lys Leu Met Ala Pro Ile Lys Asp Gly Asn Leu Lys Phe
            260                 265                 270

Glu Glu Lys Lys Val Asp Lys Leu Leu Asp Lys Asp Lys Ser Asp Val
        275                 280                 285

Lys Ser Ile Arg Trp Thr Gly Arg Ile Ile Pro Ser Lys Asp Gly Glu
    290                 295                 300

Tyr Thr Leu Ser Thr Asp Arg Asp Asp Val Leu Met Gln Val Asn Thr
305                 310                 315                 320

Glu Ser Thr Ile Ser Asn Thr Leu Lys Val Asn Met Lys Lys Gly Lys
                325                 330                 335

Glu Tyr Lys Val Arg Ile Glu Leu Gln Asp Lys Asn Leu Gly Ser Ile
            340                 345                 350

Asp Asn Leu Ser Ser Pro Asn Leu Tyr Trp Glu Leu Asp Gly Met Lys
        355                 360                 365

Lys Ile Ile Pro Glu Glu Asn Leu Phe Leu Arg Asp Tyr Ser Asn Ile
    370                 375                 380

Glu Lys Asp Asp Pro Phe Ile Pro Asn Asn Asn Phe Phe Asp Pro Lys
385                 390                 395                 400

Leu Met Ser Asp Trp Glu Asp Glu Asp Leu Asp Thr Asp Asn Asp Asn
                405                 410                 415

Ile Pro Asp Ser Tyr Glu Arg Asn Gly Tyr Thr Ile Lys Asp Leu Ile
            420                 425                 430

Ala Val Lys Trp Glu Asp Ser Phe Ala Glu Gln Gly Tyr Lys Lys Tyr
        435                 440                 445

Val Ser Asn Tyr Leu Glu Ser Asn Thr Ala Gly Asp Pro Tyr Thr Asp
    450                 455                 460

Tyr Glu Lys Ala Ser Gly Ser Phe Asp Lys Ala Ile Lys Thr Glu Ala
465                 470                 475                 480

Arg Asp Pro Leu Val Ala Ala Tyr Pro Ile Val Gly Val Gly Met Glu
                485                 490                 495

Lys Leu Ile Ile Ser Thr Asn Glu His Ala Ser Thr Asp Gln Gly Lys
            500                 505                 510

Thr Val Ser Arg Ala Thr Thr Asn Ser Lys Thr Glu Ser Asn Thr Ala
        515                 520                 525

Gly Val Ser Val Asn Val Gly Tyr Gln Asn Gly Phe Thr Ala Asn Val
    530                 535                 540

Thr Thr Asn Tyr Ser His Thr Thr Asp Asn Ser Thr Ala Val Gln Asp
545                 550                 555                 560

Ser Asn Gly Glu Ser Trp Asn Thr Gly Leu Ser Ile Asn Lys Gly Glu
                565                 570                 575

Ser Ala Tyr Ile Asn Ala Asn Val Arg Tyr Tyr Asn Thr Gly Thr Ala
            580                 585                 590

Pro Met Tyr Lys Val Thr Pro Thr Thr Asn Leu Val Leu Asp Gly Asp
        595                 600                 605

Thr Leu Ser Thr Ile Lys Ala Gln Ala Asn Gln Ile Gly Asn Asn Leu
    610                 615                 620

Ser Pro Gly Asp Thr Tyr Pro Lys Lys Gly Leu Ser Pro Leu Ala Leu
625                 630                 635                 640

Asn Thr Met Ala Gln Phe Ser Ser Arg Leu Ile Pro Ile Asn Tyr Asp
                645                 650                 655

Gln Leu Lys Lys Leu Asp Ala Gly Lys Gln Ile Lys Leu Glu Thr Thr
            660                 665                 670

Gln Val Ser Gly Asn Phe Gly Thr Lys Asn Ser Ser Gly Gln Ile Val
        675                 680                 685

Thr Glu Gly Asn Ser Trp Ser Asp Tyr Ile Ser Gln Ile Asp Ser Ile
    690                 695                 700

Ser Ala Ser Ile Ile Leu Asp Thr Glu Asn Glu Ser Tyr Glu Arg Arg
705                 710                 715                 720

Val Thr Ala Lys Asn Leu Gln Asp Pro Glu Asp Lys Thr Pro Glu Leu
                725                 730                 735

Thr Ile Gly Glu Ala Ile Glu Lys Ala Phe Gly Ala Thr Lys Lys Asp
            740                 745                 750

Gly Leu Leu Tyr Phe Asn Asp Ile Pro Ile Asp Glu Ser Cys Val Glu
        755                 760                 765

Leu Ile Phe Asp Asp Asn Thr Ala Asn Lys Ile Lys Asp Ser Leu Lys
    770                 775                 780

Thr Leu Ser Asp Lys Lys Ile Tyr Asn Val Lys Leu Glu Arg Gly Met
785                 790                 795                 800

Asn Ile Leu Ile Lys Thr Pro Thr Tyr Phe Thr Asn Phe Asp Asp Tyr
                805                 810                 815

Asn Asn Tyr Pro Ser Thr Trp Ser Asn Val Asn Thr Thr Asn Gln Asp
            820                 825                 830

Gly Leu Gln Gly Ser Ala Asn Lys Leu Asn Gly Glu Thr Lys Ile Lys
        835                 840                 845

Ile Pro Met Ser Glu Leu Lys Pro Tyr Lys Arg Tyr Val Phe Ser Gly
    850                 855                 860

Tyr Ser Lys Asp Pro Leu Thr Ser Asn Ser Ile Ile Val Lys Ile Lys
865                 870                 875                 880

Ala Lys Glu Glu Lys Thr Asp Tyr Leu Val Pro Glu Gln Gly Tyr Thr
                885                 890                 895

Lys Phe Ser Tyr Glu Phe Glu Thr Thr Glu Lys Asp Ser Ser Asn Ile
            900                 905                 910

Glu Ile Thr Leu Ile Gly Ser Gly Thr Thr Tyr Leu Asp Asn Leu Ser
        915                 920                 925

Ile Thr Glu Leu Asn Ser Thr Pro Glu Ile Leu Asp Glu Pro Glu Val
    930                 935                 940

Lys Ile Pro Thr Asp Gln Glu Ile Met Asp Ala His Lys Ile Tyr Phe
945                 950                 955                 960

Ala Asp Leu Asn Phe Asn Pro Ser Thr Gly Asn Thr Tyr Ile Asn Gly
                965                 970                 975

Met Tyr Phe Ala Pro Thr Gln Thr Asn Lys Glu Ala Leu Asp Tyr Ile
            980                 985                 990

Gln Lys Tyr Arg Val Glu Ala Thr  Leu Gln Tyr Ser Gly  Phe Lys Asp
        995                 1000                1005

Ile Gly  Thr Lys Asp Lys Glu  Met Arg Asn Tyr Leu  Gly Asp Pro
    1010                1015                1020

Asn Gln  Pro Lys Thr Asn Tyr  Val Asn Leu Arg Ser  Tyr Phe Thr

1025                    1030                    1035

Gly Gly  Glu Asn Ile Met Thr  Tyr Lys Lys Leu Arg  Ile Tyr Ala
    1040                    1045                    1050

Ile Thr  Pro Asp Asp Arg Glu  Leu Leu Val Leu Ser  Val Asp His
    1055                    1060                    1065

His His  His His His
    1070

<210>  12
<211>  833
<212>  PRT
<213>  Clostridium difficile

<400>  12

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu

165                 170                 175

Asp Leu Asp Thr Asp Asn Asp Asn Ile Pro Asp Ser Tyr Glu Arg Asn
            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Val Thr Thr Asn Tyr Ser His Thr Thr Asp
305                 310                 315                 320

Asn Ser Thr Ala Val Gln Asp Ser Asn Gly Glu Ser Trp Asn Thr Gly
                325                 330                 335

Leu Ser Ile Asn Lys Gly Glu Ser Ala Tyr Ile Asn Ala Asn Val Arg
            340                 345                 350

Tyr Tyr Asn Thr Gly Thr Ala Pro Met Tyr Lys Val Thr Pro Thr Thr
        355                 360                 365

Asn Leu Val Leu Asp Gly Asp Thr Leu Ser Thr Ile Lys Ala Gln Glu
    370                 375                 380

Asn Gln Ile Gly Asn Asn Leu Ser Pro Gly Asp Thr Tyr Pro Lys Lys
385                 390                 395                 400

Gly Leu Ser Pro Leu Ala Leu Asn Thr Met Asp Gln Phe Ser Ser Arg
                405                 410                 415

Leu Ile Pro Ile Asn Tyr Asp Gln Leu Lys Lys Leu Asp Ala Gly Lys
            420                 425                 430

Gln Ile Lys Leu Glu Thr Thr Gln Val Ser Gly Asn Phe Gly Thr Lys
        435                 440                 445

Asn Ser Ser Gly Gln Ile Val Thr Glu Gly Asn Ser Trp Ser Asp Tyr
    450                 455                 460

Ile Ser Gln Ile Asp Ser Ile Ser Ala Ser Ile Ile Leu Asp Thr Glu
465                 470                 475                 480

Asn Glu Ser Tyr Glu Arg Arg Val Thr Ala Gly Gly Gly Pro Glu Leu
                485                 490                 495

Thr Ile Gly Glu Ala Ile Glu Lys Ala Phe Gly Ala Thr Lys Lys Asp
            500                 505                 510

Gly Leu Leu Tyr Phe Asn Asp Ile Pro Ile Asp Glu Ser Cys Val Glu
        515                 520                 525

Leu Ile Phe Asp Asp Asn Thr Ala Asn Lys Ile Lys Asp Ser Leu Lys
    530                 535                 540

Thr Leu Ser Asp Lys Lys Ile Tyr Asn Val Lys Leu Glu Arg Gly Met
545                 550                 555                 560

Asn Ile Leu Ile Lys Thr Pro Thr Tyr Phe Thr Asn Phe Asp Asp Tyr
                565                 570                 575

Asn Asn Tyr Pro Ser Thr Trp Ser Asn Val Asn Thr Thr Asn Gln Asp
            580                 585                 590

Gly Leu Gln Gly Ser Ala Asn Lys Leu Asn Gly Glu Thr Lys Ile Lys
        595                 600                 605

Ile Pro Met Ser Glu Leu Lys Pro Tyr Lys Arg Tyr Val Phe Ser Gly
    610                 615                 620

Tyr Ser Lys Asp Pro Leu Thr Ser Asn Ser Ile Ile Val Lys Ile Lys
625                 630                 635                 640

Ala Lys Glu Glu Lys Thr Asp Tyr Leu Val Pro Glu Gln Gly Tyr Thr
                645                 650                 655

Lys Phe Ser Tyr Glu Phe Glu Thr Thr Glu Lys Asp Ser Ser Asn Ile
            660                 665                 670

Glu Ile Thr Leu Ile Gly Ser Gly Thr Thr Tyr Leu Asp Asn Leu Ser
        675                 680                 685

Ile Thr Glu Leu Asn Ser Thr Pro Glu Ile Leu Asp Glu Pro Glu Val
    690                 695                 700

Lys Ile Pro Thr Asp Gln Glu Ile Met Asp Ala His Lys Ile Tyr Phe
705                 710                 715                 720

Ala Asp Leu Asn Phe Asn Pro Ser Thr Gly Asn Thr Tyr Ile Asn Gly
                725                 730                 735

Met Tyr Phe Ala Pro Thr Gln Thr Asn Lys Glu Ala Leu Asp Tyr Ile
            740                 745                 750

Gln Lys Tyr Arg Val Glu Ala Thr Leu Gln Tyr Ser Gly Phe Lys Asp
        755                 760                 765

Ile Gly Thr Lys Asp Lys Glu Met Arg Asn Tyr Leu Gly Asp Pro Asn
    770                 775                 780

Gln Pro Lys Thr Asn Tyr Val Asn Leu Arg Ser Tyr Phe Thr Gly Gly
785                 790                 795                 800

Glu Asn Ile Met Thr Tyr Lys Lys Leu Arg Ile Tyr Ala Ile Thr Pro
                805                 810                 815

Asp Asp Arg Glu Leu Leu Val Leu Ser Val Asp His His His His His
            820                 825                 830

His

<210>  13
<211>  841
<212>  PRT
<213>  Clostridium difficile

<400>  13

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu
                165                 170                 175

Asp Leu Asp Thr Asp Asn Asp Asn Ile Pro Asp Ser Tyr Glu Arg Asn
            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Asn Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln
    370                 375                 380

Glu Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Asp Gln Phe Ser Ser
                405                 410                 415

Arg Leu Ile Pro Ile Asn Tyr Asp Gln Leu Lys Lys Leu Asp Ala Gly
            420                 425                 430

Lys Gln Ile Lys Leu Glu Thr Thr Gln Val Ser Gly Asn Phe Gly Thr
        435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Lys Asn Leu Gln Gly
                485                 490                 495

Pro Gly Gly Gly Thr Pro Glu Leu Thr Ile Gly Glu Ala Ile Glu Lys
            500                 505                 510

Ala Phe Gly Ala Thr Lys Lys Asp Gly Leu Leu Tyr Phe Asn Asp Ile
        515                 520                 525

Pro Ile Asp Glu Ser Cys Val Glu Leu Ile Phe Asp Asp Asn Thr Ala
    530                 535                 540

Asn Lys Ile Lys Asp Ser Leu Lys Thr Leu Ser Asp Lys Lys Ile Tyr

545                 550                 555                 560

Asn Val Lys Leu Glu Arg Gly Met Asn Ile Leu Ile Lys Thr Pro Thr
                565                 570                 575

Tyr Phe Thr Asn Phe Asp Asp Tyr Asn Asn Tyr Pro Ser Thr Trp Ser
            580                 585                 590

Asn Val Asn Thr Thr Asn Gln Asp Gly Leu Gln Gly Ser Ala Asn Lys
        595                 600                 605

Leu Asn Gly Glu Thr Lys Ile Lys Ile Pro Met Ser Glu Leu Lys Pro
    610                 615                 620

Tyr Lys Arg Tyr Val Phe Ser Gly Tyr Ser Lys Asp Pro Leu Thr Ser
625                 630                 635                 640

Asn Ser Ile Ile Val Lys Ile Lys Ala Lys Glu Glu Lys Thr Asp Tyr
                645                 650                 655

Leu Val Pro Glu Gln Gly Tyr Thr Lys Phe Ser Tyr Glu Phe Glu Thr
            660                 665                 670

Thr Glu Lys Asp Ser Ser Asn Ile Glu Ile Thr Leu Ile Gly Ser Gly
        675                 680                 685

Thr Thr Tyr Leu Asp Asn Leu Ser Ile Thr Glu Leu Asn Ser Thr Pro
    690                 695                 700

Glu Ile Leu Asp Glu Pro Glu Val Lys Ile Pro Thr Asp Gln Glu Ile
705                 710                 715                 720

Met Asp Ala His Lys Ile Tyr Phe Ala Asp Leu Asn Phe Asn Pro Ser
                725                 730                 735

Thr Gly Asn Thr Tyr Ile Asn Gly Met Tyr Phe Ala Pro Thr Gln Thr
            740                 745                 750

Asn Lys Glu Ala Leu Asp Tyr Ile Gln Lys Tyr Arg Val Glu Ala Thr
        755                 760                 765

Leu Gln Tyr Ser Gly Phe Lys Asp Ile Gly Thr Lys Asp Lys Glu Met
    770                 775                 780

Arg Asn Tyr Leu Gly Asp Pro Asn Gln Pro Lys Thr Asn Tyr Val Asn
785                 790                 795                 800

Leu Arg Ser Tyr Phe Thr Gly Gly Glu Asn Ile Met Thr Tyr Lys Lys
                805                 810                 815

Leu Arg Ile Tyr Ala Ile Thr Pro Asp Asp Arg Glu Leu Leu Val Leu
            820                 825                 830

Ser Val Asp His His His His His His
        835                 840

<210>  14
<211>  1094
<212>  PRT
<213>  Clostridium difficile

<400>  14

Met Val Tyr Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro Ser Asp Trp Glu Asp Glu Asp Leu Asp Thr Asp
            420                 425                 430

Asn Asp Asn Ile Pro Asp Ser Tyr Glu Arg Asn Gly Tyr Thr Ile Lys
        435                 440                 445

Asp Leu Ile Ala Val Lys Trp Glu Asp Ser Phe Ala Glu Gln Gly Tyr
    450                 455                 460

Lys Lys Tyr Val Ser Asn Tyr Leu Glu Ser Asn Thr Ala Gly Asp Pro
465                 470                 475                 480

Tyr Thr Asp Tyr Glu Lys Ala Ser Gly Ser Phe Asp Lys Ala Ile Lys
                485                 490                 495

Thr Glu Ala Arg Asp Pro Leu Val Ala Ala Tyr Pro Ile Val Gly Val
            500                 505                 510

Gly Met Glu Lys Leu Ile Ile Ser Thr Asn Glu His Ala Ser Thr Asp
        515                 520                 525

Gln Gly Lys Thr Val Ser Arg Ala Thr Thr Asn Ser Lys Thr Glu Ser
    530                 535                 540

Asn Thr Ala Gly Val Ser Val Asn Val Gly Tyr Gln Asn Gly Phe Thr
545                 550                 555                 560

Ala Asn Val Thr Thr Asn Tyr Ser His Thr Thr Asp Asn Ser Thr Ala
                565                 570                 575

Val Gln Asp Ser Asn Gly Glu Ser Trp Asn Thr Gly Leu Ser Ile Asn
            580                 585                 590

Lys Gly Glu Ser Ala Tyr Ile Asn Ala Asn Val Arg Tyr Tyr Asn Thr
        595                 600                 605

Gly Thr Ala Pro Met Tyr Lys Val Thr Pro Thr Thr Asn Leu Val Leu
    610                 615                 620

Asp Gly Asp Thr Leu Ser Thr Ile Lys Ala Gln Glu Asn Gln Ile Gly
625                 630                 635                 640

Asn Asn Leu Ser Pro Gly Asp Thr Tyr Pro Lys Lys Gly Leu Ser Pro
                645                 650                 655

Leu Ala Leu Asn Thr Met Asp Gln Phe Ser Ser Arg Leu Ile Pro Ile
            660                 665                 670

Asn Tyr Asp Gln Leu Lys Lys Leu Asp Ala Gly Lys Gln Ile Lys Leu
        675                 680                 685

Glu Thr Thr Gln Val Ser Gly Asn Phe Gly Thr Lys Asn Ser Ser Gly
    690                 695                 700

Gln Ile Val Thr Glu Gly Asn Ser Trp Ser Asp Tyr Ile Ser Gln Ile
705                 710                 715                 720

Asp Ser Ile Ser Ala Ser Ile Ile Leu Asp Thr Glu Asn Glu Ser Tyr
                725                 730                 735

Glu Arg Arg Val Thr Ala Lys Asn Leu Gln Asp Pro Glu Asp Lys Thr
            740                 745                 750

Pro Glu Leu Thr Ile Gly Glu Ala Ile Glu Lys Ala Phe Gly Ala Thr
        755                 760                 765

Lys Lys Asp Gly Leu Leu Tyr Phe Asn Asp Ile Pro Ile Asp Glu Ser
    770                 775                 780

Cys Val Glu Leu Ile Phe Asp Asp Asn Thr Ala Asn Lys Ile Lys Asp
785                 790                 795                 800

Ser Leu Lys Thr Leu Ser Asp Lys Lys Ile Tyr Asn Val Lys Leu Glu
                805                 810                 815

Arg Gly Met Asn Ile Leu Ile Lys Thr Pro Thr Tyr Phe Thr Asn Phe
            820                 825                 830

Asp Asp Tyr Asn Asn Tyr Pro Ser Thr Trp Ser Asn Val Asn Thr Thr
        835                 840                 845

Asn Gln Asp Gly Leu Gln Gly Ser Ala Asn Lys Leu Asn Gly Glu Thr
    850                 855                 860

Lys Ile Lys Ile Pro Met Ser Glu Leu Lys Pro Tyr Lys Arg Tyr Val
865                 870                 875                 880

Phe Ser Gly Tyr Ser Lys Asp Pro Leu Thr Ser Asn Ser Ile Ile Val
                885                 890                 895

Lys Ile Lys Ala Lys Glu Glu Lys Thr Asp Tyr Leu Val Pro Glu Gln
            900                 905                 910

Gly Tyr Thr Lys Phe Ser Tyr Glu Phe Glu Thr Thr Glu Lys Asp Ser
        915                 920                 925

Ser Asn Ile Glu Ile Thr Leu Ile Gly Ser Gly Thr Thr Tyr Leu Asp

930                 935                 940

Asn Leu Ser Ile Thr Glu Leu Asn Ser Thr Pro Glu Ile Leu Asp Glu
945                 950                 955                 960

Pro Glu Val Lys Ile Pro Thr Asp Gln Glu Ile Met Asp Ala His Lys
                965                 970                 975

Ile Tyr Phe Ala Asp Leu Asn Phe Asn Pro Ser Thr Gly Asn Thr Tyr
            980                 985                 990

Ile Asn Gly Met Tyr Phe Ala Pro  Thr Gln Thr Asn Lys  Glu Ala Leu
        995                 1000                1005

Asp Tyr  Ile Gln Lys Tyr Arg  Val Glu Ala Thr Leu  Gln Tyr Ser
    1010                1015                1020

Gly Phe  Lys Asp Ile Gly Thr  Lys Asp Lys Glu Met  Arg Asn Tyr
    1025                1030                1035

Leu Gly  Asp Pro Asn Gln Pro  Lys Thr Asn Tyr Val  Asn Leu Arg
    1040                1045                1050

Ser Tyr  Phe Thr Gly Gly Glu  Asn Ile Met Thr Tyr  Lys Lys Leu
    1055                1060                1065

Arg Ile  Tyr Ala Ile Thr Pro  Asp Asp Arg Glu Leu  Leu Val Leu
    1070                1075                1080

Ser Val  Asp Gly Gly His His  His His His His
    1085                1090

<210>  15
<211>  1016
<212>  PRT
<213>  Clostridium difficile

<400>  15
   Met Val Tyr Asn Thr Thr Tyr Lys Ala Pro Ile Glu Arg Pro Glu Asp
   1               5                   10                  15
   Phe Leu Lys Asp Lys Glu Lys Ala Lys Glu Trp Glu Arg Lys Glu Ala
               20                  25                  30
   Glu Arg Ile Glu Gln Lys Leu Glu Arg Ser Glu Lys Glu Ala Leu Glu
           35                  40                  45
   Ser Tyr Lys Lys Asp Ser Val Glu Ile Ser Lys Tyr Ser Gln Thr Arg
   50                  55                  60
   Asn Tyr Phe Tyr Asp Tyr Gln Ile Glu Ala Asn Ser Arg Glu Lys Glu
   65                  70                  75                  80
   Tyr Lys Glu Leu Arg Asn Ala Ile Ser Lys Asn Lys Ile Asp Lys Pro
                   85                  90                  95
   Met Tyr Val Tyr Tyr Phe Glu Ser Pro Glu Lys Phe Ala Phe Asn Lys
               100                 105                 110
   Val Ile Arg Thr Glu Asn Gln Asn Glu Ile Ser Leu Glu Lys Phe Asn
           115                 120                 125
   Glu Phe Lys Glu Thr Ile Gln Asn Lys Leu Phe Lys Gln Asp Gly Phe
       130                 135                 140
   Lys Asp Ile Ser Leu Tyr Glu Pro Gly Lys Gly Asp Glu Lys Pro Thr
   145                 150                 155                 160
   Pro Leu Leu Met His Leu Lys Leu Pro Arg Asn Thr Gly Met Leu Pro
                   165                 170                 175
   Tyr Thr Asn Thr Asn Asn Val Ser Thr Leu Ile Glu Gln Gly Tyr Ser
               180                 185                 190
   Ile Lys Ile Asp Lys Ile Val Arg Ile Val Ile Asp Gly Lys His Tyr
           195                 200                 205
   Ile Lys Ala Glu Ala Ser Val Val Ser Ser Leu Asp Phe Lys Asp Asp
       210                 215                 220
   Val Ser Lys Gly Asp Ser Trp Gly Lys Ala Asn Tyr Asn Asp Trp Ser
   225                 230                 235                 240
   Asn Lys Leu Thr Pro Asn Glu Leu Ala Asp Val Asn Asp Tyr Met Arg
                   245                 250                 255
   Gly Gly Tyr Thr Ala Ile Asn Asn Tyr Leu Ile Ser Asn Gly Pro Val
               260                 265                 270
   Asn Asn Pro Asn Pro Glu Leu Asp Ser Lys Ile Thr Asn Ile Glu Asn
           275                 280                 285
   Ala Leu Lys Arg Glu Pro Ile Pro Thr Asn Leu Thr Val Tyr Arg Arg
       290                 295                 300
   Ser Gly Pro Gln Glu Phe Gly Leu Thr Leu Thr Ser Pro Glu Tyr Asp
   305                 310                 315                 320
   Phe Asn Lys Leu Glu Asn Ile Asp Ala Phe Lys Ser Lys Trp Glu Gly
                   325                 330                 335
   Gln Ala Leu Ser Tyr Pro Asn Phe Ile Ser Thr Ser Ile Gly Ser Val
               340                 345                 350
   Asn Met Ser Ala Phe Ala Lys Arg Lys Ile Val Leu Arg Ile Thr Ile
           355                 360                 365
   Pro Lys Gly Ser Pro Gly Ala Tyr Leu Ser Ala Ile Pro Gly Tyr Ala
       370                 375                 380
   Gly Gln Tyr Glu Val Leu Leu Asn His Gly Ser Lys Phe Lys Ile Asn
   385                 390                 395                 400
   Lys Ile Asp Ser Tyr Lys Asp Gly Thr Ile Thr Lys Leu Ile Val Asp
                   405                 410                 415
   Ala Thr Leu Ile Pro Gly Gly Gly Gly Gly Gly Ala Tyr Pro Ile Val
               420                 425                 430
   Gly Val Gly Met Glu Lys Leu Ile Ile Ser Thr Asn Glu His Ala Ser
           435                 440                 445
   Thr Asp Gln Gly Lys Thr Val Ser Arg Ala Thr Thr Asn Ser Lys Thr
       450                 455                 460
   Glu Ser Asn Thr Ala Gly Val Ser Val Asn Val Gly Tyr Gln Asn Gly
   465                 470                 475                 480
   Phe Thr Ala Asn Val Thr Thr Asn Tyr Ser His Thr Thr Asp Asn Ser
                   485                 490                 495
   Thr Ala Val Gln Asp Ser Asn Gly Glu Ser Trp Asn Thr Gly Leu Ser
               500                 505                 510
   Ile Asn Lys Gly Glu Ser Ala Tyr Ile Asn Ala Asn Val Arg Tyr Tyr
           515                 520                 525
   Asn Thr Gly Thr Ala Pro Met Tyr Lys Val Thr Pro Thr Thr Asn Leu
       530                 535                 540
   Val Leu Asp Gly Asp Thr Leu Ser Thr Ile Lys Ala Gln Glu Asn Gln
   545                 550                 555                 560
   Ile Gly Asn Asn Leu Ser Pro Gly Asp Thr Tyr Pro Lys Lys Gly Leu
                   565                 570                 575
   Ser Pro Leu Ala Leu Asn Thr Met Asp Gln Phe Ser Ser Arg Leu Ile
               580                 585                 590
   Pro Ile Asn Tyr Asp Gln Leu Lys Lys Leu Asp Ala Gly Lys Gln Ile
           595                 600                 605
   Lys Leu Glu Thr Thr Gln Val Ser Gly Asn Phe Gly Thr Lys Asn Ser
       610                 615                 620
   Ser Gly Gln Ile Val Thr Glu Gly Asn Ser Trp Ser Asp Tyr Ile Ser
   625                 630                 635                 640
   Gln Ile Asp Ser Ile Ser Ala Ser Ile Ile Leu Asp Thr Glu Asn Glu
                   645                 650                 655
   Ser Tyr Glu Arg Arg Val Thr Ala Lys Asn Leu Gln Asp Pro Glu Asp
               660                 665                 670
   Lys Thr Pro Glu Leu Thr Ile Gly Glu Ala Ile Glu Lys Ala Phe Gly
           675                 680                 685
   Ala Thr Lys Lys Asp Gly Leu Leu Tyr Phe Asn Asp Ile Pro Ile Asp
       690                 695                 700
   Glu Ser Cys Val Glu Leu Ile Phe Asp Asp Asn Thr Ala Asn Lys Ile
   705                 710                 715                 720
   Lys Asp Ser Leu Lys Thr Leu Ser Asp Lys Lys Ile Tyr Asn Val Lys
                   725                 730                 735
   Leu Glu Arg Gly Met Asn Ile Leu Ile Lys Thr Pro Thr Tyr Phe Thr
               740                 745                 750
   Asn Phe Asp Asp Tyr Asn Asn Tyr Pro Ser Thr Trp Ser Asn Val Asn
           755                 760                 765
   Thr Thr Asn Gln Asp Gly Leu Gln Gly Ser Ala Asn Lys Leu Asn Gly
       770                 775                 780
   Glu Thr Lys Ile Lys Ile Pro Met Ser Glu Leu Lys Pro Tyr Lys Arg
   785                 790                 795                 800
   Tyr Val Phe Ser Gly Tyr Ser Lys Asp Pro Leu Thr Ser Asn Ser Ile
                   805                 810                 815
   Ile Val Lys Ile Lys Ala Lys Glu Glu Lys Thr Asp Tyr Leu Val Pro
               820                 825                 830
   Glu Gln Gly Tyr Thr Lys Phe Ser Tyr Glu Phe Glu Thr Thr Glu Lys
           835                 840                 845
   Asp Ser Ser Asn Ile Glu Ile Thr Leu Ile Gly Ser Gly Thr Thr Tyr
       850                 855                 860
   Leu Asp Asn Leu Ser Ile Thr Glu Leu Asn Ser Thr Pro Glu Ile Leu
   865                 870                 875                 880
   Asp Glu Pro Glu Val Lys Ile Pro Thr Asp Gln Glu Ile Met Asp Ala
                   885                 890                 895
   His Lys Ile Tyr Phe Ala Asp Leu Asn Phe Asn Pro Ser Thr Gly Asn
               900                 905                 910
   Thr Tyr Ile Asn Gly Met Tyr Phe Ala Pro Thr Gln Thr Asn Lys Glu
           915                 920                 925
   Ala Leu Asp Tyr Ile Gln Lys Tyr Arg Val Glu Ala Thr Leu Gln Tyr
       930                 935                 940
   Ser Gly Phe Lys Asp Ile Gly Thr Lys Asp Lys Glu Met Arg Asn Tyr
   945                 950                 955                 960
   Leu Gly Asp Pro Asn Gln Pro Lys Thr Asn Tyr Val Asn Leu Arg Ser
                   965                 970                 975
   Tyr Phe Thr Gly Gly Glu Asn Ile Met Thr Tyr Lys Lys Leu Arg Ile
               980                 985                 990
   Tyr Ala Ile Thr Pro Asp Asp Arg  Glu Leu Leu Val Leu  Ser Val Asp
           995                 1000                1005
   Gly Gly  His His His His His  His
       1010                1015

<210>  16
<211>  686
<212>  PRT
<213>  Clostridium difficile

<400>  16

Met Val Tyr Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val

            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro Thr Asn Phe Asp Asp Tyr Asn Asn Tyr Pro Ser
            420                 425                 430

Thr Trp Ser Asn Val Asn Thr Thr Asn Gln Asp Gly Leu Gln Gly Ser
        435                 440                 445

Ala Asn Lys Leu Asn Gly Glu Thr Lys Ile Lys Ile Pro Met Ser Glu
    450                 455                 460

Leu Lys Pro Tyr Lys Arg Tyr Val Phe Ser Gly Tyr Ser Lys Asp Pro
465                 470                 475                 480

Leu Thr Ser Asn Ser Ile Ile Val Lys Ile Lys Ala Lys Glu Glu Lys
                485                 490                 495

Thr Asp Tyr Leu Val Pro Glu Gln Gly Tyr Thr Lys Phe Ser Tyr Glu
            500                 505                 510

Phe Glu Thr Thr Glu Lys Asp Ser Ser Asn Ile Glu Ile Thr Leu Ile
        515                 520                 525

Gly Ser Gly Thr Thr Tyr Leu Asp Asn Leu Ser Ile Thr Glu Leu Asn
    530                 535                 540

Ser Thr Pro Glu Ile Leu Asp Glu Pro Glu Val Lys Ile Pro Thr Asp
545                 550                 555                 560

Gln Glu Ile Met Asp Ala His Lys Ile Tyr Phe Ala Asp Leu Asn Phe
                565                 570                 575

Asn Pro Ser Thr Gly Asn Thr Tyr Ile Asn Gly Met Tyr Phe Ala Pro
            580                 585                 590

Thr Gln Thr Asn Lys Glu Ala Leu Asp Tyr Ile Gln Lys Tyr Arg Val
        595                 600                 605

Glu Ala Thr Leu Gln Tyr Ser Gly Phe Lys Asp Ile Gly Thr Lys Asp
    610                 615                 620

Lys Glu Met Arg Asn Tyr Leu Gly Asp Pro Asn Gln Pro Lys Thr Asn
625                 630                 635                 640

Tyr Val Asn Leu Arg Ser Tyr Phe Thr Gly Gly Glu Asn Ile Met Thr
                645                 650                 655

Tyr Lys Lys Leu Arg Ile Tyr Ala Ile Thr Pro Asp Asp Arg Glu Leu
            660                 665                 670

Leu Val Leu Ser Val Asp Gly Gly His His His His His His
        675                 680                 685

<210>  17
<211>  676
<212>  PRT
<213>  Clostridium difficile

<400>  17

Met Val Tyr Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro Gly Gly Gly Gly Gly Gly Asn Thr Thr Asn Gln
            420                 425                 430

Asp Gly Leu Gln Gly Ser Ala Asn Lys Leu Asn Gly Glu Thr Lys Ile
        435                 440                 445

Lys Ile Pro Met Ser Glu Leu Lys Pro Tyr Lys Arg Tyr Val Phe Ser
    450                 455                 460

Gly Tyr Ser Lys Asp Pro Leu Thr Ser Asn Ser Ile Ile Val Lys Ile
465                 470                 475                 480

Lys Ala Lys Glu Glu Lys Thr Asp Tyr Leu Val Pro Glu Gln Gly Tyr
                485                 490                 495

Thr Lys Phe Ser Tyr Glu Phe Glu Thr Thr Glu Lys Asp Ser Ser Asn
            500                 505                 510

Ile Glu Ile Thr Leu Ile Gly Ser Gly Thr Thr Tyr Leu Asp Asn Leu
        515                 520                 525

Ser Ile Thr Glu Leu Asn Ser Thr Pro Glu Ile Leu Asp Glu Pro Glu
    530                 535                 540

Val Lys Ile Pro Thr Asp Gln Glu Ile Met Asp Ala His Lys Ile Tyr
545                 550                 555                 560

Phe Ala Asp Leu Asn Phe Asn Pro Ser Thr Gly Asn Thr Tyr Ile Asn
                565                 570                 575

Gly Met Tyr Phe Ala Pro Thr Gln Thr Asn Lys Glu Ala Leu Asp Tyr
            580                 585                 590

Ile Gln Lys Tyr Arg Val Glu Ala Thr Leu Gln Tyr Ser Gly Phe Lys
        595                 600                 605

Asp Ile Gly Thr Lys Asp Lys Glu Met Arg Asn Tyr Leu Gly Asp Pro
    610                 615                 620

Asn Gln Pro Lys Thr Asn Tyr Val Asn Leu Arg Ser Tyr Phe Thr Gly
625                 630                 635                 640

Gly Glu Asn Ile Met Thr Tyr Lys Lys Leu Arg Ile Tyr Ala Ile Thr
                645                 650                 655

Pro Asp Asp Arg Glu Leu Leu Val Leu Ser Val Asp Gly Gly His His
            660                 665                 670

His His His His
        675

<210>  18
<211>  232
<212>  PRT
<213>  Clostridium difficile

<400>  18

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser His His His His His His
225                 230

<210>  19
<211>  224
<212>  PRT
<213>  Clostridium difficile

<400>  19

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser His His His His His His
    210                 215                 220

<210>  20
<211>  204
<212>  PRT
<213>  Clostridium difficile

<400>  20

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile His His His His His His
        195                 200

<210>  21
<211>  427
<212>  PRT
<213>  Clostridium difficile

<400>  21

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val

340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
            420                 425

<210>  22
<211>  427
<212>  PRT
<213>  Clostridium difficile

<400>  22

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe

130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Phe Thr Ser Ile Gly Ser Val
            340                 345                 350

Asn Met Ser Ala Phe Ala Lys Ala Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
            420                 425

<210>  23
<211>  427
<212>  PRT
<213>  Clostridium difficile

<400>  23

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
        180                 185                 190

Ile Lys Ile Asp Lys Ile Val Arg Ile Val Ile Asp Gly Lys His Tyr
    195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
        260                 265                 270

Asn Asn Pro Asn Pro Glu Leu Asp Ser Lys Ile Thr Asn Ile Glu Asn
    275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Phe Thr Ser Ile Gly Ser Val
        340                 345                 350

Asn Met Ser Ala Phe Ala Lys Ala Lys Ile Val Leu Arg Ile Thr Ile
    355                 360                 365

Pro Lys Gly Ser Pro Gly Ala Tyr Leu Ser Ala Ile Pro Gly Tyr Ala
    370                 375                 380

Gly Glu Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
        420                 425

<210>  24
<211>  427
<212>  PRT
<213>  Clostridium difficile

<400>  24

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Phe Thr Ser Ile Gly Ser Val
            340                 345                 350

Asn Met Ser Ala Phe Ala Lys Ala Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Glu Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
            420                 425

<210>  25
<211>  841
<212>  PRT
<213>  Clostridium difficile

<400>  25

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu
                165                 170                 175

Asp Leu Asp Thr Asp Asn Asp Asn Ile Pro Asp Ser Tyr Glu Arg Asn
            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Asn Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln
    370                 375                 380

Glu Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Asp Gln Arg Ser Ser
                405                 410                 415

Arg Leu Ile Pro Ile Asn Tyr Asp Gln Leu Lys Lys Leu Asp Ala Gly
            420                 425                 430

Lys Gln Ile Lys Leu Glu Thr Thr Gln Val Ser Gly Asn Phe Gly Thr
        435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Lys Asn Leu Gln Asp
                485                 490                 495

Pro Glu Asp Lys Thr Pro Glu Leu Thr Ile Gly Glu Ala Ile Glu Lys
            500                 505                 510

Ala Phe Gly Ala Thr Lys Lys Asp Gly Leu Leu Tyr Phe Asn Asp Ile

        515                 520                 525

Pro Ile Asp Glu Ser Cys Val Glu Leu Ile Phe Asp Asp Asn Thr Ala
    530                 535                 540

Asn Lys Ile Lys Asp Ser Leu Lys Thr Leu Ser Asp Lys Lys Ile Tyr
545                 550                 555                 560

Asn Val Lys Leu Glu Arg Gly Met Asn Ile Leu Ile Lys Thr Pro Thr
                565                 570                 575

Tyr Phe Thr Asn Phe Asp Asp Tyr Asn Asn Tyr Pro Ser Thr Trp Ser
            580                 585                 590

Asn Val Asn Thr Thr Asn Gln Asp Gly Leu Gln Gly Ser Ala Asn Lys
        595                 600                 605

Leu Asn Gly Glu Thr Lys Ile Lys Ile Pro Met Ser Glu Leu Lys Pro
    610                 615                 620

Tyr Lys Arg Tyr Val Phe Ser Gly Tyr Ser Lys Asp Pro Leu Thr Ser
625                 630                 635                 640

Asn Ser Ile Ile Val Lys Ile Lys Ala Lys Glu Glu Lys Thr Asp Tyr
                645                 650                 655

Leu Val Pro Glu Gln Gly Tyr Thr Lys Phe Ser Tyr Glu Phe Glu Thr
            660                 665                 670

Thr Glu Lys Asp Ser Ser Asn Ile Glu Ile Thr Leu Ile Gly Ser Gly
        675                 680                 685

Thr Thr Tyr Leu Asp Asn Leu Ser Ile Thr Glu Leu Asn Ser Thr Pro
    690                 695                 700

Glu Ile Leu Asp Glu Pro Glu Val Lys Ile Pro Thr Asp Gln Glu Ile
705                 710                 715                 720

Met Asp Ala His Lys Ile Tyr Phe Ala Asp Leu Asn Phe Asn Pro Ser
                725                 730                 735

Thr Gly Asn Thr Tyr Ile Asn Gly Met Tyr Phe Ala Pro Thr Gln Thr
            740                 745                 750

Asn Lys Glu Ala Leu Asp Tyr Ile Gln Lys Tyr Arg Val Glu Ala Thr
        755                 760                 765

Leu Gln Tyr Ser Gly Phe Lys Asp Ile Gly Thr Lys Asp Lys Glu Met
    770                 775                 780

Arg Asn Tyr Leu Gly Asp Pro Asn Gln Pro Lys Thr Asn Tyr Val Asn
785                 790                 795                 800

Leu Arg Ser Tyr Phe Thr Gly Gly Glu Asn Ile Met Thr Tyr Lys Lys
                805                 810                 815

Leu Arg Ile Tyr Ala Ile Thr Pro Asp Asp Arg Glu Leu Leu Val Leu
            820                 825                 830

Ser Val Asp His His His His His His
        835                 840

<210>  26
<211>  841
<212>  PRT
<213>  Clostridium difficile

<400>  26

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu
                165                 170                 175

Asp Leu Asp Thr Asp Asn Asp Asn Ile Pro Asp Ser Tyr Glu Arg Asn
            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Asn Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln
    370                 375                 380

Ala Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Ala Gln Phe Ser Ser
                405                 410                 415

Arg Leu Ile Pro Ile Asn Tyr Asp Gln Leu Lys Lys Leu Asp Ala Gly
            420                 425                 430

Lys Gln Ile Lys Leu Glu Thr Thr Gln Val Ser Gly Asn Phe Gly Thr
        435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Lys Asn Leu Gln Asp
                485                 490                 495

Pro Glu Asp Lys Thr Pro Glu Leu Thr Ile Gly Glu Ala Ile Glu Lys
            500                 505                 510

Ala Phe Gly Ala Thr Lys Lys Asp Gly Leu Leu Tyr Phe Asn Asp Ile
        515                 520                 525

Pro Ile Asp Glu Ser Cys Val Glu Leu Ile Phe Asp Asp Asn Thr Ala
    530                 535                 540

Asn Lys Ile Lys Asp Ser Leu Lys Thr Leu Ser Asp Lys Lys Ile Tyr
545                 550                 555                 560

Asn Val Lys Leu Glu Arg Gly Met Asn Ile Leu Ile Lys Thr Pro Thr
                565                 570                 575

Tyr Phe Thr Asn Phe Asp Asp Tyr Asn Asn Tyr Pro Ser Thr Trp Ser
            580                 585                 590

Asn Val Asn Thr Thr Asn Gln Asp Gly Leu Gln Gly Ser Ala Asn Lys
        595                 600                 605

Leu Asn Gly Glu Thr Lys Ile Lys Ile Pro Met Ser Glu Leu Lys Pro
    610                 615                 620

Tyr Lys Arg Tyr Val Phe Ser Gly Tyr Ser Lys Asp Pro Leu Thr Ser
625                 630                 635                 640

Asn Ser Ile Ile Val Lys Ile Lys Ala Lys Glu Glu Lys Thr Asp Tyr
                645                 650                 655

Leu Val Pro Glu Gln Gly Tyr Thr Lys Phe Ser Tyr Glu Phe Glu Thr
            660                 665                 670

Thr Glu Lys Asp Ser Ser Asn Ile Glu Ile Thr Leu Ile Gly Ser Gly
        675                 680                 685

Thr Thr Tyr Leu Asp Asn Leu Ser Ile Thr Glu Leu Asn Ser Thr Pro
    690                 695                 700

Glu Ile Leu Asp Glu Pro Glu Val Lys Ile Pro Thr Asp Gln Glu Ile
705                 710                 715                 720

Met Asp Ala His Lys Ile Tyr Phe Ala Asp Leu Asn Phe Asn Pro Ser
                725                 730                 735

Thr Gly Asn Thr Tyr Ile Asn Gly Met Tyr Phe Ala Pro Thr Gln Thr
            740                 745                 750

Asn Lys Glu Ala Leu Asp Tyr Ile Gln Lys Tyr Arg Val Glu Ala Thr
        755                 760                 765

Leu Gln Tyr Ser Gly Phe Lys Asp Ile Gly Thr Lys Asp Lys Glu Met
    770                 775                 780

Arg Asn Tyr Leu Gly Asp Pro Asn Gln Pro Lys Thr Asn Tyr Val Asn
785                 790                 795                 800

Leu Arg Ser Tyr Phe Thr Gly Gly Glu Asn Ile Met Thr Tyr Lys Lys
                805                 810                 815

Leu Arg Ile Tyr Ala Ile Thr Pro Asp Asp Arg Glu Leu Leu Val Leu
            820                 825                 830

Ser Val Asp His His His His His His
        835                 840

<210> 27
<211> 266
<212> PRT
<213> Clostridium difficile

<400> 27

Met Thr Asn Phe Asp Asp Tyr Asn Asn Tyr Pro Ser Thr Trp Ser Asn
1               5                   10                  15

Val Asn Thr Thr Asn Gln Asp Gly Leu Gln Gly Ser Ala Asn Lys Leu
            20                  25                  30

Asn Gly Glu Thr Lys Ile Lys Ile Pro Met Ser Glu Leu Lys Pro Tyr
        35                  40                  45

Lys Arg Tyr Val Phe Ser Gly Tyr Ser Lys Asp Pro Leu Thr Ser Asn
    50                  55                  60

Ser Ile Ile Val Lys Ile Lys Ala Lys Glu Glu Lys Thr Asp Tyr Leu
65                  70                  75                  80

Val Pro Glu Gln Gly Tyr Thr Lys Phe Ser Tyr Glu Phe Glu Thr Thr
                85                  90                  95

Glu Lys Asp Ser Ser Asn Ile Glu Ile Thr Leu Ile Gly Ser Gly Thr
            100                 105                 110

Thr Tyr Leu Asp Asn Leu Ser Ile Thr Glu Leu Asn Ser Thr Pro Glu
        115                 120                 125

Ile Leu Asp Glu Pro Glu Val Lys Ile Pro Thr Asp Gln Glu Ile Met
    130                 135                 140

Asp Ala His Lys Ile Tyr Phe Ala Asp Leu Asn Phe Asn Pro Ser Thr
145                 150                 155                 160

Gly Asn Thr Tyr Ile Asn Gly Met Tyr Phe Ala Pro Thr Gln Thr Asn
                165                 170                 175

Lys Glu Ala Leu Asp Tyr Ile Gln Lys Tyr Arg Val Glu Ala Thr Leu
            180                 185                 190

Gln Tyr Ser Gly Phe Lys Asp Ile Gly Thr Lys Asp Lys Glu Met Arg
        195                 200                 205

Asn Tyr Leu Gly Asp Pro Asn Gln Pro Lys Thr Asn Tyr Val Asn Leu
    210                 215                 220

Arg Ser Tyr Phe Thr Gly Gly Glu Asn Ile Met Thr Tyr Lys Lys Leu
225                 230                 235                 240

Arg Ile Tyr Ala Ile Thr Pro Asp Asp Arg Glu Leu Leu Val Leu Ser
                245                 250                 255

Val Asp Gly Gly His His His His His His
            260                 265

<210>  28

<211>  250
<212>  PRT
<213>  Clostridium difficile

<400>  28

Met Asn Thr Thr Asn Gln Asp Gly Leu Gln Gly Ser Ala Asn Lys Leu
1               5                   10                  15

Asn Gly Glu Thr Lys Ile Lys Ile Pro Met Ser Glu Leu Lys Pro Tyr
            20                  25                  30

Lys Arg Tyr Val Phe Ser Gly Tyr Ser Lys Asp Pro Leu Thr Ser Asn
        35                  40                  45

Ser Ile Ile Val Lys Ile Lys Ala Lys Glu Glu Lys Thr Asp Tyr Leu
    50                  55                  60

Val Pro Glu Gln Gly Tyr Thr Lys Phe Ser Tyr Glu Phe Glu Thr Thr
65                  70                  75                  80

Glu Lys Asp Ser Ser Asn Ile Glu Ile Thr Leu Ile Gly Ser Gly Thr
                85                  90                  95

Thr Tyr Leu Asp Asn Leu Ser Ile Thr Glu Leu Asn Ser Thr Pro Glu
            100                 105                 110

Ile Leu Asp Glu Pro Glu Val Lys Ile Pro Thr Asp Gln Glu Ile Met
        115                 120                 125

Asp Ala His Lys Ile Tyr Phe Ala Asp Leu Asn Phe Asn Pro Ser Thr
    130                 135                 140

Gly Asn Thr Tyr Ile Asn Gly Met Tyr Phe Ala Pro Thr Gln Thr Asn
145                 150                 155                 160

Lys Glu Ala Leu Asp Tyr Ile Gln Lys Tyr Arg Val Glu Ala Thr Leu
                165                 170                 175

Gln Tyr Ser Gly Phe Lys Asp Ile Gly Thr Lys Asp Lys Glu Met Arg
            180                 185                 190

Asn Tyr Leu Gly Asp Pro Asn Gln Pro Lys Thr Asn Tyr Val Asn Leu
        195                 200                 205

Arg Ser Tyr Phe Thr Gly Gly Glu Asn Ile Met Thr Tyr Lys Lys Leu
    210                 215                 220

Arg Ile Tyr Ala Ile Thr Pro Asp Asp Arg Glu Leu Leu Val Leu Ser

225                 230                 235                 240

Val Asp Gly Gly His His His His His His
                245                 250

<210>  29
<211>  2710
<212>  PRT
<213>  Clostridium difficile

<400>  29

Met Ser Leu Ile Ser Lys Glu Glu Leu Ile Lys Leu Ala Tyr Ser Ile
1               5                   10                  15

Arg Pro Arg Glu Asn Glu Tyr Lys Thr Ile Leu Thr Asn Leu Asp Glu
            20                  25                  30

Tyr Asn Lys Leu Thr Thr Asn Asn Asn Glu Asn Lys Tyr Leu Gln Leu
        35                  40                  45

Lys Lys Leu Asn Glu Ser Ile Asp Val Phe Met Asn Lys Tyr Lys Thr
    50                  55                  60

Ser Ser Arg Asn Arg Ala Leu Ser Asn Leu Lys Lys Asp Ile Leu Lys
65                  70                  75                  80

Glu Val Ile Leu Ile Lys Asn Ser Asn Thr Ser Pro Val Glu Lys Asn
                85                  90                  95

Leu His Phe Val Trp Ile Gly Gly Glu Val Ser Asp Ile Ala Leu Glu
            100                 105                 110

Tyr Ile Lys Gln Trp Ala Asp Ile Asn Ala Glu Tyr Asn Ile Lys Leu
        115                 120                 125

Trp Tyr Asp Ser Glu Ala Phe Leu Val Asn Thr Leu Lys Lys Ala Ile
    130                 135                 140

Val Glu Ser Ser Thr Thr Glu Ala Leu Gln Leu Leu Glu Glu Glu Ile
145                 150                 155                 160

Gln Asn Pro Gln Phe Asp Asn Met Lys Phe Tyr Lys Lys Arg Met Glu
                165                 170                 175

Phe Ile Tyr Asp Arg Gln Lys Arg Phe Ile Asn Tyr Tyr Lys Ser Gln
            180                 185                 190

Ile Asn Lys Pro Thr Val Pro Thr Ile Asp Asp Ile Ile Lys Ser His

195                 200                 205

Leu Val Ser Glu Tyr Asn Arg Asp Glu Thr Val Leu Glu Ser Tyr Arg
    210                 215                 220

Thr Asn Ser Leu Arg Lys Ile Asn Ser Asn His Gly Ile Asp Ile Arg
225                 230                 235                 240

Ala Asn Ser Leu Phe Thr Glu Gln Glu Leu Leu Asn Ile Tyr Ser Gln
                245                 250                 255

Glu Leu Leu Asn Arg Gly Asn Leu Ala Ala Ala Ser Asp Ile Val Arg
            260                 265                 270

Leu Leu Ala Leu Lys Asn Phe Gly Gly Val Tyr Leu Asp Val Asp Met
        275                 280                 285

Leu Pro Gly Ile His Ser Asp Leu Phe Lys Thr Ile Ser Arg Pro Ser
    290                 295                 300

Ser Ile Gly Leu Asp Arg Trp Glu Met Ile Lys Leu Glu Ala Ile Met
305                 310                 315                 320

Lys Tyr Lys Lys Tyr Ile Asn Asn Tyr Thr Ser Glu Asn Phe Asp Lys
                325                 330                 335

Leu Asp Gln Gln Leu Lys Asp Asn Phe Lys Leu Ile Ile Glu Ser Lys
            340                 345                 350

Ser Glu Lys Ser Glu Ile Phe Ser Lys Leu Glu Asn Leu Asn Val Ser
        355                 360                 365

Asp Leu Glu Ile Lys Ile Ala Phe Ala Leu Gly Ser Val Ile Asn Gln
    370                 375                 380

Ala Leu Ile Ser Lys Gln Gly Ser Tyr Leu Thr Asn Leu Val Ile Glu
385                 390                 395                 400

Gln Val Lys Asn Arg Tyr Gln Phe Leu Asn Gln His Leu Asn Pro Ala
                405                 410                 415

Ile Glu Ser Asp Asn Asn Phe Thr Asp Thr Thr Lys Ile Phe His Asp
            420                 425                 430

Ser Leu Phe Asn Ser Ala Thr Ala Glu Asn Ser Met Phe Leu Thr Lys
        435                 440                 445

Ile Ala Pro Tyr Leu Gln Val Gly Phe Met Pro Glu Ala Arg Ser Thr
    450                 455                 460

Ile Ser Leu Ser Gly Pro Gly Ala Tyr Ala Ser Ala Tyr Tyr Asp Phe
465                 470                 475                 480

Ile Asn Leu Gln Glu Asn Thr Ile Glu Lys Thr Leu Lys Ala Ser Asp
                485                 490                 495

Leu Ile Glu Phe Lys Phe Pro Glu Asn Asn Leu Ser Gln Leu Thr Glu
            500                 505                 510

Gln Glu Ile Asn Ser Leu Trp Ser Phe Asp Gln Ala Ser Ala Lys Tyr
        515                 520                 525

Gln Phe Glu Lys Tyr Val Arg Asp Tyr Thr Gly Gly Ser Leu Ser Glu
    530                 535                 540

Asp Asn Gly Val Asp Phe Asn Lys Asn Thr Ala Leu Asp Lys Asn Tyr
545                 550                 555                 560

Leu Leu Asn Asn Lys Ile Pro Ser Asn Asn Val Glu Glu Ala Gly Ser
                565                 570                 575

Lys Asn Tyr Val His Tyr Ile Ile Gln Leu Gln Gly Asp Asp Ile Ser
            580                 585                 590

Tyr Glu Ala Thr Cys Asn Leu Phe Ser Lys Asn Pro Lys Asn Ser Ile
        595                 600                 605

Ile Ile Gln Arg Asn Met Asn Glu Ser Ala Lys Ser Tyr Phe Leu Ser
    610                 615                 620

Asp Asp Gly Glu Ser Ile Leu Glu Leu Asn Lys Tyr Arg Ile Pro Glu
625                 630                 635                 640

Arg Leu Lys Asn Lys Glu Lys Val Lys Val Thr Phe Ile Gly His Gly
                645                 650                 655

Lys Asp Glu Phe Asn Thr Ser Glu Phe Ala Arg Leu Ser Val Asp Ser
            660                 665                 670

Leu Ser Asn Glu Ile Ser Ser Phe Leu Asp Thr Ile Lys Leu Asp Ile
        675                 680                 685

Ser Pro Lys Asn Val Glu Val Asn Leu Leu Gly Cys Asn Met Phe Ser
    690                 695                 700

Tyr Asp Phe Asn Val Glu Glu Thr Tyr Pro Gly Lys Leu Leu Leu Ser
705                 710                 715                 720

Ile Met Asp Lys Ile Thr Ser Thr Leu Pro Asp Val Asn Lys Asn Ser
                725                 730                 735

Ile Thr Ile Gly Ala Asn Gln Tyr Glu Val Arg Ile Asn Ser Glu Gly
            740                 745                 750

Arg Lys Glu Leu Leu Ala His Ser Gly Lys Trp Ile Asn Lys Glu Glu
        755                 760                 765

Ala Ile Met Ser Asp Leu Ser Ser Lys Glu Tyr Ile Phe Phe Asp Ser
    770                 775                 780

Ile Asp Asn Lys Leu Lys Ala Lys Ser Lys Asn Ile Pro Gly Leu Ala
785                 790                 795                 800

Ser Ile Ser Glu Asp Ile Lys Thr Leu Leu Leu Asp Ala Ser Val Ser
                805                 810                 815

Pro Asp Thr Lys Phe Ile Leu Asn Asn Leu Lys Leu Asn Ile Glu Ser
            820                 825                 830

Ser Ile Gly Asp Tyr Ile Tyr Tyr Glu Lys Leu Glu Pro Val Lys Asn
        835                 840                 845

Ile Ile His Asn Ser Ile Asp Asp Leu Ile Asp Glu Phe Asn Leu Leu
    850                 855                 860

Glu Asn Val Ser Asp Glu Leu Tyr Glu Leu Lys Lys Leu Asn Asn Leu
865                 870                 875                 880

Asp Glu Lys Tyr Leu Ile Ser Phe Glu Asp Ile Ser Lys Asn Asn Ser
                885                 890                 895

Thr Tyr Ser Val Arg Phe Ile Asn Lys Ser Asn Gly Glu Ser Val Tyr
            900                 905                 910

Val Glu Thr Glu Lys Glu Ile Phe Ser Lys Tyr Ser Glu His Ile Thr
        915                 920                 925

Lys Glu Ile Ser Thr Ile Lys Asn Ser Ile Ile Thr Asp Val Asn Gly
    930                 935                 940

Asn Leu Leu Asp Asn Ile Gln Leu Asp His Thr Ser Gln Val Asn Thr
945                 950                 955                 960

Leu Asn Ala Ala Phe Phe Ile Gln Ser Leu Ile Asp Tyr Ser Ser Asn
                965                 970                 975

Lys Asp Val Leu Asn Asp Leu Ser Thr Ser Val Lys Val Gln Leu Tyr
            980                 985                 990

Ala Gln Leu Phe Ser Thr Gly Leu  Asn Thr Ile Tyr Asp  Ser Ile Gln
        995                 1000                 1005

Leu Val  Asn Leu Ile Ser Asn  Ala Val Asn Asp Thr  Ile Asn Val
    1010                 1015                 1020

Leu Pro  Thr Ile Thr Glu Gly  Ile Pro Ile Val Ser  Thr Ile Leu
    1025                 1030                 1035

Asp Gly  Ile Asn Leu Gly Ala  Ala Ile Lys Glu Leu  Leu Asp Glu
    1040                 1045                 1050

His Asp  Pro Leu Leu Lys Lys  Glu Leu Glu Ala Lys  Val Gly Val
    1055                 1060                 1065

Leu Ala  Ile Asn Met Ser Leu  Ser Ile Ala Ala Thr  Val Ala Ser
    1070                 1075                 1080

Ile Val  Gly Ile Gly Ala Glu  Val Thr Ile Phe Leu  Leu Pro Ile
    1085                 1090                 1095

Ala Gly  Ile Ser Ala Gly Ile  Pro Ser Leu Val Asn  Asn Glu Leu
    1100                 1105                 1110

Ile Leu  His Asp Lys Ala Thr  Ser Val Val Asn Tyr  Phe Asn His
    1115                 1120                 1125

Leu Ser  Glu Ser Lys Lys Tyr  Gly Pro Leu Lys Thr  Glu Asp Asp
    1130                 1135                 1140

Lys Ile  Leu Val Pro Ile Asp  Asp Leu Val Ile Ser  Glu Ile Asp
    1145                 1150                 1155

Phe Asn  Asn Asn Ser Ile Lys  Leu Gly Thr Cys Asn  Ile Leu Ala
    1160                 1165                 1170

Met Glu  Gly Gly Ser Gly His  Thr Val Thr Gly Asn  Ile Asp His
    1175                 1180                 1185

Phe Phe  Ser Ser Pro Ser Ile  Ser Ser His Ile Pro  Ser Leu Ser

1190                1195                1200

Ile Tyr Ser Ala Ile Gly Ile Glu Thr Glu Asn Leu Asp Phe Ser
    1205                1210                1215

Lys Lys Ile Met Met Leu Pro Asn Ala Pro Ser Arg Val Phe Trp
    1220                1225                1230

Trp Glu Thr Gly Ala Val Pro Gly Leu Arg Ser Leu Glu Asn Asp
    1235                1240                1245

Gly Thr Arg Leu Leu Asp Ser Ile Arg Asp Leu Tyr Pro Gly Lys
    1250                1255                1260

Phe Tyr Trp Arg Phe Tyr Ala Phe Phe Asp Tyr Ala Ile Thr Thr
    1265                1270                1275

Leu Lys Pro Val Tyr Glu Asp Thr Asn Ile Lys Ile Lys Leu Asp
    1280                1285                1290

Lys Asp Thr Arg Asn Phe Ile Met Pro Thr Ile Thr Thr Asn Glu
    1295                1300                1305

Ile Arg Asn Lys Leu Ser Tyr Ser Phe Asp Gly Ala Gly Gly Thr
    1310                1315                1320

Tyr Ser Leu Leu Leu Ser Ser Tyr Pro Ile Ser Thr Asn Ile Asn
    1325                1330                1335

Leu Ser Lys Asp Asp Leu Trp Ile Phe Asn Ile Asp Asn Glu Val
    1340                1345                1350

Arg Glu Ile Ser Ile Glu Asn Gly Thr Ile Lys Lys Gly Lys Leu
    1355                1360                1365

Ile Lys Asp Val Leu Ser Lys Ile Asp Ile Asn Lys Asn Lys Leu
    1370                1375                1380

Ile Ile Gly Asn Gln Thr Ile Asp Phe Ser Gly Asp Ile Asp Asn
    1385                1390                1395

Lys Asp Arg Tyr Ile Phe Leu Thr Cys Glu Leu Asp Asp Lys Ile
    1400                1405                1410

Ser Leu Ile Ile Glu Ile Asn Leu Val Ala Lys Ser Tyr Ser Leu
    1415                1420                1425

Leu Leu Ser Gly Asp Lys Asn Tyr Leu Ile Ser Asn Leu Ser Asn
    1430                1435                1440

Thr Ile Glu Lys Ile Asn Thr Leu Gly Leu Asp Ser Lys Asn Ile
    1445                1450                1455

Ala Tyr Asn Tyr Thr Asp Glu Ser Asn Asn Lys Tyr Phe Gly Ala
    1460                1465                1470

Ile Ser Lys Thr Ser Gln Lys Ser Ile Ile His Tyr Lys Lys Asp
    1475                1480                1485

Ser Lys Asn Ile Leu Glu Phe Tyr Asn Asp Ser Thr Leu Glu Phe
    1490                1495                1500

Asn Ser Lys Asp Phe Ile Ala Glu Asp Ile Asn Val Phe Met Lys
    1505                1510                1515

Asp Asp Ile Asn Thr Ile Thr Gly Lys Tyr Tyr Val Asp Asn Asn
    1520                1525                1530

Thr Asp Lys Ser Ile Asp Phe Ser Ile Ser Leu Val Ser Lys Asn
    1535                1540                1545

Gln Val Lys Val Asn Gly Leu Tyr Leu Asn Glu Ser Val Tyr Ser
    1550                1555                1560

Ser Tyr Leu Asp Phe Val Lys Asn Ser Asp Gly His His Asn Thr
    1565                1570                1575

Ser Asn Phe Met Asn Leu Phe Leu Asp Asn Ile Ser Phe Trp Lys
    1580                1585                1590

Leu Phe Gly Phe Glu Asn Ile Asn Phe Val Ile Asp Lys Tyr Phe
    1595                1600                1605

Thr Leu Val Gly Lys Thr Asn Leu Gly Tyr Val Glu Phe Ile Cys
    1610                1615                1620

Asp Asn Asn Lys Asn Ile Asp Ile Tyr Phe Gly Glu Trp Lys Thr
    1625                1630                1635

Ser Ser Ser Lys Ser Thr Ile Phe Ser Gly Asn Gly Arg Asn Val
    1640                1645                1650

Val Val Glu Pro Ile Tyr Asn Pro Asp Thr Gly Glu Asp Ile Ser
    1655                1660                1665

Thr Ser Leu Asp Phe Ser Tyr Glu Pro Leu Tyr Gly Ile Asp Arg
    1670                1675                1680

Tyr Ile Asn Lys Val Leu Ile Ala Pro Asp Leu Tyr Thr Ser Leu
    1685                1690                1695

Ile Asn Ile Asn Thr Asn Tyr Tyr Ser Asn Glu Tyr Tyr Pro Glu
    1700                1705                1710

Ile Ile Val Leu Asn Pro Asn Thr Phe His Lys Lys Val Asn Ile
    1715                1720                1725

Asn Leu Asp Ser Ser Ser Phe Glu Tyr Lys Trp Ser Thr Glu Gly
    1730                1735                1740

Ser Asp Phe Ile Leu Val Arg Tyr Leu Glu Glu Ser Asn Lys Lys
    1745                1750                1755

Ile Leu Gln Lys Ile Arg Ile Lys Gly Ile Leu Ser Asn Thr Gln
    1760                1765                1770

Ser Phe Asn Lys Met Ser Ile Asp Phe Lys Asp Ile Lys Lys Leu
    1775                1780                1785

Ser Leu Gly Tyr Ile Met Ser Asn Phe Lys Ser Phe Asn Ser Glu
    1790                1795                1800

Asn Glu Leu Asp Arg Asp His Leu Gly Phe Lys Ile Ile Asp Asn
    1805                1810                1815

Lys Thr Tyr Tyr Tyr Asp Glu Asp Ser Lys Leu Val Lys Gly Leu
    1820                1825                1830

Ile Asn Ile Asn Asn Ser Leu Phe Tyr Phe Asp Pro Ile Glu Phe
    1835                1840                1845

Asn Leu Val Thr Gly Trp Gln Thr Ile Asn Gly Lys Lys Tyr Tyr
    1850                1855                1860

Phe Asp Ile Asn Thr Gly Ala Ala Leu Thr Ser Tyr Lys Ile Ile
    1865                1870                1875

Asn Gly Lys His Phe Tyr Phe Asn Asn Asp Gly Val Met Gln Leu
    1880                1885                1890

Gly Val Phe Lys Gly Pro Asp Gly Phe Glu Tyr Phe Ala Pro Ala
    1895                1900                1905

Asn Thr Gln Asn Asn Asn Ile Glu Gly Gln Ala Ile Val Tyr Gln
    1910                1915                1920

Ser Lys Phe Leu Thr Leu Asn Gly Lys Lys Tyr Tyr Phe Asp Asn
    1925                1930                1935

Asn Ser Lys Ala Val Thr Gly Trp Arg Ile Ile Asn Asn Glu Lys
    1940                1945                1950

Tyr Tyr Phe Asn Pro Asn Asn Ala Ile Ala Ala Val Gly Leu Gln
    1955                1960                1965

Val Ile Asp Asn Asn Lys Tyr Tyr Phe Asn Pro Asp Thr Ala Ile
    1970                1975                1980

Ile Ser Lys Gly Trp Gln Thr Val Asn Gly Ser Arg Tyr Tyr Phe
    1985                1990                1995

Asp Thr Asp Thr Ala Ile Ala Phe Asn Gly Tyr Lys Thr Ile Asp
    2000                2005                2010

Gly Lys His Phe Tyr Phe Asp Ser Asp Cys Val Val Lys Ile Gly
    2015                2020                2025

Val Phe Ser Thr Ser Asn Gly Phe Glu Tyr Phe Ala Pro Ala Asn
    2030                2035                2040

Thr Tyr Asn Asn Asn Ile Glu Gly Gln Ala Ile Val Tyr Gln Ser
    2045                2050                2055

Lys Phe Leu Thr Leu Asn Gly Lys Lys Tyr Tyr Phe Asp Asn Asn
    2060                2065                2070

Ser Lys Ala Val Thr Gly Leu Gln Thr Ile Asp Ser Lys Lys Tyr
    2075                2080                2085

Tyr Phe Asn Thr Asn Thr Ala Glu Ala Ala Thr Gly Trp Gln Thr
    2090                2095                2100

Ile Asp Gly Lys Lys Tyr Tyr Phe Asn Thr Asn Thr Ala Glu Ala
    2105                2110                2115

Ala Thr Gly Trp Gln Thr Ile Asp Gly Lys Lys Tyr Tyr Phe Asn
    2120                2125                2130

Thr Asn Thr Ala Ile Ala Ser Thr Gly Tyr Thr Ile Ile Asn Gly

2135                2140                2145

Lys His Phe Tyr Phe Asn Thr Asp Gly Ile Met Gln Ile Gly Val
    2150                2155                2160

Phe Lys Gly Pro Asn Gly Phe Glu Tyr Phe Ala Pro Ala Asn Thr
    2165                2170                2175

Asp Ala Asn Asn Ile Glu Gly Gln Ala Ile Leu Tyr Gln Asn Glu
    2180                2185                2190

Phe Leu Thr Leu Asn Gly Lys Lys Tyr Tyr Phe Gly Ser Asp Ser
    2195                2200                2205

Lys Ala Val Thr Gly Trp Arg Ile Ile Asn Asn Lys Lys Tyr Tyr
    2210                2215                2220

Phe Asn Pro Asn Asn Ala Ile Ala Ala Ile His Leu Cys Thr Ile
    2225                2230                2235

Asn Asn Asp Lys Tyr Tyr Phe Ser Tyr Asp Gly Ile Leu Gln Asn
    2240                2245                2250

Gly Tyr Ile Thr Ile Glu Arg Asn Asn Phe Tyr Phe Asp Ala Asn
    2255                2260                2265

Asn Glu Ser Lys Met Val Thr Gly Val Phe Lys Gly Pro Asn Gly
    2270                2275                2280

Phe Glu Tyr Phe Ala Pro Ala Asn Thr His Asn Asn Asn Ile Glu
    2285                2290                2295

Gly Gln Ala Ile Val Tyr Gln Asn Lys Phe Leu Thr Leu Asn Gly
    2300                2305                2310

Lys Lys Tyr Tyr Phe Asp Asn Asp Ser Lys Ala Val Thr Gly Trp
    2315                2320                2325

Gln Thr Ile Asp Gly Lys Lys Tyr Tyr Phe Asn Leu Asn Thr Ala
    2330                2335                2340

Glu Ala Ala Thr Gly Trp Gln Thr Ile Asp Gly Lys Lys Tyr Tyr
    2345                2350                2355

Phe Asn Leu Asn Thr Ala Glu Ala Ala Thr Gly Trp Gln Thr Ile
    2360                2365                2370

Asp Gly Lys Lys Tyr Tyr Phe Asn Thr Asn Thr Phe Ile Ala Ser
    2375                2380                2385

Thr Gly Tyr Thr Ser Ile Asn Gly Lys His Phe Tyr Phe Asn Thr
    2390                2395                2400

Asp Gly Ile Met Gln Ile Gly Val Phe Lys Gly Pro Asn Gly Phe
    2405                2410                2415

Glu Tyr Phe Ala Pro Ala Asn Thr Asp Ala Asn Asn Ile Glu Gly
    2420                2425                2430

Gln Ala Ile Leu Tyr Gln Asn Lys Phe Leu Thr Leu Asn Gly Lys
    2435                2440                2445

Lys Tyr Tyr Phe Gly Ser Asp Ser Lys Ala Val Thr Gly Leu Arg
    2450                2455                2460

Thr Ile Asp Gly Lys Lys Tyr Tyr Phe Asn Thr Asn Thr Ala Val
    2465                2470                2475

Ala Val Thr Gly Trp Gln Thr Ile Asn Gly Lys Lys Tyr Tyr Phe
    2480                2485                2490

Asn Thr Asn Thr Ser Ile Ala Ser Thr Gly Tyr Thr Ile Ile Ser
    2495                2500                2505

Gly Lys His Phe Tyr Phe Asn Thr Asp Gly Ile Met Gln Ile Gly
    2510                2515                2520

Val Phe Lys Gly Pro Asp Gly Phe Glu Tyr Phe Ala Pro Ala Asn
    2525                2530                2535

Thr Asp Ala Asn Asn Ile Glu Gly Gln Ala Ile Arg Tyr Gln Asn
    2540                2545                2550

Arg Phe Leu Tyr Leu His Asp Asn Ile Tyr Tyr Phe Gly Asn Asn
    2555                2560                2565

Ser Lys Ala Ala Thr Gly Trp Val Thr Ile Asp Gly Asn Arg Tyr
    2570                2575                2580

Tyr Phe Glu Pro Asn Thr Ala Met Gly Ala Asn Gly Tyr Lys Thr
    2585                2590                2595

Ile Asp Asn Lys Asn Phe Tyr Phe Arg Asn Gly Leu Pro Gln Ile
    2600                2605                2610

Gly Val Phe Lys Gly Ser Asn Gly Phe Glu Tyr Phe Ala Pro Ala
    2615                2620                2625

Asn Thr Asp Ala Asn Asn Ile Glu Gly Gln Ala Ile Arg Tyr Gln
    2630                2635                2640

Asn Arg Phe Leu His Leu Leu Gly Lys Ile Tyr Tyr Phe Gly Asn
    2645                2650                2655

Asn Ser Lys Ala Val Thr Gly Trp Gln Thr Ile Asn Gly Lys Val
    2660                2665                2670

Tyr Tyr Phe Met Pro Asp Thr Ala Met Ala Ala Ala Gly Gly Leu
    2675                2680                2685

Phe Glu Ile Asp Gly Val Ile Tyr Phe Phe Gly Val Asp Gly Val
    2690                2695                2700

Lys Ala Pro Gly Ile Tyr Gly
    2705                2710

<210>  30
<211>  2366
<212>  PRT
<213>  Clostridium difficile

<400>  30

Met Ser Leu Val Asn Arg Lys Gln Leu Glu Lys Met Ala Asn Val Arg
1               5                   10                  15

Phe Arg Thr Gln Glu Asp Glu Tyr Val Ala Ile Leu Asp Ala Leu Glu
            20                  25                  30

Glu Tyr His Asn Met Ser Glu Asn Thr Val Val Glu Lys Tyr Leu Lys
        35                  40                  45

Leu Lys Asp Ile Asn Ser Leu Thr Asp Ile Tyr Ile Asp Thr Tyr Lys
    50                  55                  60

Lys Ser Gly Arg Asn Lys Ala Leu Lys Lys Phe Lys Glu Tyr Leu Val
65                  70                  75                  80

Thr Glu Val Leu Glu Leu Lys Asn Asn Asn Leu Thr Pro Val Glu Lys
                85                  90                  95

Asn Leu His Phe Val Trp Ile Gly Gly Gln Ile Asn Asp Thr Ala Ile
            100                 105                 110

Asn Tyr Ile Asn Gln Trp Lys Asp Val Asn Ser Asp Tyr Asn Val Asn
        115                 120                 125

Val Phe Tyr Asp Ser Asn Ala Phe Leu Ile Asn Thr Leu Lys Lys Thr
    130                 135                 140

Val Val Glu Ser Ala Ile Asn Asp Thr Leu Glu Ser Phe Arg Glu Asn
145                 150                 155                 160

Leu Asn Asp Pro Arg Phe Asp Tyr Asn Lys Phe Phe Arg Lys Arg Met
                165                 170                 175

Glu Ile Ile Tyr Asp Lys Gln Lys Asn Phe Ile Asn Tyr Tyr Lys Ala
            180                 185                 190

Gln Arg Glu Glu Asn Pro Glu Leu Ile Ile Asp Asp Ile Val Lys Thr
        195                 200                 205

Tyr Leu Ser Asn Glu Tyr Ser Lys Glu Ile Asp Glu Leu Asn Thr Tyr
    210                 215                 220

Ile Glu Glu Ser Leu Asn Lys Ile Thr Gln Asn Ser Gly Asn Asp Val
225                 230                 235                 240

Arg Asn Phe Glu Glu Phe Lys Asn Gly Glu Ser Phe Asn Leu Tyr Glu
                245                 250                 255

Gln Glu Leu Val Glu Arg Trp Asn Leu Ala Ala Ala Ser Asp Ile Leu
            260                 265                 270

Arg Ile Ser Ala Leu Lys Glu Ile Gly Gly Met Tyr Leu Asp Val Asp
        275                 280                 285

Met Leu Pro Gly Ile Gln Pro Asp Leu Phe Glu Ser Ile Glu Lys Pro
    290                 295                 300

Ser Ser Val Thr Val Asp Phe Trp Glu Met Thr Lys Leu Glu Ala Ile
305                 310                 315                 320

Met Lys Tyr Lys Glu Tyr Ile Pro Glu Tyr Thr Ser Glu His Phe Asp
                325                 330                 335

Met Leu Asp Glu Glu Val Gln Ser Ser Phe Glu Ser Val Leu Ala Ser
            340                 345                 350

Lys Ser Asp Lys Ser Glu Ile Phe Ser Ser Leu Gly Asp Met Glu Ala
        355                 360                 365

Ser Pro Leu Glu Val Lys Ile Ala Phe Asn Ser Lys Gly Ile Ile Asn
    370                 375                 380

Gln Gly Leu Ile Ser Val Lys Asp Ser Tyr Cys Ser Asn Leu Ile Val
385                 390                 395                 400

Lys Gln Ile Glu Asn Arg Tyr Lys Ile Leu Asn Asn Ser Leu Asn Pro
                405                 410                 415

Ala Ile Ser Glu Asp Asn Asp Phe Asn Thr Thr Thr Asn Thr Phe Ile
            420                 425                 430

Asp Ser Ile Met Ala Glu Ala Asn Ala Asp Asn Gly Arg Phe Met Met
        435                 440                 445

Glu Leu Gly Lys Tyr Leu Arg Val Gly Phe Phe Pro Asp Val Lys Thr
    450                 455                 460

Thr Ile Asn Leu Ser Gly Pro Glu Ala Tyr Ala Ala Ala Tyr Gln Asp
465                 470                 475                 480

Leu Leu Met Phe Lys Glu Gly Ser Met Asn Ile His Leu Ile Glu Ala
                485                 490                 495

Asp Leu Arg Asn Phe Glu Ile Ser Lys Thr Asn Ile Ser Gln Ser Thr
            500                 505                 510

Glu Gln Glu Met Ala Ser Leu Trp Ser Phe Asp Asp Ala Arg Ala Lys
        515                 520                 525

Ala Gln Phe Glu Glu Tyr Lys Arg Asn Tyr Phe Glu Gly Ser Leu Gly
    530                 535                 540

Glu Asp Asp Asn Leu Asp Phe Ser Gln Asn Ile Val Val Asp Lys Glu
545                 550                 555                 560

Tyr Leu Leu Glu Lys Ile Ser Ser Leu Ala Arg Ser Ser Glu Arg Gly
                565                 570                 575

Tyr Ile His Tyr Ile Val Gln Leu Gln Gly Asp Lys Ile Ser Tyr Glu
            580                 585                 590

Ala Ala Cys Asn Leu Phe Ala Lys Thr Pro Tyr Asp Ser Val Leu Phe
        595                 600                 605

Gln Lys Asn Ile Glu Asp Ser Glu Ile Ala Tyr Tyr Tyr Asn Pro Gly

610                 615                 620

Asp Gly Glu Ile Gln Glu Ile Asp Lys Tyr Lys Ile Pro Ser Ile Ile
625                 630                 635                 640

Ser Asp Arg Pro Lys Ile Lys Leu Thr Phe Ile Gly His Gly Lys Asp
                645                 650                 655

Glu Phe Asn Thr Asp Ile Phe Ala Gly Phe Asp Val Asp Ser Leu Ser
            660                 665                 670

Thr Glu Ile Glu Ala Ala Ile Asp Leu Ala Lys Glu Asp Ile Ser Pro
        675                 680                 685

Lys Ser Ile Glu Ile Asn Leu Leu Gly Cys Asn Met Phe Ser Tyr Ser
    690                 695                 700

Ile Asn Val Glu Glu Thr Tyr Pro Gly Lys Leu Leu Leu Lys Val Lys
705                 710                 715                 720

Asp Lys Ile Ser Glu Leu Met Pro Ser Ile Ser Gln Asp Ser Ile Ile
                725                 730                 735

Val Ser Ala Asn Gln Tyr Glu Val Arg Ile Asn Ser Glu Gly Arg Arg
            740                 745                 750

Glu Leu Leu Asp His Ser Gly Glu Trp Ile Asn Lys Glu Glu Ser Ile
        755                 760                 765

Ile Lys Asp Ile Ser Ser Lys Glu Tyr Ile Ser Phe Asn Pro Lys Glu
    770                 775                 780

Asn Lys Ile Thr Val Lys Ser Lys Asn Leu Pro Glu Leu Ser Thr Leu
785                 790                 795                 800

Leu Gln Glu Ile Arg Asn Asn Ser Asn Ser Ser Asp Ile Glu Leu Glu
                805                 810                 815

Glu Lys Val Met Leu Thr Glu Cys Glu Ile Asn Val Ile Ser Asn Ile
            820                 825                 830

Asp Thr Gln Ile Val Glu Glu Arg Ile Glu Glu Ala Lys Asn Leu Thr
        835                 840                 845

Ser Asp Ser Ile Asn Tyr Ile Lys Asp Glu Phe Lys Leu Ile Glu Ser
    850                 855                 860

Ile Ser Asp Ala Leu Cys Asp Leu Lys Gln Gln Asn Glu Leu Glu Asp
865                 870                 875                 880

Ser His Phe Ile Ser Phe Glu Asp Ile Ser Glu Thr Asp Glu Gly Phe
                885                 890                 895

Ser Ile Arg Phe Ile Asn Lys Glu Thr Gly Glu Ser Ile Phe Val Glu
            900                 905                 910

Thr Glu Lys Thr Ile Phe Ser Glu Tyr Ala Asn His Ile Thr Glu Glu
        915                 920                 925

Ile Ser Lys Ile Lys Gly Thr Ile Phe Asp Thr Val Asn Gly Lys Leu
    930                 935                 940

Val Lys Lys Val Asn Leu Asp Thr Thr His Glu Val Asn Thr Leu Asn
945                 950                 955                 960

Ala Ala Phe Phe Ile Gln Ser Leu Ile Glu Tyr Asn Ser Ser Lys Glu
                965                 970                 975

Ser Leu Ser Asn Leu Ser Val Ala Met Lys Val Gln Val Tyr Ala Gln
            980                 985                 990

Leu Phe Ser Thr Gly Leu Asn Thr  Ile Thr Asp Ala Ala  Lys Val Val
        995                 1000                 1005

Glu Leu  Val Ser Thr Ala Leu  Asp Glu Thr Ile Asp  Leu Leu Pro
    1010                 1015                 1020

Thr Leu  Ser Glu Gly Leu Pro  Ile Ile Ala Thr Ile  Ile Asp Gly
    1025                 1030                 1035

Val Ser  Leu Gly Ala Ala Ile  Lys Glu Leu Ser Glu  Thr Ser Asp
    1040                 1045                 1050

Pro Leu  Leu Arg Gln Glu Ile  Glu Ala Lys Ile Gly  Ile Met Ala
    1055                 1060                 1065

Val Asn  Leu Thr Thr Ala Thr  Thr Ala Ile Ile Thr  Ser Ser Leu
    1070                 1075                 1080

Gly Ile  Ala Ser Gly Phe Ser  Ile Leu Leu Val Pro  Leu Ala Gly
    1085                 1090                 1095

Ile Ser  Ala Gly Ile Pro Ser  Leu Val Asn Asn Glu  Leu Val Leu
    1100                 1105                 1110

Arg Asp Lys Ala Thr Lys Val Val Asp Tyr Phe Lys His Val Ser
    1115                1120                1125

Leu Val Glu Thr Glu Gly Val Phe Thr Leu Leu Asp Asp Lys Ile
    1130                1135                1140

Met Met Pro Gln Asp Asp Leu Val Ile Ser Glu Ile Asp Phe Asn
    1145                1150                1155

Asn Asn Ser Ile Val Leu Gly Lys Cys Glu Ile Trp Arg Met Glu
    1160                1165                1170

Gly Gly Ser Gly His Thr Val Thr Asp Asp Ile Asp His Phe Phe
    1175                1180                1185

Ser Ala Pro Ser Ile Thr Tyr Arg Glu Pro His Leu Ser Ile Tyr
    1190                1195                1200

Asp Val Leu Glu Val Gln Lys Glu Glu Leu Asp Leu Ser Lys Asp
    1205                1210                1215

Leu Met Val Leu Pro Asn Ala Pro Asn Arg Val Phe Ala Trp Glu
    1220                1225                1230

Thr Gly Trp Thr Pro Gly Leu Arg Ser Leu Glu Asn Asp Gly Thr
    1235                1240                1245

Lys Leu Leu Asp Arg Ile Arg Asp Asn Tyr Glu Gly Glu Phe Tyr
    1250                1255                1260

Trp Arg Tyr Phe Ala Phe Ile Ala Asp Ala Leu Ile Thr Thr Leu
    1265                1270                1275

Lys Pro Arg Tyr Glu Asp Thr Asn Ile Arg Ile Asn Leu Asp Ser
    1280                1285                1290

Asn Thr Arg Ser Phe Ile Val Pro Ile Ile Thr Thr Glu Tyr Ile
    1295                1300                1305

Arg Glu Lys Leu Ser Tyr Ser Phe Tyr Gly Ser Gly Gly Thr Tyr
    1310                1315                1320

Ala Leu Ser Leu Ser Gln Tyr Asn Met Gly Ile Asn Ile Glu Leu
    1325                1330                1335

Ser Glu Ser Asp Val Trp Ile Ile Asp Val Asp Asn Val Val Arg
    1340                1345                1350

Asp Val Thr Ile Glu Ser Asp Lys Ile Lys Lys Gly Asp Leu Ile
    1355                1360                1365

Glu Gly Ile Leu Ser Thr Leu Ser Ile Glu Glu Asn Lys Ile Ile
    1370                1375                1380

Leu Asn Ser His Glu Ile Asn Phe Ser Gly Glu Val Asn Gly Ser
    1385                1390                1395

Asn Gly Phe Val Ser Leu Thr Phe Ser Ile Leu Glu Gly Ile Asn
    1400                1405                1410

Ala Ile Ile Glu Val Asp Leu Leu Ser Lys Ser Tyr Lys Leu Leu
    1415                1420                1425

Ile Ser Gly Glu Leu Lys Ile Leu Met Leu Asn Ser Asn His Ile
    1430                1435                1440

Gln Gln Lys Ile Asp Tyr Ile Gly Phe Asn Ser Glu Leu Gln Lys
    1445                1450                1455

Asn Ile Pro Tyr Ser Phe Val Asp Ser Glu Gly Lys Glu Asn Gly
    1460                1465                1470

Phe Ile Asn Gly Ser Thr Lys Glu Gly Leu Phe Val Ser Glu Leu
    1475                1480                1485

Pro Asp Val Val Leu Ile Ser Lys Val Tyr Met Asp Asp Ser Lys
    1490                1495                1500

Pro Ser Phe Gly Tyr Tyr Ser Asn Asn Leu Lys Asp Val Lys Val
    1505                1510                1515

Ile Thr Lys Asp Asn Val Asn Ile Leu Thr Gly Tyr Tyr Leu Lys
    1520                1525                1530

Asp Asp Ile Lys Ile Ser Leu Ser Leu Thr Leu Gln Asp Glu Lys
    1535                1540                1545

Thr Ile Lys Leu Asn Ser Val His Leu Asp Glu Ser Gly Val Ala
    1550                1555                1560

Glu Ile Leu Lys Phe Met Asn Arg Lys Gly Asn Thr Asn Thr Ser
    1565                1570                1575

Asp Ser Leu Met Ser Phe Leu Glu Ser Met Asn Ile Lys Ser Ile

1580                1585                1590

Phe Val Asn Phe Leu Gln Ser Asn Ile Lys Phe Ile Leu Asp Ala
    1595                1600                1605

Asn Phe Ile Ile Ser Gly Thr Thr Ser Ile Gly Gln Phe Glu Phe
    1610                1615                1620

Ile Cys Asp Glu Asn Asp Asn Ile Gln Pro Tyr Phe Ile Lys Phe
    1625                1630                1635

Asn Thr Leu Glu Thr Asn Tyr Thr Leu Tyr Val Gly Asn Arg Gln
    1640                1645                1650

Asn Met Ile Val Glu Pro Asn Tyr Asp Leu Asp Asp Ser Gly Asp
    1655                1660                1665

Ile Ser Ser Thr Val Ile Asn Phe Ser Gln Lys Tyr Leu Tyr Gly
    1670                1675                1680

Ile Asp Ser Cys Val Asn Lys Val Val Ile Ser Pro Asn Ile Tyr
    1685                1690                1695

Thr Asp Glu Ile Asn Ile Thr Pro Val Tyr Glu Thr Asn Asn Thr
    1700                1705                1710

Tyr Pro Glu Val Ile Val Leu Asp Ala Asn Tyr Ile Asn Glu Lys
    1715                1720                1725

Ile Asn Val Asn Ile Asn Asp Leu Ser Ile Arg Tyr Val Trp Ser
    1730                1735                1740

Asn Asp Gly Asn Asp Phe Ile Leu Met Ser Thr Ser Glu Glu Asn
    1745                1750                1755

Lys Val Ser Gln Val Lys Ile Arg Phe Val Asn Val Phe Lys Asp
    1760                1765                1770

Lys Thr Leu Ala Asn Lys Leu Ser Phe Asn Phe Ser Asp Lys Gln
    1775                1780                1785

Asp Val Pro Val Ser Glu Ile Ile Leu Ser Phe Thr Pro Ser Tyr
    1790                1795                1800

Tyr Glu Asp Gly Leu Ile Gly Tyr Asp Leu Gly Leu Val Ser Leu
    1805                1810                1815

Tyr Asn Glu Lys Phe Tyr Ile Asn Asn Phe Gly Met Met Val Ser
    1820                1825                1830

Gly Leu Ile Tyr Ile Asn Asp Ser Leu Tyr Tyr Phe Lys Pro Pro
    1835                1840                1845

Val Asn Asn Leu Ile Thr Gly Phe Val Thr Val Gly Asp Asp Lys
    1850                1855                1860

Tyr Tyr Phe Asn Pro Ile Asn Gly Gly Ala Ala Ser Ile Gly Glu
    1865                1870                1875

Thr Ile Ile Asp Asp Lys Asn Tyr Tyr Phe Asn Gln Ser Gly Val
    1880                1885                1890

Leu Gln Thr Gly Val Phe Ser Thr Glu Asp Gly Phe Lys Tyr Phe
    1895                1900                1905

Ala Pro Ala Asn Thr Leu Asp Glu Asn Leu Glu Gly Glu Ala Ile
    1910                1915                1920

Asp Phe Thr Gly Lys Leu Ile Ile Asp Glu Asn Ile Tyr Tyr Phe
    1925                1930                1935

Asp Asp Asn Tyr Arg Gly Ala Val Glu Trp Lys Glu Leu Asp Gly
    1940                1945                1950

Glu Met His Tyr Phe Ser Pro Glu Thr Gly Lys Ala Phe Lys Gly
    1955                1960                1965

Leu Asn Gln Ile Gly Asp Tyr Lys Tyr Tyr Phe Asn Ser Asp Gly
    1970                1975                1980

Val Met Gln Lys Gly Phe Val Ser Ile Asn Asp Asn Lys His Tyr
    1985                1990                1995

Phe Asp Asp Ser Gly Val Met Lys Val Gly Tyr Thr Glu Ile Asp
    2000                2005                2010

Gly Lys His Phe Tyr Phe Ala Glu Asn Gly Glu Met Gln Ile Gly
    2015                2020                2025

Val Phe Asn Thr Glu Asp Gly Phe Lys Tyr Phe Ala His His Asn
    2030                2035                2040

Glu Asp Leu Gly Asn Glu Glu Gly Glu Glu Ile Ser Tyr Ser Gly
    2045                2050                2055

Ile Leu Asn Phe Asn Asn Lys Ile Tyr Tyr Phe Asp Asp Ser Phe
    2060                2065                2070

Thr Ala Val Val Gly Trp Lys Asp Leu Glu Asp Gly Ser Lys Tyr
    2075                2080                2085

Tyr Phe Asp Glu Asp Thr Ala Glu Ala Tyr Ile Gly Leu Ser Leu
    2090                2095                2100

Ile Asn Asp Gly Gln Tyr Tyr Phe Asn Asp Asp Gly Ile Met Gln
    2105                2110                2115

Val Gly Phe Val Thr Ile Asn Asp Lys Val Phe Tyr Phe Ser Asp
    2120                2125                2130

Ser Gly Ile Ile Glu Ser Gly Val Gln Asn Ile Asp Asp Asn Tyr
    2135                2140                2145

Phe Tyr Ile Asp Asp Asn Gly Ile Val Gln Ile Gly Val Phe Asp
    2150                2155                2160

Thr Ser Asp Gly Tyr Lys Tyr Phe Ala Pro Ala Asn Thr Val Asn
    2165                2170                2175

Asp Asn Ile Tyr Gly Gln Ala Val Glu Tyr Ser Gly Leu Val Arg
    2180                2185                2190

Val Gly Glu Asp Val Tyr Tyr Phe Gly Glu Thr Tyr Thr Ile Glu
    2195                2200                2205

Thr Gly Trp Ile Tyr Asp Met Glu Asn Glu Ser Asp Lys Tyr Tyr
    2210                2215                2220

Phe Asn Pro Glu Thr Lys Lys Ala Cys Lys Gly Ile Asn Leu Ile
    2225                2230                2235

Asp Asp Ile Lys Tyr Tyr Phe Asp Glu Lys Gly Ile Met Arg Thr
    2240                2245                2250

Gly Leu Ile Ser Phe Glu Asn Asn Asn Tyr Tyr Phe Asn Glu Asn
    2255                2260                2265

Gly Glu Met Gln Phe Gly Tyr Ile Asn Ile Glu Asp Lys Met Phe
    2270                2275                2280

Tyr Phe Gly Glu Asp Gly Val Met Gln Ile Gly Val Phe Asn Thr
    2285                2290                2295

Pro Asp Gly Phe Lys Tyr Phe Ala His Gln Asn Thr Leu Asp Glu
    2300                2305                2310

Asn Phe Glu Gly Glu Ser Ile Asn Tyr Thr Gly Trp Leu Asp Leu
    2315                2320                2325

Asp Glu Lys Arg Tyr Tyr Phe Thr Asp Glu Tyr Ile Ala Ala Thr
    2330                2335                2340

Gly Ser Val Ile Ile Asp Gly Glu Glu Tyr Tyr Phe Asp Pro Asp
    2345                2350                2355

Thr Ala Gln Leu Val Ile Ser Glu
    2360                2365

<210>  31
<211>  966
<212>  PRT
<213>  Clostridium difficile

<400>  31

Met Gly Trp Gln Thr Ile Asp Gly Lys Lys Tyr Tyr Phe Asn Thr Asn
1               5                   10                  15

Thr Ala Ile Ala Ser Thr Gly Tyr Thr Ile Ile Asn Gly Lys His Phe
            20                  25                  30

Tyr Phe Asn Thr Asp Gly Ile Met Gln Ile Gly Val Phe Lys Gly Pro
        35                  40                  45

Asn Gly Phe Glu Tyr Phe Ala Pro Ala Asn Thr Asp Ala Asn Asn Ile
    50                  55                  60

Glu Gly Gln Ala Ile Leu Tyr Gln Asn Glu Phe Leu Thr Leu Asn Gly
65                  70                  75                  80

Lys Lys Tyr Tyr Phe Gly Ser Asp Ser Lys Ala Val Thr Gly Trp Arg
                85                  90                  95

Ile Ile Asn Asn Lys Lys Tyr Tyr Phe Asn Pro Asn Asn Ala Ile Ala
            100                 105                 110

Ala Ile His Leu Cys Thr Ile Asn Asn Asp Lys Tyr Tyr Phe Ser Tyr
        115                 120                 125

Asp Gly Ile Leu Gln Asn Gly Tyr Ile Thr Ile Glu Arg Asn Asn Phe
    130                 135                 140

Tyr Phe Asp Ala Asn Asn Glu Ser Lys Met Val Thr Gly Val Phe Lys
145                 150                 155                 160

Gly Pro Asn Gly Phe Glu Tyr Phe Ala Pro Ala Asn Thr His Asn Asn
                165                 170                 175

Asn Ile Glu Gly Gln Ala Ile Val Tyr Gln Asn Lys Phe Leu Thr Leu
            180                 185                 190

Asn Gly Lys Lys Tyr Tyr Phe Asp Asn Asp Ser Lys Ala Val Thr Gly
        195                 200                 205

Trp Gln Thr Ile Asp Gly Lys Lys Tyr Tyr Phe Asn Leu Asn Thr Ala
    210                 215                 220

Glu Ala Ala Thr Gly Trp Gln Thr Ile Asp Gly Lys Lys Tyr Tyr Phe
225                 230                 235                 240

Asn Leu Asn Thr Ala Glu Ala Ala Thr Gly Trp Gln Thr Ile Asp Gly
                245                 250                 255

Lys Lys Tyr Tyr Phe Asn Thr Asn Thr Phe Ile Ala Ser Thr Gly Tyr
            260                 265                 270

Thr Ser Ile Asn Gly Lys His Phe Tyr Phe Asn Thr Asp Gly Ile Met
        275                 280                 285

Gln Ile Gly Val Phe Lys Gly Pro Asn Gly Phe Glu Tyr Phe Ala Pro
    290                 295                 300

Ala Asn Thr Asp Ala Asn Asn Ile Glu Gly Gln Ala Ile Leu Tyr Gln
305                 310                 315                 320

Asn Lys Phe Leu Thr Leu Asn Gly Lys Lys Tyr Tyr Phe Gly Ser Asp
                325                 330                 335

Ser Lys Ala Val Thr Gly Leu Arg Thr Ile Asp Gly Lys Lys Tyr Tyr
            340                 345                 350

Phe Asn Thr Asn Thr Ala Val Ala Val Thr Gly Trp Gln Thr Ile Asn
        355                 360                 365

Gly Lys Lys Tyr Tyr Phe Asn Thr Asn Thr Ser Ile Ala Ser Thr Gly
    370                 375                 380

Tyr Thr Ile Ile Ser Gly Lys His Phe Tyr Phe Asn Thr Asp Gly Ile

385                 390                 395                 400

Met Gln Ile Gly Val Phe Lys Gly Pro Asp Gly Phe Glu Tyr Phe Ala
                405                 410                 415

Pro Ala Asn Thr Asp Ala Asn Asn Ile Glu Gly Gln Ala Ile Arg Tyr
            420                 425                 430

Gln Asn Arg Phe Leu Tyr Leu His Asp Asn Ile Tyr Tyr Phe Gly Asn
        435                 440                 445

Asn Ser Lys Ala Ala Thr Gly Trp Val Thr Ile Asp Gly Asn Arg Tyr
    450                 455                 460

Tyr Phe Glu Pro Asn Thr Ala Met Gly Ala Asn Gly Tyr Lys Thr Ile
465                 470                 475                 480

Asp Asn Lys Asn Phe Tyr Phe Arg Asn Gly Leu Pro Gln Ile Gly Val
                485                 490                 495

Phe Lys Gly Ser Asn Gly Phe Glu Tyr Phe Ala Pro Ala Asn Thr Asp
            500                 505                 510

Ala Asn Asn Ile Glu Gly Gln Ala Ile Arg Tyr Gln Asn Arg Phe Leu
        515                 520                 525

His Leu Leu Gly Lys Ile Tyr Tyr Phe Gly Asn Asn Ser Lys Ala Val
    530                 535                 540

Thr Gly Trp Gln Thr Ile Asn Gly Lys Val Tyr Tyr Phe Met Pro Asp
545                 550                 555                 560

Thr Ala Met Ala Ala Ala Gly Gly Leu Phe Glu Ile Asp Gly Val Ile
                565                 570                 575

Tyr Phe Phe Gly Val Asp Gly Val Lys Ala Pro Gly Phe Val Ser Ile
            580                 585                 590

Asn Asp Asn Lys His Tyr Phe Asp Asp Ser Gly Val Met Lys Val Gly
        595                 600                 605

Tyr Thr Glu Ile Asp Gly Lys His Phe Tyr Phe Ala Glu Asn Gly Glu
    610                 615                 620

Met Gln Ile Gly Val Phe Asn Thr Glu Asp Gly Phe Lys Tyr Phe Ala
625                 630                 635                 640

His His Asn Glu Asp Leu Gly Asn Glu Glu Gly Glu Glu Ile Ser Tyr
                645                 650                 655

Ser Gly Ile Leu Asn Phe Asn Asn Lys Ile Tyr Tyr Phe Asp Asp Ser
            660                 665                 670

Phe Thr Ala Val Val Gly Trp Lys Asp Leu Glu Asp Gly Ser Lys Tyr
        675                 680                 685

Tyr Phe Asp Glu Asp Thr Ala Glu Ala Tyr Ile Gly Leu Ser Leu Ile
    690                 695                 700

Asn Asp Gly Gln Tyr Tyr Phe Asn Asp Asp Gly Ile Met Gln Val Gly
705                 710                 715                 720

Phe Val Thr Ile Asn Asp Lys Val Phe Tyr Phe Ser Asp Ser Gly Ile
                725                 730                 735

Ile Glu Ser Gly Val Gln Asn Ile Asp Asp Asn Tyr Phe Tyr Ile Asp
            740                 745                 750

Asp Asn Gly Ile Val Gln Ile Gly Val Phe Asp Thr Ser Asp Gly Tyr
        755                 760                 765

Lys Tyr Phe Ala Pro Ala Asn Thr Val Asn Asp Asn Ile Tyr Gly Gln
    770                 775                 780

Ala Val Glu Tyr Ser Gly Leu Val Arg Val Gly Glu Asp Val Tyr Tyr
785                 790                 795                 800

Phe Gly Glu Thr Tyr Thr Ile Glu Thr Gly Trp Ile Tyr Asp Met Glu
                805                 810                 815

Asn Glu Ser Asp Lys Tyr Tyr Phe Asn Pro Glu Thr Lys Lys Ala Cys
            820                 825                 830

Lys Gly Ile Asn Leu Ile Asp Asp Ile Lys Tyr Tyr Phe Asp Glu Lys
        835                 840                 845

Gly Ile Met Arg Thr Gly Leu Ile Ser Phe Glu Asn Asn Asn Tyr Tyr
    850                 855                 860

Phe Asn Glu Asn Gly Glu Met Gln Phe Gly Tyr Ile Asn Ile Glu Asp
865                 870                 875                 880

Lys Met Phe Tyr Phe Gly Glu Asp Gly Val Met Gln Ile Gly Val Phe
                885                 890                 895

Asn Thr Pro Asp Gly Phe Lys Tyr Phe Ala His Gln Asn Thr Leu Asp
            900                 905                 910

Glu Asn Phe Glu Gly Glu Ser Ile Asn Tyr Thr Gly Trp Leu Asp Leu
        915                 920                 925

Asp Glu Lys Arg Tyr Tyr Phe Thr Asp Glu Tyr Ile Ala Ala Thr Gly
    930                 935                 940

Ser Val Ile Ile Asp Gly Glu Glu Tyr Tyr Phe Asp Pro Asp Thr Ala
945                 950                 955                 960

Gln Leu Val Ile Ser Glu
                965

<210> 32
<211> 966
<212> PRT
<213> Clostridium difficile

<400> 32

Met Gly Trp Gln Thr Ile Asp Gly Lys Lys Tyr Tyr Phe Asn Thr Asn
1               5                   10                  15

Thr Ala Ile Ala Ser Thr Gly Tyr Thr Ile Ile Asn Gly Lys His Phe
            20                  25                  30

Tyr Phe Asn Thr Asp Gly Ile Met Gln Ile Gly Val Phe Lys Gly Pro
        35                  40                  45

Asn Gly Phe Glu Tyr Phe Ala Pro Ala Asn Thr Asp Ala Asn Asn Ile
    50                  55                  60

Glu Gly Gln Ala Ile Leu Tyr Gln Asn Glu Phe Leu Thr Leu Asn Gly
65                  70                  75                  80

Lys Lys Tyr Tyr Phe Gly Ser Asp Ser Lys Ala Val Thr Gly Trp Arg
                85                  90                  95

Ile Ile Asn Asn Lys Lys Tyr Tyr Phe Asn Pro Asn Asn Ala Ile Ala
            100                 105                 110

Ala Ile His Leu Cys Thr Ile Asn Asn Asp Lys Tyr Tyr Phe Ser Tyr
        115                 120                 125

Asp Gly Ile Leu Gln Asn Gly Tyr Ile Thr Ile Glu Arg Asn Asn Phe
    130                 135                 140

Tyr Phe Asp Ala Asn Asn Glu Ser Lys Met Val Thr Gly Val Phe Lys
145                 150                 155                 160

Gly Pro Asn Gly Phe Glu Tyr Phe Ala Pro Ala Asn Thr His Asn Asn
                165                 170                 175

Asn Ile Glu Gly Gln Ala Ile Val Tyr Gln Asn Lys Phe Leu Thr Leu
            180                 185                 190

Asn Gly Lys Lys Tyr Tyr Phe Asp Asn Asp Ser Lys Ala Val Thr Gly
        195                 200                 205

Trp Gln Thr Ile Asp Gly Lys Lys Tyr Tyr Phe Asn Leu Asn Thr Ala
    210                 215                 220

Glu Ala Ala Thr Gly Trp Gln Thr Ile Asp Gly Lys Lys Tyr Tyr Phe
225                 230                 235                 240

Asn Leu Asn Thr Ala Glu Ala Ala Thr Gly Trp Gln Thr Ile Asp Gly
                245                 250                 255

Lys Lys Tyr Tyr Phe Asn Thr Asn Thr Phe Ile Ala Ser Thr Gly Tyr
            260                 265                 270

Thr Ser Ile Asn Gly Lys His Phe Tyr Phe Asn Thr Asp Gly Ile Met
        275                 280                 285

Gln Ile Gly Val Phe Lys Gly Pro Asn Gly Phe Glu Tyr Phe Ala Pro
    290                 295                 300

Ala Asn Thr Asp Ala Asn Asn Ile Glu Gly Gln Ala Ile Leu Tyr Gln
305                 310                 315                 320

Asn Lys Phe Leu Thr Leu Asn Gly Lys Lys Tyr Tyr Phe Gly Ser Asp
                325                 330                 335

Ser Lys Ala Val Thr Gly Leu Arg Thr Ile Asp Gly Lys Lys Tyr Tyr
            340                 345                 350

Phe Asn Thr Asn Thr Ala Val Ala Val Thr Gly Trp Gln Thr Ile Asn
        355                 360                 365

Gly Lys Lys Tyr Tyr Phe Asn Thr Asn Thr Ser Ile Ala Ser Thr Gly
    370                 375                 380

Tyr Thr Ile Ile Ser Gly Lys His Phe Tyr Phe Asn Thr Asp Gly Ile
385                 390                 395                 400

Met Gln Ile Gly Val Phe Lys Gly Pro Asp Gly Phe Glu Tyr Phe Ala
                405                 410                 415

Pro Ala Asn Thr Asp Ala Asn Asn Ile Glu Gly Gln Ala Ile Arg Tyr
            420                 425                 430

Gln Asn Arg Phe Leu Tyr Leu His Asp Asn Ile Tyr Tyr Phe Gly Asn
        435                 440                 445

Asn Ser Lys Ala Ala Thr Gly Trp Val Thr Ile Asp Gly Asn Arg Tyr
    450                 455                 460

Tyr Phe Glu Pro Asn Thr Ala Met Gly Ala Asn Gly Tyr Lys Thr Ile
465                 470                 475                 480

Asp Asn Lys Asn Phe Tyr Phe Arg Asn Gly Leu Pro Gln Ile Gly Val
                485                 490                 495

Phe Lys Gly Ser Asn Gly Phe Glu Tyr Phe Ala Pro Ala Asn Thr Asp
            500                 505                 510

Ala Asn Asn Ile Glu Gly Gln Ala Ile Arg Tyr Gln Asn Arg Phe Leu
        515                 520                 525

His Leu Leu Gly Lys Ile Tyr Tyr Phe Gly Asn Asn Ser Lys Ala Val
    530                 535                 540

Thr Gly Trp Gln Thr Ile Asn Gly Lys Val Tyr Tyr Phe Met Pro Asp
545                 550                 555                 560

Thr Ala Met Ala Ala Ala Gly Gly Leu Asn Gln Ile Gly Asp Tyr Lys
                565                 570                 575

Tyr Tyr Phe Asn Ser Asp Gly Val Met Gln Lys Gly Phe Val Ser Ile
            580                 585                 590

Asn Asp Asn Lys His Tyr Phe Asp Asp Ser Gly Val Met Lys Val Gly
        595                 600                 605

Tyr Thr Glu Ile Asp Gly Lys His Phe Tyr Phe Ala Glu Asn Gly Glu
    610                 615                 620

Met Gln Ile Gly Val Phe Asn Thr Glu Asp Gly Phe Lys Tyr Phe Ala
625                 630                 635                 640

His His Asn Glu Asp Leu Gly Asn Glu Glu Gly Glu Glu Ile Ser Tyr

645                 650                 655

Ser Gly Ile Leu Asn Phe Asn Asn Lys Ile Tyr Tyr Phe Asp Asp Ser
            660                 665                 670

Phe Thr Ala Val Val Gly Trp Lys Asp Leu Glu Asp Gly Ser Lys Tyr
        675                 680                 685

Tyr Phe Asp Glu Asp Thr Ala Glu Ala Tyr Ile Gly Leu Ser Leu Ile
    690                 695                 700

Asn Asp Gly Gln Tyr Tyr Phe Asn Asp Asp Gly Ile Met Gln Val Gly
705                 710                 715                 720

Phe Val Thr Ile Asn Asp Lys Val Phe Tyr Phe Ser Asp Ser Gly Ile
                725                 730                 735

Ile Glu Ser Gly Val Gln Asn Ile Asp Asp Asn Tyr Phe Tyr Ile Asp
            740                 745                 750

Asp Asn Gly Ile Val Gln Ile Gly Val Phe Asp Thr Ser Asp Gly Tyr
        755                 760                 765

Lys Tyr Phe Ala Pro Ala Asn Thr Val Asn Asp Asn Ile Tyr Gly Gln
    770                 775                 780

Ala Val Glu Tyr Ser Gly Leu Val Arg Val Gly Glu Asp Val Tyr Tyr
785                 790                 795                 800

Phe Gly Glu Thr Tyr Thr Ile Glu Thr Gly Trp Ile Tyr Asp Met Glu
                805                 810                 815

Asn Glu Ser Asp Lys Tyr Tyr Phe Asn Pro Glu Thr Lys Lys Ala Cys
            820                 825                 830

Lys Gly Ile Asn Leu Ile Asp Asp Ile Lys Tyr Tyr Phe Asp Glu Lys
        835                 840                 845

Gly Ile Met Arg Thr Gly Leu Ile Ser Phe Glu Asn Asn Asn Tyr Tyr
    850                 855                 860

Phe Asn Glu Asn Gly Glu Met Gln Phe Gly Tyr Ile Asn Ile Glu Asp
865                 870                 875                 880

Lys Met Phe Tyr Phe Gly Glu Asp Gly Val Met Gln Ile Gly Val Phe
                885                 890                 895

Asn Thr Pro Asp Gly Phe Lys Tyr Phe Ala His Gln Asn Thr Leu Asp
            900                 905                 910

Glu Asn Phe Glu Gly Glu Ser Ile Asn Tyr Thr Gly Trp Leu Asp Leu
        915                 920                 925

Asp Glu Lys Arg Tyr Tyr Phe Thr Asp Glu Tyr Ile Ala Ala Thr Gly
    930                 935                 940

Ser Val Ile Ile Asp Gly Glu Glu Tyr Tyr Phe Asp Pro Asp Thr Ala
945                 950                 955                 960

Gln Leu Val Ile Ser Glu
                965

<210> 33
<211> 833
<212> PRT
<213> Clostridium difficile

<400> 33

Met Gly Trp Gln Thr Ile Asp Gly Lys Lys Tyr Tyr Phe Asn Thr Asn
1               5                   10                  15

Thr Ala Ile Ala Ser Thr Gly Tyr Thr Ile Ile Asn Gly Lys His Phe
            20                  25                  30

Tyr Phe Asn Thr Asp Gly Ile Met Gln Ile Gly Val Phe Lys Gly Pro
        35                  40                  45

Asn Gly Phe Glu Tyr Phe Ala Pro Ala Asn Thr Asp Ala Asn Asn Ile
    50                  55                  60

Glu Gly Gln Ala Ile Leu Tyr Gln Asn Glu Phe Leu Thr Leu Asn Gly
65                  70                  75                  80

Lys Lys Tyr Tyr Phe Gly Ser Asp Ser Lys Ala Val Thr Gly Trp Arg
                85                  90                  95

Ile Ile Asn Asn Lys Lys Tyr Tyr Phe Asn Pro Asn Asn Ala Ile Ala
            100                 105                 110

Ala Ile His Leu Cys Thr Ile Asn Asn Asp Lys Tyr Tyr Phe Ser Tyr
        115                 120                 125

Asp Gly Ile Leu Gln Asn Gly Tyr Ile Thr Ile Glu Arg Asn Asn Phe
    130                 135                 140

Tyr Phe Asp Ala Asn Asn Glu Ser Lys Met Val Thr Gly Val Phe Lys
145                 150                 155                 160

Gly Pro Asn Gly Phe Glu Tyr Phe Ala Pro Ala Asn Thr His Asn Asn
                165                 170                 175

Asn Ile Glu Gly Gln Ala Ile Val Tyr Gln Asn Lys Phe Leu Thr Leu
            180                 185                 190

Asn Gly Lys Lys Tyr Tyr Phe Asp Asn Asp Ser Lys Ala Val Thr Gly
        195                 200                 205

Trp Gln Thr Ile Asp Gly Lys Lys Tyr Tyr Phe Asn Leu Asn Thr Ala
    210                 215                 220

Glu Ala Ala Thr Gly Trp Gln Thr Ile Asp Gly Lys Lys Tyr Tyr Phe
225                 230                 235                 240

Asn Leu Asn Thr Ala Glu Ala Ala Thr Gly Trp Gln Thr Ile Asp Gly
                245                 250                 255

Lys Lys Tyr Tyr Phe Asn Thr Asn Thr Phe Ile Ala Ser Thr Gly Tyr
            260                 265                 270

Thr Ser Ile Asn Gly Lys His Phe Tyr Phe Asn Thr Asp Gly Ile Met
        275                 280                 285

Gln Ile Gly Val Phe Lys Gly Pro Asn Gly Phe Glu Tyr Phe Ala Pro
    290                 295                 300

Ala Asn Thr Asp Ala Asn Asn Ile Glu Gly Gln Ala Ile Leu Tyr Gln
305                 310                 315                 320

Asn Lys Phe Leu Thr Leu Asn Gly Lys Lys Tyr Tyr Phe Gly Ser Asp
                325                 330                 335

Ser Lys Ala Val Thr Gly Leu Arg Thr Ile Asp Gly Lys Lys Tyr Tyr
            340                 345                 350

Phe Asn Thr Asn Thr Ala Val Ala Val Thr Gly Trp Gln Thr Ile Asn
        355                 360                 365

Gly Lys Lys Tyr Tyr Phe Asn Thr Asn Thr Ser Ile Ala Ser Thr Gly
    370                 375                 380

Tyr Thr Ile Ile Ser Gly Lys His Phe Tyr Phe Asn Thr Asp Gly Ile
385                 390                 395                 400

Met Gln Ile Gly Val Phe Lys Gly Pro Asp Gly Phe Glu Tyr Phe Ala
                405                 410                 415

Pro Ala Asn Thr Asp Ala Asn Asn Ile Glu Gly Gln Ala Ile Arg Tyr
            420                 425                 430

Gln Asn Arg Phe Leu Tyr Leu His Asp Asn Ile Tyr Tyr Phe Gly Asn
        435                 440                 445

Asn Ser Lys Ala Ala Thr Gly Trp Val Thr Ile Asp Gly Asn Arg Tyr
    450                 455                 460

Tyr Phe Glu Pro Asn Thr Ala Met Gly Ala Asn Gly Tyr Lys Thr Ile
465                 470                 475                 480

Asp Asn Lys Asn Phe Tyr Phe Arg Asn Gly Leu Pro Gln Ile Gly Val
                485                 490                 495

Phe Lys Gly Ser Asn Gly Phe Glu Tyr Phe Ala His His Asn Glu Asp
            500                 505                 510

Leu Gly Asn Glu Glu Gly Glu Glu Ile Ser Tyr Ser Gly Ile Leu Asn
        515                 520                 525

Phe Asn Asn Lys Ile Tyr Tyr Phe Asp Asp Ser Phe Thr Ala Val Val
    530                 535                 540

Gly Trp Lys Asp Leu Glu Asp Gly Ser Lys Tyr Tyr Phe Asp Glu Asp
545                 550                 555                 560

Thr Ala Glu Ala Tyr Ile Gly Leu Ser Leu Ile Asn Asp Gly Gln Tyr
                565                 570                 575

Tyr Phe Asn Asp Asp Gly Ile Met Gln Val Gly Phe Val Thr Ile Asn
            580                 585                 590

Asp Lys Val Phe Tyr Phe Ser Asp Ser Gly Ile Ile Glu Ser Gly Val
        595                 600                 605

Gln Asn Ile Asp Asp Asn Tyr Phe Tyr Ile Asp Asp Asn Gly Ile Val
    610                 615                 620

Gln Ile Gly Val Phe Asp Thr Ser Asp Gly Tyr Lys Tyr Phe Ala Pro
625                 630                 635                 640

Ala Asn Thr Val Asn Asp Asn Ile Tyr Gly Gln Ala Val Glu Tyr Ser
                645                 650                 655

Gly Leu Val Arg Val Gly Glu Asp Val Tyr Tyr Phe Gly Glu Thr Tyr
            660                 665                 670

Thr Ile Glu Thr Gly Trp Ile Tyr Asp Met Glu Asn Glu Ser Asp Lys
        675                 680                 685

Tyr Tyr Phe Asn Pro Glu Thr Lys Lys Ala Cys Lys Gly Ile Asn Leu
    690                 695                 700

Ile Asp Asp Ile Lys Tyr Tyr Phe Asp Glu Lys Gly Ile Met Arg Thr
705                 710                 715                 720

Gly Leu Ile Ser Phe Glu Asn Asn Asn Tyr Tyr Phe Asn Glu Asn Gly
                725                 730                 735

Glu Met Gln Phe Gly Tyr Ile Asn Ile Glu Asp Lys Met Phe Tyr Phe
            740                 745                 750

Gly Glu Asp Gly Val Met Gln Ile Gly Val Phe Asn Thr Pro Asp Gly
        755                 760                 765

Phe Lys Tyr Phe Ala His Gln Asn Thr Leu Asp Glu Asn Phe Glu Gly
    770                 775                 780

Glu Ser Ile Asn Tyr Thr Gly Trp Leu Asp Leu Asp Glu Lys Arg Tyr
785                 790                 795                 800

Tyr Phe Thr Asp Glu Tyr Ile Ala Ala Thr Gly Ser Val Ile Ile Asp
                805                 810                 815

Gly Glu Glu Tyr Tyr Phe Asp Pro Asp Thr Ala Gln Leu Val Ile Ser
            820                 825                 830

Glu

<210> 34
<211> 1057
<212> PRT
<213> Clostridium difficile

<400> 34

Met Gly Trp Gln Thr Ile Asp Gly Lys Lys Tyr Tyr Phe Asn Thr Asn
1               5                   10                  15

Thr Ala Ile Ala Ser Thr Gly Tyr Thr Ile Ile Asn Gly Lys His Phe
            20                  25                  30

Tyr Phe Asn Thr Asp Gly Ile Met Gln Ile Gly Val Phe Lys Gly Pro
        35                  40                  45

Asn Gly Phe Glu Tyr Phe Ala Pro Ala Asn Thr Asp Ala Asn Asn Ile
    50                  55                  60

Glu Gly Gln Ala Ile Leu Tyr Gln Asn Glu Phe Leu Thr Leu Asn Gly
65                  70                  75                  80

Lys Lys Tyr Tyr Phe Gly Ser Asp Ser Lys Ala Val Thr Gly Trp Arg
                85                  90                  95

Ile Ile Asn Asn Lys Lys Tyr Tyr Phe Asn Pro Asn Asn Ala Ile Ala
            100                 105                 110

Ala Ile His Leu Cys Thr Ile Asn Asn Asp Lys Tyr Tyr Phe Ser Tyr
        115                 120                 125

Asp Gly Ile Leu Gln Asn Gly Tyr Ile Thr Ile Glu Arg Asn Asn Phe
    130                 135                 140

Tyr Phe Asp Ala Asn Asn Glu Ser Lys Met Val Thr Gly Val Phe Lys
145                 150                 155                 160

Gly Pro Asn Gly Phe Glu Tyr Phe Ala Pro Ala Asn Thr His Asn Asn
                165                 170                 175

Asn Ile Glu Gly Gln Ala Ile Val Tyr Gln Asn Lys Phe Leu Thr Leu
            180                 185                 190

Asn Gly Lys Lys Tyr Tyr Phe Asp Asn Asp Ser Lys Ala Val Thr Gly
        195                 200                 205

Trp Gln Thr Ile Asp Gly Lys Lys Tyr Tyr Phe Asn Leu Asn Thr Ala
    210                 215                 220

Glu Ala Ala Thr Gly Trp Gln Thr Ile Asp Gly Lys Lys Tyr Tyr Phe
225                 230                 235                 240

Asn Leu Asn Thr Ala Glu Ala Ala Thr Gly Trp Gln Thr Ile Asp Gly
                245                 250                 255

Lys Lys Tyr Tyr Phe Asn Thr Asn Thr Phe Ile Ala Ser Thr Gly Tyr
            260                 265                 270

Thr Ser Ile Asn Gly Lys His Phe Tyr Phe Asn Thr Asp Gly Ile Met

        275                 280                 285

Gln Ile Gly Val Phe Lys Gly Pro Asn Gly Phe Glu Tyr Phe Ala Pro
    290                 295                 300

Ala Asn Thr Asp Ala Asn Asn Ile Glu Gly Gln Ala Ile Leu Tyr Gln
305                 310                 315                 320

Asn Lys Phe Leu Thr Leu Asn Gly Lys Lys Tyr Tyr Phe Gly Ser Asp
                325                 330                 335

Ser Lys Ala Val Thr Gly Leu Arg Thr Ile Asp Gly Lys Lys Tyr Tyr
            340                 345                 350

Phe Asn Thr Asn Thr Ala Val Ala Val Thr Gly Trp Gln Thr Ile Asn
        355                 360                 365

Gly Lys Lys Tyr Tyr Phe Asn Thr Asn Thr Ser Ile Ala Ser Thr Gly
    370                 375                 380

Tyr Thr Ile Ile Ser Gly Lys His Phe Tyr Phe Asn Thr Asp Gly Ile
385                 390                 395                 400

Met Gln Ile Gly Val Phe Lys Gly Pro Asp Gly Phe Glu Tyr Phe Ala
                405                 410                 415

Pro Ala Asn Thr Asp Ala Asn Asn Ile Glu Gly Gln Ala Ile Arg Tyr
            420                 425                 430

Gln Asn Arg Phe Leu Tyr Leu His Asp Asn Ile Tyr Tyr Phe Gly Asn
        435                 440                 445

Asn Ser Lys Ala Ala Thr Gly Trp Val Thr Ile Asp Gly Asn Arg Tyr
    450                 455                 460

Tyr Phe Glu Pro Asn Thr Ala Met Gly Ala Asn Gly Tyr Lys Thr Ile
465                 470                 475                 480

Asp Asn Lys Asn Phe Tyr Phe Arg Asn Gly Leu Pro Gln Ile Gly Val
                485                 490                 495

Phe Lys Gly Ser Asn Gly Phe Glu Tyr Phe Ala Pro Ala Asn Thr Asp
            500                 505                 510

Ala Asn Asn Ile Glu Gly Gln Ala Ile Arg Tyr Gln Asn Arg Phe Leu
        515                 520                 525

His Leu Leu Gly Lys Ile Tyr Tyr Phe Gly Asn Asn Ser Lys Ala Val
    530                 535                 540

Thr Gly Trp Gln Thr Ile Asn Gly Lys Val Tyr Tyr Phe Met Pro Asp
545                 550                 555                 560

Thr Ala Met Ala Ala Ala Gly Gly Glu Thr Ile Ile Asp Asp Lys Asn
                565                 570                 575

Tyr Tyr Phe Asn Gln Ser Gly Val Leu Gln Thr Gly Val Phe Ser Thr
            580                 585                 590

Glu Asp Gly Phe Lys Tyr Phe Ala Pro Ala Asn Thr Leu Asp Glu Asn
        595                 600                 605

Leu Glu Gly Glu Ala Ile Asp Phe Thr Gly Lys Leu Ile Ile Asp Glu
    610                 615                 620

Asn Ile Tyr Tyr Phe Asp Asp Asn Tyr Arg Gly Ala Val Glu Trp Lys
625                 630                 635                 640

Glu Leu Asp Gly Glu Met His Tyr Phe Ser Pro Glu Thr Gly Lys Ala
                645                 650                 655

Phe Lys Gly Leu Asn Gln Ile Gly Asp Tyr Lys Tyr Tyr Phe Asn Ser
            660                 665                 670

Asp Gly Val Met Gln Lys Gly Phe Val Ser Ile Asn Asp Asn Lys His
        675                 680                 685

Tyr Phe Asp Asp Ser Gly Val Met Lys Val Gly Tyr Thr Glu Ile Asp
    690                 695                 700

Gly Lys His Phe Tyr Phe Ala Glu Asn Gly Glu Met Gln Ile Gly Val
705                 710                 715                 720

Phe Asn Thr Glu Asp Gly Phe Lys Tyr Phe Ala His His Asn Glu Asp
                725                 730                 735

Leu Gly Asn Glu Glu Gly Glu Glu Ile Ser Tyr Ser Gly Ile Leu Asn
            740                 745                 750

Phe Asn Asn Lys Ile Tyr Tyr Phe Asp Asp Ser Phe Thr Ala Val Val
        755                 760                 765

Gly Trp Lys Asp Leu Glu Asp Gly Ser Lys Tyr Tyr Phe Asp Glu Asp
    770                 775                 780

Thr Ala Glu Ala Tyr Ile Gly Leu Ser Leu Ile Asn Asp Gly Gln Tyr
785                 790                 795                 800

Tyr Phe Asn Asp Asp Gly Ile Met Gln Val Gly Phe Val Thr Ile Asn
                805                 810                 815

Asp Lys Val Phe Tyr Phe Ser Asp Ser Gly Ile Ile Glu Ser Gly Val
            820                 825                 830

Gln Asn Ile Asp Asp Asn Tyr Phe Tyr Ile Asp Asp Asn Gly Ile Val
        835                 840                 845

Gln Ile Gly Val Phe Asp Thr Ser Asp Gly Tyr Lys Tyr Phe Ala Pro
    850                 855                 860

Ala Asn Thr Val Asn Asp Asn Ile Tyr Gly Gln Ala Val Glu Tyr Ser
865                 870                 875                 880

Gly Leu Val Arg Val Gly Glu Asp Val Tyr Tyr Phe Gly Glu Thr Tyr
                885                 890                 895

Thr Ile Glu Thr Gly Trp Ile Tyr Asp Met Glu Asn Glu Ser Asp Lys
            900                 905                 910

Tyr Tyr Phe Asn Pro Glu Thr Lys Lys Ala Cys Lys Gly Ile Asn Leu
        915                 920                 925

Ile Asp Asp Ile Lys Tyr Tyr Phe Asp Glu Lys Gly Ile Met Arg Thr
    930                 935                 940

Gly Leu Ile Ser Phe Glu Asn Asn Asn Tyr Tyr Phe Asn Glu Asn Gly
945                 950                 955                 960

Glu Met Gln Phe Gly Tyr Ile Asn Ile Glu Asp Lys Met Phe Tyr Phe
                965                 970                 975

Gly Glu Asp Gly Val Met Gln Ile Gly Val Phe Asn Thr Pro Asp Gly
            980                 985                 990

Phe Lys Tyr Phe Ala His Gln Asn  Thr Leu Asp Glu Asn  Phe Glu Gly
        995                 1000                1005

Glu Ser  Ile Asn Tyr Thr Gly  Trp Leu Asp Leu Asp  Glu Lys Arg
    1010                1015                1020

Tyr Tyr  Phe Thr Asp Glu Tyr  Ile Ala Ala Thr Gly  Ser Val Ile
    1025                1030                1035

Ile Asp Gly Glu Glu Tyr Tyr Phe Asp Pro Asp Thr Ala Gln Leu
    1040                1045                1050

Val Ile Ser Glu
    1055

<210> 35
<211> 971
<212> PRT
<213> Clostridium difficile

<400> 35

Met Gly Trp Gln Thr Ile Asp Gly Lys Lys Tyr Tyr Phe Asn Thr Asn
1               5                   10                  15

Thr Ala Ile Ala Ser Thr Gly Tyr Thr Ile Ile Asn Gly Lys His Phe
            20                  25                  30

Tyr Phe Asn Thr Asp Gly Ile Met Gln Ile Gly Val Phe Lys Gly Pro
        35                  40                  45

Asn Gly Phe Glu Tyr Phe Ala Pro Ala Asn Thr Asp Ala Asn Asn Ile
    50                  55                  60

Glu Gly Gln Ala Ile Leu Tyr Gln Asn Glu Phe Leu Thr Leu Asn Gly
65                  70                  75                  80

Lys Lys Tyr Tyr Phe Gly Ser Asp Ser Lys Ala Val Thr Gly Trp Arg
                85                  90                  95

Ile Ile Asn Asn Lys Lys Tyr Tyr Phe Asn Pro Asn Asn Ala Ile Ala
            100                 105                 110

Ala Ile His Leu Cys Thr Ile Asn Asn Asp Lys Tyr Tyr Phe Ser Tyr
        115                 120                 125

Asp Gly Ile Leu Gln Asn Gly Tyr Ile Thr Ile Glu Arg Asn Asn Phe
    130                 135                 140

Tyr Phe Asp Ala Asn Asn Glu Ser Lys Met Val Thr Gly Val Phe Lys
145                 150                 155                 160

Gly Pro Asn Gly Phe Glu Tyr Phe Ala Pro Ala Asn Thr His Asn Asn
                165                 170                 175

Asn Ile Glu Gly Gln Ala Ile Val Tyr Gln Asn Lys Phe Leu Thr Leu
            180                 185                 190

Asn Gly Lys Lys Tyr Tyr Phe Asp Asn Asp Ser Lys Ala Val Thr Gly
        195                 200                 205

Trp Gln Thr Ile Asp Gly Lys Lys Tyr Tyr Phe Asn Leu Asn Thr Ala
    210                 215                 220

Glu Ala Ala Thr Gly Trp Gln Thr Ile Asp Gly Lys Lys Tyr Tyr Phe
225                 230                 235                 240

Asn Leu Asn Thr Ala Glu Ala Ala Thr Gly Trp Gln Thr Ile Asp Gly
                245                 250                 255

Lys Lys Tyr Tyr Phe Asn Thr Asn Thr Phe Ile Ala Ser Thr Gly Tyr
            260                 265                 270

Thr Ser Ile Asn Gly Lys His Phe Tyr Phe Asn Thr Asp Gly Ile Met
        275                 280                 285

Gln Ile Gly Val Phe Lys Gly Pro Asn Gly Phe Glu Tyr Phe Ala Pro
    290                 295                 300

Ala Asn Thr Asp Ala Asn Asn Ile Glu Gly Gln Ala Ile Leu Tyr Gln
305                 310                 315                 320

Asn Lys Phe Leu Thr Leu Asn Gly Lys Lys Tyr Tyr Phe Gly Ser Asp
                325                 330                 335

Ser Lys Ala Val Thr Gly Leu Arg Thr Ile Asp Gly Lys Lys Tyr Tyr
            340                 345                 350

Phe Asn Thr Asn Thr Ala Val Ala Val Thr Gly Trp Gln Thr Ile Asn
        355                 360                 365

Gly Lys Lys Tyr Tyr Phe Asn Thr Asn Thr Ser Ile Ala Ser Thr Gly
    370                 375                 380

Tyr Thr Ile Ile Ser Gly Lys His Phe Tyr Phe Asn Thr Asp Gly Ile
385                 390                 395                 400

Met Gln Ile Gly Val Phe Lys Gly Pro Asp Gly Phe Glu Tyr Phe Ala
                405                 410                 415

Pro Ala Asn Thr Asp Ala Asn Asn Ile Glu Gly Gln Ala Ile Arg Tyr
            420                 425                 430

Gln Asn Arg Phe Leu Tyr Leu His Asp Asn Ile Tyr Tyr Phe Gly Asn

435                 440                 445

Asn Ser Lys Ala Ala Thr Gly Trp Val Thr Ile Asp Gly Asn Arg Tyr
    450                 455                 460

Tyr Phe Glu Pro Asn Thr Ala Met Gly Ala Asn Gly Tyr Lys Thr Ile
465                 470                 475                 480

Asp Asn Lys Asn Phe Tyr Phe Arg Asn Gly Leu Pro Gln Ile Gly Val
                485                 490                 495

Phe Lys Gly Ser Asn Gly Phe Glu Tyr Phe Ala Pro Ala Asn Thr Asp
            500                 505                 510

Ala Asn Asn Ile Glu Gly Gln Ala Ile Arg Tyr Gln Asn Arg Phe Leu
        515                 520                 525

His Leu Leu Gly Lys Ile Tyr Tyr Phe Gly Asn Asn Ser Lys Ala Val
    530                 535                 540

Thr Gly Trp Gln Thr Ile Asn Gly Lys Val Tyr Tyr Phe Met Pro Asp
545                 550                 555                 560

Thr Ala Met Ala Ala Ala Gly Gly Leu Phe Glu Ile Asp Gly Val Ile
                565                 570                 575

Tyr Phe Phe Gly Val Asp Gly Val Lys Ala Pro Gly Ile Tyr Gly Gly
            580                 585                 590

Gly Phe Val Ser Ile Asn Asp Asn Lys His Tyr Phe Asp Asp Ser Gly
        595                 600                 605

Val Met Lys Val Gly Tyr Thr Glu Ile Asp Gly Lys His Phe Tyr Phe
    610                 615                 620

Ala Glu Asn Gly Glu Met Gln Ile Gly Val Phe Asn Thr Glu Asp Gly
625                 630                 635                 640

Phe Lys Tyr Phe Ala His His Asn Glu Asp Leu Gly Asn Glu Glu Gly
                645                 650                 655

Glu Glu Ile Ser Tyr Ser Gly Ile Leu Asn Phe Asn Asn Lys Ile Tyr
            660                 665                 670

Tyr Phe Asp Asp Ser Phe Thr Ala Val Val Gly Trp Lys Asp Leu Glu
        675                 680                 685

Asp Gly Ser Lys Tyr Tyr Phe Asp Glu Asp Thr Ala Glu Ala Tyr Ile
    690                 695                 700

Gly Leu Ser Leu Ile Asn Asp Gly Gln Tyr Tyr Phe Asn Asp Asp Gly
705                 710                 715                 720

Ile Met Gln Val Gly Phe Val Thr Ile Asn Asp Lys Val Phe Tyr Phe
                725                 730                 735

Ser Asp Ser Gly Ile Ile Glu Ser Gly Val Gln Asn Ile Asp Asp Asn
            740                 745                 750

Tyr Phe Tyr Ile Asp Asp Asn Gly Ile Val Gln Ile Gly Val Phe Asp
        755                 760                 765

Thr Ser Asp Gly Tyr Lys Tyr Phe Ala Pro Ala Asn Thr Val Asn Asp
    770                 775                 780

Asn Ile Tyr Gly Gln Ala Val Glu Tyr Ser Gly Leu Val Arg Val Gly
785                 790                 795                 800

Glu Asp Val Tyr Tyr Phe Gly Glu Thr Tyr Thr Ile Glu Thr Gly Trp
                805                 810                 815

Ile Tyr Asp Met Glu Asn Glu Ser Asp Lys Tyr Tyr Phe Asn Pro Glu
            820                 825                 830

Thr Lys Lys Ala Cys Lys Gly Ile Asn Leu Ile Asp Asp Ile Lys Tyr
        835                 840                 845

Tyr Phe Asp Glu Lys Gly Ile Met Arg Thr Gly Leu Ile Ser Phe Glu
    850                 855                 860

Asn Asn Asn Tyr Tyr Phe Asn Glu Asn Gly Glu Met Gln Phe Gly Tyr
865                 870                 875                 880

Ile Asn Ile Glu Asp Lys Met Phe Tyr Phe Gly Glu Asp Gly Val Met
                885                 890                 895

Gln Ile Gly Val Phe Asn Thr Pro Asp Gly Phe Lys Tyr Phe Ala His
            900                 905                 910

Gln Asn Thr Leu Asp Glu Asn Phe Glu Gly Glu Ser Ile Asn Tyr Thr
        915                 920                 925

Gly Trp Leu Asp Leu Asp Glu Lys Arg Tyr Tyr Phe Thr Asp Glu Tyr
    930                 935                 940

Ile Ala Ala Thr Gly Ser Val Ile Ile Asp Gly Glu Glu Tyr Tyr Phe
945                 950                 955                 960

Asp Pro Asp Thr Ala Gln Leu Val Ile Ser Glu
                965                 970

<210>  36
<211>  427
<212>  PRT
<213>  Clostridium difficile

<400>  36

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Glu Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
            420                 425

<210>  37
<211>  330

<212>  PRT
<213>  Clostridium difficile

<400>  37

Met Ala Tyr Pro Ile Val Gly Val Gly Met Glu Lys Leu Ile Ile Ser
1               5                   10                  15

Thr Asn Glu His Ala Ser Thr Asp Gln Gly Lys Thr Val Ser Arg Ala
            20                  25                  30

Thr Thr Asn Ser Lys Thr Glu Ser Asn Thr Ala Gly Val Ser Val Asn
        35                  40                  45

Val Gly Tyr Gln Asn Gly Phe Thr Ala Asn Val Thr Thr Asn Tyr Ser
    50                  55                  60

His Thr Thr Asp Asn Ser Thr Ala Val Gln Asp Ser Asn Gly Glu Ser
65                  70                  75                  80

Trp Asn Thr Gly Leu Ser Ile Asn Lys Gly Glu Ser Ala Tyr Ile Asn
                85                  90                  95

Ala Asn Val Arg Tyr Tyr Asn Thr Gly Thr Ala Pro Met Tyr Lys Val
            100                 105                 110

Thr Pro Thr Thr Asn Leu Val Leu Asp Gly Asp Thr Leu Ser Thr Ile
        115                 120                 125

Lys Ala Gln Glu Asn Gln Ile Gly Asn Asn Leu Ser Pro Gly Asp Thr
    130                 135                 140

Tyr Pro Lys Lys Gly Leu Ser Pro Leu Ala Leu Asn Thr Met Asp Gln
145                 150                 155                 160

Phe Ser Ser Arg Leu Ile Pro Ile Asn Tyr Asp Gln Leu Lys Lys Leu
                165                 170                 175

Asp Ala Gly Lys Gln Ile Lys Leu Glu Thr Thr Gln Val Ser Gly Asn
            180                 185                 190

Phe Gly Thr Lys Asn Ser Ser Gly Gln Ile Val Thr Glu Gly Asn Ser
        195                 200                 205

Trp Ser Asp Tyr Ile Ser Gln Ile Asp Ser Ile Ser Ala Ser Ile Ile
    210                 215                 220

Leu Asp Thr Glu Asn Glu Ser Tyr Glu Arg Arg Val Thr Ala Lys Asn
225                 230                 235                 240

Leu Gln Asp Pro Glu Asp Lys Thr Pro Glu Leu Thr Ile Gly Glu Ala
                245                 250                 255

Ile Glu Lys Ala Phe Gly Ala Thr Lys Lys Asp Gly Leu Leu Tyr Phe
            260                 265                 270

Asn Asp Ile Pro Ile Asp Glu Ser Cys Val Glu Leu Ile Phe Asp Asp
        275                 280                 285

Asn Thr Ala Asn Lys Ile Lys Asp Ser Leu Lys Thr Leu Ser Asp Lys
    290                 295                 300

Lys Ile Tyr Asn Val Lys Leu Glu Arg Gly Met Asn Ile Leu Ile Lys
305                 310                 315                 320

Thr Pro Gly Gly His His His His His His
                325                 330

<210>  38
<211>  841
<212>  PRT
<213>  Clostridium difficile

<400>  38

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu
                165                 170                 175

Asp Leu Asp Thr Asp Asn Asp Asn Ile Pro Asp Ser Tyr Glu Arg Asn
            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Asn Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln

370                 375                 380

Glu Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Asp Gln Phe Ser Ser
                405                 410                 415

Arg Leu Ile Pro Ile Asn Tyr Asp Gln Leu Lys Lys Leu Asp Ala Gly
            420                 425                 430

Lys Gln Ile Lys Leu Glu Thr Thr Gln Val Ser Gly Asn Phe Gly Thr
        435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Lys Asn Leu Gln Asp
                485                 490                 495

Pro Glu Asp Lys Thr Pro Glu Leu Thr Ile Gly Glu Ala Ile Glu Lys
            500                 505                 510

Ala Phe Gly Ala Thr Lys Lys Asp Gly Leu Leu Tyr Phe Asn Asp Ile
        515                 520                 525

Pro Ile Asp Glu Ser Cys Val Glu Leu Ile Phe Asp Asp Asn Thr Ala
    530                 535                 540

Asn Lys Ile Lys Asp Ser Leu Lys Thr Leu Ser Asp Lys Lys Ile Tyr
545                 550                 555                 560

Asn Val Lys Leu Glu Arg Gly Met Asn Ile Leu Ile Lys Thr Pro Thr
                565                 570                 575

Tyr Phe Thr Asn Phe Asp Asp Tyr Asn Asn Tyr Pro Ser Thr Trp Ser
            580                 585                 590

Asn Val Asn Thr Thr Asn Gln Asp Gly Leu Gln Gly Ser Ala Asn Lys
        595                 600                 605

Leu Asn Gly Glu Thr Lys Ile Lys Ile Pro Met Ser Glu Leu Lys Pro
    610                 615                 620

Tyr Lys Arg Tyr Val Phe Ser Gly Tyr Ser Lys Asp Pro Leu Thr Ser
625                 630                 635                 640

Asn Ser Ile Ile Val Lys Ile Lys Ala Lys Glu Glu Lys Thr Asp Tyr
                645                 650                 655

Leu Val Pro Glu Gln Gly Tyr Thr Lys Phe Ser Tyr Glu Phe Glu Thr
            660                 665                 670

Thr Glu Lys Asp Ser Ser Asn Ile Glu Ile Thr Leu Ile Gly Ser Gly
        675                 680                 685

Thr Thr Tyr Leu Asp Asn Leu Ser Ile Thr Glu Leu Asn Ser Thr Pro
    690                 695                 700

Glu Ile Leu Asp Glu Pro Glu Val Lys Ile Pro Thr Asp Gln Glu Ile
705                 710                 715                 720

Met Asp Ala His Lys Ile Tyr Phe Ala Asp Leu Asn Phe Asn Pro Ser
                725                 730                 735

Thr Gly Asn Thr Tyr Ile Asn Gly Met Tyr Phe Ala Pro Thr Gln Thr
            740                 745                 750

Asn Lys Glu Ala Leu Asp Tyr Ile Gln Lys Tyr Arg Val Glu Ala Thr
        755                 760                 765

Leu Gln Tyr Ser Gly Phe Lys Asp Ile Gly Thr Lys Asp Lys Glu Met
    770                 775                 780

Arg Asn Tyr Leu Gly Asp Pro Asn Gln Pro Lys Thr Asn Tyr Val Asn
785                 790                 795                 800

Leu Arg Ser Tyr Phe Thr Gly Gly Glu Asn Ile Met Thr Tyr Lys Lys
                805                 810                 815

Leu Arg Ile Tyr Ala Ile Thr Pro Asp Asp Arg Glu Leu Leu Val Leu
            820                 825                 830

Ser Val Asp His His His His His His
        835                 840

<210>  39
<211>  427
<212>  PRT
<213>  Clostridium difficile

<400>  39

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
            420                 425

<210>  40
<211>  427
<212>  PRT
<213>  Clostridium difficile

<400>  40

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Glu Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
            420                 425

<210>  41
<211>  427
<212>  PRT
<213>  Clostridium difficile

<400>  41

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Ser Thr Ser Ile Gly Ser Val

340                 345                 350

Asn Met Ser Ala Phe Ala Lys Ala Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
            420                 425

<210>  42
<211>  427
<212>  PRT
<213>  Clostridium difficile

<400>  42

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe

130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Phe Thr Ser Ile Gly Ser Val
            340                 345                 350

Asn Met Ser Ala Phe Ala Lys Ala Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro His His His His His His
            420                 425

<210>  43
<211>  666
<212>  PRT
<213>  Clostridium difficile

<400>  43

Met Ser Asp Trp Glu Asp Glu Asp Leu Asp Thr Asp Asn Asp Asn Ile
1               5                   10                  15

Pro Asp Ser Tyr Glu Arg Asn Gly Tyr Thr Ile Lys Asp Leu Ile Ala
            20                  25                  30

Val Lys Trp Glu Asp Ser Phe Ala Glu Gln Gly Tyr Lys Lys Tyr Val
        35                  40                  45

Ser Asn Tyr Leu Glu Ser Asn Thr Ala Gly Asp Pro Tyr Thr Asp Tyr
    50                  55                  60

Glu Lys Ala Ser Gly Ser Phe Asp Lys Ala Ile Lys Thr Glu Ala Arg
65                  70                  75                  80

Asp Pro Leu Val Ala Ala Tyr Pro Ile Val Gly Val Gly Met Glu Lys
                85                  90                  95

Leu Ile Ile Ser Thr Asn Glu His Ala Ser Thr Asp Gln Gly Lys Thr
            100                 105                 110

Val Ser Arg Ala Thr Thr Asn Ser Lys Thr Glu Ser Asn Thr Ala Gly
        115                 120                 125

Val Ser Val Asn Val Gly Tyr Gln Asn Gly Phe Thr Ala Asn Val Thr
    130                 135                 140

Thr Asn Tyr Ser His Thr Thr Asp Asn Ser Thr Ala Val Gln Asp Ser
145                 150                 155                 160

Asn Gly Glu Ser Trp Asn Thr Gly Leu Ser Ile Asn Lys Gly Glu Ser
                165                 170                 175

Ala Tyr Ile Asn Ala Asn Val Arg Tyr Tyr Asn Thr Gly Thr Ala Pro
        180                 185                 190

Met Tyr Lys Val Thr Pro Thr Thr Asn Leu Val Leu Asp Gly Asp Thr
    195                 200                 205

Leu Ser Thr Ile Lys Ala Gln Glu Asn Gln Ile Gly Asn Asn Leu Ser
    210                 215                 220

Pro Gly Asp Thr Tyr Pro Lys Lys Gly Leu Ser Pro Leu Ala Leu Asn
225                 230                 235                 240

Thr Met Asp Gln Arg Ser Ser Arg Leu Ile Pro Ile Asn Tyr Asp Gln
                245                 250                 255

Leu Lys Lys Leu Asp Ala Gly Lys Gln Ile Lys Leu Glu Thr Thr Gln
            260                 265                 270

Val Ser Gly Asn Phe Gly Thr Lys Asn Ser Ser Gly Gln Ile Val Thr
        275                 280                 285

Glu Gly Asn Ser Trp Ser Asp Tyr Ile Ser Gln Ile Asp Ser Ile Ser
    290                 295                 300

Ala Ser Ile Ile Leu Asp Thr Glu Asn Glu Ser Tyr Glu Arg Arg Val
305                 310                 315                 320

Thr Ala Lys Asn Leu Gln Asp Pro Glu Asp Lys Thr Pro Glu Leu Thr
                325                 330                 335

Ile Gly Glu Ala Ile Glu Lys Ala Phe Gly Ala Thr Lys Lys Asp Gly
            340                 345                 350

Leu Leu Tyr Phe Asn Asp Ile Pro Ile Asp Glu Ser Cys Val Glu Leu
        355                 360                 365

Ile Phe Asp Asp Asn Thr Ala Asn Lys Ile Lys Asp Ser Leu Lys Thr
    370                 375                 380

Leu Ser Asp Lys Lys Ile Tyr Asn Val Lys Leu Glu Arg Gly Met Asn
385                 390                 395                 400

Ile Leu Ile Lys Thr Pro Thr Tyr Phe Thr Asn Phe Asp Asp Tyr Asn
                405                 410                 415

Asn Tyr Pro Ser Thr Trp Ser Asn Val Asn Thr Thr Asn Gln Asp Gly
            420                 425                 430

Leu Gln Gly Ser Ala Asn Lys Leu Asn Gly Glu Thr Lys Ile Lys Ile
        435                 440                 445

Pro Met Ser Glu Leu Lys Pro Tyr Lys Arg Tyr Val Phe Ser Gly Tyr
    450                 455                 460

Ser Lys Asp Pro Leu Thr Ser Asn Ser Ile Ile Val Lys Ile Lys Ala
465                 470                 475                 480

Lys Glu Glu Lys Thr Asp Tyr Leu Val Pro Glu Gln Gly Tyr Thr Lys
                485                 490                 495

Phe Ser Tyr Glu Phe Glu Thr Thr Glu Lys Asp Ser Ser Asn Ile Glu
            500                 505                 510

Ile Thr Leu Ile Gly Ser Gly Thr Thr Tyr Leu Asp Asn Leu Ser Ile
        515                 520                 525

Thr Glu Leu Asn Ser Thr Pro Glu Ile Leu Asp Glu Pro Glu Val Lys
    530                 535                 540

Ile Pro Thr Asp Gln Glu Ile Met Asp Ala His Lys Ile Tyr Phe Ala
545                 550                 555                 560

Asp Leu Asn Phe Asn Pro Ser Thr Gly Asn Thr Tyr Ile Asn Gly Met
                565                 570                 575

Tyr Phe Ala Pro Thr Gln Thr Asn Lys Glu Ala Leu Asp Tyr Ile Gln
            580                 585                 590

Lys Tyr Arg Val Glu Ala Thr Leu Gln Tyr Ser Gly Phe Lys Asp Ile
        595                 600                 605

Gly Thr Lys Asp Lys Glu Met Arg Asn Tyr Leu Gly Asp Pro Asn Gln
    610                 615                 620

Pro Lys Thr Asn Tyr Val Asn Leu Arg Ser Tyr Phe Thr Gly Gly Glu
625                 630                 635                 640

Asn Ile Met Thr Tyr Lys Lys Leu Arg Ile Tyr Ala Ile Thr Pro Asp
                645                 650                 655

Asp Arg Glu Leu Leu Val Leu Ser Val Asp
            660                 665

<210>  44
<211>  666

<212>  PRT
<213>  Clostridium difficile

<400>  44

Met Ser Asp Trp Glu Asp Glu Asp Leu Asp Thr Asp Asn Asp Asn Ile
1               5                   10                  15

Pro Asp Ser Tyr Glu Arg Asn Gly Tyr Thr Ile Lys Asp Leu Ile Ala
            20                  25                  30

Val Lys Trp Glu Asp Ser Phe Ala Glu Gln Gly Tyr Lys Lys Tyr Val
        35                  40                  45

Ser Asn Tyr Leu Glu Ser Asn Thr Ala Gly Asp Pro Tyr Thr Asp Tyr
    50                  55                  60

Glu Lys Ala Ser Gly Ser Phe Asp Lys Ala Ile Lys Thr Glu Ala Arg
65                  70                  75                  80

Asp Pro Leu Val Ala Ala Tyr Pro Ile Val Gly Val Gly Met Glu Lys
                85                  90                  95

Leu Ile Ile Ser Thr Asn Glu His Ala Ser Thr Asp Gln Gly Lys Thr
            100                 105                 110

Val Ser Arg Ala Thr Thr Asn Ser Lys Thr Glu Ser Asn Thr Ala Gly
        115                 120                 125

Val Ser Val Asn Val Gly Tyr Gln Asn Gly Phe Thr Ala Asn Val Thr
    130                 135                 140

Thr Asn Tyr Ser His Thr Thr Asp Asn Ser Thr Ala Val Gln Asp Ser
145                 150                 155                 160

Asn Gly Glu Ser Trp Asn Thr Gly Leu Ser Ile Asn Lys Gly Glu Ser
                165                 170                 175

Ala Tyr Ile Asn Ala Asn Val Arg Tyr Tyr Asn Thr Gly Thr Ala Pro
            180                 185                 190

Met Tyr Lys Val Thr Pro Thr Thr Asn Leu Val Leu Asp Gly Asp Thr
        195                 200                 205

Leu Ser Thr Ile Lys Ala Gln Ala Asn Gln Ile Gly Asn Asn Leu Ser
    210                 215                 220

Pro Gly Asp Thr Tyr Pro Lys Lys Gly Leu Ser Pro Leu Ala Leu Asn
225                 230                 235                 240

Thr Met Ala Gln Phe Ser Ser Arg Leu Ile Pro Ile Asn Tyr Asp Gln
                245                 250                 255

Leu Lys Lys Leu Asp Ala Gly Lys Gln Ile Lys Leu Glu Thr Thr Gln
            260                 265                 270

Val Ser Gly Asn Phe Gly Thr Lys Asn Ser Ser Gly Gln Ile Val Thr
        275                 280                 285

Glu Gly Asn Ser Trp Ser Asp Tyr Ile Ser Gln Ile Asp Ser Ile Ser
    290                 295                 300

Ala Ser Ile Ile Leu Asp Thr Glu Asn Glu Ser Tyr Glu Arg Arg Val
305                 310                 315                 320

Thr Ala Lys Asn Leu Gln Asp Pro Glu Asp Lys Thr Pro Glu Leu Thr
                325                 330                 335

Ile Gly Glu Ala Ile Glu Lys Ala Phe Gly Ala Thr Lys Lys Asp Gly
            340                 345                 350

Leu Leu Tyr Phe Asn Asp Ile Pro Ile Asp Glu Ser Cys Val Glu Leu
        355                 360                 365

Ile Phe Asp Asp Asn Thr Ala Asn Lys Ile Lys Asp Ser Leu Lys Thr
    370                 375                 380

Leu Ser Asp Lys Lys Ile Tyr Asn Val Lys Leu Glu Arg Gly Met Asn
385                 390                 395                 400

Ile Leu Ile Lys Thr Pro Thr Tyr Phe Thr Asn Phe Asp Asp Tyr Asn
                405                 410                 415

Asn Tyr Pro Ser Thr Trp Ser Asn Val Asn Thr Thr Asn Gln Asp Gly
            420                 425                 430

Leu Gln Gly Ser Ala Asn Lys Leu Asn Gly Glu Thr Lys Ile Lys Ile
        435                 440                 445

Pro Met Ser Glu Leu Lys Pro Tyr Lys Arg Tyr Val Phe Ser Gly Tyr
    450                 455                 460

Ser Lys Asp Pro Leu Thr Ser Asn Ser Ile Ile Val Lys Ile Lys Ala
465                 470                 475                 480

Lys Glu Glu Lys Thr Asp Tyr Leu Val Pro Glu Gln Gly Tyr Thr Lys

485                 490                 495

Phe Ser Tyr Glu Phe Glu Thr Thr Glu Lys Asp Ser Ser Asn Ile Glu
            500                 505                 510

Ile Thr Leu Ile Gly Ser Gly Thr Thr Tyr Leu Asp Asn Leu Ser Ile
        515                 520                 525

Thr Glu Leu Asn Ser Thr Pro Glu Ile Leu Asp Glu Pro Glu Val Lys
    530                 535                 540

Ile Pro Thr Asp Gln Glu Ile Met Asp Ala His Lys Ile Tyr Phe Ala
545                 550                 555                 560

Asp Leu Asn Phe Asn Pro Ser Thr Gly Asn Thr Tyr Ile Asn Gly Met
                565                 570                 575

Tyr Phe Ala Pro Thr Gln Thr Asn Lys Glu Ala Leu Asp Tyr Ile Gln
            580                 585                 590

Lys Tyr Arg Val Glu Ala Thr Leu Gln Tyr Ser Gly Phe Lys Asp Ile
        595                 600                 605

Gly Thr Lys Asp Lys Glu Met Arg Asn Tyr Leu Gly Asp Pro Asn Gln
    610                 615                 620

Pro Lys Thr Asn Tyr Val Asn Leu Arg Ser Tyr Phe Thr Gly Gly Glu
625                 630                 635                 640

Asn Ile Met Thr Tyr Lys Lys Leu Arg Ile Tyr Ala Ile Thr Pro Asp
                645                 650                 655

Asp Arg Glu Leu Leu Val Leu Ser Val Asp
            660                 665

<210>  45
<211>  2000
<212>  DNA
<213>  Clostridium difficile

<400>  45
atgagcgatt gggaagatga agatctggat accgataatg ataatattcc ggatagctat      60

gaacgcaatg gctataccat taaagatctg attgccgtga aatgggaaga tagctttgca     120

gaacagggct ataagaaata tgtgagcaat tatctggaaa gcaataccgc aggcgatccg     180

tataccgatt atgaaaaagc aagcggcagc tttgataaag ccattaaaac cgaagcacgt     240

gatccgctgg ttgcagcata tccgattgtt ggtgttggta tggaaaaact gattattagc     300

accaatgaac atgcaagcac cgatcagggt aaaaccgtta gccgtgcaac caccaatagc 360

aaaaccgaaa gcaatacagc cggtgttagc gttaatgttg gttatcagaa tggttttacc 420

gccaatgtga ccaccaatta tagccatacc accgataata gcaccgcagt tcaggatagc 480

aatggtgaaa gctggaatac cggtctgagc attaacaaag gtgaaagcgc atatatcaat 540

gccaatgtgc gctattataa caccggcacc gcaccgatgt ataaagttac cccgaccacc 600

aatctggttc tggatggtga taccctgagt accattaaag cacaagaaaa tcagattggc 660

aataatctga gtccgggtga tacctatccg aaaaaaggtc tgagtccgct ggcactgaat 720

accatggatc agcgcagcag ccgtctgatt ccgattaact atgatcagct gaaaaaactg 780

gatgccggta aacaaatcaa actggaaacc acccaggtta gcggtaattt tggcaccaaa 840

aattcaagcg gtcagattgt taccgaaggt aatagctggt cagattatat cagccagatt 900

gatagcatta gcgccagcat tattctggat acagaaaatg aaagctatga acgtcgtgtg 960

accgcaaaaa atctgcagga cccggaagat aaaacaccgg aactgaccat tggtgaagca 1020

attgaaaaag catttggtgc caccaaaaaa gatggcctgc tgtattttaa cgatattccg 1080

attgatgaaa gctgcgtgga actgattttt gatgataata ccgccaataa aatcaaagat 1140

agcctgaaaa ccctgagcga caaaaaaatc tataatgtga aattagaacg cggtatgaat 1200

attctaatta aaaccccgac ctattttacc aattttgatg attataacaa ttatccgagc 1260

acttggagca atgtgaatac caccaatcag gatggtctgc agggtagcgc aaataaactg 1320

aatggtgaaa ccaaaatcaa aattccgatg agcgaactga aaccgtataa acgttatgtg 1380

tttagcggct atagcaaaga tccgctgacc agcaatagca ttattgtgaa aatcaaagcc 1440

aaagaagaaa aaaccgatta tctggttccg gaacagggtt ataccaaatt tagctatgaa 1500

tttgaaacca ccgaaaaaga tagcagtaat attgaaatta ccctgattgg tagcggcacc 1560

acctatctgg ataatctgag tattaccgaa ctgaatagca caccggaaat tctggatgaa 1620

ccggaagtga aaattccgac cgatcaagaa attatggatg cccataaaat ctattttgcc 1680

gatctgaact ttaatccgag caccggcaat acctatatta acggcatgta ttttgcaccg 1740

acccagacca ataaagaagc cctggattat attcagaaat atcgtgttga agccaccctg 1800

cagtatagcg gttttaaaga tattggcacc aaagataaag aaatgcgtaa ttatctgggc 1860

gatccgaatc agccgaaaac caattatgtt aatctgcgca gctattttac cggtggcgaa 1920

aacattatga cctacaaaaa actgcgcatt tatgccatta caccggatga tcgtgaactg 1980

ctggttctga gcgttgatca 2000

<210> 46
<211> 2001
<212> DNA
<213> Clostridium difficile

<400> 46
atgagcgatt gggaagatga agatctggat accgataatg ataatattcc ggatagctat 60
gaacgcaatg gctataccat taaagatctg attgccgtga aatgggaaga tagctttgca 120
gaacagggct ataagaaata tgtgagcaat tatctggaaa gcaataccgc aggcgatccg 180
tataccgatt atgaaaaagc aagcggcagc tttgataaag ccattaaaac cgaagcacgt 240
gatccgctgg ttgcagcata tccgattgtt ggtgttggta tggaaaaact gattattagc 300
accaatgaac atgcaagcac cgatcagggt aaaaccgtta gccgtgcaac caccaatagc 360
aaaaccgaaa gcaatacagc cggtgttagc gttaatgttg gttatcagaa tggttttacc 420
gccaatgtga ccaccaatta tagccatacc accgataata gcaccgcagt tcaggatagc 480
aatggtgaaa gctggaatac cggtctgagc attaacaaag gtgaaagcgc atatatcaat 540
gccaatgtgc gctattataa caccggcacc gcaccgatgt ataaagttac cccgaccacc 600
aatctggttc tggatggtga taccctgagt accattaaag cacaagcraa tcagattggc 660
aataatctga gtccgggtga tacctatccg aaaaaaggtc tgagtccgct ggcactgaat 720
accatggcgc agtttagcag ccgtctgatt ccgattaact atgatcagct gaaaaaactg 780
gatgccggta aacaaatcaa actggaaacc acccaggtta gcggtaattt tggcaccaaa 840
aattcaagcg gtcagattgt taccgaaggt aatagctggt cagattatat cagccagatt 900
gatagcatta gcgccagcat tattctggat acagaaaatg aaagctatga acgtcgtgtg 960
accgcaaaaa atctgcagga cccggaagat aaaacaccgg aactgaccat tggtgaagca 1020
attgaaaaag catttggtgc caccaaaaaa gatggcctgc tgtattttaa cgatattccg 1080
attgatgaaa gctgcgtgga actgattttt gatgataata ccgccaataa aatcaaagat 1140
agcctgaaaa ccctgagcga caaaaaaatc tataatgtga aattagaacg cggtatgaat 1200
attctaatta aaaccccgac ctattttacc aattttgatg attataacaa ttatccgagc 1260
acttggagca atgtgaatac caccaatcag gatggtctgc agggtagcgc aaataaactg 1320
aatggtgaaa ccaaaatcaa aattccgatg agcgaactga aaccgtataa acgttatgtg 1380
tttagcggct atagcaaaga tccgctgacc agcaatagca ttattgtgaa aatcaaagcc 1440
aaagaagaaa aaaccgatta tctggttccg gaacagggtt ataccaaatt tagctatgaa 1500
tttgaaacca ccgaaaaaga tagcagtaat attgaaatta ccctgattgg tagcggcacc 1560
acctatctgg ataatctgag tattaccgaa ctgaatagca caccggaaat tctggatgaa 1620
ccggaagtga aaattccgac cgatcaagaa attatggatg cccataaaat ctattttgcc 1680
gatctgaact ttaatccgag caccggcaat acctatatta acggcatgta ttttgcaccg 1740
acccagacca ataaagaagc cctggattat attcagaaat atcgtgttga agccaccctg 1800
cagtatagcg gttttaaaga tattggcacc aaagataaag aaatgcgtaa ttatctgggc 1860

gatccgaatc agccgaaaac caattatgtt aatctgcgca gctattttac cggtggcgaa     1920

aacattatga cctacaaaaa actgcgcatt tatgccatta caccggatga tcgtgaactg     1980

ctggttctga gcgttgatca c                                               2001

<210>  47
<211>  1067
<212>  PRT
<213>  Clostridium difficile

<400>  47

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205
Thr Phe Gly Gly Gly Asp His Pro Pro Lys Ser Asp Leu Glu Val Leu
    210                 215                 220
Phe Gln Gly Pro Leu Gly Ser His Met Glu Ile Val Asn Glu Asp Ile
225                 230                 235                 240
Leu Pro Asn Asn Gly Leu Met Gly Tyr Tyr Phe Thr Asp Glu His Phe
                245                 250                 255
Lys Asp Leu Lys Leu Met Ala Pro Ile Lys Asp Gly Asn Leu Lys Phe
            260                 265                 270
Glu Glu Lys Lys Val Asp Lys Leu Leu Asp Lys Asp Lys Ser Asp Val
        275                 280                 285
Lys Ser Ile Arg Trp Thr Gly Arg Ile Ile Pro Ser Lys Asp Gly Glu
    290                 295                 300
Tyr Thr Leu Ser Thr Asp Arg Asp Asp Val Leu Met Gln Val Asn Thr
305                 310                 315                 320
Glu Ser Thr Ile Ser Asn Thr Leu Lys Val Asn Met Lys Lys Gly Lys
                325                 330                 335
Glu Tyr Lys Val Arg Ile Glu Leu Gln Asp Lys Asn Leu Gly Ser Ile
            340                 345                 350
Asp Asn Leu Ser Ser Pro Asn Leu Tyr Trp Glu Leu Asp Gly Met Lys
        355                 360                 365
Lys Ile Ile Pro Glu Glu Asn Leu Phe Leu Arg Asp Tyr Ser Asn Ile
    370                 375                 380
Glu Lys Asp Asp Pro Phe Ile Pro Asn Asn Asn Phe Phe Asp Pro Lys
385                 390                 395                 400
Leu Met Ser Asp Trp Glu Asp Glu Asp Leu Asp Thr Asp Asn Asp Asn
                405                 410                 415
Ile Pro Asp Ser Tyr Glu Arg Asn Gly Tyr Thr Ile Lys Asp Leu Ile
            420                 425                 430
Ala Val Lys Trp Glu Asp Ser Phe Ala Glu Gln Gly Tyr Lys Lys Tyr
        435                 440                 445

Val Ser Asn Tyr Leu Glu Ser Asn Thr Ala Gly Asp Pro Tyr Thr Asp
    450                 455                 460

Tyr Glu Lys Ala Ser Gly Ser Phe Asp Lys Ala Ile Lys Thr Glu Ala
465                 470                 475                 480

Arg Asp Pro Leu Val Ala Ala Tyr Pro Ile Val Gly Val Gly Met Glu
                485                 490                 495

Lys Leu Ile Ile Ser Thr Asn Glu His Ala Ser Thr Asp Gln Gly Lys
            500                 505                 510

Thr Val Ser Arg Ala Thr Thr Asn Ser Lys Thr Glu Ser Asn Thr Ala
        515                 520                 525

Gly Val Ser Val Asn Val Gly Tyr Gln Asn Gly Phe Thr Ala Asn Val
    530                 535                 540

Thr Thr Asn Tyr Ser His Thr Thr Asp Asn Ser Thr Ala Val Gln Asp
545                 550                 555                 560

Ser Asn Gly Glu Ser Trp Asn Thr Gly Leu Ser Ile Asn Lys Gly Glu
                565                 570                 575

Ser Ala Tyr Ile Asn Ala Asn Val Arg Tyr Tyr Asn Thr Gly Thr Ala
            580                 585                 590

Pro Met Tyr Lys Val Thr Pro Thr Thr Asn Leu Val Leu Asp Gly Asp
        595                 600                 605

Thr Leu Ser Thr Ile Lys Ala Gln Glu Asn Gln Ile Gly Asn Asn Leu
    610                 615                 620

Ser Pro Gly Asp Thr Tyr Pro Lys Lys Gly Leu Ser Pro Leu Ala Leu
625                 630                 635                 640

Asn Thr Met Asp Gln Phe Ser Ser Arg Leu Ile Pro Ile Asn Tyr Asp
                645                 650                 655

Gln Leu Lys Lys Leu Asp Ala Gly Lys Gln Ile Lys Leu Glu Thr Thr
            660                 665                 670

Gln Val Ser Gly Asn Phe Gly Thr Lys Asn Ser Ser Gly Gln Ile Val
        675                 680                 685

Thr Glu Gly Asn Ser Trp Ser Asp Tyr Ile Ser Gln Ile Asp Ser Ile
    690                 695                 700

Ser Ala Ser Ile Ile Leu Asp Thr Glu Asn Glu Ser Tyr Glu Arg Arg
705                 710                 715                 720

Val Thr Ala Lys Asn Leu Gln Asp Pro Glu Asp Lys Thr Pro Glu Leu
                725                 730                 735

Thr Ile Gly Glu Ala Ile Glu Lys Ala Phe Gly Ala Thr Lys Lys Asp
            740                 745                 750

Gly Leu Leu Tyr Phe Asn Asp Ile Pro Ile Asp Glu Ser Cys Val Glu
        755                 760                 765

Leu Ile Phe Asp Asp Asn Thr Ala Asn Lys Ile Lys Asp Ser Leu Lys
    770                 775                 780

Thr Leu Ser Asp Lys Lys Ile Tyr Asn Val Lys Leu Glu Arg Gly Met
785                 790                 795                 800

Asn Ile Leu Ile Lys Thr Pro Thr Tyr Phe Thr Asn Phe Asp Asp Tyr
                805                 810                 815

Asn Asn Tyr Pro Ser Thr Trp Ser Asn Val Asn Thr Thr Asn Gln Asp
            820                 825                 830

Gly Leu Gln Gly Ser Ala Asn Lys Leu Asn Gly Glu Thr Lys Ile Lys
        835                 840                 845

Ile Pro Met Ser Glu Leu Lys Pro Tyr Lys Arg Tyr Val Phe Ser Gly
    850                 855                 860

Tyr Ser Lys Asp Pro Leu Thr Ser Asn Ser Ile Ile Val Lys Ile Lys
865                 870                 875                 880

Ala Lys Glu Glu Lys Thr Asp Tyr Leu Val Pro Glu Gln Gly Tyr Thr
                885                 890                 895

Lys Phe Ser Tyr Glu Phe Glu Thr Thr Glu Lys Asp Ser Ser Asn Ile
            900                 905                 910

Glu Ile Thr Leu Ile Gly Ser Gly Thr Thr Tyr Leu Asp Asn Leu Ser
        915                 920                 925

Ile Thr Glu Leu Asn Ser Thr Pro Glu Ile Leu Asp Glu Pro Glu Val
    930                 935                 940

Lys Ile Pro Thr Asp Gln Glu Ile Met Asp Ala His Lys Ile Tyr Phe

945                 950                 955                 960

Ala Asp Leu Asn Phe Asn Pro Ser Thr Gly Asn Thr Tyr Ile Asn Gly
                965                 970                 975

Met Tyr Phe Ala Pro Thr Gln Thr Asn Lys Glu Ala Leu Asp Tyr Ile
            980                 985                 990

Gln Lys Tyr Arg Val Glu Ala Thr  Leu Gln Tyr Ser Gly  Phe Lys Asp
        995                 1000                1005

Ile Gly  Thr Lys Asp Lys Glu  Met Arg Asn Tyr Leu  Gly Asp Pro
    1010                1015                1020

Asn Gln  Pro Lys Thr Asn Tyr  Val Asn Leu Arg Ser  Tyr Phe Thr
    1025                1030                1035

Gly Gly  Glu Asn Ile Met Thr  Tyr Lys Lys Leu Arg  Ile Tyr Ala
    1040                1045                1050

Ile Thr  Pro Asp Asp Arg Glu  Leu Leu Val Leu Ser  Val Asp
    1055                1060                1065

<210>  48
<211>  666
<212>  PRT
<213>  Clostridium difficile

<400>  48

Met Ser Asp Trp Glu Asp Glu Asp Leu Asp Thr Asp Asn Asp Asn Ile
1               5                   10                  15

Pro Asp Ser Tyr Glu Arg Asn Gly Tyr Thr Ile Lys Asp Leu Ile Ala
            20                  25                  30

Val Lys Trp Glu Asp Ser Phe Ala Glu Gln Gly Tyr Lys Lys Tyr Val
        35                  40                  45

Ser Asn Tyr Leu Glu Ser Asn Thr Ala Gly Asp Pro Tyr Thr Asp Tyr
    50                  55                  60

Glu Lys Ala Ser Gly Ser Phe Asp Lys Ala Ile Lys Thr Glu Ala Arg
65                  70                  75                  80

Asp Pro Leu Val Ala Ala Tyr Pro Ile Val Gly Val Gly Met Glu Lys
                85                  90                  95

Leu Ile Ile Ser Thr Asn Glu His Ala Ser Thr Asp Gln Gly Lys Thr

            100                 105                 110

Val Ser Arg Ala Thr Thr Asn Ser Lys Thr Glu Ser Asn Thr Ala Gly
        115                 120                 125

Val Ser Val Asn Val Gly Tyr Gln Asn Gly Phe Thr Ala Asn Val Thr
    130                 135                 140

Thr Asn Tyr Ser His Thr Thr Asp Asn Ser Thr Ala Val Gln Asp Ser
145                 150                 155                 160

Asn Gly Glu Ser Trp Asn Thr Gly Leu Ser Ile Asn Lys Gly Glu Ser
                165                 170                 175

Ala Tyr Ile Asn Ala Asn Val Arg Tyr Tyr Asn Thr Gly Thr Ala Pro
            180                 185                 190

Met Tyr Lys Val Thr Pro Thr Thr Asn Leu Val Leu Asp Gly Asp Thr
        195                 200                 205

Leu Ser Thr Ile Lys Ala Gln Glu Asn Gln Ile Gly Asn Asn Leu Ser
    210                 215                 220

Pro Gly Asp Thr Tyr Pro Lys Lys Gly Leu Ser Pro Leu Ala Leu Asn
225                 230                 235                 240

Thr Met Asp Gln Phe Ser Ser Arg Leu Ile Pro Ile Asn Tyr Asp Gln
                245                 250                 255

Leu Lys Lys Leu Asp Ala Gly Lys Gln Ile Lys Leu Glu Thr Thr Gln
            260                 265                 270

Val Ser Gly Asn Phe Gly Thr Lys Asn Ser Ser Gly Gln Ile Val Thr
        275                 280                 285

Glu Gly Asn Ser Trp Ser Asp Tyr Ile Ser Gln Ile Asp Ser Ile Ser
    290                 295                 300

Ala Ser Ile Ile Leu Asp Thr Glu Asn Glu Ser Tyr Glu Arg Arg Val
305                 310                 315                 320

Thr Ala Lys Asn Leu Gln Asp Pro Glu Asp Lys Thr Pro Glu Leu Thr
                325                 330                 335

Ile Gly Glu Ala Ile Glu Lys Ala Phe Gly Ala Thr Lys Lys Asp Gly
            340                 345                 350

Leu Leu Tyr Phe Asn Asp Ile Pro Ile Asp Glu Ser Cys Val Glu Leu
        355                 360                 365

Ile Phe Asp Asp Asn Thr Ala Asn Lys Ile Lys Asp Ser Leu Lys Thr
    370                 375                 380

Leu Ser Asp Lys Lys Ile Tyr Asn Val Lys Leu Glu Arg Gly Met Asn
385                 390                 395                 400

Ile Leu Ile Lys Thr Pro Thr Tyr Phe Thr Asn Phe Asp Asp Tyr Asn
                405                 410                 415

Asn Tyr Pro Ser Thr Trp Ser Asn Val Asn Thr Thr Asn Gln Asp Gly
            420                 425                 430

Leu Gln Gly Ser Ala Asn Lys Leu Asn Gly Glu Thr Lys Ile Lys Ile
        435                 440                 445

Pro Met Ser Glu Leu Lys Pro Tyr Lys Arg Tyr Val Phe Ser Gly Tyr
    450                 455                 460

Ser Lys Asp Pro Leu Thr Ser Asn Ser Ile Ile Val Lys Ile Lys Ala
465                 470                 475                 480

Lys Glu Glu Lys Thr Asp Tyr Leu Val Pro Glu Gln Gly Tyr Thr Lys
                485                 490                 495

Phe Ser Tyr Glu Phe Glu Thr Thr Glu Lys Asp Ser Ser Asn Ile Glu
            500                 505                 510

Ile Thr Leu Ile Gly Ser Gly Thr Thr Tyr Leu Asp Asn Leu Ser Ile
        515                 520                 525

Thr Glu Leu Asn Ser Thr Pro Glu Ile Leu Asp Glu Pro Glu Val Lys
    530                 535                 540

Ile Pro Thr Asp Gln Glu Ile Met Asp Ala His Lys Ile Tyr Phe Ala
545                 550                 555                 560

Asp Leu Asn Phe Asn Pro Ser Thr Gly Asn Thr Tyr Ile Asn Gly Met
                565                 570                 575

Tyr Phe Ala Pro Thr Gln Thr Asn Lys Glu Ala Leu Asp Tyr Ile Gln
            580                 585                 590

Lys Tyr Arg Val Glu Ala Thr Leu Gln Tyr Ser Gly Phe Lys Asp Ile
        595                 600                 605

Gly Thr Lys Asp Lys Glu Met Arg Asn Tyr Leu Gly Asp Pro Asn Gln
    610                 615                 620

Pro Lys Thr Asn Tyr Val Asn Leu Arg Ser Tyr Phe Thr Gly Gly Glu
625                 630                 635                 640

Asn Ile Met Thr Tyr Lys Lys Leu Arg Ile Tyr Ala Ile Thr Pro Asp
                645                 650                 655

Asp Arg Glu Leu Leu Val Leu Ser Val Asp
            660                 665

<210>  49
<211>  408
<212>  PRT
<213>  Clostridium difficile

<400>  49

Met Ser Asp Trp Glu Asp Glu Asp Leu Asp Thr Asp Asn Asp Asn Ile
1               5                   10                  15

Pro Asp Ser Tyr Glu Arg Asn Gly Tyr Thr Ile Lys Asp Leu Ile Ala
            20                  25                  30

Val Lys Trp Glu Asp Ser Phe Ala Glu Gln Gly Tyr Lys Lys Tyr Val
        35                  40                  45

Ser Asn Tyr Leu Glu Ser Asn Thr Ala Gly Asp Pro Tyr Thr Asp Tyr
    50                  55                  60

Glu Lys Ala Ser Gly Ser Phe Asp Lys Ala Ile Lys Thr Glu Ala Arg
65                  70                  75                  80

Asp Pro Leu Val Ala Ala Tyr Pro Ile Val Gly Val Gly Met Glu Lys
                85                  90                  95

Leu Ile Ile Ser Thr Asn Glu His Ala Ser Thr Asp Gln Gly Lys Thr
            100                 105                 110

Val Ser Arg Ala Thr Thr Asn Ser Lys Thr Glu Ser Asn Thr Ala Gly
        115                 120                 125

Val Ser Val Asn Val Gly Tyr Gln Asn Gly Phe Thr Ala Asn Val Thr
    130                 135                 140

Thr Asn Tyr Ser His Thr Thr Asp Asn Ser Thr Ala Val Gln Asp Ser
145                 150                 155                 160

Asn Gly Glu Ser Trp Asn Thr Gly Leu Ser Ile Asn Lys Gly Glu Ser
                165                 170                 175

Ala Tyr Ile Asn Ala Asn Val Arg Tyr Tyr Asn Thr Gly Thr Ala Pro
        180                 185                 190

Met Tyr Lys Val Thr Pro Thr Thr Asn Leu Val Leu Asp Gly Asp Thr
    195                 200                 205

Leu Ser Thr Ile Lys Ala Gln Glu Asn Gln Ile Gly Asn Asn Leu Ser
    210                 215                 220

Pro Gly Asp Thr Tyr Pro Lys Lys Gly Leu Ser Pro Leu Ala Leu Asn
225                 230                 235                 240

Thr Met Asp Gln Phe Ser Ser Arg Leu Ile Pro Ile Asn Tyr Asp Gln
                245                 250                 255

Leu Lys Lys Leu Asp Ala Gly Lys Gln Ile Lys Leu Glu Thr Thr Gln
            260                 265                 270

Val Ser Gly Asn Phe Gly Thr Lys Asn Ser Ser Gly Gln Ile Val Thr
        275                 280                 285

Glu Gly Asn Ser Trp Ser Asp Tyr Ile Ser Gln Ile Asp Ser Ile Ser
    290                 295                 300

Ala Ser Ile Ile Leu Asp Thr Glu Asn Glu Ser Tyr Glu Arg Arg Val
305                 310                 315                 320

Thr Ala Lys Asn Leu Gln Asp Pro Glu Asp Lys Thr Pro Glu Leu Thr
                325                 330                 335

Ile Gly Glu Ala Ile Glu Lys Ala Phe Gly Ala Thr Lys Lys Asp Gly
            340                 345                 350

Leu Leu Tyr Phe Asn Asp Ile Pro Ile Asp Glu Ser Cys Val Glu Leu
        355                 360                 365

Ile Phe Asp Asp Asn Thr Ala Asn Lys Ile Lys Asp Ser Leu Lys Thr
    370                 375                 380

Leu Ser Asp Lys Lys Ile Tyr Asn Val Lys Leu Glu Arg Gly Met Asn
385                 390                 395                 400

Ile Leu Ile Lys Thr Pro Gly Gly
                405

<210>  50
<211>  582
<212>  PRT
<213>  Clostridium difficile

<400>  50

Met Ala Tyr Pro Ile Val Gly Val Gly Met Glu Lys Leu Ile Ile Ser
1               5                   10                  15

Thr Asn Glu His Ala Ser Thr Asp Gln Gly Lys Thr Val Ser Arg Ala
            20                  25                  30

Thr Thr Asn Ser Lys Thr Glu Ser Asn Thr Ala Gly Val Ser Val Asn
        35                  40                  45

Val Gly Tyr Gln Asn Gly Phe Thr Ala Asn Val Thr Thr Asn Tyr Ser
    50                  55                  60

His Thr Thr Asp Asn Ser Thr Ala Val Gln Asp Ser Asn Gly Glu Ser
65                  70                  75                  80

Trp Asn Thr Gly Leu Ser Ile Asn Lys Gly Glu Ser Ala Tyr Ile Asn
                85                  90                  95

Ala Asn Val Arg Tyr Tyr Asn Thr Gly Thr Ala Pro Met Tyr Lys Val
            100                 105                 110

Thr Pro Thr Thr Asn Leu Val Leu Asp Gly Asp Thr Leu Ser Thr Ile
        115                 120                 125

Lys Ala Gln Glu Asn Gln Ile Gly Asn Asn Leu Ser Pro Gly Asp Thr
    130                 135                 140

Tyr Pro Lys Lys Gly Leu Ser Pro Leu Ala Leu Asn Thr Met Asp Gln
145                 150                 155                 160

Phe Ser Ser Arg Leu Ile Pro Ile Asn Tyr Asp Gln Leu Lys Lys Leu
                165                 170                 175

Asp Ala Gly Lys Gln Ile Lys Leu Glu Thr Thr Gln Val Ser Gly Asn
            180                 185                 190

Phe Gly Thr Lys Asn Ser Ser Gly Gln Ile Val Thr Glu Gly Asn Ser
        195                 200                 205

Trp Ser Asp Tyr Ile Ser Gln Ile Asp Ser Ile Ser Ala Ser Ile Ile
    210                 215                 220

Leu Asp Thr Glu Asn Glu Ser Tyr Glu Arg Arg Val Thr Ala Lys Asn
225                 230                 235                 240

Leu Gln Asp Pro Glu Asp Lys Thr Pro Glu Leu Thr Ile Gly Glu Ala
                245                 250                 255

Ile Glu Lys Ala Phe Gly Ala Thr Lys Lys Asp Gly Leu Leu Tyr Phe
            260                 265                 270

Asn Asp Ile Pro Ile Asp Glu Ser Cys Val Glu Leu Ile Phe Asp Asp
        275                 280                 285

Asn Thr Ala Asn Lys Ile Lys Asp Ser Leu Lys Thr Leu Ser Asp Lys
    290                 295                 300

Lys Ile Tyr Asn Val Lys Leu Glu Arg Gly Met Asn Ile Leu Ile Lys
305                 310                 315                 320

Thr Pro Thr Tyr Phe Thr Asn Phe Asp Asp Tyr Asn Asn Tyr Pro Ser
                325                 330                 335

Thr Trp Ser Asn Val Asn Thr Thr Asn Gln Asp Gly Leu Gln Gly Ser
            340                 345                 350

Ala Asn Lys Leu Asn Gly Glu Thr Lys Ile Lys Ile Pro Met Ser Glu
        355                 360                 365

Leu Lys Pro Tyr Lys Arg Tyr Val Phe Ser Gly Tyr Ser Lys Asp Pro
    370                 375                 380

Leu Thr Ser Asn Ser Ile Ile Val Lys Ile Lys Ala Lys Glu Glu Lys
385                 390                 395                 400

Thr Asp Tyr Leu Val Pro Glu Gln Gly Tyr Thr Lys Phe Ser Tyr Glu
                405                 410                 415

Phe Glu Thr Thr Glu Lys Asp Ser Ser Asn Ile Glu Ile Thr Leu Ile
            420                 425                 430

Gly Ser Gly Thr Thr Tyr Leu Asp Asn Leu Ser Ile Thr Glu Leu Asn
        435                 440                 445

Ser Thr Pro Glu Ile Leu Asp Glu Pro Glu Val Lys Ile Pro Thr Asp
    450                 455                 460

Gln Glu Ile Met Asp Ala His Lys Ile Tyr Phe Ala Asp Leu Asn Phe

465                 470                 475                 480

Asn Pro Ser Thr Gly Asn Thr Tyr Ile Asn Gly Met Tyr Phe Ala Pro
                485                 490                 495

Thr Gln Thr Asn Lys Glu Ala Leu Asp Tyr Ile Gln Lys Tyr Arg Val
            500                 505                 510

Glu Ala Thr Leu Gln Tyr Ser Gly Phe Lys Asp Ile Gly Thr Lys Asp
        515                 520                 525

Lys Glu Met Arg Asn Tyr Leu Gly Asp Pro Asn Gln Pro Lys Thr Asn
    530                 535                 540

Tyr Val Asn Leu Arg Ser Tyr Phe Thr Gly Gly Glu Asn Ile Met Thr
545                 550                 555                 560

Tyr Lys Lys Leu Arg Ile Tyr Ala Ile Thr Pro Asp Asp Arg Glu Leu
                565                 570                 575

Leu Val Leu Ser Val Asp
            580

<210>  51
<211>  1067
<212>  PRT
<213>  Clostridium difficile

<400>  51

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser

            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His Met Glu Ile Val Asn Glu Asp Ile
225                 230                 235                 240

Leu Pro Asn Asn Gly Leu Met Gly Tyr Tyr Phe Thr Asp Glu His Phe
                245                 250                 255

Lys Asp Leu Lys Leu Met Ala Pro Ile Lys Asp Gly Asn Leu Lys Phe
            260                 265                 270

Glu Glu Lys Lys Val Asp Lys Leu Leu Asp Lys Asp Lys Ser Asp Val
        275                 280                 285

Lys Ser Ile Arg Trp Thr Gly Arg Ile Ile Pro Ser Lys Asp Gly Glu
    290                 295                 300

Tyr Thr Leu Ser Thr Asp Arg Asp Asp Val Leu Met Gln Val Asn Thr
305                 310                 315                 320

Glu Ser Thr Ile Ser Asn Thr Leu Lys Val Asn Met Lys Lys Gly Lys
                325                 330                 335

Glu Tyr Lys Val Arg Ile Glu Leu Gln Asp Lys Asn Leu Gly Ser Ile
            340                 345                 350

Asp Asn Leu Ser Ser Pro Asn Leu Tyr Trp Glu Leu Asp Gly Met Lys
        355                 360                 365

Lys Ile Ile Pro Glu Glu Asn Leu Phe Leu Arg Asp Tyr Ser Asn Ile
    370                 375                 380

Glu Lys Asp Asp Pro Phe Ile Pro Asn Asn Asn Phe Phe Asp Pro Lys
385                 390                 395                 400

Leu Met Ser Asp Trp Glu Asp Glu Asp Leu Asp Thr Asp Asn Asp Asn
                405                 410                 415

Ile Pro Asp Ser Tyr Glu Arg Asn Gly Tyr Thr Ile Lys Asp Leu Ile
            420                 425                 430

Ala Val Lys Trp Glu Asp Ser Phe Ala Glu Gln Gly Tyr Lys Lys Tyr
        435                 440                 445

Val Ser Asn Tyr Leu Glu Ser Asn Thr Ala Gly Asp Pro Tyr Thr Asp
    450                 455                 460

Tyr Glu Lys Ala Ser Gly Ser Phe Asp Lys Ala Ile Lys Thr Glu Ala
465                 470                 475                 480

Arg Asp Pro Leu Val Ala Ala Tyr Pro Ile Val Gly Val Gly Met Glu
                485                 490                 495

Lys Leu Ile Ile Ser Thr Asn Glu His Ala Ser Thr Asp Gln Gly Lys
            500                 505                 510

Thr Val Ser Arg Ala Thr Thr Asn Ser Lys Thr Glu Ser Asn Thr Ala
        515                 520                 525

Gly Val Ser Val Asn Val Gly Tyr Gln Asn Gly Phe Thr Ala Asn Val
    530                 535                 540

Thr Thr Asn Tyr Ser His Thr Thr Asp Asn Ser Thr Ala Val Gln Asp
545                 550                 555                 560

Ser Asn Gly Glu Ser Trp Asn Thr Gly Leu Ser Ile Asn Lys Gly Glu
                565                 570                 575

Ser Ala Tyr Ile Asn Ala Asn Val Arg Tyr Tyr Asn Thr Gly Thr Ala
            580                 585                 590

Pro Met Tyr Lys Val Thr Pro Thr Thr Asn Leu Val Leu Asp Gly Asp
        595                 600                 605

Thr Leu Ser Thr Ile Lys Ala Gln Glu Asn Gln Ile Gly Asn Asn Leu
    610                 615                 620

Ser Pro Gly Asp Thr Tyr Pro Lys Lys Gly Leu Ser Pro Leu Ala Leu
625                 630                 635                 640

Asn Thr Met Asp Gln Arg Ser Ser Arg Leu Ile Pro Ile Asn Tyr Asp
                645                 650                 655

Gln Leu Lys Lys Leu Asp Ala Gly Lys Gln Ile Lys Leu Glu Thr Thr
            660                 665                 670

Gln Val Ser Gly Asn Phe Gly Thr Lys Asn Ser Ser Gly Gln Ile Val
        675                 680                 685

Thr Glu Gly Asn Ser Trp Ser Asp Tyr Ile Ser Gln Ile Asp Ser Ile
    690                 695                 700

Ser Ala Ser Ile Ile Leu Asp Thr Glu Asn Glu Ser Tyr Glu Arg Arg
705                 710                 715                 720

Val Thr Ala Lys Asn Leu Gln Asp Pro Glu Asp Lys Thr Pro Glu Leu
                725                 730                 735

Thr Ile Gly Glu Ala Ile Glu Lys Ala Phe Gly Ala Thr Lys Lys Asp
            740                 745                 750

Gly Leu Leu Tyr Phe Asn Asp Ile Pro Ile Asp Glu Ser Cys Val Glu
        755                 760                 765

Leu Ile Phe Asp Asp Asn Thr Ala Asn Lys Ile Lys Asp Ser Leu Lys
    770                 775                 780

Thr Leu Ser Asp Lys Lys Ile Tyr Asn Val Lys Leu Glu Arg Gly Met
785                 790                 795                 800

Asn Ile Leu Ile Lys Thr Pro Thr Tyr Phe Thr Asn Phe Asp Asp Tyr
                805                 810                 815

Asn Asn Tyr Pro Ser Thr Trp Ser Asn Val Asn Thr Thr Asn Gln Asp
            820                 825                 830

Gly Leu Gln Gly Ser Ala Asn Lys Leu Asn Gly Glu Thr Lys Ile Lys
        835                 840                 845

Ile Pro Met Ser Glu Leu Lys Pro Tyr Lys Arg Tyr Val Phe Ser Gly
    850                 855                 860

Tyr Ser Lys Asp Pro Leu Thr Ser Asn Ser Ile Ile Val Lys Ile Lys
865                 870                 875                 880

Ala Lys Glu Glu Lys Thr Asp Tyr Leu Val Pro Glu Gln Gly Tyr Thr
                885                 890                 895

Lys Phe Ser Tyr Glu Phe Glu Thr Thr Glu Lys Asp Ser Ser Asn Ile
            900                 905                 910

Glu Ile Thr Leu Ile Gly Ser Gly Thr Thr Tyr Leu Asp Asn Leu Ser
        915                 920                 925

Ile Thr Glu Leu Asn Ser Thr Pro Glu Ile Leu Asp Glu Pro Glu Val
    930                 935                 940

Lys Ile Pro Thr Asp Gln Glu Ile Met Asp Ala His Lys Ile Tyr Phe
945                 950                 955                 960

Ala Asp Leu Asn Phe Asn Pro Ser Thr Gly Asn Thr Tyr Ile Asn Gly
                965                 970                 975

Met Tyr Phe Ala Pro Thr Gln Thr Asn Lys Glu Ala Leu Asp Tyr Ile
            980                 985                 990

Gln Lys Tyr Arg Val Glu Ala Thr  Leu Gln Tyr Ser Gly  Phe Lys Asp
        995                 1000                1005

Ile Gly  Thr Lys Asp Lys Glu  Met Arg Asn Tyr Leu  Gly Asp Pro
    1010                1015                1020

Asn Gln  Pro Lys Thr Asn Tyr  Val Asn Leu Arg Ser  Tyr Phe Thr
    1025                1030                1035

Gly Gly  Glu Asn Ile Met Thr  Tyr Lys Lys Leu Arg  Ile Tyr Ala
    1040                1045                1050

Ile Thr  Pro Asp Asp Arg Glu  Leu Leu Val Leu Ser  Val Asp
    1055                1060                1065

<210>  52
<211>  1067
<212>  PRT
<213>  Clostridium difficile

<400>  52

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Glu Val Leu
    210                 215                 220

Phe Gln Gly Pro Leu Gly Ser His Met Glu Ile Val Asn Glu Asp Ile
225                 230                 235                 240

Leu Pro Asn Asn Gly Leu Met Gly Tyr Tyr Phe Thr Asp Glu His Phe
                245                 250                 255

Lys Asp Leu Lys Leu Met Ala Pro Ile Lys Asp Gly Asn Leu Lys Phe

            260                 265                 270

Glu Glu Lys Lys Val Asp Lys Leu Leu Asp Lys Asp Lys Ser Asp Val
        275                 280                 285

Lys Ser Ile Arg Trp Thr Gly Arg Ile Ile Pro Ser Lys Asp Gly Glu
    290                 295                 300

Tyr Thr Leu Ser Thr Asp Arg Asp Asp Val Leu Met Gln Val Asn Thr
305                 310                 315                 320

Glu Ser Thr Ile Ser Asn Thr Leu Lys Val Asn Met Lys Lys Gly Lys
                325                 330                 335

Glu Tyr Lys Val Arg Ile Glu Leu Gln Asp Lys Asn Leu Gly Ser Ile
            340                 345                 350

Asp Asn Leu Ser Ser Pro Asn Leu Tyr Trp Glu Leu Asp Gly Met Lys
        355                 360                 365

Lys Ile Ile Pro Glu Glu Asn Leu Phe Leu Arg Asp Tyr Ser Asn Ile
    370                 375                 380

Glu Lys Asp Asp Pro Phe Ile Pro Asn Asn Asn Phe Phe Asp Pro Lys
385                 390                 395                 400

Leu Met Ser Asp Trp Glu Asp Glu Asp Leu Asp Thr Asp Asn Asp Asn
                405                 410                 415

Ile Pro Asp Ser Tyr Glu Arg Asn Gly Tyr Thr Ile Lys Asp Leu Ile
            420                 425                 430

Ala Val Lys Trp Glu Asp Ser Phe Ala Glu Gln Gly Tyr Lys Lys Tyr
        435                 440                 445

Val Ser Asn Tyr Leu Glu Ser Asn Thr Ala Gly Asp Pro Tyr Thr Asp
    450                 455                 460

Tyr Glu Lys Ala Ser Gly Ser Phe Asp Lys Ala Ile Lys Thr Glu Ala
465                 470                 475                 480

Arg Asp Pro Leu Val Ala Ala Tyr Pro Ile Val Gly Val Gly Met Glu
                485                 490                 495

Lys Leu Ile Ile Ser Thr Asn Glu His Ala Ser Thr Asp Gln Gly Lys
            500                 505                 510

Thr Val Ser Arg Ala Thr Thr Asn Ser Lys Thr Glu Ser Asn Thr Ala
        515                 520                 525

Gly Val Ser Val Asn Val Gly Tyr Gln Asn Gly Phe Thr Ala Asn Val
    530                 535                 540

Thr Thr Asn Tyr Ser His Thr Thr Asp Asn Ser Thr Ala Val Gln Asp
545                 550                 555                 560

Ser Asn Gly Glu Ser Trp Asn Thr Gly Leu Ser Ile Asn Lys Gly Glu
                565                 570                 575

Ser Ala Tyr Ile Asn Ala Asn Val Arg Tyr Tyr Asn Thr Gly Thr Ala
            580                 585                 590

Pro Met Tyr Lys Val Thr Pro Thr Thr Asn Leu Val Leu Asp Gly Asp
        595                 600                 605

Thr Leu Ser Thr Ile Lys Ala Gln Ala Asn Gln Ile Gly Asn Asn Leu
    610                 615                 620

Ser Pro Gly Asp Thr Tyr Pro Lys Lys Gly Leu Ser Pro Leu Ala Leu
625                 630                 635                 640

Asn Thr Met Ala Gln Phe Ser Ser Arg Leu Ile Pro Ile Asn Tyr Asp
                645                 650                 655

Gln Leu Lys Lys Leu Asp Ala Gly Lys Gln Ile Lys Leu Glu Thr Thr
            660                 665                 670

Gln Val Ser Gly Asn Phe Gly Thr Lys Asn Ser Ser Gly Gln Ile Val
        675                 680                 685

Thr Glu Gly Asn Ser Trp Ser Asp Tyr Ile Ser Gln Ile Asp Ser Ile
    690                 695                 700

Ser Ala Ser Ile Ile Leu Asp Thr Glu Asn Glu Ser Tyr Glu Arg Arg
705                 710                 715                 720

Val Thr Ala Lys Asn Leu Gln Asp Pro Glu Asp Lys Thr Pro Glu Leu
                725                 730                 735

Thr Ile Gly Glu Ala Ile Glu Lys Ala Phe Gly Ala Thr Lys Lys Asp
            740                 745                 750

Gly Leu Leu Tyr Phe Asn Asp Ile Pro Ile Asp Glu Ser Cys Val Glu
        755                 760                 765

Leu Ile Phe Asp Asp Asn Thr Ala Asn Lys Ile Lys Asp Ser Leu Lys
    770                 775                 780

Thr Leu Ser Asp Lys Lys Ile Tyr Asn Val Lys Leu Glu Arg Gly Met
785                 790                 795                 800

Asn Ile Leu Ile Lys Thr Pro Thr Tyr Phe Thr Asn Phe Asp Asp Tyr
                805                 810                 815

Asn Asn Tyr Pro Ser Thr Trp Ser Asn Val Asn Thr Thr Asn Gln Asp
            820                 825                 830

Gly Leu Gln Gly Ser Ala Asn Lys Leu Asn Gly Glu Thr Lys Ile Lys
        835                 840                 845

Ile Pro Met Ser Glu Leu Lys Pro Tyr Lys Arg Tyr Val Phe Ser Gly
    850                 855                 860

Tyr Ser Lys Asp Pro Leu Thr Ser Asn Ser Ile Ile Val Lys Ile Lys
865                 870                 875                 880

Ala Lys Glu Glu Lys Thr Asp Tyr Leu Val Pro Glu Gln Gly Tyr Thr
                885                 890                 895

Lys Phe Ser Tyr Glu Phe Glu Thr Thr Glu Lys Asp Ser Ser Asn Ile
            900                 905                 910

Glu Ile Thr Leu Ile Gly Ser Gly Thr Thr Tyr Leu Asp Asn Leu Ser
        915                 920                 925

Ile Thr Glu Leu Asn Ser Thr Pro Glu Ile Leu Asp Glu Pro Glu Val
    930                 935                 940

Lys Ile Pro Thr Asp Gln Glu Ile Met Asp Ala His Lys Ile Tyr Phe
945                 950                 955                 960

Ala Asp Leu Asn Phe Asn Pro Ser Thr Gly Asn Thr Tyr Ile Asn Gly
                965                 970                 975

Met Tyr Phe Ala Pro Thr Gln Thr Asn Lys Glu Ala Leu Asp Tyr Ile
            980                 985                 990

Gln Lys Tyr Arg Val Glu Ala Thr  Leu Gln Tyr Ser Gly  Phe Lys Asp
        995                 1000                1005

Ile Gly  Thr Lys Asp Lys Glu  Met Arg Asn Tyr Leu  Gly Asp Pro
    1010                1015                1020

Asn Gln  Pro Lys Thr Asn Tyr  Val Asn Leu Arg Ser  Tyr Phe Thr
    1025                 1030                 1035

Gly Gly  Glu Asn Ile Met Thr  Tyr Lys Lys Leu Arg  Ile Tyr Ala
    1040                 1045                 1050

Ile Thr  Pro Asp Asp Arg Glu  Leu Leu Val Leu Ser  Val Asp
    1055                 1060                 1065

<210>  53
<211>  828
<212>  PRT
<213>  Clostridium difficile

<400>  53

Met Met Glu Ile Val Asn Glu Asp Ile Leu Pro Asn Asn Gly Leu Met
1               5                   10                  15

Gly Tyr Tyr Phe Thr Asp Glu His Phe Lys Asp Leu Lys Leu Met Ala
            20                  25                  30

Pro Ile Lys Asp Gly Asn Leu Lys Phe Glu Glu Lys Lys Val Asp Lys
        35                  40                  45

Leu Leu Asp Lys Asp Lys Ser Asp Val Lys Ser Ile Arg Trp Thr Gly
    50                  55                  60

Arg Ile Ile Pro Ser Lys Asp Gly Glu Tyr Thr Leu Ser Thr Asp Arg
65                  70                  75                  80

Asp Asp Val Leu Met Gln Val Asn Thr Glu Ser Thr Ile Ser Asn Thr
                85                  90                  95

Leu Lys Val Asn Met Lys Lys Gly Lys Glu Tyr Lys Val Arg Ile Glu
            100                 105                 110

Leu Gln Asp Lys Asn Leu Gly Ser Ile Asp Asn Leu Ser Ser Pro Asn
        115                 120                 125

Leu Tyr Trp Glu Leu Asp Gly Met Lys Lys Ile Ile Pro Glu Glu Asn
    130                 135                 140

Leu Phe Leu Arg Asp Tyr Ser Asn Ile Glu Lys Asp Asp Pro Phe Ile
145                 150                 155                 160

Pro Asn Asn Asn Phe Phe Asp Pro Lys Leu Met Ser Asp Trp Glu Asp
                165                 170                 175

Glu Asp Leu Asp Thr Asp Asn Asp Asn Ile Pro Asp Ser Tyr Glu Arg
        180                 185                 190

Asn Gly Tyr Thr Ile Lys Asp Leu Ile Ala Val Lys Trp Glu Asp Ser
    195                 200                 205

Phe Ala Glu Gln Gly Tyr Lys Lys Tyr Val Ser Asn Tyr Leu Glu Ser
    210                 215                 220

Asn Thr Ala Gly Asp Pro Tyr Thr Asp Tyr Glu Lys Ala Ser Gly Ser
225                 230                 235                 240

Phe Asp Lys Ala Ile Lys Thr Glu Ala Arg Asp Pro Leu Val Ala Ala
                245                 250                 255

Tyr Pro Ile Val Gly Val Gly Met Glu Lys Leu Ile Ile Ser Thr Asn
            260                 265                 270

Glu His Ala Ser Thr Asp Gln Gly Lys Thr Val Ser Arg Ala Thr Thr
        275                 280                 285

Asn Ser Lys Thr Glu Ser Asn Thr Ala Gly Val Ser Val Asn Val Gly
    290                 295                 300

Tyr Gln Asn Gly Phe Thr Ala Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln
    370                 375                 380

Glu Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Asp Gln Phe Ser Ser
                405                 410                 415

Arg Leu Ile Pro Ile Asn Tyr Asp Gln Leu Lys Lys Leu Asp Ala Gly

            420                 425                 430

Lys Gln Ile Lys Leu Glu Thr Thr Gln Val Ser Gly Asn Phe Gly Thr
        435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Gly Gly Gly Pro Glu
                485                 490                 495

Leu Thr Ile Gly Glu Ala Ile Glu Lys Ala Phe Gly Ala Thr Lys Lys
            500                 505                 510

Asp Gly Leu Leu Tyr Phe Asn Asp Ile Pro Ile Asp Glu Ser Cys Val
        515                 520                 525

Glu Leu Ile Phe Asp Asp Asn Thr Ala Asn Lys Ile Lys Asp Ser Leu
    530                 535                 540

Lys Thr Leu Ser Asp Lys Lys Ile Tyr Asn Val Lys Leu Glu Arg Gly
545                 550                 555                 560

Met Asn Ile Leu Ile Lys Thr Pro Thr Tyr Phe Thr Asn Phe Asp Asp
                565                 570                 575

Tyr Asn Asn Tyr Pro Ser Thr Trp Ser Asn Val Asn Thr Thr Asn Gln
            580                 585                 590

Asp Gly Leu Gln Gly Ser Ala Asn Lys Leu Asn Gly Glu Thr Lys Ile
        595                 600                 605

Lys Ile Pro Met Ser Glu Leu Lys Pro Tyr Lys Arg Tyr Val Phe Ser
    610                 615                 620

Gly Tyr Ser Lys Asp Pro Leu Thr Ser Asn Ser Ile Ile Val Lys Ile
625                 630                 635                 640

Lys Ala Lys Glu Glu Lys Thr Asp Tyr Leu Val Pro Glu Gln Gly Tyr
                645                 650                 655

Thr Lys Phe Ser Tyr Glu Phe Glu Thr Thr Glu Lys Asp Ser Ser Asn
            660                 665                 670

Ile Glu Ile Thr Leu Ile Gly Ser Gly Thr Thr Tyr Leu Asp Asn Leu
        675                 680                 685

Ser Ile Thr Glu Leu Asn Ser Thr Pro Glu Ile Leu Asp Glu Pro Glu
    690                 695                 700

Val Lys Ile Pro Thr Asp Gln Glu Ile Met Asp Ala His Lys Ile Tyr
705                 710                 715                 720

Phe Ala Asp Leu Asn Phe Asn Pro Ser Thr Gly Asn Thr Tyr Ile Asn
                725                 730                 735

Gly Met Tyr Phe Ala Pro Thr Gln Thr Asn Lys Glu Ala Leu Asp Tyr
            740                 745                 750

Ile Gln Lys Tyr Arg Val Glu Ala Thr Leu Gln Tyr Ser Gly Phe Lys
        755                 760                 765

Asp Ile Gly Thr Lys Asp Lys Glu Met Arg Asn Tyr Leu Gly Asp Pro
    770                 775                 780

Asn Gln Pro Lys Thr Asn Tyr Val Asn Leu Arg Ser Tyr Phe Thr Gly
785                 790                 795                 800

Gly Glu Asn Ile Met Thr Tyr Lys Lys Leu Arg Ile Tyr Ala Ile Thr
                805                 810                 815

Pro Asp Asp Arg Glu Leu Leu Val Leu Ser Val Asp
            820                 825

<210> 54
<211> 835
<212> PRT
<213> Clostridium difficile

<400> 54

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu
                165                 170                 175

Asp Leu Asp Thr Asp Asn Asp Asn Ile Pro Asp Ser Tyr Glu Arg Asn
            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Asn Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln
    370                 375                 380

Glu Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Asp Gln Phe Ser Ser
                405                 410                 415

Arg Leu Ile Pro Ile Asn Tyr Asp Gln Leu Lys Lys Leu Asp Ala Gly
            420                 425                 430

Lys Gln Ile Lys Leu Glu Thr Thr Gln Val Ser Gly Asn Phe Gly Thr
        435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Lys Asn Leu Gln Gly
                485                 490                 495

Pro Gly Gly Gly Thr Pro Glu Leu Thr Ile Gly Glu Ala Ile Glu Lys
            500                 505                 510

Ala Phe Gly Ala Thr Lys Lys Asp Gly Leu Leu Tyr Phe Asn Asp Ile
        515                 520                 525

Pro Ile Asp Glu Ser Cys Val Glu Leu Ile Phe Asp Asp Asn Thr Ala
    530                 535                 540

Asn Lys Ile Lys Asp Ser Leu Lys Thr Leu Ser Asp Lys Lys Ile Tyr
545                 550                 555                 560

Asn Val Lys Leu Glu Arg Gly Met Asn Ile Leu Ile Lys Thr Pro Thr
                565                 570                 575

Tyr Phe Thr Asn Phe Asp Asp Tyr Asn Asn Tyr Pro Ser Thr Trp Ser
            580                 585                 590

Asn Val Asn Thr Thr Asn Gln Asp Gly Leu Gln Gly Ser Ala Asn Lys
        595                 600                 605

Leu Asn Gly Glu Thr Lys Ile Lys Ile Pro Met Ser Glu Leu Lys Pro
    610                 615                 620

Tyr Lys Arg Tyr Val Phe Ser Gly Tyr Ser Lys Asp Pro Leu Thr Ser
625                 630                 635                 640

Asn Ser Ile Ile Val Lys Ile Lys Ala Lys Glu Glu Lys Thr Asp Tyr
                645                 650                 655

Leu Val Pro Glu Gln Gly Tyr Thr Lys Phe Ser Tyr Glu Phe Glu Thr
            660                 665                 670

Thr Glu Lys Asp Ser Ser Asn Ile Glu Ile Thr Leu Ile Gly Ser Gly
        675                 680                 685

Thr Thr Tyr Leu Asp Asn Leu Ser Ile Thr Glu Leu Asn Ser Thr Pro
    690                 695                 700

Glu Ile Leu Asp Glu Pro Glu Val Lys Ile Pro Thr Asp Gln Glu Ile
705                 710                 715                 720

Met Asp Ala His Lys Ile Tyr Phe Ala Asp Leu Asn Phe Asn Pro Ser
                725                 730                 735

Thr Gly Asn Thr Tyr Ile Asn Gly Met Tyr Phe Ala Pro Thr Gln Thr
            740                 745                 750

Asn Lys Glu Ala Leu Asp Tyr Ile Gln Lys Tyr Arg Val Glu Ala Thr
        755                 760                 765

Leu Gln Tyr Ser Gly Phe Lys Asp Ile Gly Thr Lys Asp Lys Glu Met
    770                 775                 780

Arg Asn Tyr Leu Gly Asp Pro Asn Gln Pro Lys Thr Asn Tyr Val Asn
785                 790                 795                 800

Leu Arg Ser Tyr Phe Thr Gly Gly Glu Asn Ile Met Thr Tyr Lys Lys
                805                 810                 815

Leu Arg Ile Tyr Ala Ile Thr Pro Asp Asp Arg Glu Leu Leu Val Leu

820                 825                 830

Ser Val Asp
        835

<210>  55
<211>  1088
<212>  PRT
<213>  Clostridium difficile

<400>  55

Met Val Tyr Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr

        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro Ser Asp Trp Glu Asp Glu Asp Leu Asp Thr Asp
            420                 425                 430

Asn Asp Asn Ile Pro Asp Ser Tyr Glu Arg Asn Gly Tyr Thr Ile Lys
        435                 440                 445

Asp Leu Ile Ala Val Lys Trp Glu Asp Ser Phe Ala Glu Gln Gly Tyr
    450                 455                 460

Lys Lys Tyr Val Ser Asn Tyr Leu Glu Ser Asn Thr Ala Gly Asp Pro
465                 470                 475                 480

Tyr Thr Asp Tyr Glu Lys Ala Ser Gly Ser Phe Asp Lys Ala Ile Lys
                485                 490                 495

Thr Glu Ala Arg Asp Pro Leu Val Ala Ala Tyr Pro Ile Val Gly Val
            500                 505                 510

Gly Met Glu Lys Leu Ile Ile Ser Thr Asn Glu His Ala Ser Thr Asp
        515                 520                 525

Gln Gly Lys Thr Val Ser Arg Ala Thr Thr Asn Ser Lys Thr Glu Ser
    530                 535                 540

Asn Thr Ala Gly Val Ser Val Asn Val Gly Tyr Gln Asn Gly Phe Thr
545                 550                 555                 560

Ala Asn Val Thr Thr Asn Tyr Ser His Thr Thr Asp Asn Ser Thr Ala
                565                 570                 575

Val Gln Asp Ser Asn Gly Glu Ser Trp Asn Thr Gly Leu Ser Ile Asn
            580                 585                 590

Lys Gly Glu Ser Ala Tyr Ile Asn Ala Asn Val Arg Tyr Tyr Asn Thr
        595                 600                 605

Gly Thr Ala Pro Met Tyr Lys Val Thr Pro Thr Thr Asn Leu Val Leu
    610                 615                 620

Asp Gly Asp Thr Leu Ser Thr Ile Lys Ala Gln Glu Asn Gln Ile Gly
625                 630                 635                 640

Asn Asn Leu Ser Pro Gly Asp Thr Tyr Pro Lys Lys Gly Leu Ser Pro
                645                 650                 655

Leu Ala Leu Asn Thr Met Asp Gln Phe Ser Ser Arg Leu Ile Pro Ile
            660                 665                 670

Asn Tyr Asp Gln Leu Lys Lys Leu Asp Ala Gly Lys Gln Ile Lys Leu
        675                 680                 685

Glu Thr Thr Gln Val Ser Gly Asn Phe Gly Thr Lys Asn Ser Ser Gly
    690                 695                 700

Gln Ile Val Thr Glu Gly Asn Ser Trp Ser Asp Tyr Ile Ser Gln Ile
705                 710                 715                 720

Asp Ser Ile Ser Ala Ser Ile Ile Leu Asp Thr Glu Asn Glu Ser Tyr
                725                 730                 735

Glu Arg Arg Val Thr Ala Lys Asn Leu Gln Asp Pro Glu Asp Lys Thr
            740                 745                 750

Pro Glu Leu Thr Ile Gly Glu Ala Ile Glu Lys Ala Phe Gly Ala Thr
        755                 760                 765

Lys Lys Asp Gly Leu Leu Tyr Phe Asn Asp Ile Pro Ile Asp Glu Ser
    770                 775                 780

Cys Val Glu Leu Ile Phe Asp Asp Asn Thr Ala Asn Lys Ile Lys Asp
785                 790                 795                 800

Ser Leu Lys Thr Leu Ser Asp Lys Lys Ile Tyr Asn Val Lys Leu Glu
                805                 810                 815

Arg Gly Met Asn Ile Leu Ile Lys Thr Pro Thr Tyr Phe Thr Asn Phe
            820                 825                 830

Asp Asp Tyr Asn Asn Tyr Pro Ser Thr Trp Ser Asn Val Asn Thr Thr
        835                 840                 845

Asn Gln Asp Gly Leu Gln Gly Ser Ala Asn Lys Leu Asn Gly Glu Thr
    850                 855                 860

Lys Ile Lys Ile Pro Met Ser Glu Leu Lys Pro Tyr Lys Arg Tyr Val
865                 870                 875                 880

Phe Ser Gly Tyr Ser Lys Asp Pro Leu Thr Ser Asn Ser Ile Ile Val
                885                 890                 895

Lys Ile Lys Ala Lys Glu Glu Lys Thr Asp Tyr Leu Val Pro Glu Gln
            900                 905                 910

Gly Tyr Thr Lys Phe Ser Tyr Glu Phe Glu Thr Thr Glu Lys Asp Ser
        915                 920                 925

Ser Asn Ile Glu Ile Thr Leu Ile Gly Ser Gly Thr Thr Tyr Leu Asp
    930                 935                 940

Asn Leu Ser Ile Thr Glu Leu Asn Ser Thr Pro Glu Ile Leu Asp Glu
945                 950                 955                 960

Pro Glu Val Lys Ile Pro Thr Asp Gln Glu Ile Met Asp Ala His Lys
                965                 970                 975

Ile Tyr Phe Ala Asp Leu Asn Phe Asn Pro Ser Thr Gly Asn Thr Tyr
            980                 985                 990

Ile Asn Gly Met Tyr Phe Ala Pro  Thr Gln Thr Asn Lys  Glu Ala Leu
        995                 1000                 1005

Asp Tyr  Ile Gln Lys Tyr Arg  Val Glu Ala Thr Leu  Gln Tyr Ser
    1010                 1015                 1020

Gly Phe  Lys Asp Ile Gly Thr  Lys Asp Lys Glu Met  Arg Asn Tyr
    1025                 1030                 1035

Leu Gly  Asp Pro Asn Gln Pro  Lys Thr Asn Tyr Val  Asn Leu Arg
    1040                 1045                 1050

Ser Tyr  Phe Thr Gly Gly Glu  Asn Ile Met Thr Tyr  Lys Lys Leu
    1055                 1060                 1065

Arg Ile  Tyr Ala Ile Thr Pro  Asp Asp Arg Glu Leu  Leu Val Leu
    1070                 1075                 1080

Ser Val  Asp Gly Gly
    1085

<210>  56
<211>  1010
<212>  PRT
<213>  Clostridium difficile

<400>  56

Met Val Tyr Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly

                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro Gly Gly Gly Gly Gly Gly Ala Tyr Pro Ile Val
            420                 425                 430

Gly Val Gly Met Glu Lys Leu Ile Ile Ser Thr Asn Glu His Ala Ser
        435                 440                 445

Thr Asp Gln Gly Lys Thr Val Ser Arg Ala Thr Thr Asn Ser Lys Thr
    450                 455                 460

Glu Ser Asn Thr Ala Gly Val Ser Val Asn Val Gly Tyr Gln Asn Gly
465                 470                 475                 480

Phe Thr Ala Asn Val Thr Thr Asn Tyr Ser His Thr Thr Asp Asn Ser
                485                 490                 495

Thr Ala Val Gln Asp Ser Asn Gly Glu Ser Trp Asn Thr Gly Leu Ser
            500                 505                 510

Ile Asn Lys Gly Glu Ser Ala Tyr Ile Asn Ala Asn Val Arg Tyr Tyr
        515                 520                 525

Asn Thr Gly Thr Ala Pro Met Tyr Lys Val Thr Pro Thr Thr Asn Leu
    530                 535                 540

Val Leu Asp Gly Asp Thr Leu Ser Thr Ile Lys Ala Gln Glu Asn Gln
545                 550                 555                 560

Ile Gly Asn Asn Leu Ser Pro Gly Asp Thr Tyr Pro Lys Lys Gly Leu
                565                 570                 575

Ser Pro Leu Ala Leu Asn Thr Met Asp Gln Phe Ser Ser Arg Leu Ile
            580                 585                 590

Pro Ile Asn Tyr Asp Gln Leu Lys Lys Leu Asp Ala Gly Lys Gln Ile
        595                 600                 605

Lys Leu Glu Thr Thr Gln Val Ser Gly Asn Phe Gly Thr Lys Asn Ser
    610                 615                 620

Ser Gly Gln Ile Val Thr Glu Gly Asn Ser Trp Ser Asp Tyr Ile Ser
625                 630                 635                 640

Gln Ile Asp Ser Ile Ser Ala Ser Ile Ile Leu Asp Thr Glu Asn Glu
                645                 650                 655

Ser Tyr Glu Arg Arg Val Thr Ala Lys Asn Leu Gln Asp Pro Glu Asp
            660                 665                 670

Lys Thr Pro Glu Leu Thr Ile Gly Glu Ala Ile Glu Lys Ala Phe Gly
        675                 680                 685

Ala Thr Lys Lys Asp Gly Leu Leu Tyr Phe Asn Asp Ile Pro Ile Asp
    690                 695                 700

Glu Ser Cys Val Glu Leu Ile Phe Asp Asp Asn Thr Ala Asn Lys Ile
705                 710                 715                 720

Lys Asp Ser Leu Lys Thr Leu Ser Asp Lys Lys Ile Tyr Asn Val Lys
                725                 730                 735

Leu Glu Arg Gly Met Asn Ile Leu Ile Lys Thr Pro Thr Tyr Phe Thr
            740                 745                 750

Asn Phe Asp Asp Tyr Asn Asn Tyr Pro Ser Thr Trp Ser Asn Val Asn
        755                 760                 765

Thr Thr Asn Gln Asp Gly Leu Gln Gly Ser Ala Asn Lys Leu Asn Gly
    770                 775                 780

Glu Thr Lys Ile Lys Ile Pro Met Ser Glu Leu Lys Pro Tyr Lys Arg
785                 790                 795                 800

Tyr Val Phe Ser Gly Tyr Ser Lys Asp Pro Leu Thr Ser Asn Ser Ile
                805                 810                 815

Ile Val Lys Ile Lys Ala Lys Glu Glu Lys Thr Asp Tyr Leu Val Pro
            820                 825                 830

Glu Gln Gly Tyr Thr Lys Phe Ser Tyr Glu Phe Glu Thr Thr Glu Lys
        835                 840                 845

Asp Ser Ser Asn Ile Glu Ile Thr Leu Ile Gly Ser Gly Thr Thr Tyr
    850                 855                 860

Leu Asp Asn Leu Ser Ile Thr Glu Leu Asn Ser Thr Pro Glu Ile Leu
865                 870                 875                 880

Asp Glu Pro Glu Val Lys Ile Pro Thr Asp Gln Glu Ile Met Asp Ala
                885                 890                 895

His Lys Ile Tyr Phe Ala Asp Leu Asn Phe Asn Pro Ser Thr Gly Asn
            900                 905                 910

Thr Tyr Ile Asn Gly Met Tyr Phe Ala Pro Thr Gln Thr Asn Lys Glu
        915                 920                 925

Ala Leu Asp Tyr Ile Gln Lys Tyr Arg Val Glu Ala Thr Leu Gln Tyr
    930                 935                 940

Ser Gly Phe Lys Asp Ile Gly Thr Lys Asp Lys Glu Met Arg Asn Tyr
945                 950                 955                 960

Leu Gly Asp Pro Asn Gln Pro Lys Thr Asn Tyr Val Asn Leu Arg Ser
                965                 970                 975

Tyr Phe Thr Gly Gly Glu Asn Ile Met Thr Tyr Lys Lys Leu Arg Ile
            980                 985                 990

Tyr Ala Ile Thr Pro Asp Asp Arg  Glu Leu Leu Val Leu  Ser Val Asp
        995                 1000                1005

Gly Gly
    1010

<210>  57
<211>  680
<212>  PRT
<213>  Clostridium difficile

<400>  57

Met Val Tyr Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro Thr Asn Phe Asp Asp Tyr Asn Asn Tyr Pro Ser
            420                 425                 430

Thr Trp Ser Asn Val Asn Thr Thr Asn Gln Asp Gly Leu Gln Gly Ser
        435                 440                 445

Ala Asn Lys Leu Asn Gly Glu Thr Lys Ile Lys Ile Pro Met Ser Glu
    450                 455                 460

Leu Lys Pro Tyr Lys Arg Tyr Val Phe Ser Gly Tyr Ser Lys Asp Pro
465                 470                 475                 480

Leu Thr Ser Asn Ser Ile Ile Val Lys Ile Lys Ala Lys Glu Glu Lys
                485                 490                 495

Thr Asp Tyr Leu Val Pro Glu Gln Gly Tyr Thr Lys Phe Ser Tyr Glu
            500                 505                 510

Phe Glu Thr Thr Glu Lys Asp Ser Ser Asn Ile Glu Ile Thr Leu Ile
        515                 520                 525

Gly Ser Gly Thr Thr Tyr Leu Asp Asn Leu Ser Ile Thr Glu Leu Asn

530                 535                 540

Ser Thr Pro Glu Ile Leu Asp Glu Pro Glu Val Lys Ile Pro Thr Asp
545                 550                 555                 560

Gln Glu Ile Met Asp Ala His Lys Ile Tyr Phe Ala Asp Leu Asn Phe
                565                 570                 575

Asn Pro Ser Thr Gly Asn Thr Tyr Ile Asn Gly Met Tyr Phe Ala Pro
            580                 585                 590

Thr Gln Thr Asn Lys Glu Ala Leu Asp Tyr Ile Gln Lys Tyr Arg Val
        595                 600                 605

Glu Ala Thr Leu Gln Tyr Ser Gly Phe Lys Asp Ile Gly Thr Lys Asp
    610                 615                 620

Lys Glu Met Arg Asn Tyr Leu Gly Asp Pro Asn Gln Pro Lys Thr Asn
625                 630                 635                 640

Tyr Val Asn Leu Arg Ser Tyr Phe Thr Gly Gly Glu Asn Ile Met Thr
                645                 650                 655

Tyr Lys Lys Leu Arg Ile Tyr Ala Ile Thr Pro Asp Asp Arg Glu Leu
            660                 665                 670

Leu Val Leu Ser Val Asp Gly Gly
        675                 680

<210>  58
<211>  670
<212>  PRT
<213>  Clostridium difficile

<400>  58

Met Val Tyr Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu

65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro Gly Gly Gly Gly Gly Gly Asn Thr Thr Asn Gln
            420                 425                 430

Asp Gly Leu Gln Gly Ser Ala Asn Lys Leu Asn Gly Glu Thr Lys Ile
        435                 440                 445

Lys Ile Pro Met Ser Glu Leu Lys Pro Tyr Lys Arg Tyr Val Phe Ser
    450                 455                 460

Gly Tyr Ser Lys Asp Pro Leu Thr Ser Asn Ser Ile Ile Val Lys Ile
465                 470                 475                 480

Lys Ala Lys Glu Glu Lys Thr Asp Tyr Leu Val Pro Glu Gln Gly Tyr
                485                 490                 495

Thr Lys Phe Ser Tyr Glu Phe Glu Thr Thr Glu Lys Asp Ser Ser Asn
            500                 505                 510

Ile Glu Ile Thr Leu Ile Gly Ser Gly Thr Thr Tyr Leu Asp Asn Leu
        515                 520                 525

Ser Ile Thr Glu Leu Asn Ser Thr Pro Glu Ile Leu Asp Glu Pro Glu
    530                 535                 540

Val Lys Ile Pro Thr Asp Gln Glu Ile Met Asp Ala His Lys Ile Tyr
545                 550                 555                 560

Phe Ala Asp Leu Asn Phe Asn Pro Ser Thr Gly Asn Thr Tyr Ile Asn
                565                 570                 575

Gly Met Tyr Phe Ala Pro Thr Gln Thr Asn Lys Glu Ala Leu Asp Tyr
            580                 585                 590

Ile Gln Lys Tyr Arg Val Glu Ala Thr Leu Gln Tyr Ser Gly Phe Lys
        595                 600                 605

Asp Ile Gly Thr Lys Asp Lys Glu Met Arg Asn Tyr Leu Gly Asp Pro
    610                 615                 620

Asn Gln Pro Lys Thr Asn Tyr Val Asn Leu Arg Ser Tyr Phe Thr Gly
625                 630                 635                 640

Gly Glu Asn Ile Met Thr Tyr Lys Lys Leu Arg Ile Tyr Ala Ile Thr
                645                 650                 655

Pro Asp Asp Arg Glu Leu Leu Val Leu Ser Val Asp Gly Gly
            660                 665                 670

<210>  59
<211>  226
<212>  PRT
<213>  Clostridium difficile

<400>  59

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser
225

<210>  60
<211>  218
<212>  PRT
<213>  Clostridium difficile

<400>  60

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser
    210                 215

<210> 61
<211> 198
<212> PRT
<213> Clostridium difficile

<400> 61

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile
        195

<210>  62
<211>  421
<212>  PRT
<213>  Clostridium difficile

<400>  62

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro
            420

<210>  63
<211>  421
<212>  PRT
<213>  Clostridium difficile

<400>  63

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Phe Thr Ser Ile Gly Ser Val
            340                 345                 350

Asn Met Ser Ala Phe Ala Lys Ala Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro

            420

<210>  64
<211>  421
<212>  PRT
<213>  Clostridium difficile

<400>  64

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp

210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Phe Thr Ser Ile Gly Ser Val
            340                 345                 350

Asn Met Ser Ala Phe Ala Lys Ala Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Glu Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro
            420

<210>  65
<211>  421
<212>  PRT
<213>  Clostridium difficile

<400>  65

Met Val Cys Asn Thr Thr Tyr Lys Ala Pro Ile Glu Arg Pro Glu Asp

1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Phe Thr Ser Ile Gly Ser Val
            340                 345                 350

Asn Met Ser Ala Phe Ala Lys Ala Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Glu Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro
            420

<210>  66
<211>  835
<212>  PRT
<213>  Clostridium difficile

<400>  66

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu
                165                 170                 175

Asp Leu Asp Thr Asp Asn Asp Asn Ile Pro Asp Ser Tyr Glu Arg Asn
            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Asn Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln
    370                 375                 380

Glu Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Asp Gln Arg Ser Ser
                405                 410                 415

Arg Leu Ile Pro Ile Asn Tyr Asp Gln Leu Lys Lys Leu Asp Ala Gly
            420                 425                 430

Lys Gln Ile Lys Leu Glu Thr Thr Gln Val Ser Gly Asn Phe Gly Thr
        435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Lys Asn Leu Gln Asp
                485                 490                 495

Pro Glu Asp Lys Thr Pro Glu Leu Thr Ile Gly Glu Ala Ile Glu Lys
            500                 505                 510

Ala Phe Gly Ala Thr Lys Lys Asp Gly Leu Leu Tyr Phe Asn Asp Ile
        515                 520                 525

Pro Ile Asp Glu Ser Cys Val Glu Leu Ile Phe Asp Asp Asn Thr Ala
    530                 535                 540

Asn Lys Ile Lys Asp Ser Leu Lys Thr Leu Ser Asp Lys Lys Ile Tyr
545                 550                 555                 560

Asn Val Lys Leu Glu Arg Gly Met Asn Ile Leu Ile Lys Thr Pro Thr
                565                 570                 575

Tyr Phe Thr Asn Phe Asp Asp Tyr Asn Asn Tyr Pro Ser Thr Trp Ser
            580                 585                 590

Asn Val Asn Thr Thr Asn Gln Asp Gly Leu Gln Gly Ser Ala Asn Lys
        595                 600                 605

Leu Asn Gly Glu Thr Lys Ile Lys Ile Pro Met Ser Glu Leu Lys Pro
    610                 615                 620

Tyr Lys Arg Tyr Val Phe Ser Gly Tyr Ser Lys Asp Pro Leu Thr Ser
625                 630                 635                 640

Asn Ser Ile Ile Val Lys Ile Lys Ala Lys Glu Glu Lys Thr Asp Tyr
                645                 650                 655

Leu Val Pro Glu Gln Gly Tyr Thr Lys Phe Ser Tyr Glu Phe Glu Thr
            660                 665                 670

Thr Glu Lys Asp Ser Ser Asn Ile Glu Ile Thr Leu Ile Gly Ser Gly
        675                 680                 685

Thr Thr Tyr Leu Asp Asn Leu Ser Ile Thr Glu Leu Asn Ser Thr Pro
    690                 695                 700

Glu Ile Leu Asp Glu Pro Glu Val Lys Ile Pro Thr Asp Gln Glu Ile
705                 710                 715                 720

Met Asp Ala His Lys Ile Tyr Phe Ala Asp Leu Asn Phe Asn Pro Ser
                725                 730                 735

Thr Gly Asn Thr Tyr Ile Asn Gly Met Tyr Phe Ala Pro Thr Gln Thr
            740                 745                 750

Asn Lys Glu Ala Leu Asp Tyr Ile Gln Lys Tyr Arg Val Glu Ala Thr
        755                 760                 765

Leu Gln Tyr Ser Gly Phe Lys Asp Ile Gly Thr Lys Asp Lys Glu Met
    770                 775                 780

Arg Asn Tyr Leu Gly Asp Pro Asn Gln Pro Lys Thr Asn Tyr Val Asn
785                 790                 795                 800

Leu Arg Ser Tyr Phe Thr Gly Gly Glu Asn Ile Met Thr Tyr Lys Lys

805                 810                 815

Leu Arg Ile Tyr Ala Ile Thr Pro Asp Asp Arg Glu Leu Leu Val Leu
            820                 825                 830

Ser Val Asp
        835

<210>  67
<211>  835
<212>  PRT
<213>  Clostridium difficile

<400>  67

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu
                165                 170                 175

Asp Leu Asp Thr Asp Asn Asp Asn Ile Pro Asp Ser Tyr Glu Arg Asn

            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Asn Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln
    370                 375                 380

Ala Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Ala Gln Phe Ser Ser
                405                 410                 415

Arg Leu Ile Pro Ile Asn Tyr Asp Gln Leu Lys Lys Leu Asp Ala Gly
            420                 425                 430

Lys Gln Ile Lys Leu Glu Thr Thr Gln Val Ser Gly Asn Phe Gly Thr
        435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Lys Asn Leu Gln Asp
                485                 490                 495

Pro Glu Asp Lys Thr Pro Glu Leu Thr Ile Gly Glu Ala Ile Glu Lys
            500                 505                 510

Ala Phe Gly Ala Thr Lys Lys Asp Gly Leu Leu Tyr Phe Asn Asp Ile
        515                 520                 525

Pro Ile Asp Glu Ser Cys Val Glu Leu Ile Phe Asp Asp Asn Thr Ala
    530                 535                 540

Asn Lys Ile Lys Asp Ser Leu Lys Thr Leu Ser Asp Lys Lys Ile Tyr
545                 550                 555                 560

Asn Val Lys Leu Glu Arg Gly Met Asn Ile Leu Ile Lys Thr Pro Thr
                565                 570                 575

Tyr Phe Thr Asn Phe Asp Asp Tyr Asn Asn Tyr Pro Ser Thr Trp Ser
            580                 585                 590

Asn Val Asn Thr Thr Asn Gln Asp Gly Leu Gln Gly Ser Ala Asn Lys
        595                 600                 605

Leu Asn Gly Glu Thr Lys Ile Lys Ile Pro Met Ser Glu Leu Lys Pro
    610                 615                 620

Tyr Lys Arg Tyr Val Phe Ser Gly Tyr Ser Lys Asp Pro Leu Thr Ser
625                 630                 635                 640

Asn Ser Ile Ile Val Lys Ile Lys Ala Lys Glu Glu Lys Thr Asp Tyr
                645                 650                 655

Leu Val Pro Glu Gln Gly Tyr Thr Lys Phe Ser Tyr Glu Phe Glu Thr
            660                 665                 670

Thr Glu Lys Asp Ser Ser Asn Ile Glu Ile Thr Leu Ile Gly Ser Gly
        675                 680                 685

Thr Thr Tyr Leu Asp Asn Leu Ser Ile Thr Glu Leu Asn Ser Thr Pro
    690                 695                 700

Glu Ile Leu Asp Glu Pro Glu Val Lys Ile Pro Thr Asp Gln Glu Ile
705                 710                 715                 720

Met Asp Ala His Lys Ile Tyr Phe Ala Asp Leu Asn Phe Asn Pro Ser
                725                 730                 735

Thr Gly Asn Thr Tyr Ile Asn Gly Met Tyr Phe Ala Pro Thr Gln Thr
            740                 745                 750

Asn Lys Glu Ala Leu Asp Tyr Ile Gln Lys Tyr Arg Val Glu Ala Thr
        755                 760                 765

Leu Gln Tyr Ser Gly Phe Lys Asp Ile Gly Thr Lys Asp Lys Glu Met
    770                 775                 780

Arg Asn Tyr Leu Gly Asp Pro Asn Gln Pro Lys Thr Asn Tyr Val Asn
785                 790                 795                 800

Leu Arg Ser Tyr Phe Thr Gly Gly Glu Asn Ile Met Thr Tyr Lys Lys
                805                 810                 815

Leu Arg Ile Tyr Ala Ile Thr Pro Asp Asp Arg Glu Leu Leu Val Leu
            820                 825                 830

Ser Val Asp
        835

<210>  68
<211>  260
<212>  PRT
<213>  Clostridium difficile

<400>  68

Met Thr Asn Phe Asp Asp Tyr Asn Asn Tyr Pro Ser Thr Trp Ser Asn
1               5                   10                  15

Val Asn Thr Thr Asn Gln Asp Gly Leu Gln Gly Ser Ala Asn Lys Leu
            20                  25                  30

Asn Gly Glu Thr Lys Ile Lys Ile Pro Met Ser Glu Leu Lys Pro Tyr
        35                  40                  45

Lys Arg Tyr Val Phe Ser Gly Tyr Ser Lys Asp Pro Leu Thr Ser Asn
    50                  55                  60

Ser Ile Ile Val Lys Ile Lys Ala Lys Glu Glu Lys Thr Asp Tyr Leu
65                  70                  75                  80

Val Pro Glu Gln Gly Tyr Thr Lys Phe Ser Tyr Glu Phe Glu Thr Thr
                85                  90                  95

Glu Lys Asp Ser Ser Asn Ile Glu Ile Thr Leu Ile Gly Ser Gly Thr
            100                 105                 110

Thr Tyr Leu Asp Asn Leu Ser Ile Thr Glu Leu Asn Ser Thr Pro Glu
        115                 120                 125

Ile Leu Asp Glu Pro Glu Val Lys Ile Pro Thr Asp Gln Glu Ile Met
    130                 135                 140

Asp Ala His Lys Ile Tyr Phe Ala Asp Leu Asn Phe Asn Pro Ser Thr
145                 150                 155                 160

Gly Asn Thr Tyr Ile Asn Gly Met Tyr Phe Ala Pro Thr Gln Thr Asn
                165                 170                 175

Lys Glu Ala Leu Asp Tyr Ile Gln Lys Tyr Arg Val Glu Ala Thr Leu
            180                 185                 190

Gln Tyr Ser Gly Phe Lys Asp Ile Gly Thr Lys Asp Lys Glu Met Arg
        195                 200                 205

Asn Tyr Leu Gly Asp Pro Asn Gln Pro Lys Thr Asn Tyr Val Asn Leu
    210                 215                 220

Arg Ser Tyr Phe Thr Gly Gly Glu Asn Ile Met Thr Tyr Lys Lys Leu
225                 230                 235                 240

Arg Ile Tyr Ala Ile Thr Pro Asp Asp Arg Glu Leu Leu Val Leu Ser
                245                 250                 255

Val Asp Gly Gly
            260

<210>  69
<211>  244
<212>  PRT
<213>  Clostridium difficile

<400>  69

Met Asn Thr Thr Asn Gln Asp Gly Leu Gln Gly Ser Ala Asn Lys Leu
1               5                   10                  15

Asn Gly Glu Thr Lys Ile Lys Ile Pro Met Ser Glu Leu Lys Pro Tyr
            20                  25                  30

Lys Arg Tyr Val Phe Ser Gly Tyr Ser Lys Asp Pro Leu Thr Ser Asn
        35                  40                  45

Ser Ile Ile Val Lys Ile Lys Ala Lys Glu Glu Lys Thr Asp Tyr Leu
    50                  55                  60

Val Pro Glu Gln Gly Tyr Thr Lys Phe Ser Tyr Glu Phe Glu Thr Thr
65                  70                  75                  80

Glu Lys Asp Ser Ser Asn Ile Glu Ile Thr Leu Ile Gly Ser Gly Thr
                85                  90                  95

Thr Tyr Leu Asp Asn Leu Ser Ile Thr Glu Leu Asn Ser Thr Pro Glu
            100                 105                 110

Ile Leu Asp Glu Pro Glu Val Lys Ile Pro Thr Asp Gln Glu Ile Met
        115                 120                 125

Asp Ala His Lys Ile Tyr Phe Ala Asp Leu Asn Phe Asn Pro Ser Thr
    130                 135                 140

Gly Asn Thr Tyr Ile Asn Gly Met Tyr Phe Ala Pro Thr Gln Thr Asn
145                 150                 155                 160

Lys Glu Ala Leu Asp Tyr Ile Gln Lys Tyr Arg Val Glu Ala Thr Leu
                165                 170                 175

Gln Tyr Ser Gly Phe Lys Asp Ile Gly Thr Lys Asp Lys Glu Met Arg
            180                 185                 190

Asn Tyr Leu Gly Asp Pro Asn Gln Pro Lys Thr Asn Tyr Val Asn Leu
        195                 200                 205

Arg Ser Tyr Phe Thr Gly Gly Glu Asn Ile Met Thr Tyr Lys Lys Leu
    210                 215                 220

Arg Ile Tyr Ala Ile Thr Pro Asp Asp Arg Glu Leu Leu Val Leu Ser
225                 230                 235                 240

Val Asp Gly Gly

<210>  70
<211>  421

<212>  PRT
<213>  Clostridium difficile

<400>  70

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Glu Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro
            420

<210>  71
<211>  324
<212>  PRT
<213>  Clostridium difficile

<400>  71

Met Ala Tyr Pro Ile Val Gly Val Gly Met Glu Lys Leu Ile Ile Ser
1               5                   10                  15

Thr Asn Glu His Ala Ser Thr Asp Gln Gly Lys Thr Val Ser Arg Ala
            20                  25                  30

Thr Thr Asn Ser Lys Thr Glu Ser Asn Thr Ala Gly Val Ser Val Asn
        35                  40                  45

Val Gly Tyr Gln Asn Gly Phe Thr Ala Asn Val Thr Thr Asn Tyr Ser
    50                  55                  60

His Thr Thr Asp Asn Ser Thr Ala Val Gln Asp Ser Asn Gly Glu Ser
65                  70                  75                  80

Trp Asn Thr Gly Leu Ser Ile Asn Lys Gly Glu Ser Ala Tyr Ile Asn
                85                  90                  95

Ala Asn Val Arg Tyr Tyr Asn Thr Gly Thr Ala Pro Met Tyr Lys Val
            100                 105                 110

Thr Pro Thr Thr Asn Leu Val Leu Asp Gly Asp Thr Leu Ser Thr Ile
        115                 120                 125

Lys Ala Gln Glu Asn Gln Ile Gly Asn Asn Leu Ser Pro Gly Asp Thr
    130                 135                 140

Tyr Pro Lys Lys Gly Leu Ser Pro Leu Ala Leu Asn Thr Met Asp Gln
145                 150                 155                 160

Phe Ser Ser Arg Leu Ile Pro Ile Asn Tyr Asp Gln Leu Lys Lys Leu
                165                 170                 175

Asp Ala Gly Lys Gln Ile Lys Leu Glu Thr Thr Gln Val Ser Gly Asn
            180                 185                 190

Phe Gly Thr Lys Asn Ser Ser Gly Gln Ile Val Thr Glu Gly Asn Ser
        195                 200                 205

Trp Ser Asp Tyr Ile Ser Gln Ile Asp Ser Ile Ser Ala Ser Ile Ile
    210                 215                 220

Leu Asp Thr Glu Asn Glu Ser Tyr Glu Arg Arg Val Thr Ala Lys Asn
225                 230                 235                 240

Leu Gln Asp Pro Glu Asp Lys Thr Pro Glu Leu Thr Ile Gly Glu Ala
                245                 250                 255

Ile Glu Lys Ala Phe Gly Ala Thr Lys Lys Asp Gly Leu Leu Tyr Phe
            260                 265                 270

Asn Asp Ile Pro Ile Asp Glu Ser Cys Val Glu Leu Ile Phe Asp Asp

275                 280                 285

Asn Thr Ala Asn Lys Ile Lys Asp Ser Leu Lys Thr Leu Ser Asp Lys
    290                 295                 300

Lys Ile Tyr Asn Val Lys Leu Glu Arg Gly Met Asn Ile Leu Ile Lys
305                 310                 315                 320

Thr Pro Gly Gly

<210>  72
<211>  835
<212>  PRT
<213>  Clostridium difficile

<400>  72

Met Glu Ile Val Asn Glu Asp Ile Leu Pro Asn Asn Gly Leu Met Gly
1               5                   10                  15

Tyr Tyr Phe Thr Asp Glu His Phe Lys Asp Leu Lys Leu Met Ala Pro
            20                  25                  30

Ile Lys Asp Gly Asn Leu Lys Phe Glu Glu Lys Lys Val Asp Lys Leu
        35                  40                  45

Leu Asp Lys Asp Lys Ser Asp Val Lys Ser Ile Arg Trp Thr Gly Arg
    50                  55                  60

Ile Ile Pro Ser Lys Asp Gly Glu Tyr Thr Leu Ser Thr Asp Arg Asp
65                  70                  75                  80

Asp Val Leu Met Gln Val Asn Thr Glu Ser Thr Ile Ser Asn Thr Leu
                85                  90                  95

Lys Val Asn Met Lys Lys Gly Lys Glu Tyr Lys Val Arg Ile Glu Leu
            100                 105                 110

Gln Asp Lys Asn Leu Gly Ser Ile Asp Asn Leu Ser Ser Pro Asn Leu
        115                 120                 125

Tyr Trp Glu Leu Asp Gly Met Lys Lys Ile Ile Pro Glu Glu Asn Leu
    130                 135                 140

Phe Leu Arg Asp Tyr Ser Asn Ile Glu Lys Asp Asp Pro Phe Ile Pro
145                 150                 155                 160

Asn Asn Asn Phe Phe Asp Pro Lys Leu Met Ser Asp Trp Glu Asp Glu

                165                 170                 175

Asp Leu Asp Thr Asp Asn Asp Asn Ile Pro Asp Ser Tyr Glu Arg Asn
            180                 185                 190

Gly Tyr Thr Ile Lys Asp Leu Ile Ala Val Lys Trp Glu Asp Ser Phe
        195                 200                 205

Ala Glu Gln Gly Tyr Lys Lys Tyr Val Ser Asn Tyr Leu Glu Ser Asn
    210                 215                 220

Thr Ala Gly Asp Pro Tyr Thr Asp Tyr Glu Lys Ala Ser Gly Ser Phe
225                 230                 235                 240

Asp Lys Ala Ile Lys Thr Glu Ala Arg Asp Pro Leu Val Ala Ala Tyr
                245                 250                 255

Pro Ile Val Gly Val Gly Met Glu Lys Leu Ile Ile Ser Thr Asn Glu
            260                 265                 270

His Ala Ser Thr Asp Gln Gly Lys Thr Val Ser Arg Ala Thr Thr Asn
        275                 280                 285

Ser Lys Thr Glu Ser Asn Thr Ala Gly Val Ser Val Asn Val Gly Tyr
    290                 295                 300

Gln Asn Gly Phe Thr Ala Asn Val Thr Thr Asn Tyr Ser His Thr Thr
305                 310                 315                 320

Asp Asn Ser Thr Ala Val Gln Asp Ser Asn Gly Glu Ser Trp Asn Thr
                325                 330                 335

Gly Leu Ser Ile Asn Lys Gly Glu Ser Ala Tyr Ile Asn Ala Asn Val
            340                 345                 350

Arg Tyr Tyr Asn Thr Gly Thr Ala Pro Met Tyr Lys Val Thr Pro Thr
        355                 360                 365

Thr Asn Leu Val Leu Asp Gly Asp Thr Leu Ser Thr Ile Lys Ala Gln
    370                 375                 380

Glu Asn Gln Ile Gly Asn Asn Leu Ser Pro Gly Asp Thr Tyr Pro Lys
385                 390                 395                 400

Lys Gly Leu Ser Pro Leu Ala Leu Asn Thr Met Asp Gln Phe Ser Ser
                405                 410                 415

Arg Leu Ile Pro Ile Asn Tyr Asp Gln Leu Lys Lys Leu Asp Ala Gly
        420                 425                 430

Lys Gln Ile Lys Leu Glu Thr Thr Gln Val Ser Gly Asn Phe Gly Thr
    435                 440                 445

Lys Asn Ser Ser Gly Gln Ile Val Thr Glu Gly Asn Ser Trp Ser Asp
    450                 455                 460

Tyr Ile Ser Gln Ile Asp Ser Ile Ser Ala Ser Ile Ile Leu Asp Thr
465                 470                 475                 480

Glu Asn Glu Ser Tyr Glu Arg Arg Val Thr Ala Lys Asn Leu Gln Asp
                485                 490                 495

Pro Glu Asp Lys Thr Pro Glu Leu Thr Ile Gly Glu Ala Ile Glu Lys
            500                 505                 510

Ala Phe Gly Ala Thr Lys Lys Asp Gly Leu Leu Tyr Phe Asn Asp Ile
        515                 520                 525

Pro Ile Asp Glu Ser Cys Val Glu Leu Ile Phe Asp Asp Asn Thr Ala
    530                 535                 540

Asn Lys Ile Lys Asp Ser Leu Lys Thr Leu Ser Asp Lys Lys Ile Tyr
545                 550                 555                 560

Asn Val Lys Leu Glu Arg Gly Met Asn Ile Leu Ile Lys Thr Pro Thr
                565                 570                 575

Tyr Phe Thr Asn Phe Asp Asp Tyr Asn Asn Tyr Pro Ser Thr Trp Ser
            580                 585                 590

Asn Val Asn Thr Thr Asn Gln Asp Gly Leu Gln Gly Ser Ala Asn Lys
        595                 600                 605

Leu Asn Gly Glu Thr Lys Ile Lys Ile Pro Met Ser Glu Leu Lys Pro
    610                 615                 620

Tyr Lys Arg Tyr Val Phe Ser Gly Tyr Ser Lys Asp Pro Leu Thr Ser
625                 630                 635                 640

Asn Ser Ile Ile Val Lys Ile Lys Ala Lys Glu Glu Lys Thr Asp Tyr
                645                 650                 655

Leu Val Pro Glu Gln Gly Tyr Thr Lys Phe Ser Tyr Glu Phe Glu Thr
            660                 665                 670

Thr Glu Lys Asp Ser Ser Asn Ile Glu Ile Thr Leu Ile Gly Ser Gly
        675                 680                 685

Thr Thr Tyr Leu Asp Asn Leu Ser Ile Thr Glu Leu Asn Ser Thr Pro
    690                 695                 700

Glu Ile Leu Asp Glu Pro Glu Val Lys Ile Pro Thr Asp Gln Glu Ile
705                 710                 715                 720

Met Asp Ala His Lys Ile Tyr Phe Ala Asp Leu Asn Phe Asn Pro Ser
                725                 730                 735

Thr Gly Asn Thr Tyr Ile Asn Gly Met Tyr Phe Ala Pro Thr Gln Thr
            740                 745                 750

Asn Lys Glu Ala Leu Asp Tyr Ile Gln Lys Tyr Arg Val Glu Ala Thr
        755                 760                 765

Leu Gln Tyr Ser Gly Phe Lys Asp Ile Gly Thr Lys Asp Lys Glu Met
    770                 775                 780

Arg Asn Tyr Leu Gly Asp Pro Asn Gln Pro Lys Thr Asn Tyr Val Asn
785                 790                 795                 800

Leu Arg Ser Tyr Phe Thr Gly Gly Glu Asn Ile Met Thr Tyr Lys Lys
                805                 810                 815

Leu Arg Ile Tyr Ala Ile Thr Pro Asp Asp Arg Glu Leu Leu Val Leu
            820                 825                 830

Ser Val Asp
        835

<210>  73
<211>  421
<212>  PRT
<213>  Clostridium difficile

<400>  73

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val
            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro
            420

<210>  74
<211>  421
<212>  PRT
<213>  Clostridium difficile

<400>  74

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Arg Arg
    290                 295                 300

Ser Gly Pro Gln Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Asn Phe Ile Ser Thr Ser Ile Gly Ser Val

            340                 345                 350

Asn Met Ser Ala Phe Ala Lys Arg Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Glu Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro
            420

<210> 75
<211> 421
<212> PRT
<213> Clostridium difficile

<400> 75

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe

130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
            180                 185                 190

Ile Lys Ile Asp Lys Ile Val Arg Ile Val Ile Asp Gly Lys His Tyr
        195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
            260                 265                 270

Asn Asn Pro Asn Pro Glu Leu Asp Ser Lys Ile Thr Asn Ile Glu Asn
        275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Ser Thr Ser Ile Gly Ser Val
            340                 345                 350

Asn Met Ser Ala Phe Ala Lys Ala Lys Ile Val Leu Arg Ile Thr Ile
        355                 360                 365

Pro Lys Gly Ser Pro Gly Ala Tyr Leu Ser Ala Ile Pro Gly Tyr Ala
    370                 375                 380

Gly Gln Tyr Gln Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro
            420

<210>  76
<211>  421
<212>  PRT
<213>  Clostridium difficile

<400>  76

Met Val Cys Asn Thr Thr Tyr Lys Ala Pro Ile Glu Arg Pro Glu Asp
1               5                   10                  15

Phe Leu Lys Asp Lys Glu Lys Ala Lys Glu Trp Glu Arg Lys Glu Ala
            20                  25                  30

Glu Arg Ile Glu Gln Lys Leu Glu Arg Ser Glu Lys Glu Ala Leu Glu
        35                  40                  45

Ser Tyr Lys Lys Asp Ser Val Glu Ile Ser Lys Tyr Ser Gln Thr Arg
    50                  55                  60

Asn Tyr Phe Tyr Asp Tyr Gln Ile Glu Ala Asn Ser Arg Glu Lys Glu
65                  70                  75                  80

Tyr Lys Glu Leu Arg Asn Ala Ile Ser Lys Asn Lys Ile Asp Lys Pro
                85                  90                  95

Met Tyr Val Tyr Tyr Phe Glu Ser Pro Glu Lys Phe Ala Phe Asn Lys
            100                 105                 110

Val Ile Arg Thr Glu Asn Gln Asn Glu Ile Ser Leu Glu Lys Phe Asn
        115                 120                 125

Glu Phe Lys Glu Thr Ile Gln Asn Lys Leu Phe Lys Gln Asp Gly Phe
    130                 135                 140

Lys Asp Ile Ser Leu Tyr Glu Pro Gly Lys Gly Asp Glu Lys Pro Thr
145                 150                 155                 160

Pro Leu Leu Met His Leu Lys Leu Pro Arg Asn Thr Gly Met Leu Pro
                165                 170                 175

Tyr Thr Asn Thr Asn Asn Val Ser Thr Leu Ile Glu Gln Gly Tyr Ser
        180                 185                 190

Ile Lys Ile Asp Lys Ile Val Arg Ile Val Ile Asp Gly Lys His Tyr
    195                 200                 205

Ile Lys Ala Glu Ala Ser Val Val Ser Ser Leu Asp Phe Lys Asp Asp
    210                 215                 220

Val Ser Lys Gly Asp Ser Trp Gly Lys Ala Asn Tyr Asn Asp Trp Ser
225                 230                 235                 240

Asn Lys Leu Thr Pro Asn Glu Leu Ala Asp Val Asn Asp Tyr Met Arg
                245                 250                 255

Gly Gly Tyr Thr Ala Ile Asn Asn Tyr Leu Ile Ser Asn Gly Pro Val
        260                 265                 270

Asn Asn Pro Asn Pro Glu Leu Asp Ser Lys Ile Thr Asn Ile Glu Asn
    275                 280                 285

Ala Leu Lys Arg Glu Pro Ile Pro Thr Asn Leu Thr Val Tyr Ala Arg
    290                 295                 300

Ser Gly Pro Ala Glu Phe Gly Leu Thr Leu Thr Ser Pro Glu Tyr Asp
305                 310                 315                 320

Phe Asn Lys Leu Glu Asn Ile Asp Ala Phe Lys Ser Lys Trp Glu Gly
                325                 330                 335

Gln Ala Leu Ser Tyr Pro Ala Phe Ile Phe Thr Ser Ile Gly Ser Val
        340                 345                 350

Asn Met Ser Ala Phe Ala Lys Ala Lys Ile Val Leu Arg Ile Thr Ile
    355                 360                 365

Pro Lys Gly Ser Pro Gly Ala Tyr Leu Ser Ala Ile Pro Gly Tyr Ala
    370                 375                 380

Gly Gln Tyr Glu Val Leu Leu Asn His Gly Ser Lys Phe Lys Ile Asn
385                 390                 395                 400

Lys Ile Asp Ser Tyr Lys Asp Gly Thr Ile Thr Lys Leu Ile Val Asp
                405                 410                 415

Ala Thr Leu Ile Pro
        420

## Description

### Background

*Clostridium difficile (C. difficile)* is the most important cause of nosocomial intestinal infections and is the major cause of pseudomembranous colitis in humans (Bartlett et al Am. J. Clin. Nutr. 11 suppl:2521-6 (1980)). The overall associated mortality rate for individuals infected with *C*. *difficile* was calculated to be 5.99% within 3 months of diagnosis, with higher mortality associated with advanced age, being 13.5% in patients over 80 years (Karas et al Journal of Infection 561:1-9 (2010)).The current treatment for *C. difficile* infection is the administration of antibiotics (metronidazole and vancomycin); however there has been evidence of strains which are resistant to these antibiotics (Shah et al., Expert Rev. Anti Infect. Ther. 8(5), 555-564 (2010)). Accordingly there is a need for immunogenic compositions capable of inducing antibodies to, and /or a protective immune response to, *C. difficile.*

The enterotoxicity of *C. difficile* is primarily due to the action of two toxins, toxin A and toxin B. These are both potent cytotoxins (Lyerly et al Current Microbiology 21:29-32 (1990).
It has been demonstrated that fragments of toxin A, in particular fragments of the C-terminal domain, can lead to a protective immune response in hamsters (Lyerly et al Current Microbiology 21:29-32 (1990)), WO96/12802 and WO00/61762.

Some strains, but not all, also express *Clostridium difficile* binary toxin (CDT). Similar to many other binary toxins, CDT is composed of two components - an enzymatically active component ("binary toxin A" or "CDTa") and a catalytically inert transport and binding component ("binary toxin B" or "CDTb"). The catalytically inert component facilitates translocation of the CDTa into the target cell.

CDTa has an ADP-ribosylating activity, which transfers the ADP-ribose moiety of NAD/NADPH to monomeric actin (G-actin) in the target cell and thus preventing its polymerization to F-actin and resulting in disruption of the cytoskeleton and eventual cell death (Sundriyal et al, Protein expression and Purification 74 (2010) 42-48).

WO2013/112867 (Merck) describes vaccines against *Clostridium difficile* comprising recombinant *C. difficile* Toxin A, Toxin B and CDTa proteins, all comprising specifically defined mutations relative to the native toxin sequences that are described as substantially reducing or eliminating toxicity, in combination with binary toxin B (CDTb).

The present inventors have found that CDTb proteins comprising mutations designed to modify the pore forming ability of CDTb or modify the heptamerisation ability of CDTb have improved characteristics.

### Summary of Invention

In a first aspect of the invention there is provided an immunogenic composition comprising an isolated *Clostridium difficile* CDTb protein wherein the isolated *Clostridium difficile* CDTb protein has been mutated to modify pore forming ability.

In a second aspect of the invention there is provided an immunogenic composition comprising an isolated *Clostridium difficile* CDTb protein which is a truncated CDTb protein with the signal peptide and the prodomain removed and also the receptor binding domain removed and/or the CDTa binding domain removed.

In a third aspect of the invention there is provided an immunogenic composition comprising an isolated *Clostridium difficile* CDTb protein wherein the isolated *Clostridium difficile* CDTb protein has been mutated to modify heptamerisation ability.

In a fourth aspect of the invention there is provided an immunogenic composition comprising a fusion protein comprising a full length CDTa protein and a CDTb protein.

In a fifth aspect the present invention provides a vaccine comprising the immunogenic composition of any one of the first four aspects and a pharmaceutically acceptable excipient.

In a sixth aspect the present invention provides the immunogenic composition of any one of the first four aspects or the vaccine of the fifth aspect, for use in the treatment or prevention of disease e.g. *C. difficile* disease.

In a seventh aspect the present invention provides the use of an immunogenic composition of any one of the first four aspects or the vaccine of the fifth aspect in the preparation of a medicament for the prevention or treatment of disease e.g. *C. difficile* disease.

In an eighth aspect the present invention provides a method of preventing or treating *C. difficile* disease comprising administering an immunogenic composition of any one of the first four aspects or the vaccine of the fifth aspect to a mammalian subject.

In a ninth aspect the present invention provides novel polypeptides and nucleotides as defined herein.

In a further aspect, the present invention provides a method for the treatment or prevention of a condition or disease caused wholly or in part by *C. difficile.* The method comprises administering to a subject in need thereof a therapeutically effective amount of the proteins as described herein, the immunogenic composition of the invention or the vaccines of the invention.

### BRIEF DESCRIPTION OF THE DRAWINGS

Figure 1 - Cytotoxicity of CDTb candidates alone on HT29 cells: data from Example 7. C37, C123, C124, C126 and C128 referred to in Figure 1 are the CDTb proteins as described in Examples 2, 4 and 6, the sequences of which are set out in the sequence summary (Table A).
Figure 2 - Cytotoxicity of CDTb candidates mixed with CDTa full on HT29 cells: data from Example 7. C37, C123, C124, C126 and C128 referred to in Figure 1 are the CDTb proteins as described in Examples 2, 4 and 6, the sequences of which are set out in the sequence summary (Table A). "CDTa full" is the CDTa protein C34 the sequence of which is set out in the sequence summary (Table A).
Figure 3: Dynamic light scattering measurements of hydrodynamic radius for KO pore forming CDTb constructs C123, C126 and of KO heptamerisation CDTb construct C128. Average radius gives an indication of the homogeneity of the sample, while peak 1 radius is an estimation of the hydrodynamic radius of the protein of interest.
Figure 4: Graph showing anti-CDTb immunogenicity in mice immunised with *C. difficile* CDTa or *C. difficile* CDTb, in both cases formulated with adjuvant
Figure 5: Graph showing anti-CDTa immunogenicity in mice immunised with *C. difficile* CDTa or *C. difficile* CDTb, in both cases formulated with adjuvant
Figure 6: Cytotoxicity inhibition titres in HCT116 cells from mice immunised with *C. difficile* CDTa or *C. difficile* CDTb, in both cases formulated with adjuvant
Figure 7: Cytotoxicity inhibition titres in HT29 cells from mice immunised with *C. difficile* CDTa or *C. difficile* CDTb, in both cases formulated with adjuvant
Figure 8: Cytotoxicity of CDTb candidates alone (Figure 8a) or mixed with CDTa full (Figure 8b) on HT29 cells: data from Example 7. C37, C123, C124, C126 and C128 are the CDTb proteins as described in Examples 2, 4 and 6, the sequences of which are set out in the sequence summary (Table A). "CDTa full" is the CDTa protein C34 the sequence of which is set out in the sequence summary (Table A). C149, C152, C164, C166, C116, C117 are the CDTb proteins as described in Examples 4 and 5.
Figure 9: Cytotoxicity of CDTb candidates alone (Figure 9a) or mixed with CDTa full (Figure 9b) on HCT116 cells (as described in Example 12)
Figure 10: Cytotoxicity of CDTa-CDTb fusion proteins mixed with full activated CDTa on HT29 cells (as described in Example 13)
Figure 11 Cytotoxicity of CDTa-CDTb fusion proteins mixed with full CDTb on HT29 cells (as described in Example 13)
Figure 12: Graph showing anti-CDTa immunogenicity in mice immunised with CDTb proteins or CDTa-CDTb fusions (see Example 14)
Figure 13: Graph showing anti-CDTb immunogenicity in mice immunised with CDTb proteins or CDTa-CDTb fusions (see Example 14)
Figure 14: Cytotoxicity inhibition titres in HT29 and HCT116 cells, mice immunised with CDTb proteins or CDTa-CDTb fusions (see Example 15)

### Detailed Description

The *Clostridium difficile* binary toxin comprises two different proteins, CDTa and CDTb. CDTa comprises two domains, the C-terminal domain is responsible for the ADP ribosyltransferase activity whilst the N-terminal domain is responsible for interacting with CDTb.

During infection CDTb is activated by proteolytic cleavage by a chymotrypsin-like protease to produce a CDTb protein lacking the prodomain (referred to as CDTb"). Note that CDTb" also lacks the CDTb signal sequence. A CDTb protein lacking the signal sequence but not lacking the prodomain is referred to as CDTb'. After proteolytic activation the CDTb oligomerises and binds to CDTa to form the complete *C. difficile* binary toxin (CDT). The binding of the binary toxin to the cell receptors leads to receptor-mediated endocytosis. As the endosome acidifies the CDTb binding domain undergoes conformational changes that allow the CDTb oligomer to form a pore, the pore formation triggers translocation of the ADP-ribosyltransferase domain (CDTa) into the target cell.

In one aspect the present invention provides an immunogenic composition comprising an isolated *Clostridium difficile* CDTb protein which has been mutated to modify pore forming ability.
The term "CDTb protein which has been mutated to modify pore forming ability" refers to a CDTb protein which has been mutated to modify the ability of the CDTb protein to form a pore.

In one embodiment, the isolated *Clostridium difficile* CDTb protein has been mutated to reduce pore forming ability.

Pore forming ability may be analysed according to the methods described in The J. of Biol. Chem. 2001, vol. 276 : 8371-8376, PNAS 2004, vol. 101 : 16756-16761, or The J. of Biol. Chem. 2008, vol. 283 : 3904-3914

In this aspect the isolated *Clostridium difficile* CDTb protein may have been mutated to avoid the formation of the beta barrel structure which is composed of beta strands shared by each CDTb monomer contained in the heptamer which forms upon oligomerisation of CDTb. In this aspect the CDTb protein may be a truncated CDTb protein with the signal peptide removed. In this aspect the CDTb protein may be a truncated CDTb protein with the signal peptide removed and the prodomain removed.

The term "truncated CDTb protein with the signal peptide removed" refers to a CDTb protein with substantially all of the signal peptide removed (therefore which does not comprise amino acids corresponding to substantially all of the signal peptide. For example, the term "truncated CDTb protein with the signal peptide removed" refers to a CDTb protein with at least 25 of the amino acids of the signal peptide removed. There may be a few amino acids of the signal peptide remaining. For example 2, 5, 10, 15 or 20 amino acids of the signal peptide may remain.

The term "truncated CDTb protein with the signal peptide removed.and the prodomain removed" refers to a CDTb protein with substantially all of the signal peptide and the prodomain removed (therefore which does not comprise amino acids corresponding to substantially all of the signal peptide and substantially all of the prodomain). For example, the term "truncated CDTb protein with the signal peptide removed.and the prodomain removed" refers to a CDTb protein with all of the signal peptide removed and at least 85 of the amino acids of the prodomain removed.There may be a few amino acids of the signal peptide and/or prodomain remaining. For example 2, 5, 10, 15 or 20 amino acids of the signal peptide may remain. For example 2, 5, 10, 15 or 20 amino acids of the prodomain may remain.The signal peptide of CDTb corresponds to amino acids 1-48 (encompassing amino acids 1-42) of SEQ ID NO: 3 or their equivalents in a binary toxin protein isolated from a different strain of *C. difficile,* for example amino acids 1-42 of the amino acid sequence of CDTb from strain CD196 (Perelle, M. et al Infect. Immun., 65 (1997), pp. 1402-1407). The prodomain of CDTb corresponds to amino acids 48-211 (encompassing amino acids 48-166) of SEQ ID NO:3 or their equivalents in a binary toxin protein isolated from a different strain of *C. difficile.*

In one embodiment, the isolated *Clostridium difficile* CDTb protein comprises a mutation at position 455 of SEQ ID NO: 3 or its equivalent in a different strain of *C. difficile.* In another embodiment of this aspect, the isolated *Clostridium difficile* CDTb protein comprises a mutation at position 426 of SEQ ID NO: 3 or its equivalent in a different strain of *C. difficile.* In another embodiment of this aspect, the isolated *Clostridium difficile* CDTb protein comprises a mutation at position 453 of SEQ ID NO: 3 or its equivalent in a different strain of *C. difficile.* In another embodiment of this aspect, the isolated *Clostridium difficile* CDTb protein comprises a mutation at position 426 and a mutation at position 453 of SEQ ID NO:3 or its equivalent in a different strain of *C. difficile.*

In one embodiment, the isolated *Clostridium difficile* CDTb protein comprises at least one mutation selected from the group consisting of F455R, F455G, E426A and D453A. "F455R" means that the phenylalanine (F) at position 455 of the CDTb sequence in SEQ ID NO: 3 is mutated to arginine (R). "F455G" means that the phenylalanine (F) at position 455 of the CDTb sequence in SEQ ID NO: 3 is mutated to glycine (G). "E426A" means that the glutamate (E) at position 426 of the CDTb sequence in SEQ ID NO: 3 is mutated to alanine (A). "D453A" means that the aspartate (D) at position 453 of the CDTb sequence in SEQ ID NO: 3 is mutated to alanine (A). In one embodiment of this aspect, the isolated *Clostridium difficile* CDTb protein comprises the mutation F455R or its equivalent in a different strain of *C. difficile.* In one embodiment of this aspect, the isolated *Clostridium difficile* CDTb protein comprises the mutation F455G or its equivalent in a different strain of *C. difficile.* In one embodiment of this aspect, the isolated *Clostridium difficile* CDTb protein comprises the mutations E426A and D453A or their equivalents in a different strain of *C. difficile.*

In one embodiment the isolated *Clostridium difficile* CDTb protein is or comprises
(i) SEQ ID NO: 10 or SEQ ID NO: 11 or SEQ ID NO: 25 or SEQ ID NO: 26; or
(ii) a variant of CDTb having at least 80%, 85%, 88%, 90%, 92%, 95%, 98%, 99%, 100% sequence identity to SEQ ID NO: 10 or SEQ ID NO: 11 or SEQ ID NO: 25 or SEQ ID NO: 26; or
(iii) a fragment of CDTb having at least 30, 50, 80, 100, 120, 150, 200, 250 or 300 contiguous amino acids of SEQ ID NO: 10 or SEQ ID NO: 11 or SEQ ID NO: 25 or SEQ ID NO: 26.

In one embodiment the isolated *Clostridium difficile* CDTb protein is or comprises
(i) SEQ ID NO: 43 or SEQ ID NO: 44; or
(ii) a variant of CDTb having at least 80%, 85%, 88%, 90%, 92%, 95%, 98%, 99%, 100% sequence identity to SEQ ID NO: 43 or SEQ ID NO: 44; or
(iii) a fragment of CDTb having at least 30, 50, 80, 100, 120, 150, 200, 250 or 300 contiguous amino acids of SEQ ID NO: 43 or SEQ ID NO: 44.

In one such aspect there is provided an immunogenic composition wherein the isolated *Clostridium difficile* CDTb protein is a variant of CDTb having at least 80%, 85%, 88%, 90%, 92%, 95%, 98%, 99%, 100% sequence identity to SEQ ID NO: 10 or SEQ ID NO: 11 or SEQ ID NO: 25 or SEQ ID NO: 26.

In one such aspect there is provided an immunogenic composition wherein the isolated *Clostridium difficile* CDTb protein is a variant of CDTb having at least 80%, 85%, 88%, 90%, 92%, 95%, 98%, 99%, 100% sequence identity to SEQ ID NO: 43 or SEQ ID NO: 44.

In another aspect there is provided an immunogenic composition wherein the isolated *Clostridium difficile* CDTb protein is a fragment of CDTb having at least 30, 50, 80, 100, 120, 150, 200, 250, 300, 350, 400, 450, 500, 550, 600, 650, 700, 750, 800 or 850 contiguous amino acids of SEQ ID NO: 10 or SEQ ID NO: 11 or SEQ ID NO: 25 or SEQ ID NO: 26.

In another aspect there is provided an immunogenic composition wherein the isolated *Clostridium difficile* CDTb protein is a fragment of CDTb having at least 30, 50, 80, 100, 120, 150, 200, 250, 300, 350, 400, 450, 500, 550, 600 or 650 contiguous amino acids of SEQ ID NO: 43 or SEQ ID NO: 44.

The present invention also provides an immunogenic composition comprising an isolated *Clostridium difficile* CDTb protein which is a truncated CDTb protein with the signal peptide and the prodomain removed and also the receptor binding domain removed and/or the CDTa binding domain removed.

The term 'truncated CDTb protein with the signal peptide and the prodomain removed and also the receptor binding domain removed and/or the CDTa binding domain removed' means a truncated CDTb protein with the signal peptide and the prodomain removed, wherein also the receptor binding domain and/or the CDTa binding domain have been removed. It refers to SEQ ID NO: 3 or a fragment or variant thereof with substantially all of the signal peptide and the prodomain removed (therefore which does not comprise amino acids corresponding to substantially all of the signal peptide and substantially all of the prodomain) and also substantially all of the receptor binding domain removed and/or substantially all of the CDTa binding domain removed (therefore which does not comprise amino acids corresponding to substantially all of the receptor binding domain and/or which does not comprise amino acids corresponding to substantially all of the CDTa binding domain). For example, the term "truncated CDTb protein with the signal peptide removed and the prodomain removed" refers to a CDTb protein with all of the signal peptide removed and at least 85 of the amino acids of the prodomain removed.There may be a few amino acids of the signal peptide, prodomain, receptor binding domain or CDTa binding domain remaining. For example 2, 5, 10, 15 or 20 amino acids of the signal peptide may remain. For example 2, 5, 10, 15 or 20 amino acids of the prodomain may remain. For example 2, 5, 10, 15 or 20 amino acids of the receptor binding domain may remain. For example 2, 5, 10, 15 or 20 amino acids of the CDTa binding domain may remain.

The signal peptide of CDTb corresponds to amino acids 1-48 (encompassing amino acids 1-42) of SEQ ID NO: 3 or their equivalents in a binary toxin protein isolated from a different strain of *C. difficile,* for example amino acids 1-42 of the amino acid sequence of CDTb from strain CD196 (Perelle, M. et al Infect. Immun., 65 (1997), pp. 1402-1407).

The prodomain of CDTb corresponds to amino acids 48-211 (encompassing amino acids 48-166) of SEQ ID NO:3 or their equivalents in a binary toxin protein isolated from a different strain of *C. difficile.*

In one embodiment, the receptor binding domain of CDTb corresponds to amino acids 620-876 of SEQ ID NO:3, or their equivalents in a binary toxin protein isolated from a different strain of *C*. *difficile.* In another embodiment, the receptor binding domain corresponds to amino acids 636-876 of SEQ ID NO:3 or their equivalents in a binary toxin protein isolated from a different strain of *C. difficile.*

The CDTa binding domain of CDTb corresponds to amino acids 212-296 of SEQ ID NO: 3 or their equivalents in a binary toxin protein isolated from a different strain of *C. difficile.*

In one embodiment of this aspect the immunogenic composition comprises an isolated *Clostridium difficile* CDTb protein which is a truncated CDTb protein with the signal peptide and the prodomain removed and also either the receptor binding domain removed or the CDTa binding domain removed.

In another embodiment of this aspect the immunogenic composition comprises an isolated *Clostridium difficile* CDTb protein which is a truncated CDTb protein with the signal peptide and the prodomain removed and also the receptor binding domain removed and the CDTa binding domain removed.

Suitably the isolated *Clostridium difficile* CDTb protein is or comprises
(i) SEQ ID NO: 8 or SEQ ID NO: 9 or SEQ ID NO: 37;
(ii) a variant of CDTb having at least 80%, 85%, 88%, 90%, 92%, 95%, 98%, 99%, 100% sequence identity to SEQ ID NO: 8 or SEQ ID NO: 9 or SEQ ID NO: 37; or
(iii) a fragment of CDTb having at least 30, 50, 80, 100, 120, 150, 200, 250, 300, 350, 400, 450, 500, 550, 600, 650, 700, 750 or 800 contiguous amino acids of SEQ ID NO: 8 or SEQ ID NO: 9 or SEQ ID NO: 37.

Suitably the isolated *Clostridium difficile* CDTb protein is or comprises
(i) SEQ ID NO: 8 or SEQ ID NO: 9 or SEQ ID NO: 37;
(ii) a variant of CDTb having at least 80%, 85%, 88%, 90%, 92%, 95%, 98%, 99%, 100% sequence identity to SEQ ID NO: 8 or SEQ ID NO: 9 or SEQ ID NO: 37; or
(iii) a fragment of CDTb having at least 30, 50, 80, 100, 120, 150, 200, 250, 300, 350 or 400 contiguous amino acids of SEQ ID NO: 8 or at least 30, 50, 80, 100, 120, 150, 200, 250, 300, 350, 400, 450, 500 or, 550 contiguous amino acids of SEQ ID NO: 9 or at least 30, 50, 80, 100, 120, 150, 200, 250 or 300, contiguous amino acids of SEQ ID NO: 37

In one such aspect there is provided an immunogenic composition wherein the isolated *Clostridium difficile* CDTb protein is a variant of CDTb having at least 80%, 85%, 88%, 90%, 92%, 95%, 98%, 99%, 100% sequence identity to SEQ ID NO: 8 or SEQ ID NO: 9 or SEQ ID NO: 37.

In another aspect there is provided an immunogenic composition wherein the isolated *Clostridium difficile* CDTb protein is a fragment of CDTb having at least 30, 50, 80, 100, 120, 150, 200, 250, 300, 350, 400, 450, 500, 550, contiguous amino acids of SEQ ID NO: 8 or SEQ ID NO: 9 or SEQ ID NO: 37.

In a third aspect of the invention there is provided an immunogenic composition comprising an isolated *Clostridium difficile* CDTb protein wherein the isolated *Clostridium difficile* CDTb protein has been mutated to modify heptamerisation ability.

CDTb protein which has been mutated to modify heptamerisation ability means that the CDTb protein has been mutated to modify its ability to self-assemble into a heptameric fold. This heptamerisation is allowed by the sharing of beta-strands coming from each CDTb monomer in order to form a beta-barrel structure. In this aspect the CDTb protein may be a truncated CDTb protein with the signal peptide removed. In this aspect the CDTb protein may be a truncated CDTb protein with the signal peptide removed and the prodomain removed.

In one embodiment, the isolated *Clostridium difficile* CDTb protein has been mutated to reduce heptamerisation ability.

The ability of CDTb protein to heptamerise may be measured using a technique such as dynamic light scattering (DLS). DLS can be used to evaluate hydrodynamic radius in solution of CDTb purified proteins, in addition to providing information about homogeneity and to detect the presence of high molecular weight aggregates (such as heptamers) within a protein sample. This is based on the calculation of the diffusion coefficient of the different species that are obtained by measuring the light scattering fluctuation, which depends on protein molecular size and shape, and on the other minor constituents of the sample.

Based on a deep analysis of a 3D structural model obtained for CDTb, a loop in the oligomerisation domain may potentially be involved in the heptamerisation of CDTb. In this loop four amino acids have been identified having potential electrostatic interactions with amino acids contained in other monomers of heptameric CDTb. These residues are Asp537, Glu539, Asp540 and Lys541 of SEQ ID NO: 3.

In one embodiment the isolated *Clostridium difficile* CDTb protein comprises at least one mutation and/or truncation in the oligomerisation domain which modifies heptamerisation ability.

The oligomerisation domain corresponds to amino acids 297-619 of SEQ ID NO:3, or their equivalents in a CDTb protein isolated from a different strain of *C. difficile.*

In one embodiment the isolated *Clostridium difficile* CDTb protein comprises at least one mutation and/or truncation to the region of CDTb comprising amino acids 533-542 of SEQ ID NO: 3 or to the equivalent region in a different strain of *C. difficile.*

In one embodiment, the isolated Clostridium difficile CDTb protein comprises at least one mutation at Asp537, Glu539, Asp540 and Lys541 of SEQ ID NO: 3 or their equivalents in a different strain of *C. difficile.* In one embodiment, suitably any one or more of Asp537, Glu539, Asp540 and/or Lys541 is/are mutated to Gly or another small residue.

In another embodiment, the oligomerisation domain is truncated. For example, amino acids 533-542 of SEQ ID NO:3, or their equivalents in a CDTb protein isolated from a different strain of *C. difficile,* may be deleted and optionally replaced by other residues, e.g. by 3, 4, 5 or more Gly and/or Ala residues. In one embodiment amino acids 533-542 of SEQ ID NO:3, or their equivalents in a CDTb protein isolated from a different strain of *C. difficile,* may be deleted and optionally replaced by 3 Gly residues or 3 Ala residues.

In one embodiment, the isolated *Clostridium difficile* CDTb protein comprises mutations in four amino acids. For example, the CDTb protein comprises the mutation D537G-E539G-D540G-K541G or their equivalents in a different strain of *C. difficile.* "D537G" means that the aspartate at position 537 of the CDTb sequence in SEQ ID NO:3 is mutated to glycine. "E539G" means that the glutamate at position 539 of the CDTb sequence in SEQ ID NO:3 is mutated to glycine. "D540G" means that the aspartate at position 540 of the CDTb sequence in SEQ ID NO:3 is mutated to glycine. "K541G" means that the lysine at position 541 of the CDTb sequence in SEQ ID NO:3 is mutated to glycine.

In another embodiment of this aspect, the isolated *Clostridium difficile* CDTb protein comprises a mutation in which amino acids 533 to 542 of SEQ ID NO:3, or their equivalents in a CDTb protein isolated from a different strain of *C*. *difficile,* are replaced by 3 Gly residues.

In one embodiment the isolated *Clostridium difficile* CDTb protein is or comprises
(i) SEQ ID NO: 12 or SEQ ID NO: 13; or
(ii) a variant of CDTb having at least 80%, 85%, 88%, 90%, 92%, 95%, 98%, 99%, 100% sequence identity to SEQ ID NO: 12 or SEQ ID NO: 13; or
(iii) a fragment of CDTb having at least 30, 50, 80, 100, 120, 150, 200, 250, 300, 350, 400, 450, 500, 550, 600, 650, 700, 750, 800 or 850 contiguous amino acids of SEQ ID NO: 12 or SEQ ID NO: 13.

In one such aspect there is provided an immunogenic composition wherein the isolated *Clostridium difficile* CDTb protein is a variant of CDTb having at least 80%, 85%, 88%, 90%, 92%, 95%, 98%, 99%, 100% sequence identity to SEQ ID NO: 12 or SEQ ID NO: 13.

In another aspect there is provided an immunogenic composition wherein the isolated *Clostridium difficile* CDTb protein is a fragment of CDTb having at least 30, 50, 80, 100, 120, 150, 200, 250, 300, 350, 400, 450, 500, 550, 600, 650, 700, 750, 800 or 850 contiguous amino acids of SEQ ID NO: 12 or SEQ ID NO: 13.

The CDTb and CDTa proteins vary in amino acid sequence between different strains, for this reason the amino acid numbering may differ between strains. For this reason the term 'equivalents in a different strain' refers to amino acids which correspond to those of a reference strain (e.g., *C. difficile* R20291 from which SEQ ID NO:1 and SEQ ID NO:3 are derived), but which are found in a toxin from a different strain and which may thus be numbered differently. A region of 'equivalent' amino acids may be determined by aligning the sequences of the toxins from the different strains. Example binary toxin producing strains of *C. difficile* include CD196, CCUG 20309, R8637, IS81, IS93, IS51, IS58, R6786, R7605, R10456 and R5989. Unless otherwise indicated, the amino acids numbers provided throughout refer to those of reference strain R20291, as set forth in SEQ ID NO: 1 (CDTa) and SEQ ID NO:3 (CDTb).

In one embodiment the isolated *Clostridium difficile* CDTb protein is a monomer of CDTb. In a further embodiment the isolated *Clostridium difficile* CDTb protein is a multimer of CDTb. In a further embodiment the isolated *Clostridium difficile* CDTb protein is a heptamer of CDTb.

In one embodiment, the immunogenic composition does not comprise/does not further comprise an isolated *Clostridium difficile* CDTa protein.

The present invention provides an immunogenic composition comprising an isolated *Clostridium difficile* CDTb protein as the sole C. difficile antigen. As used herein the term "as the sole C. difficile antigen" means that the immunogenic composition does not also comprise another antigen from C. difficile e.g. the immunogenic composition does not also comprise a toxin A, toxin B or CDTa protein.

In one embodiment of, the immunogenic composition comprises/further comprises an isolated *Clostridium difficile* CDTa protein.

Suitably the isolated *Clostridium difficile* CDTa protein is a truncated CDTa protein. "A truncated CDTa protein" as used herein means a CDTa protein that does not achieve its full length or its proper form, and thus is missing some of the amino acid residues that are present in full length CDTa of SEQ ID NO: 1 or an equivalent in a different strain, and which cannot perform the function for which it was intended because its structure is incapable of doing so, e.g. ADP ribosyltransferase activity and/or interacting with CDTb. A suitable assay for ADP ribosyltransferase activity is described in PLOS pathogens 2009, vol 5(10) : e1000626.

Suitably the isolated *Clostridium difficile* CDTa protein is a truncated CDTa protein which does not comprise the C-terminal domain. The term 'truncated CDTa protein which does not comprise the C-terminal domain' refers to a fragment or variant of SEQ ID NO:1 which does not comprise a substantial portion of the C-terminal domain, there may be a few amino acids of the C-terminal domain remaining, for example, 2, 5, 10, 15, 20, 25, 30, 35 or 50 amino acids of the C-terminal domain may remain. The C-terminal domain corresponds to amino acids 269-463 of SEQ ID NO:1 or their equivalents in a CDTa protein isolated from a different strain of *C. difficile.* In this embodiment the truncated *Clostridium difficile* CDTa protein suitably is or comprises
(i) SEQ ID NO: 18 or SEQ ID NO: 19;
(i) a variant of CDTa having at least 80%, 85%, 88%, 90%, 92%, 95%, 98%, 99%, 100% sequence identity to SEQ ID NO: 18 or SEQ ID NO: 19; or
(iii) a fragment of CDTa having at least 30, 50, 80, 100, 120, 150, or 190 contiguous amino acids of SEQ ID NO: 18 or SEQ ID NO: 19.

In one embodiment the truncated CDTa protein which does not comprise the C-terminal domain is a variant of CDTa having at least 80%, 85%, 88%, 90%, 92%, 95%, 98%, 99%, 100% sequence identity to SEQ ID NO: 20. In a further embodiment the truncated CDTa protein which does not comprise the C-terminal domain is a variant of CDTa having at least 30, 50, 80, 100, 120, 150, or 190 contiguous amino acids of SEQ ID NO: 20.

In a further embodiment of any of the aspects of the invention, the isolated *Clostridium difficile* CDTa protein suitably contains a mutation which reduces its ADP-ribosyltransferase activity. For example the isolated *Clostridium difficile* CDTa protein has a mutation from glutamate to another amino acid at position 428 of SEQ ID NO: 1 or an equivalent mutation in another strain of *C. difficile.* The term 'has a mutation at position 428' refers to CDTa proteins which have a mutation at this exact location but also to a CDTa protein which is isolated from a different strain and which has a mutation at an equivalent position. The CDTa protein varies in amino acid sequence between different strains, for this reason the amino acid numbering may differ between strains, thus a CDTa protein from a different strain may have a corresponding glutamate which is not number 428 in sequence. In one embodiment the isolated *Clostridium difficile* CDTa protein has a mutation from glutamate to glutamine at position 428 of SEQ ID NO: 1.

In a further embodiment of any of the aspects of the invention, the isolated *Clostridium difficile* CDTa protein suitably has a mutation from glutamate to a different amino acid at position 430 of SEQ ID NO:1 or an equivalent mutation in another strain of *C. difficile.* The term 'has a mutation at position 430' refers to proteins which have this exact location but also to a CDTa protein which is isolated from a different strain and which has a mutation at an equivalent position. In one embodiment the isolated *Clostridium difficile* CDTa protein has a mutation from glutamate to glutamine at position 430 of SEQ ID NO: 1.

In a further embodiment of any of the aspects of the invention, the isolated *Clostridium difficile* CDTa protein suitably is or comprises
(i) SEQ ID NO: 21; SEQ ID NO: 22; SEQ ID NO: 23; or SEQ ID NO: 24; or
(ii) a variant of CDTa having at least 80%, 85%, 88%, 90%, 92%, 95%, 98%, 99%, 100% sequence identity to SEQ ID NO: 21; SEQ ID NO: 22; SEQ ID NO: 23; or SEQ ID NO: 24; or
(iii) a fragment of CDTa having at least 30, 50, 80, 100, 120, 150, 200, 250, 300, 350 or 400 contiguous amino acids of SEQ ID NO: 21; SEQ ID NO: 22; SEQ ID NO: 23; or SEQ ID NO: 24.

In a further embodiment of any of the aspects of the invention, the isolated *Clostridium difficile* CDTa protein suitably is or comprises
(i) SEQ ID NO: 22; or
(ii) a variant of CDTa having at least 80%, 85%, 88%, 90%, 92%, 95%, 98%, 99%, 100% sequence identity to SEQ ID NO: 22; or
(iii) a fragment of CDTa having at least 30, 50, 80, 100, 120, 150, 200, 250, 300, 350 or 400 contiguous amino acids of SEQ NO: 22.

### Fusion proteins comprising a CDTa protein and a CDTb protein

In another aspect, the invention provides immunogenic compositions comprising a fusion protein comprising a full length CDTa protein and a CDTb protein. In this aspect "full length CDTa" means the full length CDTa of SEQ ID NO. 36 or the equivalent in another strain of *C. difficile,* or a variant of CDTa having at least 80%, 85%, 88%, 90%, 92%, 95%, 98%, 99%, 100% sequence identity to SEQ ID NO: 36

"Fusion polypeptide" or "fusion protein" refers to a protein having at least two heterologous polypeptides (e.g. at least two *Clostridium difficile* polypeptides) covalently linked, either directly or via an amino acid linker. It may also refer to a protein having at least two heterologous polypeptides linked non-covalently. The polypeptides forming the fusion protein are typically linked C-terminus to N-terminus, although they can also be linked C-terminus to C-terminus, N-terminus to N-terminus, or N-terminus to C-terminus. The polypeptides of the fusion protein can be in any order. This term also refers to conservatively modified variants, polymorphic variants, alleles, mutants, immunogenic fragments, and interspecies homologs of the antigens that make up the fusion protein.

The term "fused" refers to the linkage e.g. covalent linkage between two polypeptides in a fusion protein. The polypeptides are typically joined via a peptide bond, either directly to each other or via an amino acid linker. Optionally, the peptides can be joined via non-peptide covalent linkages known to those of skill in the art.

A peptide linker sequence may be employed to separate the first and second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46 (1985); Murphy et al., Proc. Natl. Acad. Sci. USA 83:8258-8262 (1986); U.S. Patent No. 4,935,233 and U.S. Patent No. 4,751,180. The linker sequence may generally be from 1 to about 50 amino acids in length for example 1, 5, 10, 15, 20, 25, 30, 35 or 40 amino acids in length. Linker sequences are not required when the first and second polypeptides have non-essential N-terminal amino acid regions that can be used to separate the functional domains and prevent steric interference.

In this aspect the fusion protein may suitably comprise a linker. An example of a suitable linker comprises or consists of six consecutive Gly residues.

In this aspect the CDTa protein is suitably first in the fusion. This means that the CDTa protein is placed in the N-terminal of the fusion protein relative to the position of the CDTb protein. This improves the processabilty of the CDTa-CDTb fusion.

In one embodiment the CDTa protein has a mutation from cysteine to another amino acid at position 45 of SEQ ID NO: 1 or the equivalent amino acid in another strain of *C. difficile.* The term 'has a mutation at position 45' refers to CDTa proteins which have a mutation at this exact location of SEQ ID NO: 1 but also to a CDTa protein which is isolated from a different strain and which has a mutation at an equivalent position. In one embodiment the CDTa protein has a mutation from cysteine to tyrosine at position 45. This mutation is made in order to prevent/avoid the formation of a disulfide bond between two CDTa proteins.

In one embodiment, the full length CDTa protein comprises one or more mutations which reduce its ADP-ribosyltransferase activity.

For example, the CDTa protein may have any of the mutation sets disclosed in WO2013/112867. For example, the CDTa protein may comprise at least two mutations selected from the following mutations at positions 2, 67, 69, 253, 255, 258, 302, 307, 342, 345, 356, 359, 382, 385 and 387 (corresponding to positions 3, 68, 70, 254, 256, 259, 303, 308, 343, 346, 357, 360, 383, 386 and 388 of the sequence of SEQ ID NO: 36 of the present application:) C2A, Y67A, Y69A, Y253A, R255A, Y258A, R302A, Q307E, N342A, S345F, F356A, R359A, Y382A, E385Q, E385A, E387Q, and E387D as disclosed in WO2013/112867. "C2A" means that the cysteine at position 2 of the CDTb protein sequence disclosed in WO2013/112867 (corresponding to position 3 of SEQ ID NO:36 of the present application) is mutated to alanine, and the other mutations listed in this group should be understood similarly. In some embodiments, the CDTa mutations are selected from: C2A, R302A, S345F, E385Q, E385A, E387Q, and E387D. In embodiments of this aspect of the invention, the CDTa protein comprises a set of mutations selected from the group consisting of: (a) S345F, E385Q and E387Q; (b) R302A, E385A, E387D; (c) C2A, S345F, E385Q and E387Q; (d) R302A, S345F, E385Q and E387Q; (e) Y67A, Y69A, and R255A; (f) R359A, Y67A, and Q307E; (g) Y258A, F356A, S345F; and (h) N342A, Y253A and Y382A.

In one embodiment, the CDTa protein comprises one mutation which reduces its ADP-ribosyltransferase activity.

The term "CDTa protein comprises one mutation which reduces its ADP-ribosyltransferase activity" refers to a CDTa protein which comprises a mutation at a single position which alone is intended to reduce its ADP ribosyltransferase activity. The CDTa protein may optionally comprise other mutations not specifically intended to reduce its ADP ribosyltransferase activity. Mutations specifically intended to reduce ADP ribosyltransferase activity include E428Q and E430Q (amino acid numbering with reference to SEQ ID NO:1) or C2A, Y67A, Y69A, Y253A, R255A, Y258A, R302A, Q307E, N342A, S345F, F356A, R359A, Y382A, E385Q, E385A, E387Q, and E387D as disclosed in WO2013/112867 (amino acid numbering corresponding to positions 3, 68, 70, 254, 256, 259, 303, 308, 343, 346, 357, 360, 383, 386 and 388 with reference to SEQ ID NO 36).

For example the CDTa protein has a mutation from glutamate to another amino acid at position 428 of SEQ ID NO: 1 or the equivalent amino acid in another strain of *C. difficile.* The term 'has a mutation at position 428' refers to CDTa proteins which have a mutation at this exact location of SEQ ID NO: 1 but also to a CDTa protein which is isolated from a different strain and which has a mutation at an equivalent position. The CDTa protein varies in amino acid sequence between different strains, for this reason the amino acid numbering may differ between strains, thus a CDTa protein from a different strain may have a corresponding glutamate which is not number 428 in sequence. In one embodiment the CDTa protein has a mutation from glutamate to glutamine at position 428. In a further embodiment, the CDTa protein suitably has a mutation from glutamate to a different amino acid at position 430 of SEQ ID NO: 1 or the equivalent amino acid in another strain of *C. difficile* The term 'has a mutation at position 430' refers to proteins which have this exact location but also to a CDTa protein which is isolated from a different strain and which has a mutation at an equivalent position. In one embodiment the CDTa protein has a mutation from glutamate to glutamine at position 430.

In one embodiment, the full length CDTa protein suitably is or comprises
(i) SEQ ID NO: 39; or
(ii)a variant CDTa having at least 80%, 85%, 88%, 90%, 92%, 95%, 98%, 99%, 100% sequence identity to SEQ ID NO: 39; or
(iii) a fragment of CDTa having at least 30, 50, 80, 100, 120, 150, 200, 250, 300, 350 or 400 contiguous amino acids of SEQ ID NO: 39.

In another embodiment, the full length CDTa protein suitably is or comprises
(i) a sequence of amino acids as set forth in SEQ ID NO: 40, 42 or 67 in WO2013/112867; or
(ii) a variant of CDTa having at least 80%, 85%, 88%, 90%, 92%, 95%, 98%, 99%, 100% sequence identity to any of the sequences in (i); or
(iii) a fragment of CDTa having at least 30, 50, 80, 100, 120, 150, 200, 250, 300, 350 or 400 contiguous amino acids of any of the sequences in (i).

In one embodiment, the CDTb protein is a full length CDTb protein. In this aspect "full length CDTb" means the full length CDTb of SEQ ID NO. 7, ie CDTb with the prodomain removed.

In this embodiment of this aspect, the fusion protein comprising a full length CDTa protein and a CDTb protein suitably is or comprises
(i) SEQ ID NO: 14; or
(ii)a variant fusion protein having at least 80%, 85%, 88%, 90%, 92%, 95%, 98%, 99%, 100% sequence identity to SEQ ID NO: 14; or
(iii) a fragment having at least 30, 50, 80, 100, 120, 150, 200, 250, 300, 350, 400, 450, 500, 550, 600, 650, 700, 750, 800 or 850 contiguous amino acids of SEQ ID NO: 14.

In another embodiment, the CDTb protein is a truncated CDTb protein with the CDTa binding domain removed. The term 'with the CDTa binding domain removed' refers to a fragment or variant of SEQ ID NO: 3, or its equivalent in another strain of *C. difficile,* with substantially all of the CDTa binding domain removed (therefore which does not comprise amino acids corresponding to substantially all of the CDTa binding domain). For example, the term "truncated CDTb protein with the CDTa binding domain removed" refers to a CDTb protein with at least 65 of the amino acids of the CDTa binding domain removed.There may be a few amino acids of the CDTa binding domain remaining. For example 2, 5, 10, 15 or 20 amino acids of the CDTa binding domain may remain.

The CDTa binding domain of CDTb corresponds to amino acids 212 to 295 of SEQ ID NO: 3 or their equivalents in a binary toxin protein isolated from a different strain of *C. difficile.*

In this embodiment, the truncated CDTb protein with the CDTa binding domain removed suitably is or comprises
(i) SEQ ID NO: 9; or
(ii)a variant fusion protein having at least 80%, 85%, 88%, 90%, 92%, 95%, 98%, 99%, 100% sequence identity to SEQ ID NO: 9; or
(iii) a fragment having at least 30, 50, 80, 100, 120, 150, 200, 250, 300, 350 or 400 contiguous amino acids of SEQ ID NO: 9.

In this embodiment, the fusion protein comprising a full length CDTa protein and a truncated CDTb protein with the CDTa binding domain removed suitably is or comprises
(i) SEQ ID NO: 15; or
(ii) a variant fusion protein having at least 80%, 85%, 88%, 90%, 92%, 95%, 98%, 99%, 100% sequence identity to SEQ ID NO: 15; or
(iii) a fragment having at least 30, 50, 80, 100, 120, 150, 200, 250, 300, 350, 400, 450, 500, 550, 600, 650, 700, 750, 800 or 850contiguous amino acids of SEQ ID NO: 15.

In another embodiment the CDTb protein is a truncated CDTb protein comprising the receptor binding domain. The term 'truncated CDTb protein comprising the receptor binding domain' refers to a fragment or variant of SEQ ID NO:3, or its equivalents in another strain of *C. difficile,* with substantially all but the receptor binding domain removed (therefore which does not comprise amino acids corresponding to substantially all of the protein except for the receptor binding domain), there may be a few amino acids in addition to the receptor binding domain remaining, for example 2, 5, 10, 15 or 20 amino acids except for/in addition to the receptor binding domain. In one version, the receptor binding domain corresponds to amino acids 620-876 of SEQ ID NO:3, or their equivalents in a binary toxin protein isolated from a different strain of *C. difficile.* In another version, the receptor binding domain corresponds to amino acids 636-876 of SEQ ID NO:3 or their equivalents in a binary toxin protein isolated from a different strain of *C. difficile.*

In this embodiment the CDTb protein suitably is or comprises
(i) SEQ ID NO: 27 or SEQ ID NO: 28; or
(i) a variant of CDTb having at least 80%, 85%, 88%, 90%, 92%, 95%, 98%, 99%, 100% sequence identity to SEQ ID NO: 27 or SEQ ID NO: 28; or
(iii) a fragment of CDTb having at least 30, 50, 80, 100, 120, 150 or 200 contiguous amino acids of SEQ ID NO: 27 or SEQ ID NO: 28.

In this embodiment, the fusion protein comprising a full length CDTa protein and a truncated CDTb protein comprising the receptor binding domain suitably is or comprises
(i) SEQ ID NO: 16 or SEQ ID NO: 17; or
(ii)a variant fusion protein having at least 80%, 85%, 88%, 90%, 92%, 95%, 98%, 99%, 100% sequence identity to SEQ ID NO: 16 or SEQ ID NO: 17; or
(iii) a fragment having at least 30, 50, 80, 100, 120, 150, 200, 250, 300, 350 or 400 contiguous amino acids of SEQ ID NO: 16 or SEQ ID NO: 17.

In another aspect the invention provides novel polypeptides and nucleotides as defined herein.

In one embodiment, the invention provides a polypeptide comprising an amino acid sequence represented by any of SEQ ID NOs 8, 9, 10, 11, 12, 13, 14, 15, 16, 17, 25, 26, 37, 43, 44, 49, 50, 51, 52, 53, 54, 55, 56, 57, 58, 66, 67 or 71.
In one embodiment of any of the aspects of the invention, the immunogenic composition elicits antibodies that neutralize CDTa or CDTb or both. In a further embodiment the composition elicits antibodies that neutralize binary toxin. Whether a composition elicits antibodies against a toxin can be measured by immunising mice with the immunogenic composition, collecting sera and analysing the anti-toxin titres of the sera using by ELISA. The sera should be compared to a reference sample obtained from mice which have not been immunised. The composition of the invention elicits antibodies that neutralise CDTa if the sera against the polypeptide gives an ELISA readout more than 10%, 20%, 30%, 50%, 70%, 80%, 90%, or 100% higher than the reference sample.

In a further embodiment the immunogenic compositions of the invention elicits a protective immune response in a mammalian host against strains of *C. Difficile.* In one embodiment the mammalian host is selected from the group consisting of mouse, rabbit, guinea pig, non-human primate, monkey and human. In one embodiment the mammalian host is a mouse. In a further embodiment the mammalian host is a human.

Whether an immunogenic composition elicits a protective immune response in a mammalian host against strains of *C*. *Difficile* can be determined using a challenge assay. In such an assay the mammalian host is vaccinated with the immunogenic composition and challenged by exposure to *C. Difficile,* the time which the mammal survives after challenge is compared with the time which a reference mammal that has not been immunised with the immunogenic composition survives. An immunogenic composition elicits a protective immune response if a mammal immunised with the immunogenic composition survives at least 10%, 20%, 30%, 50%, 80%, 80%, 90%, or 100% longer than a reference mammal which has not been immunised after challenge with *C. Difficile.*

### Toxin A and Toxin B

In one embodiment of any of the aspects of the invention, the immunogenic compositions of the invention further comprise an isolated *Clostridium difficile* toxin A protein and/or an isolated *C. difficile* toxin B protein.

The term 'isolated *Clostridium difficile* toxin A protein' refers to a protein with the amino acid sequence of SEQ ID NO: 29, or a fragment or variant of SEQ ID NO: 29. In one embodiment the isolated *Clostridium difficile* toxin A protein is a fragment comprising 50, 100, 150, 200, 250, 300, 500, 750, 1000, 1250, 1500, 1750, 2000 or 2500 contiguous amino acids of SEQ ID NO: 29. In one embodiment the isolated *Clostridium difficile* toxin A protein is a variant comprising 80%, 85%, 90%, 92%, 95%, 98%, 99% or 100% identity to SEQ ID NO: 29.

The term 'isolated *Clostridium difficile* toxin B protein' refers to a protein with the amino acid sequence of SEQ ID NO: 30, or a fragment or variant of SEQ ID NO: 30. In one embodiment the isolated *Clostridium difficile* toxin B protein is a fragment comprising 50, 100, 150, 200, 250, 300, 500, 750, 1000, 1250, 1500, 1750 or 2000 SEQ ID NO: 30. In one embodiment the isolated *Clostridium difficile* toxin B protein is a variant comprising 80%, 85%, 90%, 92%, 95%, 98%, 99% or 100% identity to SEQ ID NO: 30.

In one embodiment the isolated *Clostridium difficile* toxin A protein comprises a repeating domain fragment. The term 'toxin A repeating domain' refers to the C-terminal domain of the toxin A protein from *C. difficile,* comprising repeated sequences. The toxin A repeating domain refers to amino acids 1832-2710 of toxin A from strain VPI10463 (ATCC43255) and their equivalents in a different strain The sequence of amino acids 1832-2710 from strain VPI10463 (ATCC43255) corresponds to amino acids 1832-2710 of SEQ ID NO: 29.

In a further embodiment the isolated *Clostridium difficile* toxin A protein comprises a fragment of the toxin A N-terminal domain. The toxin A N-terminal domain refers to amino acids 1-1831 of toxin A from strain VPI10463 (ATCC43255) and their equivalents in a different strain. The sequence of amino acids 1-1831 of toxin A from strain VPI10463 (ATCC43255) corresponds to amino acids 1-1831 of SEQ ID NO: 29.

In one embodiment the isolated *Clostridium difficile* toxin B protein comprises a toxin B repeating domain fragment. The term 'toxin B repeating domain' refers to the C-terminal domain of the toxin B protein from *C. difficile.* This domain refers to amino acids 1834-2366 from strain VPI10463 (ATCC43255) and their equivalents in a different strain. The sequence of amino acids 1834-2366 from strain VPI10463 (ATCC43255) corresponds to amino acids 1834-2366 of SEQ ID NO: 30.

In a further embodiment the isolated *Clostridium difficile* toxin B protein comprises a fragment of the toxin B N-terminal domain. The toxin B N-terminal domain refers to amino acids 1-1833 of toxin B from strain VBI10463 (ATCC43255) and their equivalents in a different strain. The sequence of amino acids 1-1833 of toxin B from strain VBI10463 (ATCC43255) corresponds to amino acids 1-1833 of SEQ ID NO: 30.

The *C. difficile* toxins A and B are conserved proteins, however the sequence differs a small amount between strains, moreover the amino acid sequence for toxins A and B in different strains may differ in number of amino acids.

For these reasons the terms toxin A repeating domain and/or toxin B repeating domain to refer to a sequence which is a variant with 90%, 95%, 98%, 99% or 100% sequence identity to amino acids 1832-2710 of SEQ ID NO: 29 or a variant with 90%, 95%, 98%, 99% or 100% sequence identity to amino acids 1834-2366 of SEQ ID NO: 30. Similarly the terms toxin A N-terminal domain and/or toxin B N terminal domain refer to a sequence which is a variant with 90%, 95%, 98%, 99% or 100% sequence identity to amino acids 1-1831 of SEQ ID NO: 29 or a variant with 90%, 95%, 98%, 99% or 100% sequence identity to amino acids 1-1833 of SEQ ID NO: 30.

Furthermore the amino acid numbering may differ between the C-terminal domains of toxin A (or toxin B) from one strain and toxin A (or toxin B) from another strain. For this reason the term 'equivalents in a different strain' refers to amino acids which correspond to those of a reference strain (e.g., *C. difficile* VPI10463), but which are found in a toxin from a different strain and which may thus be numbered differently. A region of 'equivalent' amino acids may be determined by aligning the sequences of the toxins from the different strains. The amino acids numbers provided throughout refer to those of strain VPI10463 (and are as set forth in SEQ ID NO: 29 and SEQ ID NO: 30).

In a further embodiment of any of the aspects of the invention, the isolated *C. difficile* toxin A protein and the isolated *C. difficile* toxin B protein form a fusion protein. In one embodiment the fusion protein is 80%, 85%, 90%, 95%, 98%, 99% or 100% identical to a sequence selected from the group consisting of SEQ ID NO: 31, 32, 33, 34, and 35. In a further embodiment the fusion protein is a fragment of at least 800, 850, 900 or 950 contiguous amino acids of a sequence selected from the group consisting of SEQ ID NO: 31, 32, 33, 34, and 35.

### Fragments

The term "fragment" as defined herein may refer to a fragment comprising a T cell epitope. T cell epitopes are short contiguous stretches of amino acids which are recognised by T cells (e.g. CD4+ or CD8+ T cells). Identification of T cell epitopes may be achieved through epitope mapping experiments which are well known to the person skilled in the art (see, for example, Paul, Fundamental Immunology, 3rd ed., 243-247 (1993); Beißbarth et al Bioinformatics 2005 21(Suppl. 1):i29-i37).

The term "fragment" when used in relation to a polypeptide comprising a histidine tag includes the polypeptide without the histidine tag, for example from which the histidine tag has been cleaved. For example, suitable fragments of polypeptides comprising an amino acid sequence represented by any of sequence ID Nos. 5, 7-28 or 36-42 (each of which comprises a histidine tag) include the corresponding polypeptide without the histidine tag represented by sequence ID Nos 47-76, as set out in the following concordance:

| Polypeptide including histag: | Suitable fragment without histag: |
|---|---|
| SEQ ID NO: 5 | SEQ ID NO: 47 |
| SEQ ID NO: 7 | SEQ ID NO: 48 |
| SEQ ID NO: 8 | SEQ ID NO: 49 |
| SEQ ID NO: 9 | SEQ ID NO: 50 |
| SEQ ID NO: 10 | SEQ ID NO: 51 |
| SEQ ID NO: 11 | SEQ ID NO: 52 |
| SEQ ID NO: 12 | SEQ ID NO: 53 |
| SEQ ID NO: 13 | SEQ ID NO: 54 |
| SEQ ID NO: 14 | SEQ ID NO: 55 |
| SEQ ID NO: 15 | SEQ ID NO: 56 |
| SEQ ID NO: 16 | SEQ ID NO: 57 |
| SEQ ID NO: 17 | SEQ ID NO: 58 |
| SEQ ID NO: 18 | SEQ ID NO: 59 |
| SEQ ID NO: 19 | SEQ ID NO: 60 |
| SEQ ID NO: 20 | SEQ ID NO: 61 |
| SEQ ID NO: 21 | SEQ ID NO: 62 |
| SEQ ID NO: 22 | SEQ ID NO: 63 |
| SEQ ID NO: 23 | SEQ ID NO: 64 |
| SEQ ID NO: 24 | SEQ ID NO: 65 |
| SEQ ID NO: 25 | SEQ ID NO: 66 |
| SEQ ID NO: 26 | SEQ ID NO: 67 |
| SEQ ID NO: 27 | SEQ ID NO: 68 |
| SEQ ID NO: 28 | SEQ ID NO: 69 |
| SEQ ID NO: 36 | SEQ ID NO: 70 |
| SEQ ID NO: 37 | SEQ ID NO: 71 |
| SEQ ID NO: 38 | SEQ ID NO: 72 |
| SEQ ID NO: 39 | SEQ ID NO: 73 |
| SEQ ID NO: 40 | SEQ ID NO: 74 |
| SEQ ID NO: 41 | SEQ ID NO: 75 |
| SEQ ID NO: 42 | SEQ ID NO: 76 |

Suitably the fragments of the invention are immunogenic fragments. "Immunogenic fragments" according to the present invention will typically comprise at least 9 contiguous amino acids from the full length polypeptide sequence (e.g. at least 10), such as at least 12 contiguous amino acids (e.g. at least 15 or at least 20 contiguous amino acids), in particular at least 50 contiguous amino acids, such as at least 100 contiguous amino acids (for example at least 200 contiguous amino acids). Suitably the immunogenic fragments will be at least 20%, such as at least 50%, at least 70% or at least 80% of the length of the full length polypeptide sequence.

It will be understood that in a diverse out-bred population, such as humans, different HLA types mean that specific epitopes may not be recognised by all members of the population. Consequently, to maximise the level of recognition and scale of immune response to a polypeptide, it is generally desirable that an immunogenic fragment contains a plurality of the epitopes from the full length sequence (suitably all epitopes).

### Variants

"Variants" or "conservatively modified variants" applies to both amino acid and nucleic acid sequences. With respect to particular nucleic acid sequences, conservatively modified variants refers to those nucleic acids which encode identical or essentially identical amino acid sequences. Where the nucleic acid does not encode an amino acid sequence, "conservatively modified variants" refers to essentially identical sequences.

In respect of variants of a protein sequence, the skilled person will recognise that individual substitutions, deletions or additions to polypeptide, which alters, adds or deletes a single amino acid or a small percentage of amino acids is a "conservatively modified variant" where the alteration(s) results in the substitution of an amino acid with a functionally similar amino acid or the substitution/deletion/addition of residues which do not substantially impact the biological function of the variant.

Conservative substitution tables providing functionally similar amino acids are well known in the art. Such conservatively modified variants are in addition to and do not exclude polymorphic variants, interspecies homologs, and alleles of the invention.

A polypeptide of the invention (such as a CDTa protein or a CDTb protein) may contain a number of conservative substitutions (for example, 1-50, such as 1-25, in particular 1-10, and especially 1 amino acid residue(s) may be altered) when compared to the reference sequence. In general, such conservative substitutions will fall within one of the amino-acid groupings specified below, though in some circumstances other substitutions may be possible without substantially affecting the immunogenic properties of the antigen. The following eight groups each contain amino acids that are typically conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
(see, e.g., Creighton, Proteins 1984).

Suitably such substitutions do not occur in the region of an epitope, and do not therefore have a significant impact on the immunogenic properties of the antigen.

Polypeptide variants may also include those wherein additional amino acids are inserted compared to the reference sequence, for example, such insertions may occur at 1-10 locations (such as 1-5 locations, suitably 1 or 2 locations, in particular 1 location) and may, for example, involve the addition of 50 or fewer amino acids at each location (such as 20 or fewer, in particular 10 or fewer, especially 5 or fewer). Suitably such insertions do not occur in the region of an epitope, and do not therefore have a significant impact on the immunogenic properties of the antigen. One example of insertions includes a short stretch of histidine residues (e.g. 2-6 residues) to aid expression and/or purification of the antigen in question.

Polypeptide variants include those wherein amino acids have been deleted compared to the reference sequence, for example, such deletions may occur at 1-10 locations (such as 1-5 locations, suitably 1 or 2 locations, in particular 1 location) and may, for example, involve the deletion of 50 or fewer amino acids at each location (such as 20 or fewer, in particular 10 or fewer, especially 5 or fewer). Suitably such deletions do not occur in the region of an epitope, and do not therefore have a significant impact on the immunogenic properties of the antigen.

The skilled person will recognise that a particular polypeptide variant may comprise substitutions, deletions and additions (or any combination thereof).

Variants preferably exhibit at least about 70% identity, more preferably at least about 80% identity and most preferably at least about 90% identity (such as at least about 95%, at least about 98% or at least about 99%) to the associated reference sequence.

The terms "identical" or percent "identity," in the context of two or more nucleic acids or polypeptide sequences, refer to two or more sequences or sub-sequences that are the same or have a specified percentage of amino acid residues or nucleotides that are the same (i.e., 70% identity, optionally 75%, 80%, 85%, 90%, 95%, 98% or 99% identity over a specified region), when compared and aligned for maximum correspondence over a comparison window, or designated region as measured using, for example, sequence comparison algorithms or by manual alignment and visual inspection. Such sequences are then said to be "substantially identical." This definition also refers to the compliment of a test sequence. Optionally, the identity exists over a region that is at least about 25 to about 50 amino acids or nucleotides in length, or optionally over a region that is 75-100 amino acids or nucleotides in length. Suitably, the comparison is performed over a window corresponding to the entire length of the reference sequence.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, references to a segment in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Current Protocols in Molecular Biology (Ausubel et al., eds. 1995 supplement)).

One example of a useful algorithm is PILEUP. PILEUP creates a multiple sequence alignment from a group of related sequences using progressive, pairwise alignments to show relationship and percent sequence identity. It also plots a tree or dendogram showing the clustering relationships used to create the alignment. PILEUP uses a simplification of the progressive alignment method of Feng & Doolittle, J. Mol. Evol. 35:351-360 (1987). The method used is similar to the method described by Higgins & Sharp, CABIOS 5:151-153 (1989). The program can align up to 300 sequences, each of a maximum length of 5,000 nucleotides or amino acids. The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid or nucleotide coordinates for regions of sequence comparison and by designating the program parameters. Using PILEUP, a reference sequence is compared to other test sequences to determine the percent sequence identity relationship using the following parameters: default gap weight (3.00), default gap length weight (0.10), and weighted end gaps. PILEUP can be obtained from the GCG sequence analysis software package, e.g., version 7.0 (Devereaux et al., Nuc. Acids Res. 12:387-395 (1984).

Another example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. Software for performing BLAST analyses is publicly available through the National Center for Biotechnology Information (website at www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands.

The BLAST algorithm also performs a statistical analysis of the similarity between two sequences (*see, e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

### POLYNUCLEOTIDE IDENTIFICATION AND CHARACTERISATION

Polynucleotides encoding the *Clostridium difficile* CDTa, CDTb, Toxin A and Toxin B proteins of the invention may be identified, prepared and/or manipulated using any of a variety of well established techniques. For example, a polynucleotide may be identified, as described in more detail below, by screening a microarray of cDNAs. Such screens may be performed, for example, using a Synteni microarray (Palo Alto, CA) according to the manufacturer's instructions (and essentially as described by Schena et al., Proc. Natl. Acad. Sci. USA 93:10614-10619 (1996) and Heller et al., Proc. Natl. Acad. Sci. USA 94:2150-2155 (1997)).

Alternatively, polynucleotides may be amplified from cDNA prepared from cells expressing the proteins described herein, such as *M. tuberculosis* cells. Such polynucleotides may be amplified via polymerase chain reaction (PCR). For this approach, sequence-specific primers may be designed based on the sequences provided herein, and may be purchased or synthesised.

An amplified portion of a polynucleotide may be used to isolate a full length gene from a suitable library (e.g., a *M. tuberculosis* cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridisation techniques, a partial sequence may be labelled (e.g., by nick-translation or end-labelling with ³²P) using well known techniques. A bacterial or bacteriophage library is then generally screened by hybridising filters containing denatured bacterial colonies (or lawns containing phage plaques) with the labelled probe (see Sambrook et al., Molecular Cloning: A Laboratory Manual (2000)). Hybridising colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences can then be assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR (see Triglia et al., Nucl. Acids Res. 16:8186 (1988)), which uses restriction enzymes to generate a fragment in the known region of the gene. The fragment is then circularised by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridises to a polyA region or vector sequence, to identify sequences that are 5' and 3' of a known sequence. Additional techniques include capture PCR (Lagerstrom et al., PCR Methods Applic. 1:111-19 (1991)) and walking PCR (Parker et al., Nucl. Acids. Res. 19:3055-60 (1991)). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (e.g., NCBI BLAST searches), and such ESTs may be used to generate a contiguous full length sequence. Full length DNA sequences may also be obtained by analysis of genomic fragments.

### POLYNUCLEOTIDE EXPRESSION IN HOST CELLS

Polynucleotide sequences or fragments thereof which encode the *Clostridium difficile* CDTa, CDTb, Toxin A and Toxin B proteins, or fusion proteins or functional equivalents thereof, may be used in recombinant DNA molecules to direct expression of a polypeptide in appropriate host cells. Due to the inherent degeneracy of the genetic code, other DNA sequences that encode substantially the same or a functionally equivalent amino acid sequence may be produced and these sequences may be used to clone and express a given polypeptide.

As will be understood by those of skill in the art, it may be advantageous in some instances to produce polypeptide-encoding nucleotide sequences possessing non-naturally occurring codons. For example, codons preferred by a particular prokaryotic or eukaryotic host can be selected to increase the rate of protein expression or to produce a recombinant RNA transcript having desirable properties, such as a half-life which is longer than that of a transcript generated from the naturally occurring sequence.

Moreover, the polynucleotide sequences can be engineered using methods generally known in the art in order to alter polypeptide encoding sequences for a variety of reasons, including but not limited to, alterations which modify the cloning, processing, and/or expression of the gene product. For example, DNA shuffling by random fragmentation and PCR reassembly of gene fragments and synthetic oligonucleotides may be used to engineer the nucleotide sequences. In addition, site-directed mutagenesis may be used to insert new restriction sites, alter glycosylation patterns, change codon preference, produce splice variants, or introduce mutations, and so forth.

Natural, modified, or recombinant nucleic acid sequences may be ligated to a heterologous sequence to encode a fusion protein. For example, to screen peptide libraries for inhibitors of polypeptide activity, it may be useful to encode a chimeric protein that can be recognised by a commercially available antibody. A fusion protein may also be engineered to contain a cleavage site located between the polypeptide-encoding sequence and the heterologous protein sequence, so that the polypeptide may be cleaved and purified away from the heterologous moiety.

Sequences encoding a desired polypeptide may be synthesised, in whole or in part, using chemical methods well known in the art (see Caruthers, M. H. et al., Nucl. Acids Res. Symp. Ser. pp. 215-223 (1980), Horn et al., Nucl. Acids Res. Symp. Ser. pp. 225-232 (1980)). Alternatively, the protein itself may be produced using chemical methods to synthesize the amino acid sequence of a polypeptide, or a portion thereof. For example, peptide synthesis can be performed using various solid-phase techniques (Roberge et al., Science 269:202-204 (1995)) and automated synthesis may be achieved, for example, using the ABI 431A Peptide Synthesizer (Perkin Elmer, Palo Alto, CA).

A newly synthesised peptide may be substantially purified by preparative high performance liquid chromatography (e.g., Creighton, Proteins, Structures and Molecular Principles (1983)) or other comparable techniques available in the art. The composition of the synthetic peptides may be confirmed by amino acid analysis or sequencing (e.g., the Edman degradation procedure). Additionally, the amino acid sequence of a polypeptide, or any part thereof, may be altered during direct synthesis and/or combined using chemical methods with sequences from other proteins, or any part thereof, to produce a variant polypeptide.

In order to express a desired polypeptide, the nucleotide sequences encoding the polypeptide, or functional equivalents, may be inserted into an appropriate expression vector, i.e., a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include *in vitro* recombinant DNA techniques, synthetic techniques, and *in vivo* genetic recombination. Such techniques are described in Sambrook et al., Molecular Cloning, A Laboratory Manual (2000), and Ausubel et al., Current Protocols in Molecular Biology (updated annually).

A variety of expression vector/host systems may be utilised to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems.

The "control elements" or "regulatory sequences" present in an expression vector are those non-translated regions of the vector--enhancers, promoters, 5' and 3' untranslated regionswhich interact with host cellular proteins to carry out transcription and translation. Such elements may vary in their strength and specificity. Depending on the vector system and host utilised, any number of suitable transcription and translation elements, including constitutive and inducible promoters, may be used. For example, when cloning in bacterial systems, inducible promoters such as the hybrid lacZ promoter of the PBLUESCRIPT phagemid (Stratagene, La Jolla, Calif.) or PSPORT1 plasmid (Gibco BRL, Gaithersburg, MD) and the like may be used. In mammalian cell systems, promoters from mammalian genes or from mammalian viruses are generally preferred. If it is necessary to generate a cell line that contains multiple copies of the sequence encoding a polypeptide, vectors based on SV40 or EBV may be advantageously used with an appropriate selectable marker.

In bacterial systems, a number of expression vectors may be selected depending upon the use intended for the expressed polypeptide. For example, when large quantities are needed, for example for the induction of antibodies, vectors which direct high level expression of fusion proteins that are readily purified may be used. Such vectors include, but are not limited to, the multifunctional *E. coli* cloning and expression vectors such as BLUESCRIPT (Stratagene), in which the sequence encoding the polypeptide of interest may be ligated into the vector in frame with sequences for the amino-terminal Met and the subsequent 7 residues of β-galactosidase so that a hybrid protein is produced; pIN vectors (Van Heeke &Schuster, J. Biol. Chem. 264:5503-5509 (1989)); and the like. pGEX Vectors (Promega, Madison, Wis; GE Healthcare.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast, *Saccharomyces cerevisiae or Pichia such as Pichia pastoris for example,* a number of vectors containing constitutive or inducible promoters such as alpha factor, alcohol oxidase, and PGH may be used. Other vectors containing constitutive or inducible promoters include GAP, PGK, GAL and ADH. For reviews, see Ausubel *et al.* (*supra*) and Grant et al., Methods Enzymol. 153:516-544 (1987) and Romas et al. Yeast 8 423-88 (1992).

In cases where plant expression vectors are used, the expression of sequences encoding polypeptides may be driven by any of a number of promoters. For example, viral promoters such as the 35S and 19S promoters of CaMV may be used alone or in combination with the omega leader sequence from TMV (Takamatsu, EMBO J. 6:307-311 (1987)). Alternatively, plant promoters such as the small subunit of RUBISCO or heat shock promoters may be used (Coruzzi et al., EMBO J. 3:1671-1680 (1984); Broglie et al., Science 224:838-843 (1984); and Winter et al., Results Probl. Cell Differ. 17:85-105 (1991)). These constructs can be introduced into plant cells by direct DNA transformation or pathogen-mediated transfection. Such techniques are described in a number of generally available reviews (*see, e.g.,* Hobbs in McGraw Hill Yearbook of Science and Technology pp. 191-196 (1992)).

An insect system may also be used to express a polypeptide of interest. For example, in one such system, *Autographa californica* nuclear polyhedrosis virus (AcNPV) is used as a vector to express foreign genes in *Spodoptera frugiperda* cells or in *Trichoplusia larvae.* The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, *S. frugiperda* cells or *Trichoplusia larvae* in which the polypeptide of interest may be expressed (Engelhard et al., Proc. Natl. Acad. Sci. U.S.A. 91 :3224-3227 (1994)).

In mammalian host cells, a number of viral-based expression systems are generally available. For example, in cases where an adenovirus is used as an expression vector, sequences encoding a polypeptide of interest may be ligated into an adenovirus transcription/translation complex consisting of the late promoter and tripartite leader sequence. Insertion in a non-essential E1 or E3 region of the viral genome may be used to obtain a viable virus which is capable of expressing the polypeptide in infected host cells (Logan & Shenk, Proc. Natl. Acad. Sci. U.S.A. 81:3655-3659 (1984)). In addition, transcription enhancers, such as the Rous sarcoma virus (RSV) enhancer, may be used to increase expression in mammalian host cells. Methods and protocols for working with adenovirus vectors are reviewed in Wold, Adenovirus Methods and Protocols, 1998. Additional references regarding use of adenovirus vectors can be found in Adenovirus: A Medical Dictionary, Bibliography, and Annotated Research Guide to Internet References, 2004.

Specific initiation signals may also be used to achieve more efficient translation of sequences encoding a polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf. et al., Results Probl. Cell Differ. 20:125-162 (1994)).

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding and/or function. Different host cells such as CHO, HeLa, MDCK, HEK293, and WI38, which have specific cellular machinery and characteristic mechanisms for such post-translational activities, may be chosen to ensure the correct modification and processing of the foreign protein.

For long-term, high-yield production of recombinant proteins, stable expression is generally preferred. For example, cell lines which stably express a polynucleotide of interest may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase (Wigler et al., Cell 11:223-32 (1977)) and adenine phosphoribosyltransferase (Lowy et al., Cell 22:817-23 (1990)) genes which can be employed in tk.sup.- or aprt.sup.- cells, respectively. Also, antimetabolite, antibiotic or herbicide resistance can be used as the basis for selection; for example, dhfr which confers resistance to methotrexate (Wigler et al., Proc. Natl. Acad. Sci. U.S.A. 77:3567-70 (1980)); npt, which confers resistance to the aminoglycosides, neomycin and G-418 (Colbere-Garapin et al., J. Mol. Biol. 150:1-14 (1981)); and als or pat, which confer resistance to chlorsulfuron and phosphinotricin acetyltransferase, respectively (Murry, *supra*). Additional selectable genes have been described, for example, trpB, which allows cells to utilise indole in place of tryptophan, or hisD, which allows cells to utilise histinol in place of histidine (Hartman & Mulligan, Proc. Natl. Acad. Sci. U.S.A. 85:8047-51 (1988)). Recently, the use of visible markers has gained popularity with such markers as anthocyanins, β-glucuronidase and its substrate GUS, and luciferase and its substrate luciferin, being widely used not only to identify transformants, but also to quantify the amount of transient or stable protein expression attributable to a specific vector system (Rhodes et al., Methods Mol. Biol. 55:121-131 (1995)).

Although the presence/absence of marker gene expression suggests that the gene of interest is also present, its presence and expression may need to be confirmed. For example, if the sequence encoding a polypeptide is inserted within a marker gene sequence, recombinant cells containing sequences can be identified by the absence of marker gene function. Alternatively, a marker gene can be placed in tandem with a polypeptide-encoding sequence under the control of a single promoter. Expression of the marker gene in response to induction or selection usually indicates expression of the tandem gene as well.

Alternatively, host cells which contain and express a desired polynucleotide sequence may be identified by a variety of procedures known to those of skill in the art. These procedures include, but are not limited to, DNA-DNA or DNA-RNA hybridisations and protein bioassay or immunoassay techniques which include membrane, solution, or chip based technologies for the detection and/or quantification of nucleic acid or protein.

A variety of protocols for detecting and measuring the expression of polynucleotide-encoded products, using either polyclonal or monoclonal antibodies specific for the product are known in the art. Examples include enzyme-linked immunosorbent assay (ELISA), radioimmunoassay (RIA), and fluorescence activated cell sorting (FACS). A two-site, monoclonal-based immunoassay utilising monoclonal antibodies reactive to two non-interfering epitopes on a given polypeptide may be preferred for some applications, but a competitive binding assay may also be employed. These and other assays are described, among other places, in Hampton et al., Serological Methods, a Laboratory Manual (1990) and Maddox et al., J. Exp. Med. 158:1211-1216 (1983).

A wide variety of labels and conjugation techniques are known by those skilled in the art and may be used in various nucleic acid and amino acid assays. Means for producing labelled hybridisation or PCR probes for detecting sequences related to polynucleotides include oligolabeling, nick translation, end-labelling or PCR amplification using a labelled nucleotide. Alternatively, the sequences, or any portions thereof may be cloned into a vector for the production of an mRNA probe. Such vectors are known in the art, are commercially available, and may be used to synthesize RNA probes *in vitro* by addition of an appropriate RNA polymerase such as T7, T3, or SP6 and labelled nucleotides. These procedures may be conducted using a variety of commercially available kits. Suitable reporter molecules or labels, which may be used include radionuclides, enzymes, fluorescent, chemiluminescent, or chromogenic agents as well as substrates, cofactors, inhibitors, magnetic particles, and the like.

Host cells transformed with a polynucleotide sequence of interest may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides may be designed to contain signal sequences which direct secretion of the encoded polypeptide through a prokaryotic or eukaryotic cell membrane. Other recombinant constructions may be used to join sequences encoding a polypeptide of interest to nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilised immunoglobulin, and the domain utilized in the FLAGS extension/affinity purification system (Immunex Corp., Seattle, Wash.). The inclusion of cleavable linker sequences such as those specific for Factor XA or enterokinase (Invitrogen. San Diego, Calif.) between the purification domain and the encoded polypeptide may be used to facilitate purification. One such expression vector provides for expression of a fusion protein containing a polypeptide of interest and a nucleic acid encoding 6 histidine residues preceding a thioredoxin or an enterokinase cleavage site. The histidine residues facilitate purification on IMIAC (immobilised metal ion affinity chromatography) as described in Porath et al., Prot. Exp. Purif. 3:263-281 (1992) while the enterokinase cleavage site provides a means for purifying the desired polypeptide from the fusion protein. A discussion of vectors which contain fusion proteins is provided in Kroll et al., DNA Cell Biol. 12:441-453 (1993)).

### POLYPEPTIDE COMPOSITIONS

Generally, a polypeptide of use in the invention (for example the *Clostridium difficile* CDTa, CDTb, Toxin A and Toxin B proteins) will be an isolated polypeptide (i.e. separated from those components with which it may usually be found in nature).

For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast, and higher eukaryotic cells, such as mammalian cells and plant cells. Preferably, the host cells employed are *E. coli,* yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Polypeptides for use in the invention, immunogenic fragments thereof, and other variants having less than about 100 amino acids, and generally less than about 50 amino acids, may also be generated by synthetic means, using techniques well known to those of ordinary skill in the art. For example, such polypeptides may be synthesised using any of the commercially available solid-phase techniques, such as the Merrifield solid-phase synthesis method, where amino acids are sequentially added to a growing amino acid chain. See Merrifield, J. Am. Chem. Soc. 85:2149-2146 (1963). Equipment for automated synthesis of polypeptides is commercially available from suppliers such as Perkin Elmer/Applied BioSystems Division (Foster City, CA), and may be operated according to the manufacturer's instructions.

Within certain specific embodiments, a polypeptide may be a fusion protein that comprises multiple polypeptides as described herein, or that comprises at least one polypeptide as described herein and an unrelated sequence, examples of such proteins include tetanus, tuberculosis and hepatitis proteins (*see, e.g.,* Stoute et al., New Engl. J. Med. 336:86-91 (1997)). A fusion partner may, for example, assist in providing T helper epitopes (an immunological fusion partner), preferably T helper epitopes recognised by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46 (1985); Murphy et al., Proc. Natl. Acad. Sci. USA 83:8258-8262 (1986); U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

### Adjuvants

In a further embodiment of any of the aspects of the invention, the immunogenic composition further comprises an adjuvant. In one embodiment the adjuvant comprises aluminium hydroxide or aluminium phosphate. Alternatively the immunogenic composition of the invention may comprise an aluminium-free adjuvant, the immunogenic composition is formulated with an adjuvant that is free of aluminum or aluminum salts, that is, an aluminum-free adjuvant or adjuvant system.

In certain embodiments, the immunogenic composition is formulated with an adjuvant comprising an immunologically active saponin fraction presented in the form of a liposome. The adjuvant may further comprise a lipopolysaccharide. The adjuvant may include QS21. For example, in one embodiment, the adjuvant contains QS21 in a liposomal formulation. In one embodiment, the adjuvant system includes 3D-MPL and QS21. For example, in one embodiment, the adjuvant contains 3D-MPL and QS21 in a liposomal formulation. Optionally, the adjuvant system also contains cholesterol. In one specific embodiment, the adjuvant includes QS21 and cholesterol. Optionally, the adjuvant system contains 1, 2-Dioleoyl-sn-Glycero-3-phosphocholine (DOPC). For example, in one specific adjuvant system contains cholesterol, DOPC, 3D-MPL and QS21.

In one specific example, the immunogenic composition includes an adjuvant formulated in a dose that includes: from about 0.1 to about 0.5 mg cholesterol; from about 0.25 to about 2 mg DOPC; from about 10 µg to about 100 µg 3D-MPL; and from about 10 µg to about 100 µg QS21. In another specific example, the immunogenic composition includes an adjuvant formulated in a dose that includes: from about 0.1 to about 0.5 mg cholesterol; from about 0.25 to about 2 mg DOPC; from about 10 µg to about 70 µg 3D-MPL; and from about 10 µg to about 70 µg QS21. In one specific formulation, the adjuvant is formulated in a single dose that contains: about 0.25 mg cholesterol; about 1.0 mg DOPC; about 50 µg 3D-MPL; and about 50 µg QS21. In other embodiments, the immunogenic composition is formulated with a fractional dose (that is a dose, which is a fraction of the preceding single dose formulations, such as one half of the preceding quantity of components (cholesterol, DOPC, 3D-MPL and QS21), ¼ of the preceding quantity of components, or another fractional dose (e.g., 1/3, 1/6, etc.) of the preceding quantity of components.

In one embodiment, the immunogenic compositions according to the invention include an adjuvant containing combinations of lipopolysaccharide and Quillaja saponins that have been disclosed previously, for example in EP0671948. This patent demonstrated a strong synergy when a lipopolysaccharide (3D-MPL) was combined with a Quillaja saponin (QS21).

The adjuvant may further comprise immunostimulatory oligonucleotides (for example, CpG) or a carrier.

A particularly suitable saponin for use in the present invention is Quil A and its derivatives. Quil A is a saponin preparation isolated from the South American tree *Quillaja Saponaria Molina* and was first described by Dalsgaard et al. in 1974 ("Saponin adjuvants", Archiv. für die gesamte Virusforschung, Vol. 44, Springer Verlag, Berlin, p243-254) to have adjuvant activity. Purified fragments of Quil A have been isolated by HPLC which retain adjuvant activity without the toxicity associated with Quil A (EP 0 362 278), for example QS7 and QS21 (also known as QA7 and QA21). QS21 is a natural saponin derived from the bark of *Quillaja saponaria* Molina, which induces CD8+ cytotoxic T cells (CTLs), Th1 cells and a predominant IgG2a antibody response and is a preferred saponin in the context of the present invention.

When the adjuvant comprises an immunologically active saponin fraction presented in the form of a liposome, the adjuvant may further comprise a sterol. Suitably the sterol is provided at a ratio of saponin:sterol of from 1:1 to 1:100 w/w, such as from 1:1 to 1:10w/w; or 1:1 to 1:5 w/w.

In a specific embodiment, QS21 is provided in its less reactogenic composition where it is quenched with an exogenous sterol, such as cholesterol for example. Several particular forms of less reactogenic compositions wherein QS21 is quenched with an exogenous cholesterol exist. In a specific embodiment, the saponin /sterol is in the form of a liposome structure (WO 96/33739, Example 1). In this embodiment the liposomes suitably contain a neutral lipid, for example phosphatidylcholine, which is suitably non-crystalline at room temperature, for example eggyolk phosphatidylcholine, dioleoyl phosphatidylcholine (DOPC) or dilauryl phosphatidylcholine. The liposomes may also contain a charged lipid which increases the stability of the lipsome-QS21 structure for liposomes composed of saturated lipids. In these cases the amount of charged lipid is suitably 1-20% w/w, preferably 5-10%. The ratio of sterol to phospholipid is 1-50% (mol/mol), suitably 20-25%.

Suitable sterols include β-sitosterol, stigmasterol, ergosterol, ergocalciferol and cholesterol. In one particular embodiment, the adjuvant composition comprises cholesterol as sterol. These sterols are well known in the art, for example cholesterol is disclosed in the Merck Index, 11th Edn., page 341, as a naturally occurring sterol found in animal fat.

Where the active saponin fraction is QS21, the ratio of QS21 : sterol will typically be in the order of 1:100 to 1:1 (w/w), suitably between 1:10 to 1:1 (w/w), and preferably 1:5 to 1:1 (w/w). Suitably excess sterol is present, the ratio of QS21:sterol being at least 1:2 (w/w). In one embodiment, the ratio of QS21:sterol is 1:5 (w/w). The sterol is suitably cholesterol.

In one embodiment, the invention provides a dose of an immunogenic composition comprising immunologically active saponin, preferably QS21, at a level of about 1 - about 70 µg per dose, for example at an amount of about 50 µg.

In one embodiment, the invention provides a dose of an immunogenic composition comprising immunologically active saponin, preferably QS21, at a level of 60µg or less, for example between 1 and 60µg. In one embodiment, the dose of the immunogenic composition comprises QS21 at a level of approximately around 50 µg, for example between 45 and 55 µg, suitably between 46 - 54 µg or between 47 and 53 µg or between 48 and 52 µg or between 49 and 51 µg, or 50 µg.

In another embodiment the dose of the immunogenic composition comprises QS21 at a level of around 25 µg, for example between 20 - 30 µg, suitably between 21 - 29 µg or between 22 and 28 µg or between 23 and 27 µg or between 24 and 26 µg, or 25 µg.

In another embodiment, the dose of the immunogenic composition comprises QS21 at a level of around 10 µg per, for example between 5 and 15 µg, suitably between 6 and 14 µg, for example between 7 and 13 µg or between 8 and 12 µg or between 9 and 11 µg, or 10µg.

Specifically, a 0.5 ml vaccine dose volume contains 25 µg or 50 µg of QS21 per dose. Specifically, a 0.5 ml vaccine dose volume contains 50 µg of QS21 per dose.

In compositions comprising a lipopolysaccharide, the lipopolysaccharide may be present at an amount of about 1 - about 70 µg per dose, for example at an amount of about 50 µg.

The lipopolysaccharide may be a non-toxic derivative of lipid A, particularly monophosphoryl lipid A or more particularly 3-Deacylated monophoshoryl lipid A (3D - MPL).

3D-MPL is sold under the name MPL by GlaxoSmithKline Biologicals S.A. and is referred throughout the document as MPL or 3D-MPL. See, for example, US Patent Nos. 4,436,727; 4,877,611; 4,866,034 and 4,912,094. 3D-MPL primarily promotes CD4+ T cell responses with an IFN-γ (Th1) phenotype. 3D-MPL can be produced according to the methods disclosed in GB 2 220 211 A. Chemically it is a mixture of 3-deacylated monophosphoryl lipid A with 3, 4, 5 or 6 acylated chains. Preferably in the compositions of the present invention small particle 3D-MPL is used. Small particle 3D-MPL has a particle size such that it may be sterile-filtered through a 0.22µm filter. Such preparations are described in WO 94/21292.

The invention therefore provides a dose of an immunogenic composition comprising lipopolysaccharide, preferably 3D-MPL, at a level of 75µg or less, for example between 1 and 60µg.

In one embodiment, the dose of the immunogenic composition comprises 3D-MPL at a level of around 50 µg, for example between 45 - 55 µg, suitably between 46 - 54 µg or between 47 and 53 µg or between 48 and 52 µg or between 49 and 51 µg, or 50 µg.

In one embodiment, the dose of the immunogenic composition comprises 3D-MPL at a level of around 25 µg, for example between 20 - 30 µg, suitably between 21 - 29 µg or between 22 and 28 µg or between 23 and 27 µg or between 24 and 26 µg, or 25 µg.

In another embodiment, the dose of the immunogenic composition comprises 3D-MPL at a level of around 10 µg, for example between 5 and 15 µg, suitably between 6 and 14 µg, for example between 7 and 13 µg or between 8 and 12 µg or between 9 and 11 µg, or 10µg.

In one embodiment, the volume of the dose is 0.5 ml. In a further embodiment, the immunogenic composition is in a volume suitable for a dose which volume is higher than 0.5 ml, for example 0.6, 0.7, 0.8, 0.9 or 1 ml. In a further embodiment, the human dose is between 1 ml and 1.5 ml.

Specifically, a 0.5 ml vaccine dose volume contains 25 µg or 50 µg of 3D-MPL per dose. Specifically, a 0.5 ml vaccine dose volume contains 50 µg of 3D-MPL per dose.

The dose of the immunogenic composition according to any aspect of the invention suitably refers to human dose. By the term "human dose" is meant a dose which is in a volume suitable for human use. Generally this is between 0.3 and 1.5 ml. In one embodiment, a human dose is 0.5 ml. In a further embodiment, a human dose is higher than 0.5 ml, for example 0.6, 0.7, 0.8, 0.9 or 1 ml. In a further embodiment, a human dose is between 1 ml and 1.5 ml.

Suitable compositions of the invention are those wherein liposomes are initially prepared without MPL (as described in WO 96/33739), and MPL is then added, suitably as small particles of below 100 nm particles or particles that are susceptible to sterile filtration through a 0.22 µm membrane. The MPL is therefore not contained within the vesicle membrane (known as MPL out). Compositions where the MPL is contained within the vesicle membrane (known as MPL in) also form an aspect of the invention. The polypeptide comprising a *C. difficile* toxin A fragment and / or a *C. difficile* toxin B fragment can be contained within the vesicle membrane or contained outside the vesicle membrane.

In a specific embodiment, QS21 and 3D-MPL are present in the same final concentration per dose of the immunogenic composition i.e. the ratio of QS21:3D-MPL is 1:1. In one aspect of this embodiment, a dose of immunogenic composition comprises a final level of 25 µg of 3D-MPL and 25 µg of QS21 or 50 µg of 3D-MPL and 50 µg of QS21.

In one embodiment, the adjuvant includes an oil-in-water emulsion. In one embodiment the adjuvant comprises an oil in water emulsion, wherein the oil in water emulsion comprises a metabolisable oil, a tocol and an emulsifier. For example, the oil-in-water emulsion can include an oil phase that incorporates a metabolisable oil, and an additional oil phase component, such as a tocol. The oil-in-water emulsion may also contain an aqueous component, such as a buffered saline solution (e.g., phosphate buffered saline). In addition, the oil-in-water emulsion typically contains an emulsifier. In one embodiment, the metabolisable oil is squalene. In one embodiment, the tocol is alpha-tocopherol. In one embodiment, the emulsifier is a nonionic surfactant emulsifier (such as polyoxyethethylene sorbitan monooleate, Polysorbate® 80 , TWEEN80™). In exemplary embodiments, the oil-in-water emulsion contains squalene and alpha tocopherol in a ratio which is equal or less than 1 (w/w).

The metabolisable oil in the oil-in-water emulsion may be present in an amount of 0.5-10mg. The tocol in the oil-in-water emulsion may be present in an amount of 0.5 - 11 mg. The emulsifying agent may be present in an amount of 0.4 - 4 mg,

In order for any oil in water composition to be suitable for human administration, the oil phase of the emulsion system has to comprise a metabolisable oil. The meaning of the term metabolisable oil is well known in the art. Metabolisable can be defined as 'being capable of being transformed by metabolism' (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts, seeds, and grains are common sources of vegetable oils. Synthetic oils are also part of this invention and can include commercially available oils such as NEOBEE® (caprylic/capric triglycerides made using glycerol from vegetable oil sources and medium-chain fatty acids (MCTs) from coconut or palm kernel oils) and others. A particularly suitable metabolisable oil is squalene. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in this invention. Squalene is a metabolisable oil by virtue of the fact that it is an intermediate in the biosynthesis of cholesterol (Merck index, 10th Edition, entry no.8619).

Suitably the metabolisable oil is present in the adjuvant composition in an amount of 0.5-10 mg, preferably 1-10, 2-10, 3-9, 4-8, 5-7, or 5-6 mg (e.g. 2-3, 5-6, or 9-10mg), specifically about 5.35 mg or about 2.14 mg per dose.

Tocols are well known in the art and are described in EP0382271. Suitably the tocol is alpha-tocopherol or a derivative thereof such as alpha-tocopherol succinate (also known as vitamin E succinate). Said tocol is suitably present in an amount of 0.5-11 mg, preferably 1-11, 2-10, 3-9, 4-8, 5-7, 5-6 mg (e.g. 10-11, 5-6, 2.5-3.5 or 1-3 mg). In a specific embodiment the tocol is present in an amount of about 5.94 mg or about 2.38 mg per dose.

The oil in water emulsion further comprises an emulsifying agent. The emulsifying agent may suitably be polyoxyethylene sorbitan monooleate. In a particular embodiment the emulsifying agent may be Polysorbate® 80 (Polyoxyethylene (20) sorbitan monooleate) or Tween® 80.

Said emulsifying agent is suitably present in the adjuvant composition in an amount of 0.1-5, 0.2-5, 0.3-4, 0.4-3 or 2-3 mg (e.g. 0.4-1.2, 2-3 or 4-5 mg) emulsifying agent. In a specific embodiment the emulsifying agent is present in an amount of about 0.97 mg or about 2.425 mg.

In one embodiment, the amounts of specific components present in the composition are the amounts present in a 0.5 ml human dose. In a further embodiment, the immunogenic composition is in a volume suitable for a human dose which volume is higher than 0.5 ml, for example 0.6, 0.7, 0.8, 0.9 or 1 ml. In a further embodiment, the human dose is between 1 ml and 1.5 ml.

Where the adjuvant is in a liquid form and is to be combined with a liquid form of a polypeptide composition, the adjuvant composition in a human dose will be a fraction of the intended final volume of the human dose, for example approximately half of the intended final volume of the human dose, for example a 350 µl volume for an intended human dose of 0.7ml, or a 250 µl volume for an intended human dose of 0.5 ml. The adjuvant composition is diluted when combined with the polypeptide antigen composition to provide the final human dose of vaccine. The final volume of such dose will of course vary dependent on the initial volume of the adjuvant composition and the volume of polypeptide antigen composition added to the adjuvant composition. In an alternative embodiment, a liquid adjuvant is used to reconstitute a lyophilised polypeptide composition. In this embodiment, the human dose of the adjuvant composition is approximately equal to the final volume of the human dose. The liquid adjuvant composition is added to the vial containing the lyophilised polypeptide composition. The final human dose can vary between 0.5 and 1.5 ml.

The method of producing oil-in-water emulsions is well known to the person skilled in the art. Commonly, the method comprises mixing the tocol-containing oil phase with a surfactant such as a PBS/ polyoxyethylene sorbitan monooleate solution, followed by homogenisation using a homogenizer. It would be clear to a man skilled in the art that a method comprising passing the mixture twice through a syringe needle would be suitable for homogenising small volumes of liquid. Equally, the emulsification process in microfluidiser (M110S Microfluidics machine, maximum of 50 passes, for a period of 2 minutes at maximum pressure input of 6 bar (output pressure of about 850 bar)) could be adapted by the man skilled in the art to produce smaller or larger volumes of emulsion. The adaptation could be achieved by routine experimentation comprising the measurement of the resultant emulsion until a preparation was achieved with oil droplets of the required diameter.

In an oil in water emulsion, the oil and emulsifier should be in an aqueous carrier. The aqueous carrier may be, for example, phosphate buffered saline.

Preferably the oil-in-water emulsion systems of the present invention have a small oil droplet size in the sub-micron range. Suitably the droplet sizes will be in the range 120 to 750 nm, more preferably sizes from 120 to 600 nm in diameter. Most preferably the oil-in water emulsion contains oil droplets of which at least 70% by intensity are less than 500 nm in diameter, more preferably at least 80% by intensity are less than 300 nm in diameter, more preferably at least 90% by intensity are in the range of 120 to 200 nm in diameter.

In one embodiment, the immunogenic composition is not 3µg or 10 µg of any of SEQ ID Nos. 1 to 7 combined with an adjuvant comprising an oil in water emulsion having 0.125 mL SB62 emulsion (Total volume), 5.35 mg squalene, 5.94 mg DL-α-tocopherol and 2.425 mg polysorbate 80 per 0.5 ml dose. In one embodiment, the immunogenic composition is not 3µg or 10 µg of any of SEQ ID Nos. 1 to 7 combined with an adjuvant comprising an oil in water emulsion 5.35 mg squalene, 5.94 mg DL-α-tocopherol and 2.425 mg polysorbate 80 per 0.5 ml dose. In one embodiment, the immunogenic composition does not contain an adjuvant comprising a oil in water emulsion having squalene, DL-α-tocopherol and polysorbate 80.

### Immunogenic compositions and vaccines of the invention

In one embodiment the immunogenic composition has a volume of 0.5 to 1.5 ml.

In one embodiment the immunogenic composition further comprises additional antigens. In one embodiment the additional antigens are antigens derived from a bacterium selected from the group consisting of *S.pneumoniae, H.influenzae, N.meningitidis, E.coli, M.catarrhalis, Clostridium tetani* (tetanus), *Corynebacterium diphtheria* (diphtheria), *Bordetella pertussis* (pertussis), *S.epidermidis,* enterococci, *S.aureus,* and *Pseudomonas aeruginosa.*

In a further embodiment the immunogenic composition of the invention may comprise further antigens from *C. difficile,* for example, the S-layer proteins (WO01/73030). Optionally the immunogenic composition further comprises a saccharide from *C. difficile.*

There is further provided a vaccine comprising an immunogenic composition of the invention and a pharmaceutically acceptable excipient.
The vaccine preparations containing immunogenic compositions of the present invention may be used to protect a mammal susceptible to *C. difficile* infection or treat a mammal with a *C. difficile* infection, by means of administering said vaccine via systemic or mucosal route. These administrations may include injection via the intramuscular, intraperitoneal, intradermal or subcutaneous routes; or via mucosal administration to the oral/alimentary, respiratory, genitourinary tracts. Although the vaccine of the invention may be administered as a single dose, components thereof may also be co-administered together at the same time or at different times (for instance pneumococcal saccharide conjugates could be administered separately, at the same time or 1-2 weeks after the administration of the any bacterial protein component of the vaccine for coordination of the immune responses with respect to each other). In addition to a single route of administration, 2 different routes of administration may be used. For example, saccharides or saccharide conjugates may be administered intramuscularly (IM) or intradermally (ID) and bacterial proteins may be administered intranasally (IN) or intradermally (ID). In addition, the vaccines of the invention may be administered IM for priming doses and IN for booster doses.

The content of toxins in the vaccine will typically be in the range 1-250µg, preferably 5-50µg, most typically in the range 5 - 25µg. Following an initial vaccination, subjects may receive one or several booster immunizations adequately spaced. Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

In one aspect of the invention is provided a vaccine kit, comprising a vial containing an immunogenic composition of the invention, optionally in lyophilised form, and further comprising a vial containing an adjuvant as described herein. It is envisioned that in this aspect of the invention, the adjuvant will be used to reconstitute the lyophilised immunogenic composition.

A further aspect of the invention is a method of preventing or treating *C. difficile* infection comprising administering to the host an immunoprotective dose of the immunogenic composition or vaccine or kit of the invention. In one embodiment there is provided a method of preventing or treating primary and/or recurrence episodes of *C. difficile* infection comprising administering to the host an immunoprotective dose of the immunogenic composition or vaccine or kit of the invention.

In one embodiment of the invention there is provided an immunogenic composition or vaccine of the invention for use in the treatment or prevention of a condition or disease caused by *C.difficile. "C.difficile* disease" means a disease caused wholly or in part by *C. difficile.* In a further embodiment of the invention there is provided an immunogenic composition or vaccine of the invention for use in the treatment or prevention of a condition or disease caused wholly or in part by a strain of *C.difficile* selected from the group consisting of 078, 019, 023, 027, 033, 034, 036, 045, 058, 059, 063, 066, 075, 078, 080, 111, 112, 203, 250 and 571. Preferably the strain is strain 078.

As used herein "treatment" means the prevention of occurrence of symptoms of the condition or disease in a subject, the prevention of recurrence of symptoms of the condition or disease in a subject, the delay of recurrence of symptoms of the condition or disease in a subject, the decrease in severity or frequency of symptoms of the condition or disease in a subject, slowing or eliminating the progression of the condition and the partial or total elimination of symptoms of the disease or condition in a subject.

In a further aspect of the invention there is provided a use of an immunogenic composition or vaccine of the invention in the preparation of a medicament for the prevention or treatment of *C. difficile* disease. In a further embodiment the disease is a disease caused by a strain of *C. difficile* selected from the group consisting of 078, 019, 023, 027, 033, 034, 036, 045, 058, 059, 063, 066, 075, 078, 080, 111, 112, 203, 250 and 571. Preferably the strain is strain 078.

In a further aspect of the invention there is provided a method of preventing or treating *C. difficile* disease comprising administering the immunogenic composition of the invention or the vaccine of the invention to a mammalian subject such as a human subject. In a further embodiment the disease is a disease caused by a strain of *C. difficile* selected from the group consisting of 078, 019, 023, 027, 033, 034, 036, 045, 058, 059, 063, 066, 075, 078, 080, 111, 112, 203, 250 and 571. Preferably the strain is strain 078.

### General

"Around" or "approximately" are defined as within 10% more or less of the given figure for the purposes of the invention.

The term "comprises" means "includes." Thus, unless the context requires otherwise, the word "comprises," and variations such as "comprise" and "comprising" will be understood to imply the inclusion of a stated compound or composition (*e.g.,* nucleic acid, polypeptide, antigen) or step, or group of compounds or steps, but not to the exclusion of any other compounds, composition, steps, or groups thereof. The abbreviation, *"e.g."* is derived from the Latin exempli gratia, and is used herein to indicate a non-limiting example. Thus, the abbreviation *"e.g."* is synonymous with the term "for example."

The amino acid numbering used herein is derived from the sequences for CDTa, CDTb, Toxin A and Toxin B presented herein as SEQ ID NO: 1, SEQ ID NO: 3, SEQ ID NO: 29 and SEQ ID NO: 30, respectively, which are to be considered as reference sequences for these proteins.

Embodiments herein relating to "vaccine compositions" of the invention are also applicable to embodiments relating to "immunogenic compositions" of the invention, and vice versa.

Unless otherwise explained, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicates otherwise. The term "plurality" refers to two or more. It is further to be understood that all base sizes or amino acid sizes, and all molecular weight or molecular mass values, given for nucleic acids or polypeptides are approximate, and are provided for description. Additionally, numerical limitations given with respect to concentrations or levels of a substance, such as an antigen, may be approximate.

The invention is further disclosed in the following paragraphs:
1. An immunogenic composition comprising an isolated *Clostridium difficile* CDTb protein wherein the isolated *Clostridium difficile* CDTb protein has been mutated to modify pore forming ability.
2. The immunogenic composition of paragraph 1 wherein the isolated *Clostridium difficile* CDTb protein comprises a mutation at position 455.
3. The immunogenic composition of paragraph 1 wherein the isolated *Clostridium difficile* CDTb protein comprises a mutation at position 426 and/or a mutation at position 453.
4. The immunogenic composition of paragraph 1 wherein the isolated *Clostridium difficile* CDTb protein comprises at least one mutation selected from the group consisting of F455R, F455G, E426A and D453A.
5. The immunogenic composition of paragraph 1 wherein the isolated *Clostridium difficile* CDTb protein comprises the mutation F455G or wherein the isolated *Clostridium difficile* CDTb protein comprises the mutations E426A and D453A.
6. The immunogenic composition of any preceding paragraph wherein the isolated *Clostridium difficile* CDTb protein comprises
   (i) SEQ ID NO: 10 or SEQ ID NO: 11 or SEQ ID NO: 25 or SEQ ID NO: 26; or
   (ii) a variant of CDTb having at least 80%, 85%, 88%, 90%, 92%, 95%, 98%, 99%, 100% sequence identity to SEQ ID NO: 10 or SEQ ID NO: 11 or SEQ ID NO: 25 or SEQ ID NO: 26; or
   (iii) a fragment of CDTb having at least 30, 50, 80, 100, 120, 150, 200, 250 or 300 contiguous amino acids of SEQ ID NO: 10 or SEQ ID NO: 11 or SEQ ID NO: 25 or SEQ ID NO: 26.
7. The immunogenic composition of any one of paragraphs 1 to 5 wherein the isolated *Clostridium difficile* CDTb protein comprises
   (i) SEQ ID NO: 43 or SEQ ID NO: 44; or
   (ii) a variant of CDTb having at least 80%, 85%, 88%, 90%, 92%, 95%, 98%, 99%, 100% sequence identity to SEQ ID NO: 43 or SEQ ID NO: 44; or
   (iii) a fragment of CDTb having at least 30, 50, 80, 100, 120, 150, 200, 250 or 300 contiguous amino acids of SEQ ID NO: 43 or SEQ ID NO: 44.
8. An immunogenic composition comprising an isolated *Clostridium difficile* CDTb protein which is a truncated CDTb protein with the signal peptide and the prodomain removed and also the receptor binding domain removed and/or the CDTa binding domain removed.
9. The immunogenic composition of paragraph 8 wherein the isolated *Clostridium difficile* CDTb protein is or comprises
   (i) SEQ ID NO: 8 or SEQ ID NO: 9 or SEQ ID NO: 37;
   (ii) a variant of CDTb having at least 80%, 85%, 88%, 90%, 92%, 95%, 98%, 99%, 100% sequence identity to SEQ ID NO: 8 or SEQ ID NO: 9 or SEQ ID NO: 37; or
   (iii) a fragment of CDTb having at least 30, 50, 80, 100, 120, 150, 200, 250, 300, 350, 400, 450, 500, 550, 600, 650, 700, 750 or 800 contiguous amino acids of SEQ ID NO: 8 or SEQ ID NO: 9 or SEQ ID NO: 37.
10. An immunogenic composition comprising an isolated *Clostridium difficile* CDTb protein wherein the isolated *Clostridium difficile* CDTb protein has been mutated to modify heptamerisation ability.
11. The immunogenic composition of paragraph 10 wherein the isolated *Clostridium difficile* CDTb protein is or comprises
   (i) SEQ ID NO: 12 or SEQ ID NO: 13; or
   (ii) a variant of CDTb having at least 80%, 85%, 88%, 90%, 92%, 95%, 98%, 99%, 100% sequence identity to SEQ ID NO: 12 or SEQ ID NO: 13; or
   (iii) a fragment of CDTb having at least 30, 50, 80, 100, 120, 150, 200, 250, 300, 350, 400, 450, 500, 550, 600, 650, 700, 750, 800 or 850 contiguous amino acids of SEQ ID NO: 12 or SEQ ID NO: 13.
12. The immunogenic composition of any preceding paragraph further comprising an isolated *Clostridium difficile* CDTa protein.
13. An immunogenic composition comprising a fusion protein comprising (i) a full length CDTa protein and (ii) a CDTb protein.
14. The immunogenic composition of paragraph 13 wherein the full length CDTa protein comprises a mutation which reduces its ADP-ribosyltransferase activity.
15. The immunogenic composition of paragraph 13 or paragraph 14 wherein the full length CDTa protein comprises at least one mutation at an amino acid position selected from the group consisting of E428Q and E430Q.
16. The immunogenic composition of paragraph 15 wherein the full length CDTa protein comprises no more than one mutation from the group consisting of E428Q and E430Q.
17. An immunogenic composition according to any one of paragraphs 13-16 wherein the CDTa protein has a mutation from glutamate to glutamine at position 428.
18. An immunogenic composition according to any one of paragraphs 13-16 wherein the CDTa protein has a mutation from glutamate to glutamine at position 430.
19. The immunogenic composition of any one of paragraphs 13-18 wherein the full length CDTa protein suitably is or comprises
   (i) SEQ ID NO: 39; or
   (ii)a variant fusion protein having at least 80%, 85%, 88%, 90%, 92%, 95%, 98%, 99%, 100% sequence identity to SEQ ID NO: 39; or
   (iii) a fragment having at least 30, 50, 80, 100, 120, 150, 200, 250, 300, 350 or 400 contiguous amino acids of SEQ ID NO: 39.
20. The immunogenic composition of any one of paragraphs 13-19 wherein the CDTb protein is a full length CDTb protein.
21. The immunogenic composition of paragraph 13 wherein the fusion protein comprising a full length CDTa protein and a CDTb protein suitably is or comprises
   (i) SEQ ID NO: 14; or
   (ii)a variant fusion protein having at least 80%, 85%, 88%, 90%, 92%, 95%, 98%, 99%, 100% sequence identity to SEQ ID NO: 14; or
   (iii) a fragment having at least 30, 50, 80, 100, 120, 150, 200, 250, 300, 350 or 400 contiguous amino acids of SEQ ID NO: 14.
22. The immunogenic composition of any one of paragraphs 13 - 19 wherein the CDTb protein is a truncated CDTb protein with the CDTa binding domain removed.
23. The immunogenic composition of any one of paragraphs 13-19 and 22 wherein the truncated CDTb protein with the CDTa binding domain removed suitably is or comprises
   (i) SEQ ID NO: 9; or
   (ii)a variant fusion protein having at least 80%, 85%, 88%, 90%, 92%, 95%, 98%, 99%, 100% sequence identity to SEQ ID NO: 9; or
   (iii) a fragment having at least 30, 50, 80, 100, 120, 150, 200, 250, 300, 350 or 400 contiguous amino acids of SEQ ID NO: 9.
24. The immunogenic composition of paragraph 13 or paragraph 22 wherein the fusion protein comprising a full length CDTa protein and a truncated CDTb protein with the CDTa binding domain removed suitably is or comprises
   (i) SEQ ID NO: 15; or
   (ii)a variant fusion protein having at least 80%, 85%, 88%, 90%, 92%, 95%, 98%, 99%, 100% sequence identity to SEQ ID NO: 15; or
   (iii) a fragment having at least 30, 50, 80, 100, 120, 150, 200, 250, 300, 350 or 400 contiguous amino acids of SEQ ID NO: 15.
25. The immunogenic composition of any one of paragraphs 13-19 wherein the CDTb protein is a truncated CDTb protein comprising the receptor binding domain.
26. The immunogenic composition of paragraph 25 wherein the truncated CDTb protein comprising the receptor binding domain suitably is or comprises
   (i) SEQ ID NO: 27 or SEQ ID NO: 28; or
   (i) a variant of CDTb having at least 80%, 85%, 88%, 90%, 92%, 95%, 98%, 99%, 100% sequence identity to SEQ ID NO: 27 or SEQ ID NO: 28; or
   (iii) a fragment of CDTb having at least 30, 50, 80, 100, 120, 150 or 200 contiguous amino acids of SEQ ID NO: 27 or SEQ ID NO: 28.
27. The immunogenic composition of paragraph 13 or paragraph 25 wherein the fusion protein comprising a full length CDTa protein and a truncated CDTb protein with the CDTa binding domain removed suitably is or comprises
   (i) SEQ ID NO: 16 or SEQ ID NO: 17; or
   (ii)a variant fusion protein having at least 80%, 85%, 88%, 90%, 92%, 95%, 98%, 99%, 100% sequence identity to SEQ ID NO: 16 or SEQ ID NO: 17; or
   (iii) a fragment having at least 30, 50, 80, 100, 120, 150, 200, 250, 300, 350 or 400 contiguous amino acids of SEQ ID NO: 16 or SEQ ID NO: 17.
28. The immunogenic composition of any preceding paragraph wherein the composition elicits antibodies that neutralize CDTa or CDTb or both.
29. The immunogenic composition of any preceding paragraph wherein the composition elicits antibodies that neutralize binary toxin.
30. The immunogenic composition of any preceding paragraph wherein the immunogenic composition further comprises an isolated *Clostridium difficile* toxin A protein and/or an isolated *C. difficile* toxin B protein.
31. The immunogenic composition of paragraph 30 wherein the immunogenic composition comprises an isolated *Clostridium difficile* toxin A protein and an isolated *C. difficile* toxin B protein wherein the isolated *Clostridium difficile* toxin A protein and the isolated *C. difficile* toxin B protein form a fusion protein.
32. The immunogenic composition of paragraph 31 wherein the fusion protein is
   (i) SEQ ID NO: 32; or
   (ii) a variant having at least 80%, 85%, 90%, 95%, 98%, 99% or 100% sequence identity to SEQ ID NO: fill in; or
   (iii) a fragment of at least 800, 850, 900 or 950 contiguous amino acids of a sequence selected from the group consisting of SEQ ID NO: fill in.
33. The immunogenic composition of any preceding paragraph wherein the immunogenic composition further comprises an adjuvant.
34. The immunogenic composition of any one of the preceding paragraphs further comprising additional antigens.
35. The immunogenic composition of any one of the preceding paragraphs further comprising a saccharide from *C. difficile.*
36. A vaccine comprising the immunogenic composition of any one of the preceding paragraphs and a pharmaceutically acceptable excipient.
37. The immunogenic composition of any one paragraphs 1-35 or the vaccine of paragraph 36 for use in the treatment or prevention of *C. difficile* disease.
38. The immunogenic composition of any one of paragraphs 1-35 or the vaccine of paragraph 36 for use in the treatment or prevention of disease caused by a strain of *C. difficile* selected from the group consisting of 078, 019, 023, 027, 033, 034, 036, 045, 058, 059, 063, 066, 075, 078, 080, 111, 112, 203, 250 and 571.
39. The immunogenic composition of any one paragraphs 1-35 or the vaccine of paragraph 36 for use in the treatment or prevention of disease caused by *C. difficile* strain 078.
40. A use of the immunogenic composition of any one of paragraphs 1-35 or the vaccine of paragraph 36 in the preparation of a medicament for the prevention or treatment of *C. difficile* disease.
41. The use of paragraph 40 wherein the disease is a disease caused by a strain of *C. difficile* selected from the group consisting of 078, 019, 023, 027, 033, 034, 036, 045, 058, 059, 063, 066, 075, 078, 080, 111, 112, 203, 250 and 571.
42. The use of paragraph 40 wherein the disease is a disease caused by *C. difficile* strain 078.
43. A method of preventing or treating *C. difficile* disease comprising administering the immunogenic composition of any one of paragraphs 1-35 or the vaccine of paragraph 36 to a mammalian subject.
44. The method of paragraph 43 wherein the disease is a disease caused by a strain of *C. difficile* selected from the group consisting of 078, 019, 023, 027, 033, 034, 036, 045, 058, 059, 063, 066, 075, 078, 080, 111, 112, 203, 250 and 571.
45. The method of paragraph 43 wherein the disease is a disease caused by *C. difficile* strain 078.
46. The isolated *Clostridium difficile* CDTb protein as defined in any one of paragraphs 1-11.
47. The fusion protein comprising (i) a full length CDTa protein and (ii) a CDTb protein as defined in any one of paragraphs 13-27.

All references or patent applications cited within this patent specification are incorporated by reference herein in their entirety.
In order that this invention may be better understood, the following examples are set forth. These examples are for purposes of illustration only, and are not to be construed as limiting the scope of the invention in any manner.

### EXAMPLES

The AS01B adjuvant referred to is an adjuvant having 50 µg QS21 presented in the form of a liposome, 50 µg 3D-MPL, 0.25 mg cholesterol and 1.0 mg DOPC per 0.5ml dose. A dose of 50µl suitable for immunizing mice contains 5µg QS21, 5µg 3D-MPL, 0.025mg cholesterol and 0.1mg DOPC.

### Example 1: Design of binary toxin antigens

The Binary Toxin (other name: ADP-ribosyltransferase toxin) is composed by two components: the enzymatic component, named CDTa and the transport and binding component, named CDTb.

Based on literature data and information available for components of other bacterial binary toxins, CDTb could be divided into five domains. The first one is the prodomain, its cleavage by an enzyme having a chymotrypsin activity allows the heptamerisation of the mature protein. The second domain allows the binding to CDTa. The third and fourth ones are involved in the oligomerisation and membrane insertion. Finally, the last domain is the host cell receptor binding domain.

### CDTb mature

In order to avoid the chymotrypsin activation step in the CDTb process, it was tried to express only the mature CDTb protein (without its signal peptide and prodomain).

In the literature (Protein Expression and Purification, 2010, vol. 74 : 42-48), the mature CDTb was described as starting at Leucine 210 of SEQ ID NO: 3. This mature CDTb was named CDTb".

Other experimental data suggest that the activated CDTb starts at Serine 212 of SEQ ID NO: 3. This result was supported by analysis of a 3D modelised structure of CDTb. This model was built using SwissModel (Bioinformatics, 2006, vol. 22 : 195-201). The template used for the homology modelling was the B component of *Bacillus anthracis,* named Protective Antigen or PA (Protein Data Bank accession number: 3TEW).

### CDTb receptor-binding domain alone

The 3D homology structure model obtained for CDTb using the PA antigen from *B. anthraxis* is accurate for the four first domains of CDTb but not for the receptor-binding domain (these domains of CDTb and PA are too different). To design constructs expressing the receptor-binding domain alone, the C-terminal part of the fourth domain was analysed on the 3D structure model in order to decide where the last domain will start.

Two versions of the CDTb-receptor binding domain were designed. In the first one, this domain starts just after the modelised 3D structure of the fourth domain. In this version, the CDTb-receptor-binding domain will probably have a long flexible peptide in its N-terminal part. The second version of the receptor-binding domain starts where the 2D predicted structures performed on the C-terminal part of CDTb (predictions done using Psipred program, Bioinformatics, 2000, vol. 16 : 404-405) become more compacts after a lack of predicted secondary structures. This could indicate the beginning of a new structural domain. In this second version, no flexible peptide is present at the N-terminal part of the isolated CDTb receptor-binding domain.

### CDTb Ca²⁺ binding motif mutation

Following literature information, mutations in the Ca²⁺ binding domain of the B component of lota toxin of *Clostridium perfringens* (lb) abolish the binding with the A component of this binary toxin (la). These mutations could be very interesting in the case of a vaccine composition containing a mixture of mature CDTb protein and a wild type CDTa protein.

Using multiple protein sequence alignment, these mutations were located on the CDTb sequence and mutated. It concerns residues Asp²²⁰ , Asp²²² and Asp²²⁴ of SEQ ID NO: 3. They were mutated into Ala residues.

### CDTb prodomain

In order to try to decrease the degradation issues observed with construct C55 (CDTb with the prodomain removed) in gel, some co-expression tests were evaluated. The working hypothesis of doing that is to improve the folding of the mature CDTb.

Two limits of prodomain were proposed. The first one starts at residue 43 of CDTb of SEQ ID NO: 3 (after the signal peptide cleavage) and finishes at residue Met²¹¹ (given that the experimentally determined first residue of the mature CDTb is Ser²¹²). The second prodomain was designed based on the predicted 3D structure of CDTb. The linker existing between the prodomain and the first structural domain of the mature CDTb protein is removed in this construct.

### Example 2 - Cloning, expression and purification of C. difficile CDTb protein

### Expression plasmid and recombinant strain: C37 and C55 (as set out in Table A).

Genes encoding the truncated protein of CDTb without signal peptide (Pro-CDTb', C37) and a His tag in C-term were cloned into the pGEX-6p1 expression vector (GE Healthcare) using the BamHl/Xhol restriction sites using standard procedures. This vector included GST (Gluthathione-S-transferase) as fusion partner in N-terminal of CDTb' (GST-Pro-CDTb'). The final construct was generated by the transformation of *E*. *coli* strain BL21 (DE3) with the recombinant expression vector according to standard method with CaCl2-treated cells (Hanahan D. « Plasmid transformation by Simanis. » In Glover, D. M. (Ed), DNA cloning. IRL Press London. (1985): p. 109-135.).

Genes encoding the truncated protein of CDTb without signal peptide and prodomain (CDTb": C55) and a His tag in C-term were cloned into the pET24b(+) expression vector (Novagen) using the Ndel/Xhol restriction sites using standard procedures. Final constructs were generated by the transformation of *E*. *coli* B834 (DE3) modified strain with the appropriate recombinant expression vectors according to standard method with CaCl2-treated cells (Hanahan D. « Plasmid transformation by Simanis. » In Glover, D. M. (Ed), DNA cloning. IRL Press London. (1985): p. 109-135.).

### Host Strain

BL21 (DE3). BL21 (DE3) is a non-methionine auxotroph derivative of B834. Strains having the designation (DE3) are lysogenic for a λ prophage that contains an IPTG inducible T7 RNA polymerase. λ DE3 lysogens are designed for protein expression from pET vectors This strain is also deficient in the *lon* and *ompT* proteases.

Genotype : *E.coli* BL21 ::DE3 strain, F⁻ *ompT hsdS*_{B}(r_{B}⁻ m_{B}⁻) *gal dcm* (*DE3*).

B834 is the parental strain for BL21. These protease-deficient hosts are methionine auxotrophs. λ DE3 lysogens are designed for protein expression from pET vectors This strain is also deficient in the *lon* and *ompT* proteases.

Modification: Including PGL gene to avoid phosphogluconoylation in the biotin locus (Strain is auxotroph for biotin).
Genotype : B834 ::DE3 strain, F- *ompT* hsdSB(rB - mB-) *gal dcm met* (DE3)
Modification : Δ(bioA-bioD)::PGL

### Expression of the recombinant proteins:

*E.coli* transformants were stripped from agar plate and used to inoculate 200 ml of LBT broth ± 1% (w/v) glucose +/- kanamycin (50 µg/ml) or ampicillin (100 µg/ml) to obtain O.D.₆₀₀ₙₘ between 0.1 -0.2. Cultures were incubated overnight at 37 °C, 250 RPM.

Overnight culture were diluted to 1:20 in 500 ml of LBT medium containing +/- kanamycin (50 µg/ml) or ampicillin (100 µg/ml) and grown at 37°C at a stirring speed of 250 rpm until O.D.₆₂₀ reached 0.5/0.6.

At an O.D.at 600nm of around 0.6, cultures were cooled down before inducing the expression of the recombinant protein by addition of 1 mM isopropyl β-D-1-thiogalactopyranoside (IPTG; EMD Chemicals Inc., catalogue number: 5815) and incubated overnight at 23 °C, 250 RPM.

After the overnight inductions (around 16 hours), O.D. at 600nm were evaluated after induction and cultures were centrifuged at 14 000 RPM for 15 minutes and pellets were frozen at -20°C separately.

### Purification

### C37 (SEQ ID NO: 5)

The bacterial pellet was re-suspended in 50mM bicine buffer (pH 8.0) containing 500mM NaCl , 5mM TCEP (Thermo Scientific Pierce, (2-carboxyethyl)phosphine hydrochloride) and a mixture of protease inhibitor (Complete, Roche). Bacteria were lysed using a French Press system 3 X 20 000 PSI. Soluble (supernatant) and insoluble (pellet) components were separated by centrifugation at 20 000g for 30 min at 4°C.

The 6-His tagged-protein was purified under native conditions on IMAC. The soluble components were loaded on a 5ml GE Histrap column (GE) pre-equilibrated with the same buffer used to bacterial re-suspension. After loading on the column, the column was washed with a 50mM bicine buffer pH8.0, containing 150mM NaCl, 25mM imidazole, 1mM TCEP. Elution was performed using a 50mM bicine buffer pH8.0 containing 150mM NaCl, 250mM imidazole, 1mM TCEP.

After desalting step (BIORAD Bio-Gel P6 Desalting) in 50mM bicine buffer pH8.0 containing 150mM NaCl and 1mM TCEP, the product was treated (overnight at 4°C) with PreScission protease (GE-Healthcare) in order to cleave the GST tag. After overnight treatment, 0.2% Tween 20 was added to the digestion mixture.

Then the protein was passed through a GST affinity column (GE GSTrap FF) pre-equilibrated with buffer 50mM bicine buffer pH8.0 containing 150mM NaCl , 1mM TCEP, 0.2% tween20 and 20mM reduced glutation, in order to remove the cleaved tag, un-cleaved fusion protein and the PreScission protease.

The GST-free protein was collected in the flow through and loaded again on a 5ml GE Histrap column (GE) pre-equilibrated with 50mM bicine buffer pH8.0 containing 150mM NaCl, 1mM TCEP, 0.2% tween20. After loading on the column, the column was washed with a 50mM bicine buffer pH8.0, containing 150mM NaCl, 0.2% tween20, 1mM TCEP and 10mM imidazole. Elution was performed using a 50mM bicine buffer pH8.0 containing 150mM NaCl, 0.2% tween20, 1mM TCEP and 500mM imidazole.

After desalting step (BIORAD Bio-Gel P6 Desalting) in 50mM bicine buffer pH8.0 containing 150 mM NaCl, 1mM TCEP and 0.2% tween 20, the product was treated with α-chymotrypsin (from bovine pancreas - Sigma), followed by trypsin inhibitor treatment (from egg white-Sigma). The complete activation of CDTb by chymotrypsin was monitored by SDS-PAGE.

The fully activated product was loaded on SEC chromatography (SUPERDEX™ 75) in 50mM bicine buffer pH8.0 containing 300mM NaCl, 1mM TCEP. Fractions containing CDTb antigen were selected on the basis of purity by SDS-PAGE. Protein concentration was determined using Lowry RC/DC Protein Assay of BioRad. The purified bulk was sterile-filtered on 0.22 µm and stored at -80°C.

### C55 (SEQ ID NO: 7)

The bacterial pellet was re-suspended in 50mM bicine buffer (pH 8.0) containing 150 mM NaCl,5mM TCEP (Thermo Scientific Pierce, (2-carboxyethyl) phosphine hydrochloride), 0.4% empigen and a mixture of protease inhibitors (Complete, Roche). Bacteria were lysed using a French Press system 3 X 20 000 PSI. Soluble (supernatant) and insoluble (pellet) components were separated by centrifugation at 20 000g for 30 min at 4°C.

The 6-His tagged-protein was purified under native conditions on IMAC. The soluble components were loaded on a 5ml GE Histrap column (GE) pre-equilibrated with 50mM bicine buffer (pH 8.0) containing 150mM NaCl , 0.15% empigen, 1mM TCEP. After loading on the column, the column was washed with a 50mM bicine buffer pH8.0, containing 150 mM NaCl, 0.2% tween 20, 20mM imidazole and 1mM TCEP. Elution was performed using a 50mM bicine buffer pH8.0 containing 150mM NaCl, 0.2% tween 20, 500mM imidazole and 1mM TCEP.

After desalting step (BIORAD Bio-Gel P6 Desalting) in 50mM bicine buffer pH8.0 containing 300mM NaCl, 1mM TCEP the product was loaded on SEC chromatography (SUPERDEX™ 75) in the same buffer. Fractions containing CDTb antigen were selected on the basis of purity by SDS-PAGE. Protein concentration was determined using Lowry RC/DC Protein Assay of BioRad. The purified bulk was sterile-filtered on 0.22 µm and stored at -80°C.

### Example 3: Cloning, expression and purification of CDTa protein

### Expression plasmid and recombinant strain: CDTa full length (C34, C44, C49, C50, C54, C67, C68, C69, C107, C108, C110 as set out in Table A)

Genes encoding the protein of full length without signal peptide of CDTa with and without mutations (see Table A) and a His tag in C-term were cloned into the pET24b(+) expression vector (Novagen) using the Ndel/Xhol restriction sites using standard procedures. Final constructs were generated by the transformation of *E. coli* strain HMS174 (DE3) or BLR (DE3) pLysS (C34) with each recombinant expression vector separately according to standard method with CaCl2-treated cells (Hanahan D. « Plasmid transformation by Simanis. » In Glover, D. M. (Ed), DNA cloning. IRL Press London. (1985): p. 109-135.).

### Host strain:

HMS 174 (DE3). HMS174 strains provide the *recA* mutation in a K-12 background. Strains having the designation (DE3) are lysogenic for a λ prophage that contains an IPTG inducible T7 RNA polymerase. λ DE3 lysogens are designed for protein expression from pET vectors **Genotype:** F⁻ *recA1 hsdR*(r_{K12}⁻ m_{K12}⁺) (Rif^{R}).

BLR(DE3) pLysS. BLR is a recA derivative of BL21. Strains having the designation (DE3) are lysogenic for a λ prophage that contains an IPTG inducible T7 RNA polymerase. λ DE3 lysogens are designed for protein expression from pET vectors This strain is also deficient in the *lon* and *ompT* proteases, pLysS strains express T7 lysozyme which further suppress basal expression of the T7 RNA polymerase prior to induction.

Genotype : *E.coli* BLR::DE3 strain, F⁻ *ompT* hsdS_{B}(r_{B}⁻ m_{B}⁻) *gal dcm (DE3)* Δ(srl-recA)306::Tn *10* pLysS (Cam^{R}, Tet^{R}).

### Expression of the recombinant proteins:

*E.coli* transformants were stripped from agar plate and used to inoculate 200 ml of LBT broth ± 1% (w/v) glucose + kanamycin (50 µg/ml) to obtain O.D.600nm between 0.1 -0.2. Cultures were incubated overnight at 37 °C, 250 RPM.

Each overnight culture were diluted to 1:20 in 500 ml of LBT medium containing kanamycin (50 µg/ml) and grown at 37°C at a stirring speed of 250 rpm until O.D.620 reached 0.5/0.6.

At O.D.600nm around 0.6, the cultures were cooled down before inducing the expression of the recombinant protein by addition of 1 mM isopropyl β-D-1-thiogalactopyranoside (IPTG; EMD Chemicals Inc., catalogue number: 5815) and incubated overnight at 23 °C, 250 RPM.

After overnight induction (around 16 hours), O.D.₆₀₀ₙₘ were evaluated after induction and culture were centrifuged at 14 000 RPM for 15 minutes and pellets were frozen at -20°C separately.

### Expression plasmid and recombinant strain: CDTa- N-term (C49 and C50 as set out in Table A)

Genes encoding protein of N-terminal, without signal peptide of CDTa (see Table A) and a His tag in C-term were cloned into the pET24b(+) expression vector (Novagen) using the Ndel/Xhol restriction sites using standard procedures. Final constructs were generated by the transformation of *E. coli* strain HMS174 (DE3) with each recombinant expression vectors separately according to standard method with CaCl2-treated cells (Hanahan D. « Plasmid transformation by Simanis. » In Glover, D. M. (Ed), DNA cloning. IRL Press London. (1985): p. 109-135.).

### Host strain:

HMS 174 (DE3). HMS174 strains provide the *recA* mutation in a K-12 background. Strains having the designation (DE3) are lysogenic for a λ prophage that contains an IPTG inducible T7 RNA polymerase. λ DE3 lysogens are designed for protein expression from pET vectors **Genotype:** F⁻ *recA1 hsdR*(r_{K12}⁻ m_{K12}⁺) (Rif^{R}).

### Expression of the recombinant proteins:

*E.coli* transformants were stripped from agar plate and used to inoculate 200 ml of LBT broth ± 1% (w/v) glucose + kanamycin (50 µg/ml) to obtain O.D.600nm between 0.1 -0.2. Cultures were incubated overnight at 37 °C, 250 RPM.

This overnight culture was diluted to 1:20 in 500 ml of LBT medium containing kanamycin (50 µg/ml) and grown at 37°C at a stirring speed of 250 rpm until O.D.620 reached 0.5/0.6.

At O.D.600nm around 0.6, the culture was cooled down before inducing the expression of the recombinant protein by addition of 1 mM isopropyl β-D-1-thiogalactopyranoside (IPTG; EMD Chemicals Inc., catalogue number: 5815) and incubated overnight at 23 °C, 250 RPM.

After overnight induction (around 16 hours), O.D.₆₀₀ₙₘ was evaluated after induction and culture was centrifuged at 14 000 RPM for 15 minutes and pellets were frozen at -20°C separately.

### Purification

The following procedure was used to purify constructs C34, C44, C49, C50, C54, C67, C69, C107 and C110.
The bacterial pellets were re-suspended in 20mM or 50mM bicine buffers (pH 7.5 or pH 8.0), containing 500mM NaCl , 0mM or 5mM TCEP (Thermo Scientific Pierce, (2-carboxyethyl)phosphine hydrochloride) and a mixture of protease inhibitors (Complete, Roche, without EDTA). Bacteria were lysed using a French Press system 3 X 20 000 PSI. Soluble (supernatant) and insoluble (pellet) components were separated by centrifugation at 20 000g for 30 min at 4°C.

The 6-His tagged-proteins were purified under native conditions on IMAC. The soluble components were loaded on a 5ml GE Histrap column (GE) pre-equilibrated with the same buffer used to bacterial re-suspension. After loading on the column, the column was washed with a 20mM or 50mM bicine buffer (pH7.5 or pH8.0), containing 500mM NaCl, 10mM imidazole, 5mM TCEP. Elution was performed using a 50mM bicine buffer pH7.6, 500mM NaCl, 1mM TCEP and imidazole (250mM or 500mM).

After desalting (BIORAD Bio-Gel P6 Desalting) and concentration (Amicon Ultra 10kDa) steps, the product was loaded on SEC chromatography (SUPERDEX™ 75 or 200) in 20mM or 50mM bicine buffer (pH7.5 or pH8.0), 150mM NaCl, 1mM TCEP, for further purification step.

Fractions containing CDTa antigen were selected on the basis of purity by SDS-PAGE. Protein concentration was determined using Lowry RC/DC Protein Assay of BioRad. The purified bulk was sterile-filtered on 0.22 µm and stored at -80°C.

### Example 4: Design, cloning, expression and purification of C. difficile CDTb protein with modified pore forming ability.

A first strategy was evaluated in order to decrease the cytotoxicity observed for CDTb alone: avoid the possibility for CDTb of modifying its structure in an endosome in order to form a pore into the membrane of this endosome. The two strategies followed in this approach were based on publication data available for B components coming from Binary toxin of *Bacillus anthracis* (C123 and C149) and *Clostridium botulinium* (C126, C152, C164 and C166).The following CDTb proteins were designed:

| Name | Length(aa)* | Location | Description | SEQ ID |
|---|---|---|---|---|
| C123 | | | pGEX-CDTb - F455R-His | SEQ ID NO:10 |
| C126 | | | pGEX-CdtB-E426A-D453A | SEQ ID NO: 11 |
| C149 | 841 | 43-876 of CDTb of SEQ ID NO: 3 | pET24b-Cdtb - F455R-His | SEQ ID NO: 25 |
| C152 | 841 | 43-876 of CDTb of SEQ ID NO: 3 | pET24b-Cdtb-E426A-D453A-His | SEQ ID NO: 26 |
| C164 | 672 | 212-876 of CDTb of SEQ ID NO: 3 | pET24b-Cdtb-w/o prdomain-F455R-His | SEQ ID NO: 43 |
| C166 | 672 | 212-876 of CDTb of SEQ ID NO: 3 | pET24b-Cdtb-w/o prodomain-E426A-D453A-His | SEQ ID NO: 44 |

| | | | | |
|---|---|---|---|---|
| * Takes into account the start Met codon and the His-tag | | | | |

### Cloning and production

Histag in C-term/ cloning in pGEX-6p1 vector in BamH1-Xho1 site / Stains B834(DE3): genotype: E.coli BL21::DE3 strain, F- ompT hsdSB(rB- mB-) gal dcm (DE3).

B834 is the parental strain for BL2. These protease-deficient hosts are methionine auxotrophs. λ DE3 lysogens are designed for protein expression from pET and pGEX vectors This strain is also deficient in the Ion and ompT proteases. Selection antibiotic: Ampicillin: 50µg/ml/ production o/n at 16C

### Purification (C123, C126, C149 and C152):

.French Press with lysis buffer: 50mM Bicine-500mM NaCl, - 5mMTCEP , Complete- pH8.0 Ni-NTA GE His trap 5ml (profinia) + Desalting
Equilibrated : 50mM Bicine - 500mM NaCl , 1mMTCEP - pH8.0
Wash:50mM Bicine - 150mM NaCl ,1mMTCEP - imidazole 0mM pH8.0
Elution: 50mM Bicine - 150mM NaCl , 1mMTCEP - imidazole 250mM pH8.0
Desalting: 50mMBicine-150mMNaCl-1mMTCEP pH8.0
Prescission Clivage (GST tag):
   +375µl (500UI/250µl) : 750 UI
   ON at 4°C + add Tween 20 0.2% final conc.
   GSTrap FF (1ml)
   Equilibrated buffer: 50mM Bicine - 150mM NaCl- 1mMTCEP - 0.2%Tween20 pH8.0
   Keep the FT
   Wash buffer: same as equil buffer
   Elution buffer: 50mM Bicine - 150mM NaCl- 1mMTCEP- 0.2%Tween 20 -250mM Imidazole, -pH8.0
   IMAC GE 5ml + Desalting
   Equilibrated buffer: 50mM Bicine - 150mM NaCl- 1mMTCEP 0.2%Tween20, -pH8.0
   Wash buffer: 50mM Bicine - 150mM NaCl- 1mMTCEP- 10mM imidazole 0.2%Tween20 , - pH8.0
   Elution buffer: 50mM Bicine - 150mM NaCl- 1 mMTCEP- 250mM Imidazole - 0.2%Tween 20, -pH8.0
   Desalting buffer: 50mM Bicine - 150mM NaCl- 1mMTCEP - 0.2%Tween20, -pH8.0 RC/DC dosage
Activation : Chymotrypsin treatment:
   100µg chymotrypsin (1µg/µl) active 1mg of protein
   (42mg + 3.6mg of chymotrypsin in 12.5ml final vol)
   50 min at RT
   + inactivation : chymotrypsin inhibitor 12.6mg
   SEC Superdex 200
   Equilibrated: 50mM Bicine - 150mM NaCl- 1mMTCEP, -pH8.0
   Concentration on IMAC GE 1ml + Desalting
   Equilibrated buffer: 50mM Bicine - 150mM NaCl- 1mMTCEP, -pH8.0
   Wash buffer: 50mM Bicine - 150mM NaCl- 1 mMTCEP, -pH8.0
   Elution buffer: 50mM Bicine - 150mM NaCl- 1 mMTCEP- 250mM Imidazole, -pH8.0
   Desalting buffer: 50mM Bicine - 150mM NaCl- 1mMTCEP, -pH8.0
   Filtration 0.22µm, RC/DC dosage

Purification of C164 was carried out in accordance with the following scheme 1:

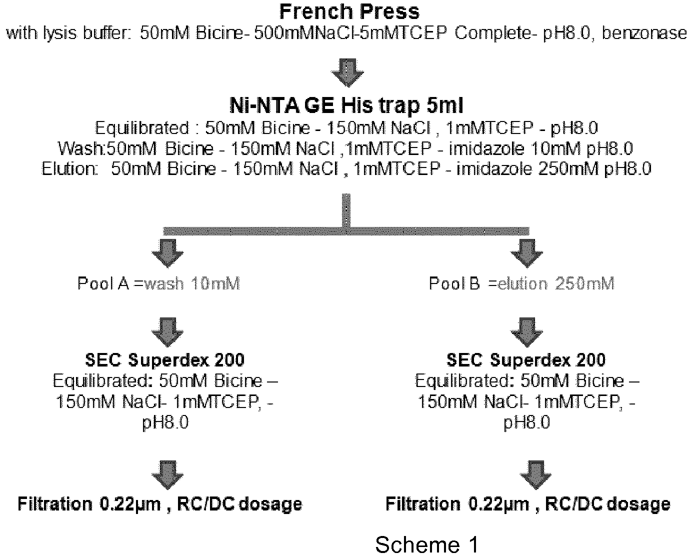

Purification of C166 was carried out in accordance with the following scheme 2:

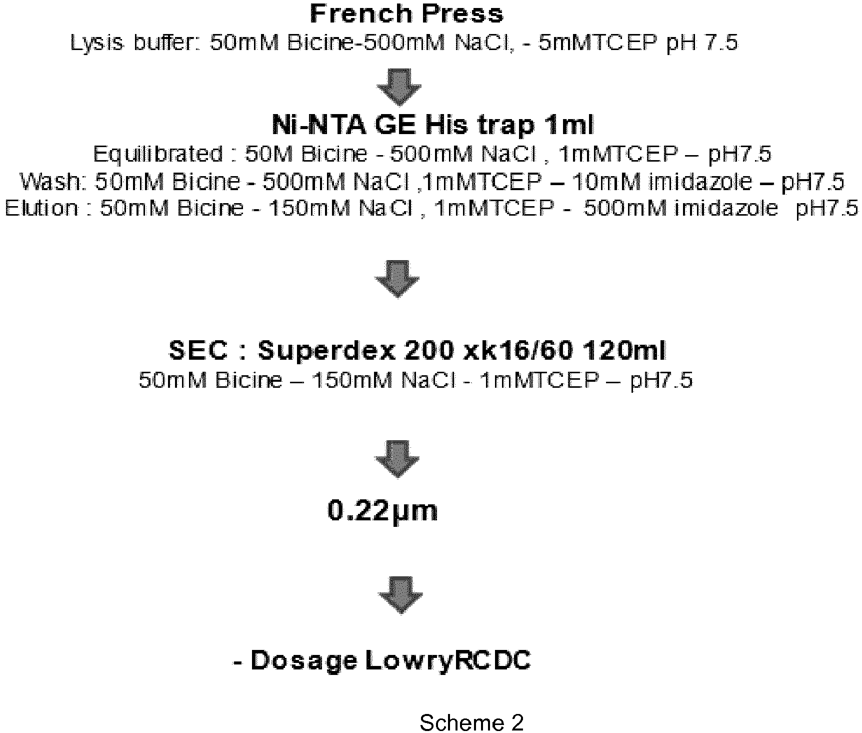

### Characterisation:

Dynamic light scattering is used to evaluate hydrodynamic radius in solution of CDTb purified proteins, in addition to provide information about homogeneity and to detect presence of high molecular weight aggregates within a protein sample. This is based on the calculation of the diffusion coefficient of the different species that are obtained by measuring the light scattering fluctuation, which depends on protein molecular size and shape, and on the other minor constituents of the sample.

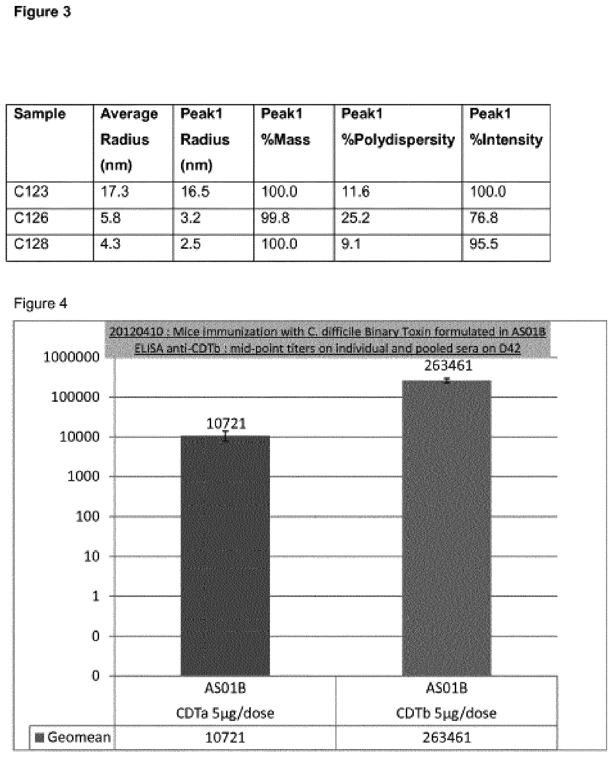

Protein samples were analysed on a Dynapro plate reader (Wyatt technology), using five acquisition of 15 seconds at 25°C
a- Buffer: 50mM Bicine- 150mM NaCl- 1mMTCEP, -pH8.0
Results are shown in Figure 3.

### Example 5: Design, cloning, expression and purification of C. difficile CDTb protein with the signal peptide and the prodomain removed and also the receptor binding domain removed and/or the CDTa binding domain removed.

The rational of expressing the different structural domains of CDTb alone was to understand which one leads to degradation and aggregation issues. Two domains were evaluated using this strategy: the receptor-binding domain and the CDTa-binding domain were removed on construct C116 and C117, respectively. The "oligomerisation" and "membrane insertion" domains of CDTb were not expressed separately because they are potentially structurally related. The following CDTb proteins were designed:

| Name | Length(aa)* | Location | Description | SEQ ID |
|---|---|---|---|---|
| C116 | 414 | 212-616 of CDTb of SEQ ID NO: 3 | pET24b-CDTb ΔRBD-His | SEQ ID NO:8 |
| C117 | 588 | 296-876 of CDTb of SEQ ID NO: 3 | pET24b-CDTb ΔCDTa bdg-His | SEQ ID NO: 9 |
| C118 | 330 | 296-616 of CDTb of SEQ ID NO: 3 | pET24b-CDTb-ΔRBD-ΔCDTa bdg-His | SEQ ID NO:37 |

| | | | | |
|---|---|---|---|---|
| *Takes into account the start Met codon and the His-tag | | | | |

### Cloning and production

Histag in C-term/ cloning in pET24b vector in Nde1-Xho1 site / Stains B834(DE3): genotype: E.coli BL21::DE3 strain, F- ompT hsdSB(rB- mB-) gal dcm (DE3).

B834 is the parental strain for BL2. These protease-deficient hosts are methionine auxotrophs. λ DE3 lysogens are designed for protein expression from pET vectors This strain is also deficient in the Ion and ompT proteases. Selection antibiotic: Kanamycin: 50µg/ml/ production o/n at 16C

### Purification and Characterisation

**C116**
**French Press**
   Lysis buffer: 50mM Bicine-500mM NaCl, - 5mMTCEP , Complete- pH8.0
**Ni-NTA GE His trap 5ml**
   Equilibrated **: 8M Urea** - 50mM Bicine - 500mM NaCl, 1mMTCEP - pH8.0
   Wash: **8M urea** -50mM Bicine - 500mM NaCl ,1mMTCEP - 5mM imidazole - pH8.0
**Refoldind on column His trap**
   Gardient : 100min at 1ml/min : 8M -50mM Bicine - 150mM NaCl - 1mM TECP - pH 8.0 1mMTCEP to 0M Urea 50mM Bicine - 150mM NaCl - 1mM TECP - pH 8.0
**Elution**
   50mM Bicine - 150mM NaCl , 1mMTCEP - imidazole 500mM pH7.5
**SEC : Superdex 200**
   50mM Bicine - 150mM NaCl - 1mMTCEP - pH7.5
**0.22µm** - **Dosage LowryRCDC**
**C117**
**French Press**
   with lysis buffer: 50mM Bicine-500mM NaCl, - 5mMTCEP , Complete- pH8.0
**Ni-NTA GE His trap 5ml (profinia)**
   Equilibrated : 50mM Bicine - 500mM NaCl , 1mMTCEP - pH8.0
   Wash:50mM Bicine - 500mM NaCl ,1mMTCEP - imidazole 0mM pH8.0
   Elution: 50mM Bicine - 150mM NaCl , 1mMTCEP - imidazole 500mM pH7.5
**SEC Superdex 200**
   Equilibrated: 50mM Bicine - 150mM NaCl- 1mMTCEP, -pH7.5
**Filtration 0.22µm** , **RC/DC dosage**

### Example 6: Design, cloning, expression and purification of C. difficile CDTb protein with modified heptamerisation ability

A second strategy was used in order to decrease the cytotoxicity observed for CDTb alone : avoid the heptamerisation of CDTb. A 3D model of a heptameric complex of CDTb were designed. Using this model, one loop was identified as potentially interacting between monomers. Two approaches were evaluated : the remove of this loop (C127) or the mutation of residues contained in this loop that are probably involved in the interaction between monomer (C128)The following proteins were designed:

| Name | Length(aa)* | Location | Description | SEQ ID |
|---|---|---|---|---|
| C127 | 834 | 43-876 of CDTb of SEQ ID NO: 3 | pET24b-Truncated CDTb-His | SEQ ID NO: 12 |
| C128 | 841 | 43-876 of CDTb of SEQ ID NO:3 | pET24b-mutated CDTb-His | SEQ ID NO: 13 |

| | | | | |
|---|---|---|---|---|
| *Takes into account the start Met codon and the His-tag | | | | |

### Cloning and production

Histag in C-term/ cloning in pET24b vector in Nde1-Xho1 site / Stains B834(DE3): genotype: E.coli BL21::DE3 strain, F- ompT hsdSB(rB- mB-) gal dcm (DE3).

B834 is the parental strain for BL2. These protease-deficient hosts are methionine auxotrophs. λ DE3 lysogens are designed for protein expression from pET vectors This strain is also deficient in the Ion and ompT proteases. Selection antibiotic: Kanamycin: 50µg/ml/ production o/n at 16C.

### Purification and Characterisation

French Press
   with lysis buffer: 50mM Bicine-150mM NaCl- 0.4% Empigen- 5mMTCEP - Complete- pH8.0 Ni-NTA GE His trap 1ml (profinia)
   Equilibrated : 50mM Bicine - 150mM NaCl -0.15% Empigen - 1mMTCEP - pH8.0
   Wash: 50mM Bicine - 150mM NaCl - 0.2% Tween 20 - 1mMTCEP - imidazole 10mM pH8.0
   Elution: 50mM Bicine - 150mM NaCl - 0.2% Tween 20 - 1mMTCEP - imidazole 500mM pH8.0
   Desalting G-25: 50mM Bicine - 150mM NaCl - 0.2% Tween 20 - 1 mMTCEP pH8.0
Chymotrypsin activation
   SEC : Superdex 200 XK16/60 (120ml)
   Equilibrated: 50mM Bicine - 150mM NaCl - 1mMTCEP, -pH8.0

   Concentration: GE-His Trap (1ml) + Desalting
   50mM Bicine - 150mM NaCl- 1mMTCEP, -pH8.0
0.22µm - Dosage LowryRCDC

### Characterization:

Dynamic light scattering is used to evaluate hydrodynamic radius in solution of CdtB purified proteins, in addition to provide information about homogeneity and to detect presence of high molecular weight aggregates within a protein sample. This is based on the calculation of the diffusion coefficient of the different species that are obtained by measuring the light scattering fluctuation, which depends on protein molecular size and shape, and on the other minor constituents of the sample.

Protein samples were analysed on a Dynapro plate reader (Wyatt technology), using five acquisition of 15 seconds at 25°C
a- Buffer: 50mM Bicine - 150mM NaCl- 1mMTCEP, -pH8.0

### Example 7: Cytotoxicity of CDTb proteins on HT29 cells.

The chymotrypsin-activated CDTb sub-units (C37) alone appear cytotoxic on HT29 cells. This suggests that the pore-forming could be responsible of the cytotoxicity.

A second generation of antigen design has been produced in order to inhibit the pore forming step by disrupting the hydrophobic interactions between beta-sheets of individual proteins.

This binary toxin subunit B (binding cells part) of binary toxins was added alone or mixed with binary toxin subunit A (enzymatic part) on Human colonic epithelial cells (HT29 cells) to evaluate their residual cytotoxicity.

The cytotoxicity of candidates C123, C126, C128, C164, C166, C116, C117, C149 and C152 was tested on HT29 cell lines.

### Binary toxin cytotoxicity assay

Human colonic epithelial cells (HT29 cells) were cultured at 37°C with 5% CO2 in DMEM + 10% fetal bovine serum + 1% glutamine + 1% antibiotics (penicillin-streptomycin-amphotericin) and were seeded in 96-well black tissue culture plates (Greiner Bio-one, Ref:655090) at a density of 4 10³ cells/well for HT29.
After 24h, 50µl of the cell media was removed from the wells.

The binary toxin candidates to characterize were diluted in order to reach 2µg/ml of CDTa and 6 µg/ml of CDTb. Further three-fold dilutions were made in microplate (NUNC, Ref: 163320). 50 µl of serial dilutions of binary toxin preparations were added to the black plates and the microplates were incubated at 37°C with 5% CO2 for 6 days.

After 6 days, the mix of binary toxin and medium were removed from the well and 100µl of Hoechst stain (BD Pharmingen, Ref. 561908) diluted 1:500 in phosphate buffer saline (PBS) was added in each well for 2 hours in the dark at room temperature.

After coloration, the Hoechst stain was removed from the wells and the cells fluorescence cells was measured using an Axiovision microscope.

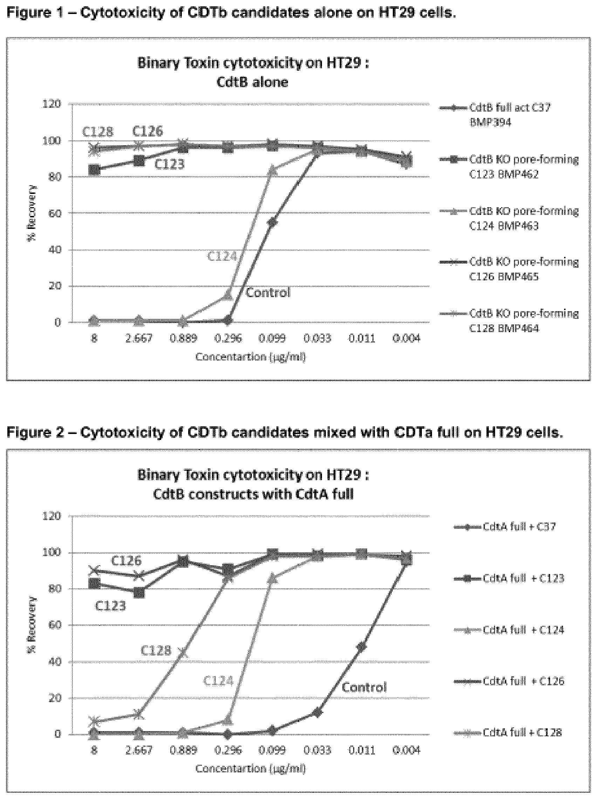

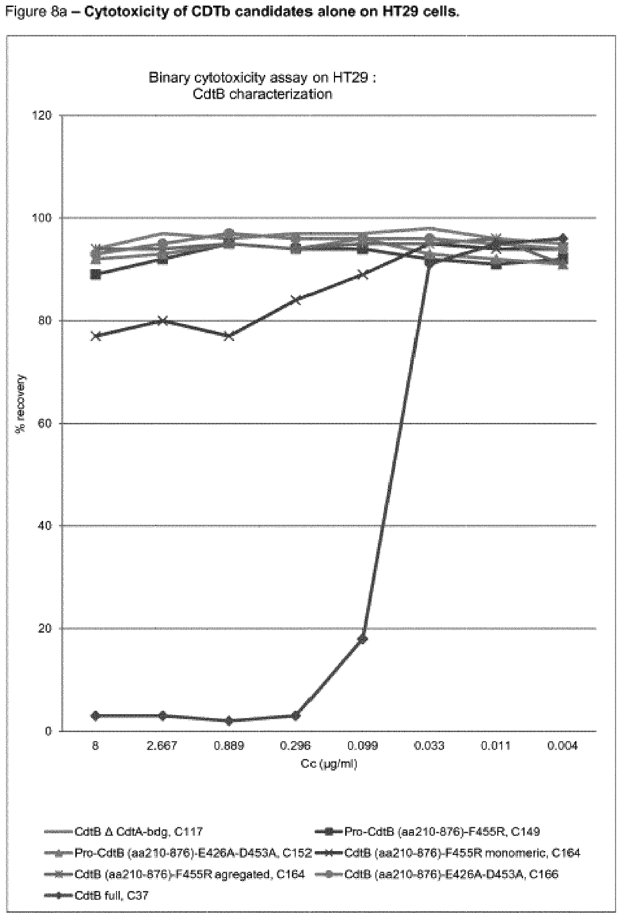

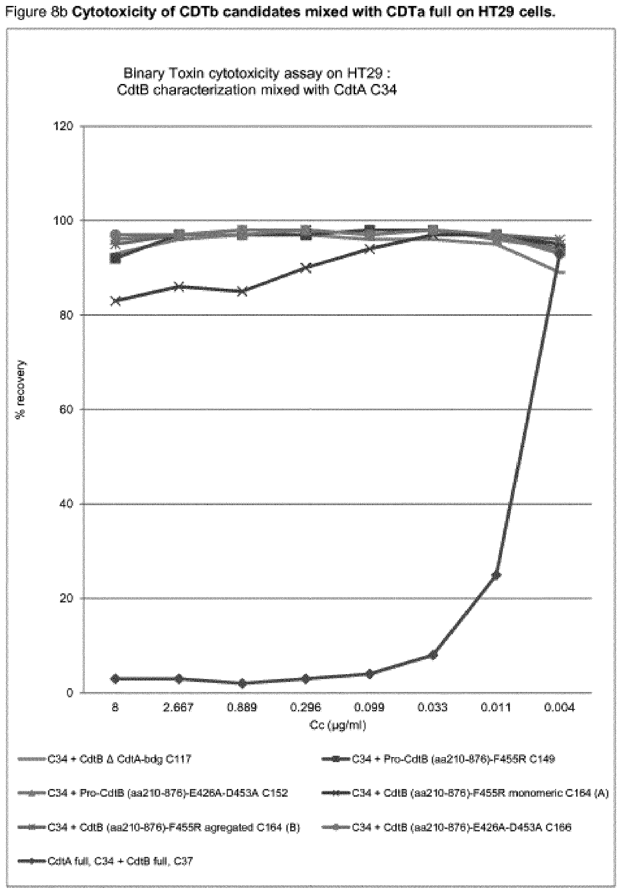

The cytotoxicity is expressed in percentage of surface covered by fluorescent staining in each well. No cytotoxic (100% of recovery) effect is obtained with cells alone. And Higher level of cytotoxicity is obtained with binary toxin full activated (CDTa C34+ CDTb C37).
Results are shown in Figure 1, Figure 2 and Figure 8.

### Example 8: Analysis of CDTb proteins by DLS

Dynamic light scattering (DLS) was used to evaluate hydrodynamic radius in solution of CDTb purified proteins C123, C126 and C128, in addition to provide information about homogeneity and to detect presence of high molecular weight aggregates within a protein sample. This is based on the calculation of the diffusion coefficient of the different species that are obtained by measuring the light scattering fluctuation, which depends on protein molecular size and shape, and on the other minor constituents of the sample.

The following protein samples were analysed on a Dynapro plate reader (Wyatt technology), using five acquisition of 15 seconds at 25°C.
All proteins are in a buffer composed of 50mM Bicine - 150mM NaCl- 1mMTCEP, -pH8.0. Measurements were performed on the purified bulk kept at 4°C that had not been frozen.

As shown on figure 3, both C126 KO pore forming construct and C128 KO heptamerisation construct have a hydrodynamic radius of the main population around 3nm that might be coherent with a monomer. The C123 KO heptamerisation construct is found as a homogeneous high molecular weight oligomer or aggregate, with a hydrodynamic radius of 15,7nm.
The fact that average hydrodynamic radius of the total samples are close to the radius of the main populations, together with their low polydispersity, suggest that both C123, C126 and C128 his tagged purified proteins have an homogeneous size distribution.

### Example 9: Design, cloning, expression and purification of C. difficile CDTb fusion protein comprising a full length CDTa protein and a CDTb protein

There are two big advantages for a fusion of CDTa and CDTb: the first one is only one process to obtain 2 proteins. The second one : CDTa is easier than CDTb to produce -> Putting the CDTa as first partner of the fusion could potentially have a positive effect on the entire processability of the fusion. In C139, both mature CDTa (without its signal peptide) and CDTb (without its signal peptide and without its prodomain) proteins were fused.

In C139 the CDTa partner is mutated at position 428 (a Glutamate is mutated into a Glutamine) in order to mutate the cytotoxic activity of CDTa, and the CDTa partner of this fusion is mutated in position 45 (a Cys was mutated into a Tyr residue) in order to avoid an observed dimerisation of CDTa.

In C145, the CDTa part of the fusion is the same as for C139. the CDTa-binding domain of CDTb partner was removed in order to potentially avoid interaction between this domain and the CDTa partner. Between both partners a linker/spacer (6 Gly residues) was added in order to improve the structural flexibility between partners and allow a independent correct folding of the two proteins.

The receptor-binding domain of CDTb has a very good expression yield, is homogenous but less immunogenic than CDTb mature. We tried to increase the immunogenicity of this domain by fusing it to the mature CDTa partner. Two different limits were designed for this receptor-binding domain so 2 fusions were evaluated. In C155 and C156 the CDTa partner is the same as for C139 and C145. In C156, between both partners of the fusion, a spacer/linker was added (6 Gly residues). This linker was not needed in the C155 construct because the receptor-binding domain of CDTb used in C155 begins by a long un-structured and flexible peptide that could be used as linker/spacer.

The following fusion proteins were designed:

| Name | Length(aa)* | Location | Description | SEQ ID |
|---|---|---|---|---|
| C139 | 1094 | CDTa: 44-463 of SEQ ID NO: 1 CDTb: 212-876 of SEQ ID NO: 3 | pET24b-CdtA-E428Q-Cdtb mature-his | SEQ ID NO: 14 |
| C145 | 1016 | CDTa: 44-463of SEQ ID NO: 1CDTb: 296-876 of SEQ ID NO: 3 | pET24b-CdtA-E428Q-Cdtb ΔCDTa bdg-His | SEQ ID NO: 15 |
| C155 | 686 | CDTa: 44-463 of SEQ ID NO: 1 CDTb: 620-876 of SEQ ID NO: 3 | pET24b-CdtA-E428Q-Cdtb RBD long | SEQ ID NO: 16 |
| C156 | 676 | CDTa: 44-463 of SEQ ID NO: 1 CDTb: 636-876 of SEQ ID NO: 3 | pET24b-CdtA-E428Q-Cdtb RBD short | SEQ ID NO: 17 |

| | | | | |
|---|---|---|---|---|
| * Takes into account the start Met codon and the His-tag | | | | |

### Cloning and production

As for Example 5.

### Example 10: Molecular weight evaluation of CDTb and CDTa-CDTb fusion constructions

Analytical ultracentrifugation may be used to determine the homogeneity and size distribution in solution of the different species within a protein sample by measuring the rate at which molecules move in response to a centrifugal force. This is based on the calculation of the coefficients of sedimentation of the different species that are obtained by sedimentation velocity experiment, which depend on their molecular shape and mass.
1. Protein samples are spun in a Beckman-Coulter ProteomeLab XL-1 analytical ultracentrifuge at 8000RPM, 25000RPM or 42000RPM depending of the target protein size, after the AN-60Ti rotor had been equilibrated to 15°C.
2. For data collection, scans are recorded at 280nm every 5 minutes.
3. Data analysis is performed using the program SEDFIT for determination of the C(S) distribution. Determination of the partial specific volume of the proteins is performed with the SEDNTERP software from their amino acid sequence. Sednterp may also be used to determine the viscosity and the density of the buffer.
4. The molecular weight of the different species may be determined from the C(S) distribution plot (concentration vs sedimentation coefficient), considering that it's a better representation of the raw data than the C(M) distribution (concentration vs molecular weight) to characterize the size distribution of a mixture.

### Example 11: Immunisation of mice with C. difficile CDTa and CDTb sub-units proteins in a AS01B formulation.

### Mice immunisation

Groups of 25 female Balb/C mice were immunized IM at days 0, 14 and 28 with 5 µg of full CDTa and CDTb binary toxin purified sub-units. These antigens were injected in an AS01B formulation.

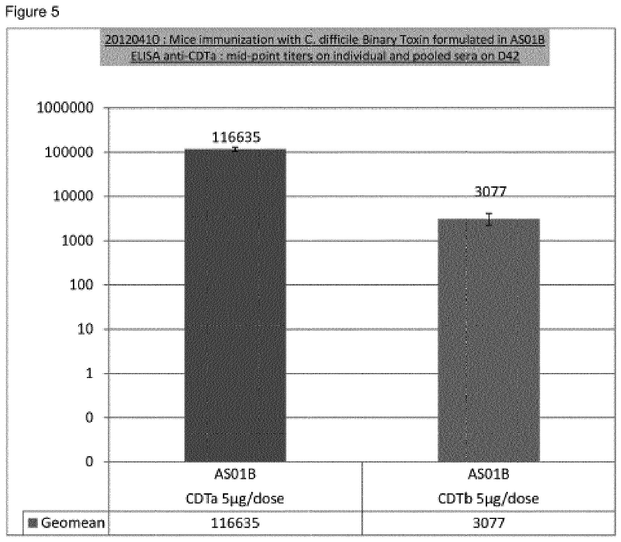

Anti-CDTa and anti-CDTb ELISA titres were determined in individual sera collected at day 42 (Post III 14). Results are shown in Figures 4-5.

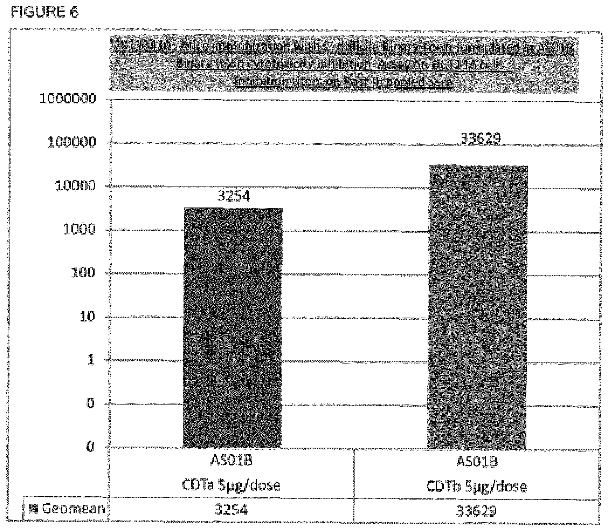

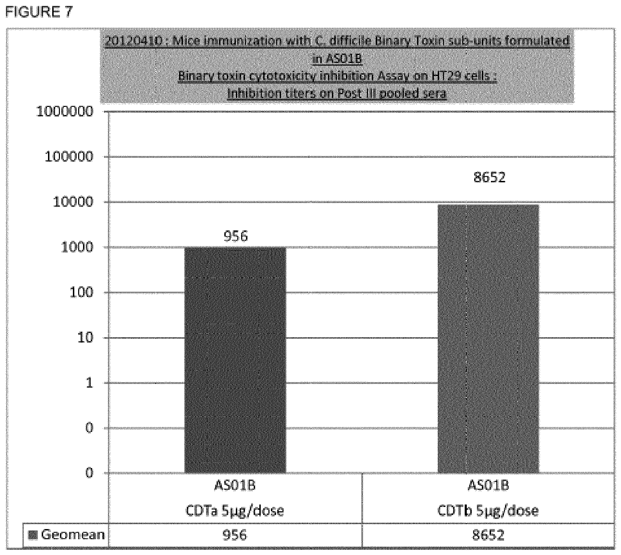

A binary toxin cytotoxicity inhibition assay was also performed on pooled Post III sera (day42). Results are shown in Figures 6-7

### Anti-CDTa and anti-CDTb ELISA response: Protocol

Full CDTa (C34) or full CDTb (C37)sub-units were coated at 1µg/ml (for CDTa) or 2µg/ml (for CDTb) in phosphate buffered saline (PBS) on high-binding microtitre plates (Nunc MAXISORP™), overnight at 4° C. The plates were blocked with PBS-BSA 1% for 30 min at RT with agitation. The mice anti-sera are prediluted 1/500 in PBS-BSA0.2%-TWEEN™ 0.05%. and then, further twofold dilutions were made in microplates and incubated at RT for 30 min. After washing, bound mouse antibody was detected using Jackson ImmunoLaboratories Inc. peroxidase-conjugated Anti-Mouse (ref: 115-035-003) diluted 1:5000 in PBS-BSA0.2%-tween 0.05%. The detection antibodies were incubated for 30 min. at room temperature (RT) with agitation. The colour was developed using 4 mg O-phenylenediamine (OPD) + 5 µl H₂O₂ per 10 ml pH 4.5 0.1M citrate buffer for 15 minutes in the dark at room temperature. The reaction was stopped with 50 µl HCl, and the optical density (OD) was read at 490 nm relative to 620 nm.

The level of anti-CDTa or anti-CDTb antibodies are expressed in mid-point titres. A GMT was calculated for the 25 samples in each treatment group.

### Binary toxin cytotoxicity inhibition assay

Human colonic epithelial cells (HT29 or HCT-116 cells) were cultured at 37°C with 5%CO₂ in DMEM +10% fetal bovine serum +1% glutamine +1% antibiotics (penicillin-streptomycin-amphotericin) and were seeded in 96-well black tissue culture plates (Greiner Bio-one, Ref : 655090) at a density of 4.10⁴ cells/well for HT29 and 1.10⁴cells/well for HCT116.
After 24h, the cell media was removed from the wells.

The mice anti-sera were prediluted 1:50 in cell media and then, further three-fold dilutions were made in microplate (NUNC, Ref: 163320). 50µl of serial dilutions of mice pooled antisera were added to the black plates. 50µl of a mix of CDTa (25ng/ml) and chymotrypsin-activated CDTb (75 ng/ml) were then added and the black plates incubated at 37°C with 5% CO₂ for 6 days.

After 6 days, the mix of antisera and toxin were removed from the wells and 100µl of Hoescht stain (BD Pharmingen, Ref : 561908) diluted 1:500 in phosphate buffer saline (PBS) was added in each well for 2 hours in the dark at room temperature.

After coloration, the Hoescht stain was removed from the wells and the cells fluorescence cells was measured using an Axiovision microscope.

The surface covered by fluorescent staining was determined in each well and cytotoxicity inhibition titres were defined as the reciprocal dilution inducing a 50% inhibition of the fluorescent signal.

### Example 12: Cytotoxicity of CDTb proteins on HCT116 cells

The chymotrypsin-activated CDTb sub-units (C37) alone appear cytotoxic on HCT116 cells. This suggests that the pore-forming could be responsible of the cytotoxicity.

A second generation of antigen design has been produced in order to inhibit the pore forming step by disrupting the hydrophobic interactions between beta-sheets of individual proteins.

This binary toxin subunit B (binding cells part) of binary toxins was added alone or mixed with binary toxin subunit A (enzymatic part) on Human colonic epithelial cells (HCT116) to evaluate their residual cytotoxicity.

The cytotoxicity of candidates C123, C126, C128, C164, C166, C116, C117, C149 and C152 was tested on HCT116 cell lines.

### Binary toxin cytotoxicity assay

Human colonic epithelial cells (HCT116 cells) were cultured at 37°C with 5% CO2 in DMEM + 10% fetal bovine serum + 1% glutamine + 1% antibiotics (penicillin-streptomycin-amphotericin) and were seeded in 96-well black tissue culture plates (Greiner Bio-one, Ref:655090) at a density of 10³ cells/well for HCT116.
After 24h, 50µl of the cell media was removed from the wells.

The binary toxin candidates to characterize were diluted in order to reach 2µg/ml of CDTa and 6 µg/ml of CDTb. Further three-fold dilutions were made in microplate (NUNC, Ref: 163320). 50 µl of serial dilutions of binary toxin preparations were added to the black plates and the microplates were incubated at 37°C with 5% CO2 for 6 days.

After 6 days, the mix of binary toxin and medium were removed from the well and 100µl of Hoechst stain (BD Pharmingen, Ref. 561908) diluted 1:500 in phosphate buffer saline (PBS) was added in each well for 2 hours in the dark at room temperature.

After coloration, the Hoechst stain was removed from the wells and the cells fluorescence cells was measured using an Axiovision microscope.

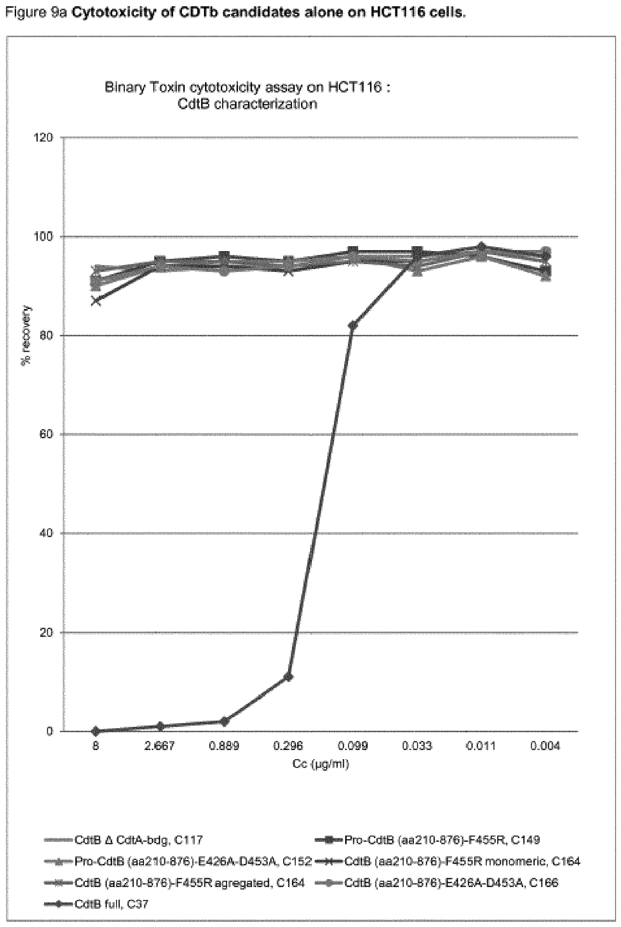

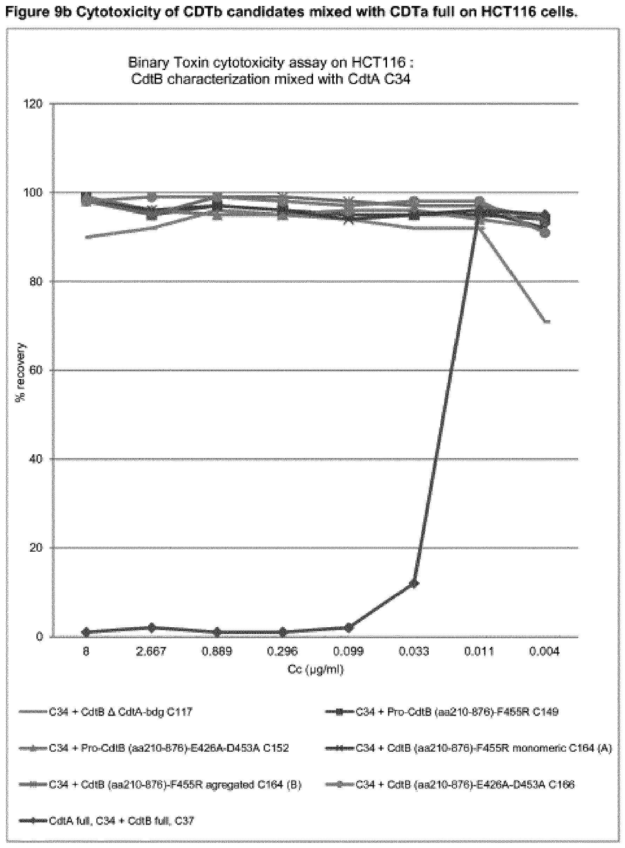

The cytotoxicity is expressed in percentage of surface covered by fluorescent staining in each well. No cytotoxic (100% of recovery) effect is obtained with cells alone. And Higher level of cytotoxicity is obtained with binary toxin full activated (CDTa C34+ CDTb C37).
Results are shown in Figure 9

### Example 13: Cytotoxicity of CDTa-CDTb fusion proteins on HT29 cells

The cytotoxicity of fusions C139, C145, C155 and C156 was tested on HT29 cell lines. The potential residual cytotoxicity associated with the CdtB sub-unit was evaluated by spiking with full CdtA sub-unit. The potential residual cytotoxicity associated with the CdtA sub-unit was evaluated by spiking with full CdtB sub-unit. Full length CDTa and full length CDTb were used as controls.

### Binary toxin cytotoxicity assay

Human colonic epithelial cells (HT29 cells) were cultured at 37°C with 5% CO2 in DMEM + 10% fetal bovine serum + 1% glutamine + 1% antibiotics (penicillin-streptomycin-amphotericin) and were seeded in 96-well black tissue culture plates (Greiner Bio-one, Ref:655090) at a density of 4 10³ cells/well for HT29.
After 24h, 50µl of the cell media was removed from the wells.

The binary toxin candidates to characterize were diluted in order to reach 2µg/ml of CDTa and 6 µg/ml of CDTb. Further three-fold dilutions were made in microplate (NUNC, Ref: 163320). 50 µl of serial dilutions of binary toxin preparations were added to the black plates and the microplates were incubated at 37°C with 5% CO2 for 6 days.

After 6 days, the mix of binary toxin and medium were removed from the well and 100µl of Hoechst stain (BD Pharmingen, Ref. 561908) diluted 1:500 in phosphate buffer saline (PBS) was added in each well for 2 hours in the dark at room temperature.

After coloration, the Hoechst stain was removed from the wells and the cells fluorescence cells was measured using an Axiovision microscope.

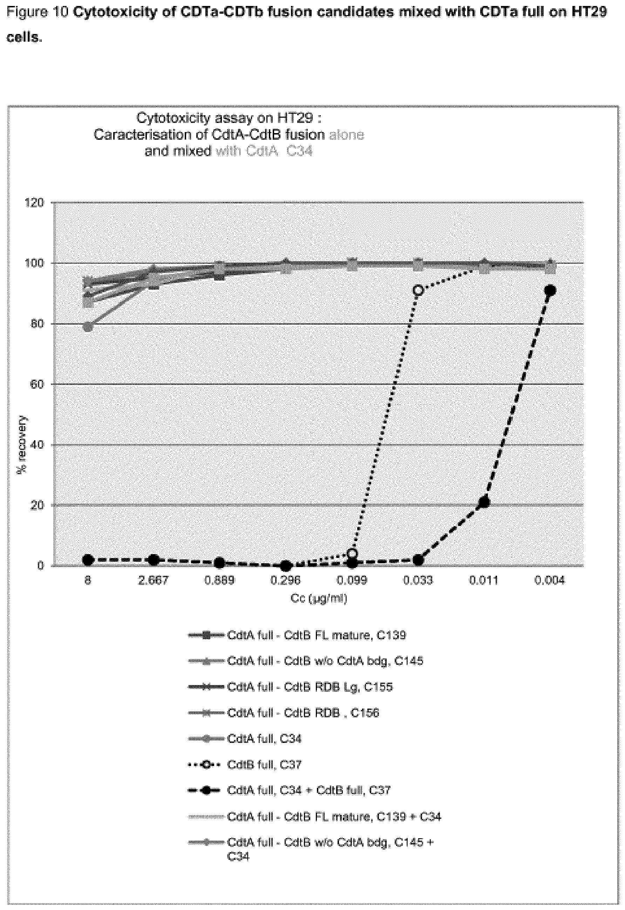

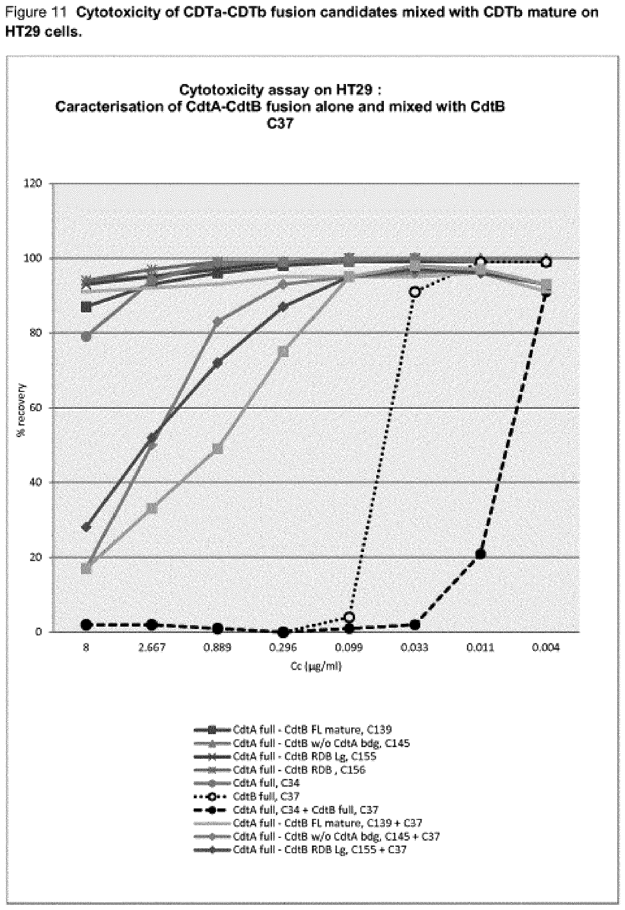

The cytotoxicity is expressed in percentage of surface covered by fluorescent staining in each well. No cytotoxic (100% of recovery) effect is obtained with cells alone. And Higher level of cytotoxicity is obtained with binary toxin full activated (CDTa C34+ CDTb C37).
None of the fusions were cytotoxic and there was no observed residual cytotoxicity associated with the CDTb sub-unit of the fusions. There was no observed residual cytotoxicity associated with the CDTa sub-unit in the CdtA full-CdtB full fusion (C139). A very low residual cytotoxicity associated with the CDTasub-unit was observed with the 3 fusions not including full lenth CdtB (C145, C155, C156).
Results are shown in Figures 10 and 11.

### Example 14: Immunisation of mice with C. difficile CDTb proteins or CDTa-CDTb fusions in AS01B formulation.

### Mice immunisation

Groups of 12 female Balb/C mice were immunized IM at days 0,14 and 28 with 1 µg of CDTa and CDTb binary toxin purified sub-units and with 2 µg of CDTa-CDTb fusions. These antigens were injected in an AS01B formulation.

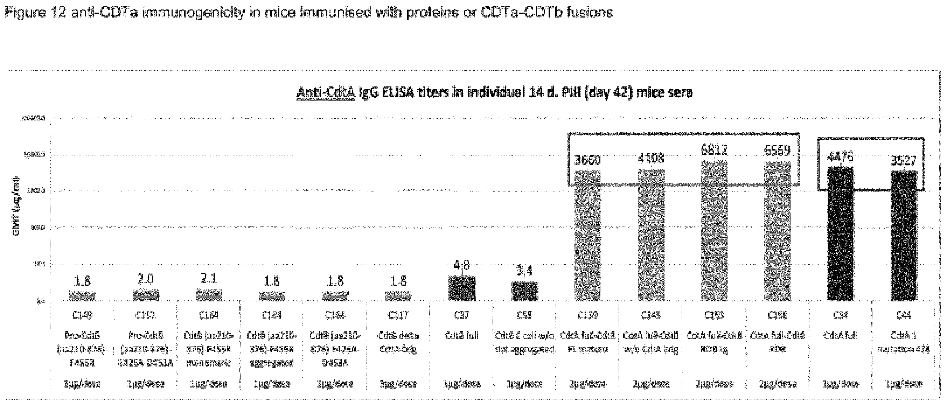

Anti-CDTa and anti-CDTb ELISA titres were determined in individual sera collected at day 42 (Post III 14). Results are shown in Figures 12-13.

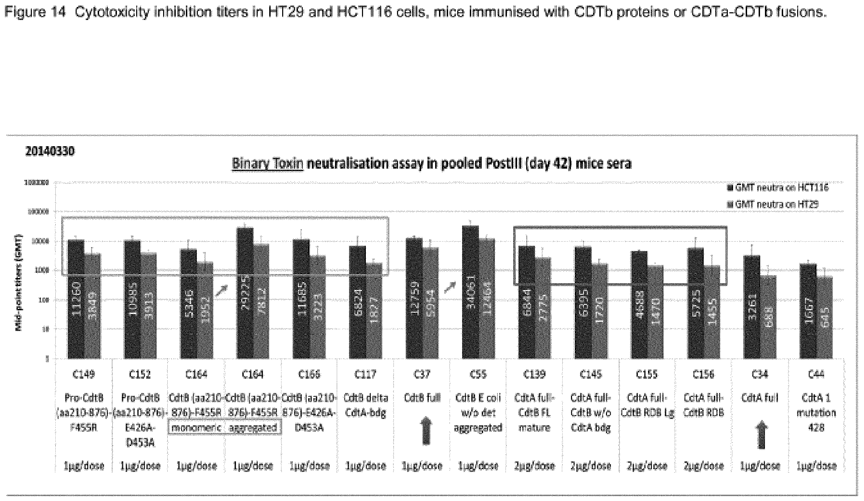

A binary toxin cytotoxicity inhibition assay was also performed on pooled Post III sera (day42) on HT29 and HCT116 cell lines. Results are shown in Figure 14.

### Anti-CDTa and anti-CDTb ELISA response: Protocol

E-428 mutated CDTa (C44) or non activated CDTb (C46)sub-units were coated at 1µg/ml in phosphate buffered saline (PBS) on high-binding microtitre plates (Nunc MAXISORP™), overnight at 4° C. The plates were blocked with PBS-BSA 1% for 30 min at RT with agitation. The mice anti-sera are prediluted 1/500 in PBS-BSA0.2%-TWEEN™ 0.05%. and then, further twofold dilutions were made in microplates and incubated at RT for 30 min. After washing, bound mouse antibody was detected using Jackson ImmunoLaboratories Inc. peroxidase-conjugated Anti-Mouse (ref: 115-035-003) diluted 1:5000 in PBS-BSA0.2%-tween 0.05%. The detection antibodies were incubated for 30 min. at room temperature (RT) with agitation. The colour was developed using 4 mg O-phenylenediamine (OPD) + 5 µl H₂O₂ per 10 ml pH 4.5 0.1M citrate buffer for 15 minutes in the dark at room temperature. The reaction was stopped with 50 µl HCl, and the optical density (OD) was read at 490 nm relative to 620 nm.

The level of anti-CDTa or anti-CDTb antibodies is expressed in mid-point titers. A GMT was calculated for the 12 samples in each treatment group.

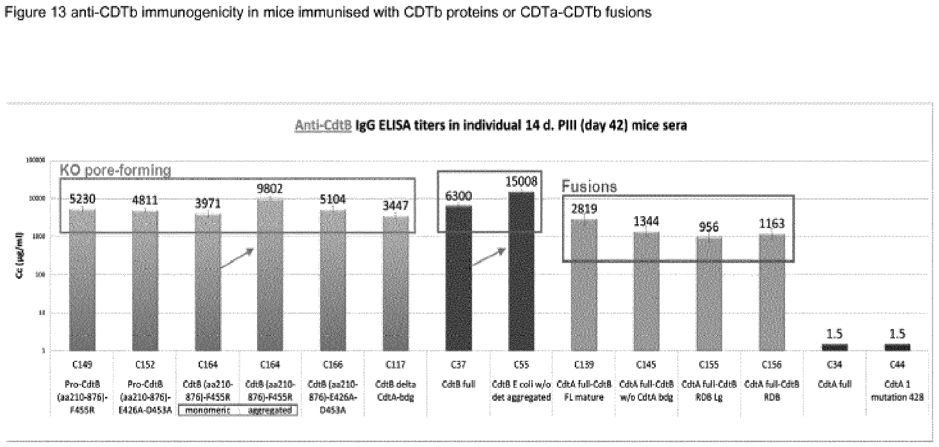

The anti-CdtA antibody titers achieved are shown in Figure 12.
The anti-CdtB antibody titers achieved are shown in Figure 13

### Example 15: CDTb and CDTa-CDTb fusion cytotoxicity inhibition assay

### Binary toxin cytotoxicity inhibition assay

Human colonic epithelial cells (HT29 or HCT-116 cells) were cultured at 37°C with 5%CO₂ in DMEM +10% fetal bovine serum +1% glutamine +1% antibiotics (penicillin-streptomycin-amphotericin) and were seeded in 96-well black tissue culture plates (Greiner Bio-one, Ref : 655090) at a density of 4.10³ cells/well for HT29 and 1.10³cells/well for HCT116.
After 24h, the cell media was removed from the wells.

The mice anti-sera were prediluted 1:50 in cell media and then, further three-fold dilutions were made in microplate (NUNC, Ref: 163320). 50µl of serial dilutions of mice pooled antisera were added to the black plates. 50µl of a mix of CDTa (25ng/ml) and chymotrypsin-activated CDTb (75 ng/ml) were then added and the black plates incubated at 37°C with 5% CO₂ for 6 days.

After 6 days, the mix of antisera and toxin were removed from the wells and 100µl of Hoescht stain (BD Pharmingen, Ref : 561908) diluted 1:500 in phosphate buffer saline (PBS) was added in each well for 2 hours in the dark at room temperature.

After coloration, the Hoescht stain was removed from the wells and the cells fluorescence cells was measured using an Axiovision microscope.

The surface covered by fluorescent staining was determined in each well and cytotoxicity inhibition titres were defined as the reciprocal dilution inducing a 50% inhibition of the fluorescent signal.
Results are shown in Figure 14

**Sequence Summary (Table A)**

| **Description** | **Construct reference** | **Amino acid sequence** | **Polynucleotide sequence** |
|---|---|---|---|
| CDTa full length (strain R20291) | N/A | SEQ.I.D.NO:1 | SEQ.I.D.NO:2 |
| CDTb full length (strain R20291) | N/A | SEQ.I.D.NO:3 | SEQ.I.D.NO:4 |
| CDTb' (minus signal peptide) ligated to Glutathione-S-transferase protein.(GST underlined) | C37 | SEQ.I.D.NO:5 | SEQ.I.D.NO:6 |
| CDTb with prodomain removed (CDTb", aa212-876) | C55 | SEQ ID NO:7 | |
| CDTb with prodomain and receptor binding domain removed (corresponding to aa 212-616 of SEQ ID NO: 3), includes histag | C116 | SEQ ID NO: 8 | |
| CDTb with prodomain and CDTa binding domain removed (corresponding to aa296-876 of SEQ ID NO: 3), includes histag | C117 | SEQ ID NO: 9 | |
| CDTb (corresponding to SEQ ID NO: 3) mut -F455R, Histag | C123 | SEQ ID NO: 10 | |
| CDTb sequence (corresponding to SEQ ID NO: 3) in which residues 423 (Glutamate) and 453 (Aspartate) are both mutated into alanine. | C126 | SEQ ID NO: 11 | |
| CDTb (corresponding to aa 43-876 of SEQ ID NO: 3) in which aa 532 to aa 542 are deleted and replaced by 3 Gly residues | C127 | SEQ ID NO: 12 | |
| CDTb (corresponding to aa43-876 SEQ ID NO: 3) mut -D537G-E539G-D540G-K541G, Histag | C128 | SEQ ID NO: 13 | |
| Fusion CdtA-E428Q-Cdtb mature, histag (CDTa: aa44-463 of SEQ ID NO: 1; CDTb: aa212-876 of SEQ ID NO: 3) | C139 | SEQ ID NO: 14 | |
| Fusion CdtA-E428Q-Cdtb withCdtA bdg domain removed,Histag (CDTa: aa44-463 of SEQ ID NO: 1; CDTb: aa296-876 of SEQ ID NO: 3) | C145 | SEQ ID NO: 15 | |
| Fusion CdtA-E428Q-Cdtb RBD long (CDTa: aa44-463 of SEQ ID NO: 1; CDTb: aa620-876 of SEQ ID NO: 3) | C155 | SEQ ID NO: 16 | |
| Fusion CdtA-E428Q-Cdtb RBD short (CDTa: aa44-463 of SEQ ID NO: 1; CDTb: aa636-876 of SEQ ID NO: 3) | C156 | SEQ ID NO: 17 | |
| CDTa linker (aa44-268 SEQ ID NO: 1) | C49 | SEQ ID NO: 18 | |
| CDTa without linker (aa44-260 SEQ ID NO:1) | C50 | SEQ ID NO: 19 | |
| CDTa N terminal domain (residue 44 to residue 240) | Gulke et al 2001 | SEQ ID NO: 20 | |
| CDTa (aa44-463 SEQ ID NO:1) mut E428Q-E430Q | C67 | SEQ ID NO: 21 | |
| CDTa (aa44-463 SEQ ID NO: 1) mut R345A-Q350A-N385A-R402A-S388F-E428Q-E430Q | C69 | SEQ ID NO: 22 | |
| CDTa (aa44-463 SEQ ID NO: 1) mut R345A-Q350A-N385A-R402A-S388F | C107 | SEQ ID NO: 23 | |
| CDTa (aa44-463 SEQ ID NO: 1) mut R345A-Q350A-N385A-R402A-S388F-E430Q | C108 | SEQ ID NO: 24 | |
| CDTb with prodomain, mut F455R | C149 | SEQ ID NO: 25 | |
| CDTb with prodomain, mutE426A-D453A | C152 | SEQ ID NO: 26 | |
| CDTb receptor binding domain with linker in N-term of sequence, from aa 620-876 SEQ ID NO: 3 | C52 | SEQ ID NO: 27 | |
| CDTb receptor binding domain without linker in N-term of sequence, from aa 636-876SEQ ID NO: 3 | C53 | SEQ ID NO: 28 | |
| Toxin A | | SEQ ID NO: 29 | |
| Toxin B | | SEQ ID NO: 30 | |
| Fusion 1 | F1 | SEQ ID NO: 31 | |
| Fusion 2 | F2 | SEQ ID NO: 32 | |
| Fusion 3 | F3 | SEQ ID NO: 33 | |
| Fusion 4 | F4 | SEQ ID NO: 34 | |
| Fusion 5 | F5 | SEQ ID NO: 35 | |
| CDTa (aa44-463 of SEQ ID NO: 1) | C34 | SEQ ID NO: 36 | |
| truncated CDTb protein with the signal peptide, prodomain, receptor binding domain and CDTa binding domain removed, corresponding to aa296 to aa 616 of SEQ ID NO: 3 | C118 | SEQ ID NO: 37 | |
| CDTb' (minus signal peptide) | C46 | SEQ ID NO: 38 | |
| CDTa (aa44-463 SEQ ID NO:1) mut E428Q | C44 | SEQ ID NO: 39 | |
| CDTa (aa44-463 SEQ ID NO:1) mut E430Q | C54 | SEQ ID NO: 40 | |
| CDTa (aa44-463 SEQ ID NO: 1) mut R345A-Q350A-N385A-R402A-E428Q-E430Q | C68 | SEQ ID NO: 41 | |
| CDTa (aa44-463 SEQ ID NO: 1) mut R345A-Q350A-N385A-R402A-S388F-E428Q | C110 | SEQ ID NO: 42 | |
| CDTb without prodomain, mut F455R | C164 | SEQ ID NO: 43 | SEQ ID NO: 45 |
| CDTb without prodomain, mutE426A-D453A | C166 | SEQ ID NO: 44 | SEQ ID NO: 46 |
| CDTb' (minus signal peptide) ligated to Glutathione-S-transferase protein.(GST underlined) (without his tag) | C37 without his tag | SEQ ID NO: 47 | |
| CDTb with prodomain removed (CDTb", aa212-876)(without histag) | C55 without histag | SEQ ID NO: 48 | |
| CDTb with prodomain and receptor binding domain removed (corresponding to aa 212-616 of SEQ ID NO: 3), without histag | C116 without histag | SEQ ID NO: 49 | |
| CDTb with prodomain and CDTa binding domain removed (corresponding to aa296-876 of SEQ ID NO: 3), without histag | C117 without histag | SEQ ID NO: 50 | |
| CDTb (corresponding to SEQ ID NO: 3) mut -F455R, without Histag | C123 without histag | SEQ ID NO: 51 | |
| CDTb sequence (corresponding to SEQ ID NO: 3) in which residues 423 (Glutamate) and 453 (Aspartate) are both mutated into alanine. Without histag | C126 without histag | SEQ ID NO: 52 | |
| CDTb (corresponding to aa 43-876 of SEQ ID NO: 3) in which aa 532 to aa 542 are deleted and replaced by 3 Gly residues, without histag | C127 without histag | SEQ ID NO: 53 | |
| CDTb (corresponding to aa43-876 SEQ ID NO: 3) mut -D537G-E539G-D540G-K541 G, without histag | C128 without histag | SEQ ID NO: 54 | |
| Fusion CdtA-E428Q-Cdtb mature, without histag (CDTa: aa44-463 of SEQ ID NO: 1; CDTb: aa212-876 of SEQ ID NO: 3) | C139 without histag | SEQ ID NO:55 | |
| Fusion CdtA-E428Q-Cdtb withCdtA bdg domain removed, without histag (CDTa: aa44-463 of SEQ ID NO: 1; CDTb: aa296-876 of SEQ ID NO: 3) | C145 without histag | SEQ ID NO:56 | |
| Fusion CdtA-E428Q-Cdtb RBD long (CDTa: aa44-463 of SEQ ID NO: 1; CDTb: aa620-876 of SEQ ID NO: 3) without histag | C155 without histag | SEQ ID NO:57 | |
| Fusion CdtA-E428Q-Cdtb RBD short (CDTa: aa44-463 of SEQ ID NO: 1; CDTb: aa636-876 of SEQ ID NO: 3) without histag | C156 without histag | SEQ ID NO:58 | |
| CDTa linker (aa44-268 SEQ ID NO: 1) without histag | C49 without histag | SEQ ID NO:59 | |
| CDTa without linker (aa44-260 SEQ ID NO:1) without histag | C50 without histag | SEQ ID NO:60 | |
| CDTa N terminal domain (residue 44 to residue 240) without histag | Gulke et al 2001 without histag | SEQ ID NO:61 | |
| CDTa (aa44-463 SEQ ID NO:1) mut E428Q-E430Q without histag | C67 without histag | SEQ ID NO:62 | |
| CDTa (aa44-463 SEQ ID NO: 1) mut R345A-Q350A-N385A-R402A-S388F-E428Q-E430Q without histag | C69 without histag | SEQ ID NO:63 | |
| CDTa (aa44-463 SEQ ID NO: 1) mut R345A-Q350A-N385A-R402A-S388F without histag | C107 without histag | SEQ ID NO:64 | |
| CDTa (aa44-463 SEQ ID NO: 1) mut R345A-Q350A-N385A-R402A-S388F-E430Q without histag | C108 without histag | SEQ ID NO:65 | |
| CDTb with prodomain, mut F455R without histag | C149 without histag | SEQ ID NO:66 | |
| CDTb with prodomain, mutE426A-D453A without histag | C152 without histag | SEQ ID NO:67 | |
| CDTb receptor binding domain with linker in N-term of sequence, from aa 620-876 SEQ ID NO: 3 without histag | C52 without histag | SEQ ID NO:68 | |
| CDTb receptor binding domain without linker in N-term of sequence, from aa 636-876SEQ ID NO: 3 without histag | C53 without histag | SEQ ID NO:69 | |
| CDTa (aa44-463 of SEQ ID NO: 1) without histag | C34 without histag | SEQ ID NO:70 | |
| truncated CDTb protein with the signal peptide, prodomain, receptor binding domain and CDTa binding domain removed, corresponding to aa296 to aa 616 of SEQ ID NO: 3, without histag | C118 without histag | SEQ ID NO:71 | |
| CDTb' (minus signal peptide), without histag | C46 without histag | SEQ ID NO:72 | |
| CDTa (aa44-463 SEQ ID NO:1) mut E428Q, without histag | C44 without histag | SEQ ID NO:73 | |
| CDTa (aa44-463 SEQ ID NO:1) mut E430Q, without histag | C54 without histag | SEQ ID NO:74 | |
| CDTa (aa44-463 SEQ ID NO: 1) mut R345A-Q350A-N385A-R402A-E428Q-E430Q, without histag | C68 without histag | SEQ ID NO:75 | |
| CDTa (aa44-463 SEQ ID NO: 1) mut R345A-Q350A-N385A-R402A-S388F-E428Q, without histag | C110 without histag | SEQ ID NO:76 | |

### SEQUENCE LISTING

SEQ ID 1 – CDTa full length polypeptide sequence
MKKFRKHKRISNCISILLILYLTLGGLLPNNIYAQDLQSYSEKVCNTTYKAPIERPEDFLKDKE
KAKEWERKEAERIEQKLERSEKEALESYKKDSVEISKYSQTRNYFDYQIEANSREKEYKEL
RNAISKNKIDKPMYVYYFESPEKFAFNKVIRTENQNEISLEKFNEFKETIQNKLFKQDGFKDIS
LYEPGKGDEKPTPLLMHLKLPRNTGMLPYTNTNNVSTLIEQGYSIKIDKIVRIVIDGKHYIKAE
ASVVSSLDFKDDVSKGDSWGKANYNDWSNKLTPNELADVNDYMRGGYTAINNYLISNGPV
NNPNPELDSKITNIENALKREPIPTNLTVYRRSGPQEFGLTLTSPEYDFNKLENIDAFKSKWE
GQALSYPNFISTSIGSVNMSAFAKRKIVLRITIPKGSPGAYLSAIPGYAGEYEVLLNHGSKFKI
NKIDSYKDGTITKLIVDATLIP

SEQ ID 2 – CDTa full length polynucleotide sequence
ATGAAAAAATTTAGGAAACATAAAAGGATTAGTAATTGTATATCTATATTGTTGATATTAT
ATCTAACTTTAGGTGGTTTGTTACCTAATAACATTTATGCACAAGACTTACAAAGCTATA
GTGAAAAAGTTTGCAATACTACTTACAAGGCTCCTATAGAAAGACCAGAAGATTTTCTTA
AAGATAAAGAAAAGGCTAAAGAATGGGAAAGAAAAGAAGCAGAAAGAATAGAGCAAAAA
CTTGAAAGATCTGAAAAAGAAGCATTAGAATCATATAAAAAAGATTCTGTAGAAATAAGT
AAATATTCTCAGACAAGAAATTATTTTTATGATTATCAAATAGAAGCAAATTCTCGAGAAA
AAGAATATAAAGAACTTCGAAATGCTATATCAAAAAATAAAATAGATAAACCTATGTATGT
CTATTATTTTGAATCTCCAGAAAAATTTGCATTTAATAAAGTAATAAGAACAGAAAATCAA
AACGAAATTTCATTAGAAAAATTTAATGAGTTTAAAGAAACTATACAAAACAAATTATTTA
AGCAAGATGGATTTAAAGATATTTCTTTATATGAACCTGGAAAAGGTGATGAAAAACCTA
CACCATTACTTATGCACTTAAAATTACCTAGAAATACTGGTATGTTACCATATACAAATAC
TAACAATGTAAGTACATTAATAGAGCAAGGATATAGTATAAAAATAGATAAAATTGTTCGT
ATAGTTATAGATGGGAAGCACTATATTAAAGCAGAAGCATCTGTTGTAAGTAGTCTTGAT
TTTAAAGATGATGTAAGTAAGGGGGATTCTTGGGGTAAAGCAAATTATAATGATTGGAG
TAATAAATTAACACCTAATGAACTTGCTGATGTAAATGATTATATGCGTGGAGGATATAC
TGCAATTAATAATTATTTAATATCAAATGGTCCAGTAAATAATCCTAACCCAGAATTAGAT
TCTAAAATCACAAACATTGAAAATGCATTAAAACGTGAACCTATTCCAACTAATTTAACTG
TATATAGAAGATCTGGTCCTCAAGAATTTGGTTTAACTCTTACTTCCCCTGAATATGATTT
TAACAAACTAGAAAATATAGATGCTTTTAAATCAAAATGGGAAGGACAAGCACTGTCTTA
TCCAAACTTTATTAGTACTAGTATTGGTAGTGTGAATATGAGTGCATTTGCTAAAAGAAA
AATAGTACTACGTATAACTATACCTAAAGGTTCTCCTGGAGCTTATCTATCAGCTATTCC
AGGTTATGCAGGTGAATATGAAGTGCTTTTAAATCATGGAAGCAAATTTAAAATCAATAA

AATTGATTCTTACAAAGATGGTACTATAACAAAATTAATTGTTGATGCAACATTGATACCT
TAA

SEQ ID 3 – CDTb full length polypeptide sequence
MKIQMRNKKVLSFLTLTAIVSQALVYPVYAQTSTSNHSNKKKEIVNEDILPNNGLMGYYFTDE
HFKDLKLMAPIKDGNLKFEEKKVDKLLDKDKSDVKSIRWTGRIIPSKDGEYTLSTDRDDVLM
QVNTESTISNTLKVNMKKGKEYKVRIELQDKNLGSIDNLSSPNLYWELDGMKKIIPEENLFLR
DYSNIEKDDPFIPNNNFFDPKLMSDWEDEDLDTDNDNIPDSYERNGYTIKDLIAVKWEDSFA
EQGYKKYVSNYLESNTAGDPYTDYEKASGSFDKAIKTEARDPLVAAYPIVGVGMEKLIISTN
EHASTDQGKTVSRATTNSKTESNTAGVSVNVGYQNGFTANVTTNYSHTTDNSTAVQDSNG
ESWNTGLSINKGESAYINANVRYYNTGTAPMYKVTPTTNLVLDGDTLSTIKAQENQIGNNLS
PGDTYPKKGLSPLALNTMDQFSSRLIPINYDQLKKLDAGKQIKLETTQVSGNFGTKNSSGQI
VTEGNSWSDYISQIDSISASIILDTENESYERRVTAKNLQDPEDKTPELTIGEAIEKAFGATKK
DGLLYFNDIPIDESCVELIFDDNTANKIKDSLKTLSDKKIYNVKLERGMNILIKTPTYFTNFDDY
NNYPSTWSNVNTTNQDGLQGSANKLNGETKIKIPMSELKPYKRYVFSGYSKDPLTSNSIIVKI
KAKEEKTDYLVPEQGYTKFSYEFETTEKDSSNIEITLIGSGTTYLDNLSITELNSTPEILDEPEV
KIPTDQEIMDAHKIYFADLNFNPSTGNTYINGMYFAPTQTNKEALDYIQKYRVEATLQYSGFK
DIGTKDKEMRNYLGDPNQPKTNYVNLRSYFTGGENIMTYKKLRIYAITPDDRELLVLSVD

SEQ ID 4 – CDTb full length polynucleotide sequence
ATGAAAATACAAATGAGGAATAAAAAGGTATTAAGTTTTTTAACACTTACAGCTATAGTTA
GTCAAGCACTAGTATATCCTGTATATGCTCAAACTAGTACAAGTAATCATTCTAATAAGA
AAAAAGAAATTGTAAATGAAGATATACTCCCAAACAATGGATTAATGGGATATTATTTCA
CAGATGAGCACTTTAAAGATTTAAAATTAATGGCACCCATAAAAGATGGTAATTTAAAAT
TTGAAGAAAAGAAAGTAGATAAACTTCTGGATAAAGACAAATCAGATGTAAAATCTATAC
GATGGACAGGAAGAATAATTCCTTCTAAGGATGGTGAATATACATTATCAACTGATAGA
GATGATGTCTTAATGCAAGTAAATACTGAGAGTACTATATCAAATACACTTAAAGTTAATA
TGAAAAAGGGTAAAGAATATAAAGTTAGAATAGAGCTACAAGATAAAAATTTAGGTTCAA
TAGATAATTTATCATCACCTAATCTTTATTGGGAATTAGATGGTATGAAGAAAATTATACC
AGAAGAAAATTTATTCTTAAGAGATTATTCTAATATAGAAAAAGATGATCCATTTATCCCA
AATAACAATTTCTTTGACCCAAAGTTGATGTCTGATTGGGAAGACGAAGATTTGGATACA
GATAATGATAATATACCAGATTCATATGAACGAAATGGATATACTATTAAGGACTTAATT
GCAGTTAAGTGGGAAGATAGTTTTGCAGAACAAGGCTATAAGAAATATGTATCAAATTAT
TTAGAGTCAAATACTGCTGGAGATCCATATACAGATTATGAAAAAGCTTCAGGTTCTTTT
GACAAGGCTATAAAGACTGAAGCAAGAGATCCGTTAGTTGCAGCATATCCAATTGTTGG
AGTAGGTATGGAAAAATTAATTATATCTACAAATGAACATGCCTCTACTGATCAAGGTAA
AACTGTTTCCAGAGCTACTACTAACAGTAAAACTGAATCTAATACAGCTGGTGTGTCTGT

TAATGTAGGATATCAAAATGGATTCACAGCTAATGTAACTACAAATTATTCCCATACAAC
AGATAATTCAACTGCTGTTCAAGATAGTAATGGAGAATCATGGAATACTGGATTAAGTAT
AAACAAAGGAGAATCTGCATATATAAATGCAAATGTTAGATATTACAACACAGGTACTGC
ACCTATGTACAAAGTGACACCAACAACAAATTTAGTGTTAGATGGAGATACATTATCAAC
TATCAAAGCACAAGAAAATCAAATTGGCAATAATCTATCTCCTGGAGATACTTATCCCAA
AAAAGGGCTTTCACCTCTAGCTCTTAACACAATGGATCAATTTAGCTCTAGACTGATTCC
TATAAATTATGATCAATTAAAAAAATTAGATGCTGGAAAGCAAATTAAATTAGAAACAACA
CAAGTAAGTGGAAATTTTGGTACAAAAAATAGTTCTGGACAAATAGTAACAGAAGGAAAT
AGTTGGTCAGACTATATAAGTCAAATTGACAGTATTTCTGCATCTATTATATTAGATACAG
AGAATGAATCTTACGAAAGAAGAGTTACTGCTAAAAATTTACAGGATCCAGAAGATAAAA
CACCTGAACTTACAATTGGAGAAGCAATTGAAAAAGCTTTTGGCGCTACTAAAAAAGAT
GGTTTGTTATATTTTAATGATATACCAATAGATGAAAGTTGTGTTGAACTCATATTTGATG
ATAATACAGCCAATAAGATTAAAGATAGTTTAAAAACTTTGTCTGATAAAAAGATATATAA
TGTTAAACTTGAAAGAGGAATGAATATACTTATAAAAACACCAACTTACTTTACTAATTTT
GATGATTATAATAATTACCCTAGTACATGGAGTAATGTCAATACTACGAATCAAGATGGT
TTACAAGGCTCAGCAAATAAATTAAATGGTGAGACGAAGATTAAAATCCCTATGTCTGAG
CTAAAACCTTATAAACGTTATGTTTTTAGTGGATATTCAAAGGATCCTTTAACATCTAATT
CAATAATTGTAAAGATAAAAGCAAAAGAAGAGAAAACGGATTATTTGGTACCAGAACAA
GGATATACAAAATTTAGTTATGAATTTGAAACTACTGAAAAAGATTCTTCTAATATAGAGA
TAACATTAATTGGTAGTGGTACAACATACTTAGATAACTTATCTATTACAGAGCTAAATAG
TACTCCTGAAATACTTGATGAACCAGAAGTTAAAATTCCAACTGACCAAGAAATAATGGA
TGCACATAAAATATATTTTGCAGATTTAAATTTTAATCCAAGTACAGGAAATACTTATATA
AATGGTATGTATTTTGCACCAACACAAACTAATAAAGAAGCTCTCGATTATATCCAAAAA
TATAGAGTTGAAGCTACTTTACAATATTCTGGATTTAAAGATATTGGAACTAAAGATAAA
GAAATGCGTAATTATTTAGGAGATCCAAATCAGCCTAAAACTAATTATGTTAATCTTAGG
AGTTATTTTACAGGTGGAGAAAATATTATGACATACAAGAAATTAAGAATATATGCAATTA
CTCCAGACGATAGAGAGTTATTAGTTCTTAGTGTTGATTAG

SEQ ID 5 – CDTb C37 construct. CDTb' (minus signal peptide) ligated to Glutathione-S-
transferase protein (GST underlined) polypeptide sequence.
MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK

WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQEN
QIGNNLSPGDTYPKKGLSPLALNTMDQFSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVDHHHHHH

SEQ ID 6 – CDTb C37construct. CDTb' (minus pro-domain) ligated to Glutathione-S-
transferase protein (GST underlined) polynucleotide sequence.

atgtcccctatactaggttattggaaaattaagggccttgtgcaacccactcgacttcttttggaatatcttgaagaaaaatatgaag
agcatttgtatgagcgcgatgaaggtgataaatggcgaaacaaaaagtttgaattgggtttggagtttcccaatcttccttattatatt
gatggtgatgttaaattaacacagtctatggccatcatacgttatatagctgacaagcacaacatgttgggtggttgtccaaaagag
cgtgcagagatttcaatgcttgaaggagcggttttggatattagatacggtgtttcgagaattgcatatagtaaagactttgaaactct
caaagttgattttcttagcaagctacctgaaatgctgaaaatgttcgaagatcgtttatgtcataaaacatatttaaatggtgatcatgt
aacccatcctgacttcatgttgtatgacgctcttgatgttgttttatacatggacccaatgtgcctggatgcgttcccaaaattagtttgttt
taaaaaacgtattgaagctatcccacaaattgataagtacttgaaatccagcaagtatatagcatggcctttgcagggctggcaa
gccacgtttggtggtggcgaccatcctccaaaatcggatctggaagttctgttccaggggcccctgggatcccatatggaaattgtg
aatgaagatattctgccgaataatggtctgatgggatactactttaccgatgaacattttaaagatctgaaactgatggcaccgatta
aagatggcaatctgaaatttgaagaaaaaaaagtggataaactgctggataaagataaaagtgatgtgaaaagcattcgttgg
accggtcgtattattccgagcaaagatggtgaatacaccctgagcaccgatcgtgatgatgttctgatgcaggttaataccgaaag
caccattagcaataccctgaaagtgaatatgaaaaaaggcaaagaatataaagtgcgcattgaactgcaggataaaaatctgg
gtagcattgataatctgagcagcccgaatctgtattgggaactggatggtatgaaaaaaatcattccggaagaaaacctgtttctg
cgcgattatagcaatattgaaaaagatgatccgtttattccgaataataactttttgatccgaaactgatgagcgattgggaagatg
aagatctggataccgataatgataatattccggatagctatgaacgcaatggctataccattaaagatctgattgccgtgaaatgg
gaagatagctttgcagaacagggctataagaaatatgtgagcaattatctggaaagcaataccgcaggcgatccgtataccgat
tatgaaaaagcaagcggcagctttgataaagccattaaaaccgaagcacgtgatccgctggttgcagcatatccgattgttggtgt
tggtatggaaaaactgattattagcaccaatgaacatgcaagcaccgatcagggtaaaaccgttagccgtgcaaccaccaata
gcaaaaccgaaagcaatacagccggtgttagcgttaatgttggttatcagaatggttttaccgccaatgtgaccaccaattatagc
cataccaccgataatagcaccgcagttcaggatagcaatggtgaaagctggaataccggtctgagcattaacaaaggtgaaag
cgcatatatcaatgccaatgtgcgctattataacaccggcaccgcaccgatgtataaagttaccccgaccaccaatctggttctgg
atggtgataccctgagtaccattaaagcacaagaaaatcagattggcaataatctgagtccgggtgatacctatccgaaaaaag

gtctgagtccgctggcactgaataccatggatcagtttagcagccgtctgattccgattaactatgatcagctgaaaaaactggatg
ccggtaaacaaatcaaactggaaaccacccaggttagcggtaattttggcaccaaaaattcaagcggtcagattgttaccgaag
gtaatagctggtcagattatatcagccagattgatagcattagcgccagcattattctggatacagaaaatgaaagctatgaacgt
cgtgtgaccgcaaaaaatctgcaggacccggaagataaaacaccggaactgaccattggtgaagcaattgaaaaagcatttg
gtgccaccaaaaaagatggcctgctgtattttaacgatattccgattgatgaaagctgcgtggaactgatttttgatgataataccgc
caataaaatcaaagatagcctgaaaaccctgagcgacaaaaaaatctataatgtgaaactggaacgcggtatgaatattctgat
taaaaccccgacctattttaccaattttgatgattataacaattatccgagcacttggagcaatgtgaataccaccaatcaggatggt
ctgcagggtagcgcaaataaactgaatggtgaaaccaaaatcaaaattccgatgagcgaactgaaaccgtataaacgttatgt
gtttagcggctatagcaaagatccgctgaccagcaatagcattattgtgaaaatcaaagccaaagaagaaaaaaccgattatct
ggttccggaacagggttataccaaatttagctatgaatttgaaaccaccgaaaaagatagcagtaatattgaaattaccctgattg
gtagcggcaccacctatctggataatctgagtattaccgaactgaatagcacaccggaaattctggatgaaccggaagtgaaaa
ttccgaccgatcaagaaattatggatgcccataaaatctattttgccgatctgaactttaatccgagcaccggcaatacctatattaa
cggcatgtattttgcaccgacccagaccaataaagaagccctggattatattcagaaatatcgtgttgaagccaccctgcagtata
gcggttttaaagatattggcaccaaagataaagaaatgcgtaattatctgggcgatccgaatcagccgaaaaccaattatgttaat
ctgcgcagctattttaccggtggcgaaaacattatgacctacaaaaaactgcgcatttatgccattacaccggatgatcgtgaact
gctggttctgagcgttgatcaccaccatcatcatcattaa

SEQ ID NO: 7 – amino acid sequence of CDTb with prodomain removed (CDTb'', aa212-
876) (C55)
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQFS
SRLIPINYDQLKKLDAGKQIKLETTQVSGNFGTKNSSGQIVTEGNSWSDYISQIDSISASIILDT
ENESYERRVTAKNLQDPEDKTPELTIGEAIEKAFGATKKDGLLYFNDIPIDESCVELIFDDNTA
NKIKDSLKTLSDKKIYNVKLERGMNILIKTPTYFTNFDDYNNYPSTWSNVNTTNQDGLQGSA
NKLNGETKIKIPMSELKPYKRYVFSGYSKDPLTSNSIIVKIKAKEEKTDYLVPEQGYTKFSYEF
ETTEKDSSNIEITLIGSGTTYLDNLSITELNSTPEILDEPEVKIPTDQEIMDAHKIYFADLNFNPS
TGNTYINGMYFAPTQTNKEALDYIQKYRVEATLQYSGFKDIGTKDKEMRNYLGDPNQPKTN
YVNLRSYFTGGENIMTYKKLRIYAITPDDRELLVLSVDHHHHHH

SEQ ID NO: 8 C116
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQFS
SRLIPINYDQLKKLDAGKQIKLETTQVSGNFGTKNSSGQIVTEGNSWSDYISQIDSISASIILDT

ENESYERRVTAKNLQDPEDKTPELTIGEAIEKAFGATKKDGLLYFNDIPIDESCVELIFDDNTA
NKIKDSLKTLSDKKIYNVKLERGMNILIKTPGGHHHHHH

SEQ ID NO: 9 C117
MAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPTYFTNFDDYNNYPSTWSNVNTTNQDGLQGSANKLNGETKIKIPMSELKPYKRY
VFSGYSKDPLTSNSIIVKIKAKEEKTDYLVPEQGYTKFSYEFETTEKDSSNIEITLIGSGTTYLD
NLSITELNSTPEILDEPEVKIPTDQEIMDAHKIYFADLNFNPSTGNTYINGMYFAPTQTNKEAL
DYIQKYRVEATLQYSGFKDIGTKDKEMRNYLGDPNQPKTNYVNLRSYFTGGENIMTYKKLRI
YAITPDDRELLVLSVDHHHHHH

SEQ ID NO: 10 C123 CDTb sequence with F455R.

MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQEN
QIGNNLSPGDTYPKKGLSPLALNTMDQRSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVDHHHHHH

SEQ ID NO: 11 C126 CDTb sequence in which residues 426 (Glutamate) and 453
(Aspartate) were both mutated into alanine.

MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQAN
QIGNNLSPGDTYPKKGLSPLALNTMAQFSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVDHHHHHH

SEQ ID NO: 12 C127
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTAV
TTNYSHTTDNSTAVQDSNGESWNTGLSINKGESAYINANVRYYNTGTAPMYKVTPTTNLVL
DGDTLSTIKAQENQIGNNLSPGDTYPKKGLSPLALNTMDQFSSRLIPINYDQLKKLDAGKQIK
LETTQVSGNFGTKNSSGQIVTEGNSWSDYISQIDSISASIILDTENESYERRVTAgggPELTIG
EAIEKAFGATKKDGLLYFNDIPIDESCVELIFDDNTANKIKDSLKTLSDKKIYNVKLERGMNILI
KTPTYFTNFDDYNNYPSTWSNVNTTNQDGLQGSANKLNGETKIKIPMSELKPYKRYVFSGY
SKDPLTSNSIIVKIKAKEEKTDYLVPEQGYTKFSYEFETTEKDSSNIEITLIGSGTTYLDNLSITE
LNSTPEILDEPEVKIPTDQEIMDAHKIYFADLNFNPSTGNTYINGMYFAPTQTNKEALDYIQKY
RVEATLQYSGFKDIGTKDKEMRNYLGDPNQPKTNYVNLRSYFTGGENIMTYKKLRIYAITPD
DRELLVLSVDHHHHHH

SEQ ID NO: 13 C128
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQFSSRLIPINYDQLKKLDAGK
QIKLETTQVSGNFGTKNSSGQIVTEGNSWSDYISQIDSISASIILDTENESYERRVTAKNLQgP
gggTPELTIGEAIEKAFGATKKDGLLYFNDIPIDESCVELIFDDNTANKIKDSLKTLSDKKIYNVK
LERGMNILIKTPTYFTNFDDYNNYPSTWSNVNTTNQDGLQGSANKLNGETKIKIPMSELKPY
KRYVFSGYSKDPLTSNSIIVKIKAKEEKTDYLVPEQGYTKFSYEFETTEKDSSNIEITLIGSGTT
YLDNLSITELNSTPEILDEPEVKIPTDQEIMDAHKIYFADLNFNPSTGNTYINGMYFAPTQTNK
EALDYIQKYRVEATLQYSGFKDIGTKDKEMRNYLGDPNQPKTNYVNLRSYFTGGENIMTYK
KLRIYAITPDDRELLVLSVDHHHHHH

SEQ ID NO: 14 C139
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPSDWEDEDLDTDNDNIPDSYE
RNGYTIKDLIAVKWEDSFAEQGYKKYVSNYLESNTAGDPYTDYEKASGSFDKAIKTEARDPL
VAAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPTYFTNFDDYNNYPSTWSNVNTTNQDGLQGSANKLNGETKIKIPMSELKPYKRY
VFSGYSKDPLTSNSIIVKIKAKEEKTDYLVPEQGYTKFSYEFETTEKDSSNIEITLIGSGTTYLD
NLSITELNSTPEILDEPEVKIPTDQEIMDAHKIYFADLNFNPSTGNTYINGMYFAPTQTNKEAL
DYIQKYRVEATLQYSGFKDIGTKDKEMRNYLGDPNQPKTNYVNLRSYFTGGENIMTYKKLRI
YAITPDDRELLVLSVDGGHHHHHH

SEQ ID NO: 15 C145
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPGGGGGGAYPIVGVGMEKLIIS
TNEHASTDQGKTVSRATTNSKTESNTAGVSVNVGYQNGFTANVTTNYSHTTDNSTAVQDS
NGESWNTGLSINKGESAYINANVRYYNTGTAPMYKVTPTTNLVLDGDTLSTIKAQENQIGNN
LSPGDTYPKKGLSPLALNTMDQFSSRLIPINYDQLKKLDAGKQIKLETTQVSGNFGTKNSSG
QIVTEGNSWSDYISQIDSISASIILDTENESYERRVTAKNLQDPEDKTPELTIGEAIEKAFGATK
KDGLLYFNDIPIDESCVELIFDDNTANKIKDSLKTLSDKKIYNVKLERGMNILIKTPTYFTNFDD
YNNYPSTWSNVNTTNQDGLQGSANKLNGETKIKIPMSELKPYKRYVFSGYSKDPLTSNSIIV
KIKAKEEKTDYLVPEQGYTKFSYEFETTEKDSSNIEITLIGSGTTYLDNLSITELNSTPEILDEP
EVKIPTDQEIMDAHKIYFADLNFNPSTGNTYINGMYFAPTQTNKEALDYIQKYRVEATLQYSG
FKDIGTKDKEMRNYLGDPNQPKTNYVNLRSYFTGGENIMTYKKLRIYAITPDDRELLVLSVD
GGHHHHHH

SEQ ID NO: 16 C155
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPTNFDDYNNYPSTWSNVNTTN
QDGLQGSANKLNGETKIKIPMSELKPYKRYVFSGYSKDPLTSNSIIVKIKAKEEKTDYLVPEQ
GYTKFSYEFETTEKDSSNIEITLIGSGTTYLDNLSITELNSTPEILDEPEVKIPTDQEIMDAHKIY
FADLNFNPSTGNTYINGMYFAPTQTNKEALDYIQKYRVEATLQYSGFKDIGTKDKEMRNYL
GDPNQPKTNYVNLRSYFTGGENIMTYKKLRIYAITPDDRELLVLSVDGGHHHHHH

SEQ ID NO: 17 C156
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE

QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPGGGGGGNTTNQDGLQGSAN
KLNGETKIKIPMSELKPYKRYVFSGYSKDPLTSNSIIVKIKAKEEKTDYLVPEQGYTKFSYEFE
TTEKDSSNIEITLIGSGTTYLDNLSITELNSTPEILDEPEVKIPTDQEIMDAHKIYFADLNFNPST
GNTYINGMYFAPTQTNKEALDYIQKYRVEATLQYSGFKDIGTKDKEMRNYLGDPNQPKTNY
VNLRSYFTGGENIMTYKKLRIYAITPDDRELLVLSVDGGHHHHHH

SEQ ID NO: 18: C49
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSHHHHHH

SEQ ID NO: 19: C50
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSHHHHHH

SEQ ID NO: 20: CDTa N terminal domain (residue 44 to residue 240) polypeptide
sequence.
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIHHHHHH

SEQ ID NO: 21: C67
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGqYqVLLNHGSKFKINKIDSYKDGTITKLIVDATLIPHHHHHH

SEQ ID NO: 22: C69
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGQYQVLLNHGSKFKINKIDSYKDGTITKLIVDATLIPHHHHHH

SEQ ID NO: 23: C107
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGEYEVLLNHGSKFKINKIDSYKDGTITKLIVDATLIPHHHHHH

SEQ ID NO: 24: C108
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGEYQVLLNHGSKFKIN
KIDSYKDGTITKLIVDATLIPHHHHHH

SEQ ID NO: 25: C149
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQRSSRLIPINYDQLKKLDAGK
QIKLETTQVSGNFGTKNSSGQIVTEGNSWSDYISQIDSISASIILDTENESYERRVTAKNLQD

PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVDHHHHHH

SEQ ID NO: 26: C152
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQANQIGNNLSPGDTYPKKGLSPLALNTMAQFSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVDHHHHHH

SEQ ID NO: 27: C52
MTNFDDYNNYPSTWSNVNTTNQDGLQGSANKLNGETKIKIPMSELKPYKRYVFSGYSKDP
LTSNSIIVKIKAKEEKTDYLVPEQGYTKFSYEFETTEKDSSNIEITLIGSGTTYLDNLSITELNST
PEILDEPEVKIPTDQEIMDAHKIYFADLNFNPSTGNTYINGMYFAPTQTNKEALDYIQKYRVE
ATLQYSGFKDIGTKDKEMRNYLGDPNQPKTNYVNLRSYFTGGENIMTYKKLRIYAITPDDRE
LLVLSVDGGHHHHHH

SEQ ID NO: 28: C53
MNTTNQDGLQGSANKLNGETKIKIPMSELKPYKRYVFSGYSKDPLTSNSIIVKIKAKEEKTDY
LVPEQGYTKFSYEFETTEKDSSNIEITLIGSGTTYLDNLSITELNSTPEILDEPEVKIPTDQEIM
DAHKIYFADLNFNPSTGNTYINGMYFAPTQTNKEALDYIQKYRVEATLQYSGFKDIGTKDKE
MRNYLGDPNQPKTNYVNLRSYFTGGENIMTYKKLRIYAITPDDRELLVLSVDGGHHHHHH

SEQ ID NO: 29 amino acid sequence of Toxin A

MSLISKEELIKLAYSIRPRENEYKTILTNLDEYNKLTTNNNENKYLQLKKLNESIDVFMN
KYKTSSRNRALSNLKKDILKEVILIKNSNTSPVEKNLHFVWIGGEVSDIALEYIKQWADI
NAEYNIKLWYDSEAFLVNTLKKAIVESSTTEALQLLEEEIQNPQFDNMKFYKKRMEFIYD
RQKRFINYYKSQINKPTVPTIDDIIKSHLVSEYNRDETVLESYRTNSLRKINSNHGIDIR
ANSLFTEQELLNIYSQELLNRGNLAAASDIVRLLALKNFGGVYLDVDMLPGIHSDLFKTI
SRPSSIGLDRWEMIKLEAIMKYKKYINNYTSENFDKLDQQLKDNFKLIIESKSEKSEIFS
KLENLNVSDLEIKIAFALGSVINQALISKQGSYLTNLVIEQVKNRYQFLNQHLNPAIESD
NNFTDTTKIFHDSLFNSATAENSMFLTKIAPYLQVGFMPEARSTISLSGPGAYASAYYDF
INLQENTIEKTLKASDLIEFKFPENNLSQLTEQEINSLWSFDQASAKYQFEKYVRDYTGG
SLSEDNGVDFNKNTALDKNYLLNNKIPSNNVEEAGSKNYVHYIIQLQGDDISYEATCNLF
SKNPKNSIIIQRNMNESAKSYFLSDDGESILELNKYRIPERLKNKEKVKVTFIGHGKDEF
NTSEFARLSVDSLSNEISSFLDTIKLDISPKNVEVNLLGCNMFSYDFNVEETYPGKLLLS
IMDKITSTLPDVNKNSITIGANQYEVRINSEGRKELLAHSGKWINKEEAIMSDLSSKEYI
FFDSIDNKLKAKSKNIPGLASISEDIKTLLLDASVSPDTKFILNNLKLNIESSIGDYIYY
EKLEPVKNIIHNSIDDLIDEFNLLENVSDELYELKKLNNLDEKYLISFEDISKNNSTYSV
RFINKSNGESVYVETEKEIFSKYSEHITKEISTIKNSIITDVNGNLLDNIQLDHTSQVNT
LNAAFFIQSLIDYSSNKDVLNDLSTSVKVQLYAQLFSTGLNTIYDSIQLVNLISNAVNDT
INVLPTITEGIPIVSTILDGINLGAAIKELLDEHDPLLKKELEAKVGVLAINMSLSIAAT
VASIVGIGAEVTIFLLPIAGISAGIPSLVNNELILHDKATSVVNYFNHLSESKKYGPLKT
EDDKILVPIDDLVISEIDFNNNSIKLGTCNILAMEGGSGHTVTGNIDHFFSSPSISSHIP
SLSIYSAIGIETENLDFSKKIMMLPNAPSRVFWWETGAVPGLRSLENDGTRLLDSIRDLY
PGKFYWRFYAFFDYAITTLKPVYEDTNIKIKLDKDTRNFIMPTITTNEIRNKLSYSFDGA
GGTYSLLLSSYPISTNINLSKDDLWIFNIDNEVREISIENGTIKKGKLIKDVLSKIDINK
NKLIIGNQTIDFSGDIDNKDRYIFLTCELDDKISLIIEINLVAKSYSLLLSGDKNYLISN
LSNTIEKINTLGLDSKNIAYNYTDESNNKYFGAISKTSQKSIIHYKKDSKNILEFYNDST
LEFNSKDFIAEDINVFMKDDINTITGKYYVDNNTDKSIDFSISLVSKNQVKVNGLYLNES
VYSSYLDFVKNSDGHHNTSNFMNLFLDNISFWKLFGFENINFVIDKYFTLVGKTNLGYVE
FICDNNKNIDIYFGEWKTSSSKSTIFSGNGRNVVVEPIYNPDTGEDISTSLDFSYEPLYG
IDRYINKVLIAPDLYTSLININTNYYSNEYYPEIIVLNPNTFHKKVNINLDSSSFEYKWS
TEGSDFILVRYLEESNKKILQKIRIKGILSNTQSFNKMSIDFKDIKKLSLGYIMSNFKSF
NSENELDRDHLGFKIIDNKTYYYDEDSKLVKGLININNSLFYFDPIEFNLVTGWQTINGK
KYYFDINTGAALTSYKIINGKHFYFNNDGVMQLGVFKGPDGFEYFAPANTQNNNIEGQAI
VYQSKFLTLNGKKYYFDNNSKAVTGWRIINNEKYYFNPNNAIAAVGLQVIDNNKYYFNPD
TAIISKGWQTVNGSRYYFDTDTAIAFNGYKTIDGKHFYFDSDCVVKIGVFSTSNGFEYFA
PANTYNNNNIEGQAIVYQSKFLTLNGKKYYFDNNSKAVTGLQTIDSKKYYFNTNTAEAATG
WQTIDGKKYYFNTNTAEAATGWQTIDGKKYYFNTNTAIASTGYTIINGKHFYFNTDGIMQ
IGVFKGPNGFEYFAPANTDANNIEGQAILYQNEFLTLNGKKYYFGSDSKAVTGWRIINNK

KYYFNPNNAIAAIHLCTINNDKYYFSYDGILQNGYITIERNNFYFDANNESKMVTGVFKG
PNGFEYFAPANTHNNNIEGQAIVYQNKFLTLNGKKYYFDNDSKAVTGWQTIDGKKYYFNL
NTAEAATGWQTIDGKKYYFNLNTAEAATGWQTIDGKKYYFNTNTFIASTGYTSINGKHFY
FNTDGIMQIGVFKGPNGFEYFAPANTDANNIEGQAILYQNKFLTLNGKKYYFGSDSKAVT
GLRTIDGKKYYFNTNTAVAVTGWQTINGKKYYFNTNTSIASTGYTIISGKHFYFNTDGIM
QIGVFKGPDGFEYFAPANTDANNIEGQAIRYQNRFLYLHDNIYYFGNNSKAATGWVTIDG
NRYYFEPNTAMGANGYKTIDNKNFYFRNGLPQIGVFKGSNGFEYFAPANTDANNIEGQAI
RYQNRFLHLLGKIYYFGNNSKAVTGWQTINGKVYYFMPDTAMAAAGGLFEIDGVIYFFGV
DGVKAPGIYG

SEQ ID NO: 30 – amino acid sequence of Toxin B
MSLVNRKQLEKMANVRFRTQEDEYVAILDALEEYHNMSENTVVEKYLKLKDINSLTDIYI
DTYKKSGRNKALKKFKEYLVTEVLELKNNNLTPVEKNLHFVWIGGQINDTAINYINQWKD
VNSDYNVNVFYDSNAFLINTLKKTVVESAINDTLESFRENLNDPRFDYNKFFRKRMEIIY
DKQKNFINYYKAQREENPELIIDDIVKTYLSNEYSKEIDELNTYIEESLNKITQNSGNDV
RNFEEFKNGESFNLYEQELVERWNLAAASDILRISALKEIGGMYLDVDMLPGIQPDLFES
IEKPSSVTVDFWEMTKLEAIMKYKEYIPEYTSEHFDMLDEEVQSSFESVLASKSDKSEIF
SSLGDMEASPLEVKIAFNSKGIINQGLISVKDSYCSNLIVKQIENRYKILNNSLNPAISE
DNDFNTTTNTFIDSIMAEANADNGRFMMELGKYLRVGFFPDVKTTINLSGPEAYAAAYQD
LLMFKEGSMNIHLIEADLRNFEISKTNISQSTEQEMASLWSFDDARAKAQFEEYKRNYFE
GSLGEDDNLDFSQNIVVDKEYLLEKISSLARSSERGYIHYIVQLQGDKISYEAACNLFAK
TPYDSVLFQKNIEDSEIAYYYNPGDGEIQEIDKYKIPSIISDRPKIKLTFIGHGKDEFNT
DIFAGFDVDSLSTEIEAAIDLAKEDISPKSIEINLLGCNMFSYSINVEETYPGKLLLKVK
DKISELMPSISQDSIIVSANQYEVRINSEGRRELLDHSGEWINKEESIIKDISSKEYISF
NPKENKITVKSKNLPELSTLLQEIRNNSNSSDIELEEKVMLTECEINVISNIDTQIVEER
IEEAKNLTSDSINYIKDEFKLIESISDALCDLKQQNELEDSHFISFEDISETDEGFSIRF
INKETGESIFVETEKTIFSEYANHITEEISKIKGTIFDTVNGKLVKKVNLDTTHEVNTLN
AAFFIQSLIEYNSSKESLSNLSVAMKVQVYAQLFSTGLNTITDAAKVVELVSTALDETID
LLPTLSEGLPIIATIIDGVSLGAAIKELSETSDPLLRQEIEAKIGIMAVNLTTATTAIIT
SSLGIASGFSILLVPLAGISAGIPSLVNNELVLRDKATKVVDYFKHVSLVETEGVFTLLD
DKIMMPQDDLVISEIDFNNNSIVLGKCEIWRMEGGSGHTVTDDIDHFFSAPSITYREPHL
SIYDVLEVQKEELDLSKDLMVLPNAPNRVFAWETGWTPGLRSLENDGTKLLDRIRDNYEG
EFYWRYFAFIADALITTLKPRYEDTNIRINLDSNTRSFIVPIITTEYIREKLSYSFYGSG
GTYALSLSQYNMGINIELSESDVWIIDVDNVVRDVTIESDKIKKGDLIEGILSTLSIEEN
KIILNSHEINFSGEVNGSNGFVSLTFSILEGINAIIEVDLLSKSYKLLISGELKILMLNS
NHIQQKIDYIGFNSELQKNIPYSFVDSEGKENGFINGSTKEGLFVSELPDVVLISKVYMD
DSKPSFGYYSNNLKDVKVITKDNVNILTGYYLKDDIKISLSLTLQDEKTIKLNSVHLDES

GVAEILKFMNRKGNTNTSDSLMSFLESMNIKSIFVNFLQSNIKFILDANFIISGTTSIGQ
FEFICDENDNIQPYFIKFNTLETNYTLYVGNRQNMIVEPNYDLDDSGDISSTVINFSQKY
LYGIDSCVNKVVISPNIYTDEINITPVYETNNTYPEVIVLDANYINEKINVNINDLSIRY
VWSNDGNDFILMSTSEENKVSQVKIRFVNVFKDKTLANKLSFNFSDKQDVPVSEIILSFT
PSYYEDGLIGYDLGLVSLYNEKFYINNFGMMVSGLIYINDSLYYFKPPVNNLITGFVTVG
DDKYYFNPINGGAASIGETIIDDKNYYFNQSGVLQTGVFSTEDGFKYFAPANTLDENLEG
EAIDFTGKLIIDENIYYFDDNYRGAVEWKELDGEMHYFSPETGKAFKGLNQIGDYKYYFN
SDGVMQKGFVSINDNKHYFDDSGVMKVGYTEIDGKHFYFAENGEMQIGVFNTEDGFKYFA
HHNEDLGNEEGEEISYSGILNFNNKIYYFDDSFTAVVGWKDLEDGSKYYFDEDTAEAYIG
LSLINDGQYYFNDDGIMQVGFVTINDKVFYFSDSGIIESGVQNIDDNYFYIDDNGIVQIG
VFDTSDGYKYFAPANTVNDNIYGQAVEYSGLVRVGEDVYYFGETYTIETGWIYDMENESD
KYYFNPETKKACKGINLIDDIKYYFDEKGIMRTGLISFENNNYYFNENGEMQFGYINIED
KMFYFGEDGVMQIGVFNTPDGFKYFAHQNTLDENFEGESINYTGWLDLDEKRYYFTDEYI
AATGSVIIDGEEYYFDPDTAQLVISE

SEQ ID NO: 31: F1

MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAPANTDANNIEGQAIRYQNRFLHLLGKIYYFGNNSKAVTGWQTINGKVYYFM
PDTAMAAAGGLFEIDGVIYFFGVDGVKAPGFVSINDNKHYFDDSGVMKVGYTEIDGKHFYF
AENGEMQIGVFNTEDGFKYFAHHNEDLGNEEGEEISYSGILNFNNKIYYFDDSFTAVVGWK
DLEDGSKYYFDEDTAEAYIGLSLINDGQYYFNDDGIMQVGFVTINDKVFYFSDSGIIESGVQN
IDDNYFYIDDNGIVQIGVFDTSDGYKYFAPANTVNDNIYGQAVEYSGLVRVGEDVYYFGETY
TIETGWIYDMENESDKYYFNPETKKACKGINLIDDIKYYFDEKGIMRTGLISFENNNYYFNEN
GEMQFGYINIEDKMFYFGEDGVMQIGVFNTPDGFKYFAHQNTLDENFEGESINYTGWLDLD
EKRYYFTDEYIAATGSVIIDGEEYYFDPDTAQLVISE

SEQ ID NO: 32: F2
MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF

SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAPANTDANNIEGQAIRYQNRFLHLLGKIYYFGNNSKAVTGWQTINGKVYYFM
PDTAMAAAGGLNQIGDYKYYFNSDGVMQKGFVSINDNKHYFDDSGVMKVGYTEIDGKHFY
FAENGEMQIGVFNTEDGFKYFAHHNEDLGNEEGEEISYSGILNFNNKIYYFDDSFTAVVGW
KDLEDGSKYYFDEDTAEAYIGLSLINDGQYYFNDDGIMQVGFVTINDKVFYFSDSGIIESGVQ
NIDDNYFYIDDNGIVQIGVFDTSDGYKYFAPANTVNDNIYGQAVEYSGLVRVGEDVYYFGET
YTIETGWIYDMENESDKYYFNPETKKACKGINLIDDIKYYFDEKGIMRTGLISFENNNYYFNE
NGEMQFGYINIEDKMFYFGEDGVMQIGVFNTPDGFKYFAHQNTLDENFEGESINYTGWLDL
DEKRYYFTDEYIAATGSVIIDGEEYYFDPDTAQLVISE

SEQ ID NO: 33: F3

MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAHHNEDLGNEEGEEISYSGILNFNNKIYYFDDSFTAVVGWKDLEDGSKYYFD
EDTAEAYIGLSLINDGQYYFNDDGIMQVGFVTINDKVFYFSDSGIIESGVQNIDDNYFYIDDN
GIVQIGVFDTSDGYKYFAPANTVNDNIYGQAVEYSGLVRVGEDVYYFGETYTIETGWIYDME
NESDKYYFNPETKKACKGINLIDDIKYYFDEKGIMRTGLISFENNNYYFNENGEMQFGYINIE
DKMFYFGEDGVMQIGVFNTPDGFKYFAHQNTLDENFEGESINYTGWLDLDEKRYYFTDEYI
AATGSVIIDGEEYYFDPDTAQLVISE

SEQ ID NO: 34: F4
MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT

GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAPANTDANNIEGQAIRYQNRFLHLLGKIYYFGNNSKAVTGWQTINGKVYYFM
PDTAMAAAGGETIIDDKNYYFNQSGVLQTGVFSTEDGFKYFAPANTLDENLEGEAIDFTGKL
IIDENIYYFDDNYRGAVEWKELDGEMHYFSPETGKAFKGLNQIGDYKYYFNSDGVMQKGFV
SINDNKHYFDDSGVMKVGYTEIDGKHFYFAENGEMQIGVFNTEDGFKYFAHHNEDLGNEE
GEEISYSGILNFNNKIYYFDDSFTAVVGWKDLEDGSKYYFDEDTAEAYIGLSLINDGQYYFND
DGIMQVGFVTINDKVFYFSDSGIIESGVQNIDDNYFYIDDNGIVQIGVFDTSDGYKYFAPANT
VNDNIYGQAVEYSGLVRVGEDVYYFGETYTIETGWIYDMENESDKYYFNPETKKACKGINLI
DDIKYYFDEKGIMRTGLISFENNNYYFNENGEMQFGYINIEDKMFYFGEDGVMQIGVFNTPD
GFKYFAHQNTLDENFEGESINYTGWLDLDEKRYYFTDEYIAATGSVIIDGEEYYFDPDTAQL
VISE

SEQ ID NO: 35: F5
MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAPANTDANNIEGQAIRYQNRFLHLLGKIYYFGNNSKAVTGWQTINGKVYYFM
PDTAMAAAGGLFEIDGVIYFFGVDGVKAPGIYGGGFVSINDNKHYFDDSGVMKVGYTEIDG
KHFYFAENGEMQIGVFNTEDGFKYFAHHNEDLGNEEGEEISYSGILNFNNKIYYFDDSFTAV
VGWKDLEDGSKYYFDEDTAEAYIGLSLINDGQYYFNDDGIMQVGFVTINDKVFYFSDSGIIE
SGVQNIDDNYFYIDDNGIVQIGVFDTSDGYKYFAPANTVNDNIYGQAVEYSGLVRVGEDVYY
FGETYTIETGWIYDMENESDKYYFNPETKKACKGINLIDDIKYYFDEKGIMRTGLISFENNNY
YFNENGEMQFGYINIEDKMFYFGEDGVMQIGVFNTPDGFKYFAHQNTLDENFEGESINYTG
WLDLDEKRYYFTDEYIAATGSVIIDGEEYYFDPDTAQLVISE

SEQ ID NO: 36: CDTa C34 construct polypeptide sequence
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE

QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGEYEVLLNHGSKFKINKIDSYKDGTITKLIVDATLIPHHHHHH

SEQ ID NO: 37: CDTb C118 construct. CDTb without signal peptide, without prodomain,
without CDTa-binding domain and without Receptor-binding domain

MAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPGGHHHHHH

SEQ ID NO: 38: CDTb C46 construct. CDTb' (minus signal peptide) polypeptide sequence
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQFSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVDHHHHHH

SEQ ID NO: 39: C44 construct. CDTa mutation E428Q polypeptide sequence.
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD

VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGqYEVLLNHGSKFKINKIDSYKDGTITKLIVDATLIPHHHHHH

SEQ ID NO: 40: C54 construct. CDTa mutation E430Q polypeptide sequence.
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGEYqVLLNHGSKFKINKIDSYKDGTITKLIVDATLIPHHHHHH

SEQ ID NO: 41: C68
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFISTSIGSVNMSAFAKAKIVLRITIPKGSPGAYL
SAIPGYAGQYQVLLNHGSKFKINKIDSYKDGTITKLIVDATLIPHHHHHH

SEQ ID NO: 42: Amino acid sequence of CDTa without signal peptide, with six
mutations (R345A, Q350A, N385A, R402A, S388F, E428Q, aa 44-463) (C110
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGQYEVLLNHGSKFKINKIDSYKDGTITKLIVDATLIPHHHHHH

SEQ ID NO: 43: Amino acid sequence of C164 (KO pore forming mutation F455R on CdtB
without prodomain)
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR

YYNTGTAPMYKVTPTTNLVLDGDTLSTIKAQENQIGNNLSPGDTYPKKGLSPLALNTMDQR
SSRLIPINYDQLKKLDAGKQIKLETTQVSGNFGTKNSSGQIVTEGNSWSDYISQIDSISASIILD
TENESYERRVTAKNLQDPEDKTPELTIGEAIEKAFGATKKDGLLYFNDIPIDESCVELIFDDNT
ANKIKDSLKTLSDKKIYNVKLERGMNILIKTPTYFTNFDDYNNYPSTWSNVNTTNQDGLQGS
ANKLNGETKIKIPMSELKPYKRYVFSGYSKDPLTSNSIIVKIKAKEEKTDYLVPEQGYTKFSYE
FETTEKDSSNIEITLIGSGTTYLDNLSITELNSTPEILDEPEVKIPTDQEIMDAHKIYFADLNFNP
STGNTYINGMYFAPTQTNKEALDYIQKYRVEATLQYSGFKDIGTKDKEMRNYLGDPNQPKT
NYVNLRSYFTGGENIMTYKKLRIYAITPDDRELLVLSVD

SEQ ID NO: 44: Amino acid sequence of C166 (KO pore forming mutation E426A-D453A
on CdtB without prodomain)

MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQANQIGNNLSPGDTYPKKGLSPLALNTMAQFS
SRLIPINYDQLKKLDAGKQIKLETTQVSGNFGTKNSSGQIVTEGNSWSDYISQIDSISASIILDT
ENESYERRVTAKNLQDPEDKTPELTIGEAIEKAFGATKKDGLLYFNDIPIDESCVELIFDDNTA
NKIKDSLKTLSDKKIYNVKLERGMNILIKTPTYFTNFDDYNNYPSTWSNVNTTNQDGLQGSA
NKLNGETKIKIPMSELKPYKRYVFSGYSKDPLTSNSIIVKIKAKEEKTDYLVPEQGYTKFSYEF
ETTEKDSSNIEITLIGSGTTYLDNLSITELNSTPEILDEPEVKIPTDQEIMDAHKIYFADLNFNPS
TGNTYINGMYFAPTQTNKEALDYIQKYRVEATLQYSGFKDIGTKDKEMRNYLGDPNQPKTN
YVNLRSYFTGGENIMTYKKLRIYAITPDDRELLVLSVD

SEQ ID NO: 45: Nucleic acid sequence of C164 (KO pore forming mutation F455R on CdtB
without prodomain)

ATGAGCGATTGGGAAGATGAAGATCTGGATACCGATAATGATAATATTCCGGATAGCTA
TGAACGCAATGGCTATACCATTAAAGATCTGATTGCCGTGAAATGGGAAGATAGCTTTG
CAGAACAGGGCTATAAGAAATATGTGAGCAATTATCTGGAAAGCAATACCGCAGGCGAT
CCGTATACCGATTATGAAAAAGCAAGCGGCAGCTTTGATAAAGCCATTAAAACCGAAGC
ACGTGATCCGCTGGTTGCAGCATATCCGATTGTTGGTGTTGGTATGGAAAAACTGATTA
TTAGCACCAATGAACATGCAAGCACCGATCAGGGTAAAACCGTTAGCCGTGCAACCAC
CAATAGCAAAACCGAAAGCAATACAGCCGGTGTTAGCGTTAATGTTGGTTATCAGAATG
GTTTTACCGCCAATGTGACCACCAATTATAGCCATACCACCGATAATAGCACCGCAGTT
CAGGATAGCAATGGTGAAAGCTGGAATACCGGTCTGAGCATTAACAAAGGTGAAAGCG
CATATATCAATGCCAATGTGCGCTATTATAACACCGGCACCGCACCGATGTATAAAGTT

ACCCCGACCACCAATCTGGTTCTGGATGGTGATACCCTGAGTACCATTAAAGCACAAGA
AAATCAGATTGGCAATAATCTGAGTCCGGGTGATACCTATCCGAAAAAGGTCTGAGTC
CGCTGGCACTGAATACCATGGATCAGCGCAGCAGCCGTCTGATTCCGATTAACTATGAT
CAGCTGAAAAAACTGGATGCCGGTAAACAAATCAAACTGGAAACCACCCAGGTTAGCG
GTAATTTTGGCACCAAAAATTCAAGCGGTCAGATTGTTACCGAAGGTAATAGCTGGTCA
GATTATATCAGCCAGATTGATAGCATTAGCGCCAGCATTATTCTGGATACAGAAAATGAA
AGCTATGAACGTCGTGTGACCGCAAAAAATCTGCAGGACCCGGAAGATAAAACACCGG
AACTGACCATTGGTGAAGCAATTGAAAAAGCATTTGGTGCCACCAAAAAAGATGGCCTG
CTGTATTTTAACGATATTCCGATTGATGAAAGCTGCGTGGAACTGATTTTTGATGATAAT
ACCGCCAATAAAATCAAAGATAGCCTGAAAACCCTGAGCGACAAAAAAATCTATAATGT
GAAATTAGAACGCGGTATGAATATTCTAATTAAAACCCCGACCTATTTTACCAATTTTGA
TGATTATAACAATTATCCGAGCACTTGGAGCAATGTGAATACCACCAATCAGGATGGTC
TGCAGGGTAGCGCAAATAAACTGAATGGTGAAACCAAAATCAAAATTCCGATGAGCGAA
CTGAAACCGTATaAACGTTATGTGTTTAGCGGCTATAGCAAAGATCCGCTGACCAGCAA
TAGCATTATTGTGAAAATCAAAGCCAAAGAAGAAAAACCGATTATCTGGTTCCGGAAC
AGGGTTATACCAAATTTAGCTATGAATTTGAAACCACCGAAAAAGATAGCAGTAATATTG
AAATTACCCTGATTGGTAGCGGCACCACCTATCTGGATAATCTGAGTATTACCGAACTG
AATAGCACACCGGAAATTCTGGATGAACCGGAAGTGAAAATTCCGACCGATCAAGAAAT
TATGGATGCCCATAAAATCTATTTTGCCGATCTGAACTTTAATCCGAGCACCGGCAATAC
CTATATTAACGGCATGTATTTTGCACCGACCCAGACCAATAAAGAAGCCCTGGATTATAT
TCAGAAATATCGTGTTGAAGCCACCCTGCAGTATAGCGGTTTTAAAGATATTGGCACCA
AAGATAAAGAAATGCGTAATTATCTGGGCGATCCGAATCAGCCGAAAACCAATTATGTT
AATCTGCGCAGCTATTTTACCGGTGGCGAAAACATTATGACCTACAAAAAACTGCGCAT
TTATGCCATTACACCGGATGATCGTGAACTGCTGGTTCTGAGCGTTGATCA

SEQ ID NO: 46: Nucleic acid sequence of C166 (KO pore forming mutation E426A-D453A
on CdtB without prodomain)

ATGAGCGATTGGGAAGATGAAGATCTGGATACCGATAATGATAATATTCCGGATAGCTA
TGAACGCAATGGCTATACCATTAAAGATCTGATTGCCGTGAAATGGGAAGATAGCTTTG
CAGAACAGGGCTATAAGAAATATGTGAGCAATTATCTGGAAAGCAATACCGCAGGCGAT
CCGTATACCGATTATGAAAAAGCAAGCGGCAGCTTTGATAAAGCCATTAAAACCGAAGC
ACGTGATCCGCTGGTTGCAGCATATCCGATTGTTGGTGTTGGTATGGAAAAACTGATTA
TTAGCACCAATGAACATGCAAGCACCGATCAGGGTAAAACCGTTAGCCGTGCAACCAC
CAATAGCAAAACCGAAAGCAATACAGCCGGTGTTAGCGTTAATGTTGGTTATCAGAATG
GTTTTACCGCCAATGTGACCACCAATTATAGCCATACCACCGATAATAGCACCGCAGTT
CAGGATAGCAATGGTGAAAGCTGGAATACCGGTCTGAGCATTAACAAAGGTGAAAGCG

CATATATCAATGCCAATGTGCGCTATTATAACACCGGCACCGCACCGATGTATAAAGTT
ACCCCGACCACCAATCTGGTTCTGGATGGTGATACCCTGAGTACCATTAAAGCACAAGC
RAATCAGATTGGCAATAATCTGAGTCCGGGTGATACCTATCCGAAAAAGGTCTGAGTC
CGCTGGCACTGAATACCATGGCGCAGTTTAGCAGCCGTCTGATTCCGATTAACTATGAT
CAGCTGAAAAACTGGATGCCGGTAAACAAATCAAACTGGAAACCACCCAGGTTAGCG
GTAATTTTGGCACCAAAAATTCAAGCGGTCAGATTGTTACCGAAGGTAATAGCTGGTCA
GATTATATCAGCCAGATTGATAGCATTAGCGCCAGCATTATTCTGGATACAGAAAATGAA
AGCTATGAACGTCGTGTGACCGCAAAAAATCTGCAGGACCCGGAAGATAAAACACCGG
AACTGACCATTGGTGAAGCAATTGAAAAAGCATTTGGTGCCACCAAAAAAGATGGCCTG
CTGTATTTTAACGATATTCCGATTGATGAAAGCTGCGTGGAACTGATTTTTGATGATAAT
ACCGCCAATAAAATCAAAGATAGCCTGAAAACCCTGAGCGACAAAAAAATCTATAATGT
GAAATTAGAACGCGGTATGAATATTCTAATTAAAACCCCGACCTATTTTACCAATTTTGA
TGATTATAACAATTATCCGAGCACTTGGAGCAATGTGAATACCACCAATCAGGATGGTC
TGCAGGGTAGCGCAAATAAACTGAATGGTGAAACCAAAATCAAAATTCCGATGAGCGAA
CTGAAACCGTATAAACGTTATGTGTTTAGCGGCTATAGCAAAGATCCGCTGACCAGCAA
TAGCATTATTGTGAAAATCAAAGCCAAAGAAGAAAAACCGATTATCTGGTTCCGGAAC
AGGGTTATACCAAATTTAGCTATGAATTTGAAACCACCGAAAAAGATAGCAGTAATATTG
AAATTACCCTGATTGGTAGCGGCACCACCTATCTGGATAATCTGAGTATTACCGAACTG
AATAGCACACCGGAAATTCTGGATGAACCGGAAGTGAAAATTCCGACCGATCAAGAAAT
TATGGATGCCCATAAAATCTATTTTGCCGATCTGAACTTTAATCCGAGCACCGGCAATAC
CTATATTAACGGCATGTATTTTGCACCGACCCAGACCAATAAAGAAGCCCTGGATTATAT
TCAGAAATATCGTGTTGAAGCCACCCTGCAGTATAGCGGTTTTAAAGATATTGGCACCA
AAGATAAAGAAATGCGTAATTATCTGGGCGATCCGAATCAGCCGAAAACCAATTATGTT
AATCTGCGCAGCTATTTTACCGGTGGCGAAAACATTATGACCTACAAAAAACTGCGCAT
TTATGCCATTACACCGGATGATCGTGAACTGCTGGTTCTGAGCGTTGATCAC

SEQ ID 47 – CDTb C37 construct. CDTb' (minus signal peptide) ligated to Glutathione-
S-transferase protein (GST underlined) polypeptide sequence, without histag.
MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA

VQDSNGESWNTGLSINKGESAYINANVRYYNTGTAPMYKVTPTTNLVLDGDTLSTIKAQEN
QIGNNLSPGDTYPKKGLSPLALNTMDQFSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVD

SEQ ID NO: 48 – amino acid sequence of CDTb with prodomain removed (CDTb'', aa212-
876) (C55), without histag
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQFS
SRLIPINYDQLKKLDAGKQIKLETTQVSGNFGTKNSSGQIVTEGNSWSDYISQIDSISASIILDT
ENESYERRVTAKNLQDPEDKTPELTIGEAIEKAFGATKKDGLLYFNDIPIDESCVELIFDDNTA
NKIKDSLKTLSDKKIYNVKLERGMNILIKTPTYFTNFDDYNNYPSTWSNVNTTNQDGLQGSA
NKLNGETKIKIPMSELKPYKRYVFSGYSKDPLTSNSIIVKIKAKEEKTDYLVPEQGYTKFSYEF
ETTEKDSSNIEITLIGSGTTYLDNLSITELNSTPEILDEPEVKIPTDQEIMDAHKIYFADLNFNPS
TGNTYINGMYFAPTQTNKEALDYIQKYRVEATLQYSGFKDIGTKDKEMRNYLGDPNQPKTN
YVNLRSYFTGGENIMTYKKLRIYAITPDDRELLVLSVD

SEQ ID NO: 49 C116 without histag
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQFS
SRLIPINYDQLKKLDAGKQIKLETTQVSGNFGTKNSSGQIVTEGNSWSDYISQIDSISASIILDT
ENESYERRVTAKNLQDPEDKTPELTIGEAIEKAFGATKKDGLLYFNDIPIDESCVELIFDDNTA
NKIKDSLKTLSDKKIYNVKLERGMNILIKTPGG

SEQ ID NO: 50 C117 without histag
MAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG

DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPTYFTNFDDYNNYPSTWSNVNTTNQDGLQGSANKLNGETKIKIPMSELKPYKRY
VFSGYSKDPLTSNSIIVKIKAKEEKTDYLVPEQGYTKFSYEFETTEKDSSNIEITLIGSGTTYLD
NLSITELNSTPEILDEPEVKIPTDQEIMDAHKIYFADLNFNPSTGNTYINGMYFAPTQTNKEAL
DYIQKYRVEATLQYSGFKDIGTKDKEMRNYLGDPNQPKTNYVNLRSYFTGGENIMTYKKLRI
YAITPDDRELLVLSVD

SEQ ID NO: 51 C123 CDTb sequence with F455R, without histag.

MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQEN
QIGNNLSPGDTYPKKGLSPLALNTMDQRSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVD

SEQ ID NO: 52 C126 CDTb sequence in which residues 426 (Glutamate) and 453
(Aspartate) were both mutated into alanine, without histag.

MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR

DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQAN
QIGNNLSPGDTYPKKGLSPLALNTMAQFSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVD

SEQ ID NO: 53 C127 without histag
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTAV
TTNYSHTTDNSTAVQDSNGESWNTGLSINKGESAYINANVRYYNTGTAPMYKVTPTTNLVL
DGDTLSTIKAQENQIGNNLSPGDTYPKKGLSPLALNTMDQFSSRLIPINYDQLKKLDAGKQIK
LETTQVSGNFGTKNSSGQIVTEGNSWSDYISQIDSISASIILDTENESYERRVTAgggPELTIG
EAIEKAFGATKKDGLLYFNDIPIDESCVELIFDDNTANKIKDSLKTLSDKKIYNVKLERGMNILI
KTPTYFTNFDDYNNYPSTWSNVNTTNQDGLQGSANKLNGETKIKIPMSELKPYKRYVFSGY
SKDPLTSNSIIVKIKAKEEKTDYLVPEQGYTKFSYEFETTEKDSSNIEITLIGSGTTYLDNLSITE
LNSTPEILDEPEVKIPTDQEIMDAHKIYFADLNFNPSTGNTYINGMYFAPTQTNKEALDYIQKY
RVEATLQYSGFKDIGTKDKEMRNYLGDPNQPKTNYVNLRSYFTGGENIMTYKKLRIYAITPD
DRELLVLSVD

SEQ ID NO: 54 C128 without histag
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA

NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQFSSRLIPINYDQLKKLDAGK
QIKLETTQVSGNFGTKNSSGQIVTEGNSWSDYISQIDSISASIILDTENESYERRVTAKNLQgP
gggTPELTIGEAIEKAFGATKKDGLLYFNDIPIDESCVELIFDDNTANKIKDSLKTLSDKKIYNVK
LERGMNILIKTPTYFTNFDDYNNYPSTWSNVNTTNQDGLQGSANKLNGETKIKIPMSELKPY
KRYVFSGYSKDPLTSNSIIVKIKAKEEKTDYLVPEQGYTKFSYEFETTEKDSSNIEITLIGSGTT
YLDNLSITELNSTPEILDEPEVKIPTDQEIMDAHKIYFADLNFNPSTGNTYINGMYFAPTQTNK
EALDYIQKYRVEATLQYSGFKDIGTKDKEMRNYLGDPNQPKTNYVNLRSYFTGGENIMTYK
KLRIYAITPDDRELLVLSVD

SEQ ID NO: 55 C139 without histag
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPSDWEDEDLDTDNDNIPDSYE
RNGYTIKDLIAVKWEDSFAEQGYKKYVSNYLESNTAGDPYTDYEKASGSFDKAIKTEARDPL
VAAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPTYFTNFDDYNNYPSTWSNVNTTNQDGLQGSANKLNGETKIKIPMSELKPYKRY
VFSGYSKDPLTSNSIIVKIKAKEEKTDYLVPEQGYTKFSYEFETTEKDSSNIEITLIGSGTTYLD
NLSITELNSTPEILDEPEVKIPTDQEIMDAHKIYFADLNFNPSTGNTYINGMYFAPTQTNKEAL
DYIQKYRVEATLQYSGFKDIGTKDKEMRNYLGDPNQPKTNYVNLRSYFTGGENIMTYKKLRI
YAITPDDRELLVLSVDGG

SEQ ID NO: 56 C145 without histag
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL

SAIPGYAGQYEVLLNHGSKFKINKIDSYKDGTITKLIVDATLIPGGGGGGAYPIVGVGMEKLIIS
TNEHASTDQGKTVSRATTNSKTESNTAGVSVNVGYQNGFTANVTTNYSHTTDNSTAVQDS
NGESWNTGLSINKGESAYINANVRYYNTGTAPMYKVTPTTNLVLDGDTLSTIKAQENQIGNN
LSPGDTYPKKGLSPLALNTMDQFSSRLIPINYDQLKKLDAGKQIKLETTQVSGNFGTKNSSG
QIVTEGNSWSDYISQIDSISASIILDTENESYERRVTAKNLQDPEDKTPELTIGEAIEKAFGATK
KDGLLYFNDIPIDESCVELIFDDNTANKIKDSLKTLSDKKIYNVKLERGMNILIKTPTYFTNFDD
YNNYPSTWSNVNTTNQDGLQGSANKLNGETKIKIPMSELKPYKRYVFSGYSKDPLTSNSIIV
KIKAKEEKTDYLVPEQGYTKFSYEFETTEKDSSNIEITLIGSGTTYLDNLSITELNSTPEILDEP
EVKIPTDQEIMDAHKIYFADLNFNPSTGNTYINGMYFAPTQTNKEALDYIQKYRVEATLQYSG
FKDIGTKDKEMRNYLGDPNQPKTNYVNLRSYFTGGENIMTYKKLRIYAITPDDRELLVLSVD
GG

SEQ ID NO: 57 C155 without histag
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPTNFDDYNNYPSTWSNVNTTN
QDGLQGSANKLNGETKIKIPMSELKPYKRYVFSGYSKDPLTSNSIIVKIKAKEEKTDYLVPEQ
GYTKFSYEFETTEKDSSNIEITLIGSGTTYLDNLSITELNSTPEILDEPEVKIPTDQEIMDAHKIY
FADLNFNPSTGNTYINGMYFAPTQTNKEALDYIQKYRVEATLQYSGFKDIGTKDKEMRNYL
GDPNQPKTNYVNLRSYFTGGENIMTYKKLRIYAITPDDRELLVLSVDGG

SEQ ID NO: 58 C156 without histag
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPGGGGGGNTTNQDGLQGSAN
KLNGETKIKIPMSELKPYKRYVFSGYSKDPLTSNSIIVKIKAKEEKTDYLVPEQGYTKFSYEFE
TTEKDSSNIEITLIGSGTTYLDNLSITELNSTPEILDEPEVKIPTDQEIMDAHKIYFADLNFNPST
GNTYINGMYFAPTQTNKEALDYIQKYRVEATLQYSGFKDIGTKDKEMRNYLGDPNQPKTNY
VNLRSYFTGGENIMTYKKLRIYAITPDDRELLVLSVDGG

SEQ ID NO: 59: C49 without histag
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVS

SEQ ID NO: 60: C50 without histag
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSS

SEQ ID NO: 61: CDTa N terminal domain (residue 44 to residue 240) polypeptide
sequence without histag.
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKI

SEQ ID NO: 62: C67 without histag
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVVYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGqYqVLLNHGSKFKINKIDSYKDGTITKLIVDATLIP

SEQ ID NO: 63: C69 without histag
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGQYQVLLNHGSKFKINKIDSYKDGTITKLIVDATLIP

SEQ ID NO: 64: C107 without histag
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGEYEVLLNHGSKFKINKIDSYKDGTITKLIVDATLIP

SEQ ID NO: 65: C108 without histag
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGEYQVLLNHGSKFKINKIDSYKDGTITKLIVDATLIP

SEQ ID NO: 66: C149 without histag
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQRSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVD

SEQ ID NO: 67: C152 without histag

MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQANQIGNNLSPGDTYPKKGLSPLALNTMAQFSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVD

SEQ ID NO: 68: C52 without histag
MTNFDDYNNYPSTWSNVNTTNQDGLQGSANKLNGETKIKIPMSELKPYKRYVFSGYSKDP
LTSNSIIVKIKAKEEKTDYLVPEQGYTKFSYEFETTEKDSSNIEITLIGSGTTYLDNLSITELNST
PEILDEPEVKIPTDQEIMDAHKIYFADLNFNPSTGNTYINGMYFAPTQTNKEALDYIQKYRVE
ATLQYSGFKDIGTKDKEMRNYLGDPNQPKTNYVNLRSYFTGGENIMTYKKLRIYAITPDDRE
LLVLSVDGG

SEQ ID NO: 69: C53 without histag
MNTTNQDGLQGSANKLNGETKIKIPMSELKPYKRYVFSGYSKDPLTSNSIIVKIKAKEEKTDY
LVPEQGYTKFSYEFETTEKDSSNIEITLIGSGTTYLDNLSITELNSTPEILDEPEVKIPTDQEIM
DAHKIYFADLNFNPSTGNTYINGMYFAPTQTNKEALDYIQKYRVEATLQYSGFKDIGTKDKE
MRNYLGDPNQPKTNYVNLRSYFTGGENIMTYKKLRIYAITPDDRELLVLSVDGG

SEQ ID NO: 70: CDTa C34 construct polypeptide sequence without histag
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGEYEVLLNHGSKFKINKIDSYKDGTITKLIVDATLIP

SEQ ID NO: 71: CDTb C118 construct. CDTb without signal peptide, without prodomain,
without CDTa-binding domain and without Receptor-binding domain, without histag

MAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPGG

SEQ ID NO: 72: CDTb C46 construct. CDTb' (minus signal peptide) polypeptide sequence without histag
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQFSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVD

SEQ ID NO: 73: C44 construct. CDTa mutation E428Q polypeptide sequence, without
histag.
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGqYEVLLNHGSKFKINKIDSYKDGTITKLIVDATLIP

SEQ ID NO: 74: C54 construct. CDTa mutation E430Q polypeptide sequence, without
histag.
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGEYqVLLNHGSKFKINKIDSYKDGTITKLIVDATLIP

SEQ ID NO: 75: C68 without histag
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFISTSIGSVNMSAFAKAKIVLRITIPKGSPGAYL
SAIPGYAGQYQVLLNHGSKFKINKIDSYKDGTITKLIVDATLIP

SEQ ID NO: 76: Amino acid sequence of CDTa without signal peptide, with six
mutations (R345A, Q350A, N385A, R402A, S388F, E428Q, aa 44-463) (C110 without
histag)
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGQYEVLLNHGSKFKINKIDSYKDGTITKLIVDATLIP

## Claims

1. An immunogenic composition comprising an isolated *Clostridium difficile* CDTb protein wherein the isolated *Clostridium difficile* CDTb protein has been mutated to modify pore forming ability wherein the CDTb protein comprises a mutation at position 426 and/or a mutation at position 453. Basis claim 3

2. The immunogenic composition of claim 1 wherein the isolated *Clostridium difficile* CDTb protein comprises the mutation F455G or wherein the isolated *Clostridium difficile* CDTb protein comprises the mutations E426A and D453A. Basis claim 5

3. The immunogenic composition of any preceding claim wherein the isolated *Clostridium difficile* CDTb protein comprises
(i) SEQ ID NO: 10 or SEQ ID NO: 11 or SEQ ID NO: 25 or SEQ ID NO: 26 or SEQ ID NO:43 or SEQ ID NO:44 or SEQ ID NO:51 or SEQ ID NO:52 or SEQ ID NO:66 or SEQ ID NO:67; or
(ii) a variant of CDTb having at least 80%, 85%, 88%, 90%, 92%, 95%, 98%, 99%, 100% sequence identity to SEQ ID NO: 10 or SEQ ID NO: 11 or SEQ ID NO: 25 or SEQ ID NO: 26 SEQ ID NO:43 or SEQ ID NO:44 or SEQ ID NO:51 or SEQ ID NO:52 or SEQ ID NO:66 or SEQ ID NO:67; or
(iii) a fragment of CDTb having at least 30, 50, 80, 100, 120, 150, 200, 250 or 300 contiguous amino acids of SEQ ID NO: 10 or SEQ ID NO: 11 or SEQ ID NO: 25 or SEQ ID NO: 26 SEQ ID NO:43 or SEQ ID NO:44 or SEQ ID NO:51 or SEQ ID NO:52 or SEQ ID NO:66 or SEQ ID NO:67. Basis claims 6 and 7 and page 23-24

4. An immunogenic composition comprising a fusion protein comprising (i) a full length CDTa protein and (ii) a CDTb protein. Basis claim 13

5. The immunogenic composition of claim 4 wherein the fusion protein comprising a full length CDTa protein and a CDTb protein suitably is or comprises
(i) SEQ ID NO:9 or SEQ ID NO:14 SEQ ID NO:15 or SEQ ID NO:16 or SEQ ID NO:17 or SEQ ID NO:27 or SEQ ID NO:28 or SEQ ID NO:50 or SEQ ID NO:55 or SEQ ID NO:56 or SEQ ID NO:57 or SEQ ID NO:58 or SEQ ID NO:68 or SEQ ID NO:69; or
(ii)a variant fusion protein having at least 80%, 85%, 88%, 90%, 92%, 95%, 98%, 99%, 100% sequence identity to SEQ ID NO:9 or SEQ ID NO:14 SEQ ID NO:15 or SEQ ID NO:16 or SEQ ID NO:17 or SEQ ID NO:27 or SEQ ID NO:28 or SEQ ID NO:50 or SEQ ID NO:55 or SEQ ID NO:56 or SEQ ID NO:57 or SEQ ID NO:58 or SEQ ID NO:68 or SEQ ID NO:69; or
(iii) a fragment having at least 30, 50, 80, 100, 120, 150, 200, 250, 300, 350 or 400 contiguous amino acids of SEQ ID NO:9 or SEQ ID NO:14 SEQ ID NO:15 or SEQ ID NO:16 or SEQ ID NO:17 or SEQ ID NO:27 or SEQ ID NO:28 or SEQ ID NO:50 or SEQ ID NO:55 or SEQ ID NO:56 or SEQ ID NO:57 or SEQ ID NO:58 or SEQ ID NO:68 or SEQ ID NO:69. Basis from claim 23, 21, 24, 26, 27 and page 24-25

6. The immunogenic composition of any preceding claim wherein the composition elicits antibodies that neutralize CDTa or CDTb or both. Basis claim 28

7. The immunogenic composition of any preceding claim wherein the composition elicits antibodies that neutralize binary toxin. Basis claim 29

8. The immunogenic composition of any preceding claim wherein the immunogenic composition further comprises an isolated *Clostridium difficile* toxin A protein and/or an isolated C. *difficile* toxin B protein. Basis claim 30

9. The immunogenic composition of claim 8 wherein the immunogenic composition comprises an isolated *Clostridium difficile* toxin A protein and an isolated *C. difficile* toxin B protein wherein the isolated *Clostridium difficile* toxin A protein and the isolated *C. difficile* toxin B protein form a fusion protein. Basis claim 31

10. The immunogenic composition of claim 9 wherein the fusion protein is
(i) SEQ ID NO: 32; or
(ii) a variant having at least 80%, 85%, 90%, 95%, 98%, 99% or 100% sequence identity to SEQ ID NO: 32; or
(iii) a fragment of at least 800, 850, 900 or 950 contiguous amino acids of a sequence selected from the group consisting of SEQ ID NO: 32. Basis claim 32

11. The immunogenic composition of any preceding claim wherein the immunogenic composition further comprises an adjuvant. Basis claim 33

12. The immunogenic composition of any one of the preceding claims further comprising additional antigens. Basis claim 34

13. A vaccine comprising the immunogenic composition of any one of the preceding claims and a pharmaceutically acceptable excipient. Basis claim36

14. The immunogenic composition of any one claims 1-12 or the vaccine of claim 13 for use in the treatment or prevention of *C. difficile* disease. Basis claim 37

15. The immunogenic composition of any one of claims 1-12 or the vaccine of claim 13 for use in the treatment or prevention of disease caused by a strain of *C. difficile* selected from the group consisting of 078, 019, 023, 027, 033, 034, 036, 045, 058, 059, 063, 066, 075, 078, 080, 111, 112, 203, 250 and 571. Basis claim 38
